(19) Europäisches Patentamt / European Patent Office / Office européen des brevets

(11) EP 4 159 723 B1

(12) **EUROPEAN PATENT SPECIFICATION**

(45) Date of publication and mention of the grant of the patent:
**23.07.2025 Bulletin 2025/30**

(21) Application number: **21817308.6**

(22) Date of filing: **02.06.2021**

(51) International Patent Classification (IPC):
*C07D 239/34* (2006.01) *C07D 239/02* (2006.01)
*C07D 403/12* (2006.01) *A61K 31/505* (2006.01)
*A61K 31/506* (2006.01) *A61P 5/14* (2006.01)
*A61P 3/04* (2006.01) *A61P 3/06* (2006.01)
*C07F 9/6512* (2006.01) *C07D 239/52* (2006.01)
*C07D 405/14* (2006.01) *C07D 413/12* (2006.01)

(52) Cooperative Patent Classification (CPC):
**A61P 3/04; A61P 3/06; A61P 5/14; C07D 239/52; C07D 403/12; C07D 405/14; C07D 413/12; C07F 9/6512**

(86) International application number:
**PCT/CN2021/097986**

(87) International publication number:
**WO 2021/244582 (09.12.2021 Gazette 2021/49)**

(54) **NOVEL THYROID HORMONE BETA RECEPTOR AGONIST**

NEUER SCHILDDRÜSENHORMON-BETA-REZEPTORAGONIST

NOUVEL AGONISTE DU RÉCEPTEUR BETA DE L'HORMONE THYROÏDIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.06.2020 CN 202010491859**
**09.02.2021 CN 202110178964**

(43) Date of publication of application:
**05.04.2023 Bulletin 2023/14**

(73) Proprietor: **Chengdu Kanghong Pharmaceutical Co., Ltd.**
**Chengdu, Sichuan 610036 (CN)**

(72) Inventors:
- **YU, Yongguo**
  **Chengdu, Sichuan 610036 (CN)**
- **WANG, Yiqian**
  **Chengdu, Sichuan 610036 (CN)**
- **KE, Zunhong**
  **Chengdu, Sichuan 610036 (CN)**

(74) Representative: **Dehns**
**10 Old Bailey**
**London EC4M 7NG (GB)**

(56) References cited:
**WO-A1-2013/097773**
**WO-A1-2020/073974**
**WO-A1-2020/077123**
**CN-A- 102 459 185**
**CN-A- 109 574 995**
**CN-A- 110 627 773**
**JP-A- 2011 088 840**
**JP-A- 2012 106 996**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

TECHNICAL FIELD

The present application relates to a novel thyroid hormone β receptor agonist, which may be used for treating obesity, hyperlipidemia, hypercholesterolemia, diabetes, liver diseases (fatty liver, NASH, NAFLD, and the like), cardiovascular diseases (atherosclerosis, and the like) and thyroid diseases (hypothyroidism, thyroid cancer, and the like).

BACKGROUNG

Thyroid hormone is a hormone secreted by a thyroid gland and acts on almost all cells of a human body. Thyroid hormone comprises: thyroxine (T4) and triiodothyronine (T3). The T4 may be subjected to deiodinination into the T3 by specific deiodinase to be effective. The T3 has a fast and strong action, and a duration shorter than that of the T4, while the T4 has a slow and weak action, and a long duration. Although the specific deiodinase exists in all tissues, more is found in liver and kidney.

The thyroid hormone is necessary for the normal growth and development of the human body.. Either insufficient or excessive secretion of the thyroid hormone may cause diseases. Insufficient thyroid hormone will affect physical and mental development, which may lead to cretinism, . Adults with insufficient thyroid hormone may suffer from myxedema, In the case of hyperthyroidism nervousness, impatience, tremor, heart rate increase, cardiac output increase and other phenomena occur. The thyroid hormone can promote substance oxidation, increase oxygen consumption, enhance a basal metabolic rate and enhance heat production.

Normally, the central nervous system controls the release of thyrotropin-releasing hormone (TRH) from the hypothalamus, which regulates the secretion of thyroid-stimulating hormone (TSH) by adenohypophysis, and TSH stimulates thyroid cells to secrete T4 and T3. When the concentration of T4 and T3 in the blood increases, the synthesis and release of TSH in the adenohypophysis are inhibited by negative feedback, and the responsiveness of the adenohypophysis to TRH is reduced, thereby reducing the secretion of TSH so that the secretion of thyroid hormones is not too high. However, when the concentrations of the T4 and the T3 in blood are reduced, the negative feedback action on the adenohypophysis is reduced. The increase of the secretion of the TSH prompt the increase of the secretion of the T4 and the T3. In short, a hypothalamus - adenohypophysis - thyroid gland regulation loop may maintain relatively constant secretion of the thyroid hormone.

The biological activity of the thyroid hormone is mediated by thyroid hormone receptors (TRs). which belong to a superfamily of nuclear receptors. The TR has a ligand binding domain, a DNA binding domain and an amino terminal domain. The TR has four subtypes, TRα1, TRα2, TRβ1 and TRβ2 respectively. The TRα1 is mainly found in heart and the TRβ1 is mainly found in liver. The mRNA expression of the TRβ2 is mostly limited to adenohypophysis and the hypothalamus. The thyroid hormone binds to the TRα1, the TRβ1 and the TRβ2 to generate corresponding physiological effects. The thyroid hormone does not bind to the TRα2

Therapeutic benefits, such as treating the obesity, may be achieved by making full use of the advantages of the thyroid hormone in increasing metabolic rate, oxygen consumption and heat release. The hyperthyroidism often results in food intake but the overall increase of a basal metabolic rate (BMR) as well. Hyperthyroidism is often accompanied with a weight loss of about 15%, while hypothyroidism is often accompanied with a weight gain of 25% to 30%. When the T3 is used for treating the hypothyroidism, most patients have the weight gain.

Furthermore, the thyroid hormone can also reduce serum low density lipoprotein (LDL) (Journal of Molecular and Celluar Cardiology 37(2004): 1137-1146). Existing studies have shown that hyperthyroidism significantly reduces total serum cholesterol, which is mainly because the thyroid hormone increases the expression of LDL receptors in liver, thus promoting a process of metabolism of cholesterol to bile acid; hypothyroidism is associated with hypercholesterolemia. Therefore, the thyroid hormone may reduce incidences of atherosclerosis and other cardiovascular diseases.

When treating diseases with thyroid hormones, due to individual differences, there are often side effects of excessive physiological doses, including heart problems (mainly refers to tachycardia), muscle weakness, excessive weight loss, etc., and long-term use of thyroid hormones may result in bone loss. Thus, it is highly required to develop new novel drugs through structure modification of the thyroid hormone to maintain its beneficial effects and reduce its side effects for the related diseases treatment, such as obesity, hyperlipidemia, hypercholesterolemia, diabetes, liver diseases (fatty liver, NASH, NAFLD, and the like), cardiovascular diseases (atherosclerosis, and the like), thyroid diseases (hypothyroidism, thyroid cancer, and the like), and other related diseases.

A pyridazinone thyroid hormone analogue, represented by structure MGL3196, was patented by Madrigal Pharmaceuticals (CN101228135B), and is currently in phase III clinical trials for NASH and NAFLD treatment. Due to its low activity and poor permeability, an oral dosage of 80 mg to 100 mg per day is required. The dose is significantly higher than other products on the same target.

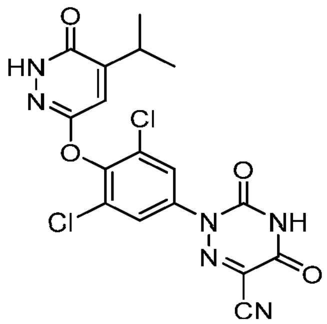

MGL3196

Compound VK2809, patented by Viking Therapeutics (CN1882327C), is in 2b clinical trial for NASH treatment. Based on phase I clinical data, the compound had safety problems and a relatively narrow therapeutic window. Liver enzyme increase, a symbol of liver injury, was observed. At the same time, cartilage damage was found in preclinical toxicological studies (J. Med. Chem. 2014, 57, 3912-3923). Eprotirome, patented by Bristol-Myers Squibb Co (CN1216857C), was terminated in phase III clinical trial. According to the reported clinical data, there was also an increase in liver enzyme. The cartilage injury was also found in the preclinical toxicological research. For other patents, such as pyridine derivatives (CN102459185) and indole derivatives (WO2002051805), there are only activity data reported without further research going on. There is no product advanced to clinical. WO 2013/097773 A1 discloses compounds used for the treatment of cardiovascular diseases.

SUMMARY

Aiming at the problems in the existing reports, the present application provides a novel thyroid hormone β receptor agonist with better activity, selectivity, or safety.

One aspect of the present application lies in providing a novel thyroid hormone β receptor agonist of Formula I, a pharmaceutically acceptable salt thereof:

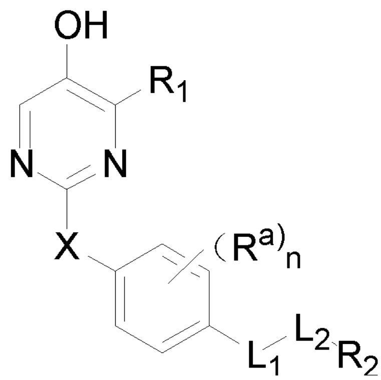

Formula I

wherein,

- $R_1$ is hydrogen, optionally substituted alkyl, optionally substituted cycloalkyl, optionally substituted aryl, optionally substituted heterocyclyl, optionally substituted heteroaryl, optionally substituted amino, optionally substituted carbamoyl, or $-COR_{10}$;
- X is optionally substituted methylene, -O-, -S-, or $-SO_2-$;
- $R^a$ is selected from hydrogen, halogen, $C_{1-6}$ linear and branched alkyl, or cycloalkyl; or two adjacent $R^a$ are bonded to form a carbocyclic ring, or heterocyclic ring;
- $L_1$ is a single bond, methylene, -CH=CH-, -O-, -CO-, $-NR_3-$, $-NR_3CO-$, $-CONR_3-$, $-CH_2NR_3-$, or -S-;
- $L_2$ is a single bond, or $-(CR_4R_5)_p$;
- $R_2$ is a carboxyl, or a group represented by the following formula:

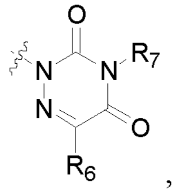,

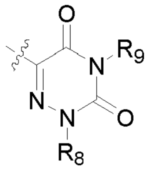, 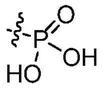, 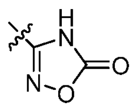, 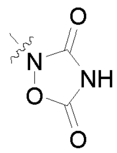, 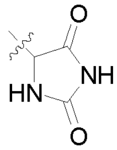, 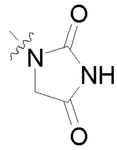, 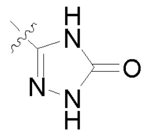,

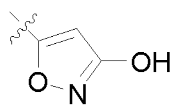, 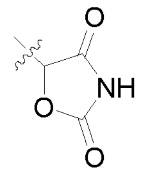, or 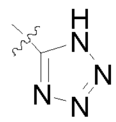;

$R_3$ is hydrogen, or optionally substituted alkyl;
$R_4$ and $R_5$ are each independently selected from hydrogen, halogen, or optionally substituted alkyl, or $R_4$ and $R_5$ are bonded to form a cycloalkyl;
$R_6$ is hydrogen, cyano, amino, COOH, $C_{1-6}$ alkyl, $C_{1-6}$ haloalkyl, $C_{3-6}$ cycloalkyl, or $C_{3-6}$ halocycloalkyl;
$R_8$ is hydrogen, cyano, COOH, $C_{1-6}$ alkyl, $C_{1-6}$ haloalkyl, $C_{3-6}$ cycloalkyl, or $C_{3-6}$ halocycloalkyl;
$R_7$ and $R_9$ are hydrogen, $C_{1-3}$ alkyl, or $C_{1-3}$ haloalkyl;
$R_{10}$ is optionally substituted alkyl, amino, hydroxyl, optionally substituted cycloalkyl, optionally substituted aryl, optionally substituted heterocyclyl, or optionally substituted heteroaryl;
n is 0, 1, 2, 3, or 4; and
p is 0, 1, or 2.

In some preferred embodiments, $R_1$ is hydrogen, or $-COR_{10}$, or alkyl, cycloalkyl, cycloalkylalkyl, aryl, arylalkyl, heterocyclyl, heteroaryl, amino, or carbamoyl optionally substituted by hydrogen, deuterium, tritium, $C_{1-6}$ alkyl, hydroxyl, halogen, or CN. In some preferred embodiments, $R_1$ is $-COR_{10}$, or $C_{1-10}$ alkyl, $C_{3-10}$ cycloalkyl, $C_{3-10}$ cycloalkyl$C_{1-6}$ alkyl, $C_{5-10}$ aryl, $C_{5-10}$ aryl$C_{1-6}$ alkyl, 5-10 membered heterocyclyl, 5-10 membered heteroaryl, amino, or carbamoyl optionally substituted by hydrogen, deuterium, tritium, $C_{1-6}$ alkyl, hydroxyl, halogen, or CN. In some preferred embodiments, $R_1$ is $-COR_{10}$, or $C_{1-8}$ alkyl, $C_{3-8}$ cycloalkyl, $C_{3-8}$ cycloalkyl$C_{1-6}$ alkyl, $C_{5-10}$ aryl, $C_{5-10}$ aryl$C_{1-6}$ alkyl, 5-10 membered heterocyclyl, or 5-10 membered heteroaryl optionally substituted by hydrogen, deuterium, tritium, $C_{1-6}$ alkyl, hydroxyl, halogen, or CN.
In some aspects, the compound of Formula I provided by the present application is shown in Formula II:

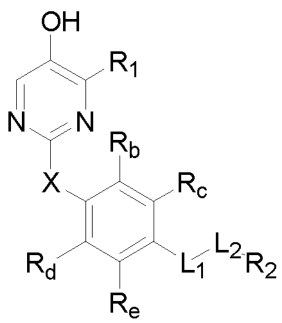

Formula II

wherein, $R_b$, $R_c$, $R_d$ and $R_e$ are hydrogen, deuterium, halogen, $C_{1-6}$ linear or branched alkyl, or cycloalkyl; or, $R_b$ and $R_c$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom; or, $R_d$ and $R_e$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom.

Other substituents are defined as in Formula I above.

In some aspects, the compound of Formula II provided by the present application is:

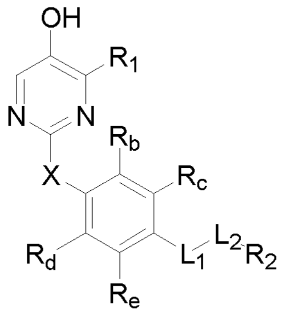

Formula II

wherein $R_1$ is optionally substituted $C_{1-6}$ linear or branched alkyl, or $C_{3-8}$ cycloalkyl;

X is O, S, or $-CH_2-$;

$R_b$, $R_c$, $R_d$ and $R_e$ are hydrogen, deuterium, halogen, $C_{1-6}$ linear or branched alkyl, or cycloalkyl; or, $R_b$ and $R_c$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom; or, $R_d$ and $R_e$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom;

$L_1$ is a single bond, $-NR_3-$, -O, or -S-;

$L_2$ is a single bond, or $-CH_2-$;

$R_2$ is a group represented by the following formula:

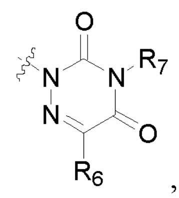 , 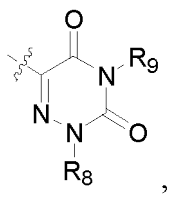 , 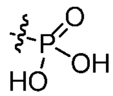 ,

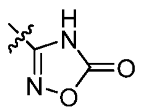 , 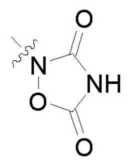 , 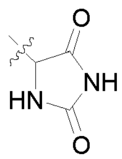 , 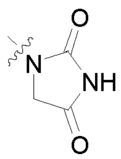 , 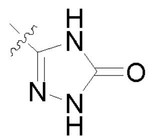 , 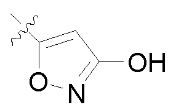 , 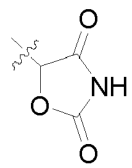 ,

or

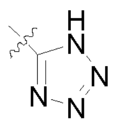 ;

$R_3$ is hydrogen, or optionally substituted $C_{1-6}$ alkyl;

$R_6$ is hydrogen, cyano, amino, COOH, $C_{1-6}$ alkyl, $C_{1-6}$ haloalkyl, $C_{3-6}$ cycloalkyl, or $C_{3-6}$ halocycloalkyl;

$R_8$ is hydrogen, cyano, COOH, $C_{1-6}$ alkyl, $C_{1-6}$ haloalkyl, $C_{3-6}$ cycloalkyl, or $C_{3-6}$ halocycloalkyl; and

$R_7$ and $R_9$ are hydrogen, $C_{1-3}$ alkyl, or $C_{1-3}$ haloalkyl.

In some aspects, the compound of Formula II provided by the present application is:

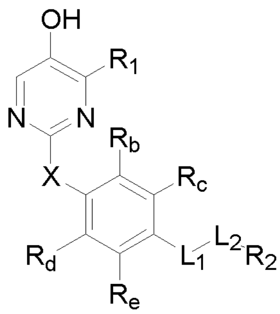

Formula II

wherein $R_1$ is optionally substituted $C_{1-6}$ linear or branched alkyl;

$R_b$, $R_c$, $R_d$ and $R_e$ are hydrogen, deuterium, halogen, $C_{1-6}$ linear or branched alkyl, or cycloalkyl; or, $R_b$ and $R_c$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom; or, $R_d$ and $R_e$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom;

X is O, S, or $-CH_2-$;

$L_1$ is a single bond, -O-, -S-, or -NH-;

$L_2$ is a single bond;

$R_2$ is a group represented by the following formula:

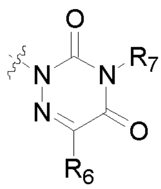 , 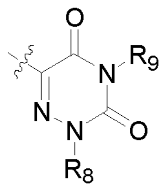 ,

or

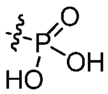 ;

$R_6$ is hydrogen, cyano, $C_{1-6}$ alkyl, or $C_{1-6}$ haloalkyl;

$R_8$ is hydrogen, cyano, $C_{1-6}$ alkyl, or $C_{1-6}$ haloalkyl; and

$R_7$ and $R_9$ are hydrogen, $C_{1-3}$ alkyl, or $C_{1-3}$ haloalkyl.

In some aspects, the compound of Formula II provided by the present application is:

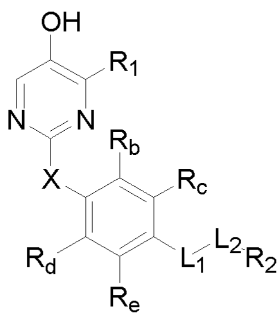

Formula II

wherein $R_1$ is $C_{1-6}$ linear or branched alkyl, benzyl, or $C_{5-6}$ cycloalkylmethylene optionally substituted by hydrogen, deuterium, tritium, $C_{1-6}$ alkyl, hydroxyl, halogen, or CN, and further preferably isopropyl, or benzyl;

$R_b$ and $R_d$ are halogen, and $R_c$ and $R_e$ are hydrogen, and $R_b$ and $R_d$ are further preferably chlorine;
X is O, S, or $-CH_2-$;
$L_1$ is a single bond, -O, -S-, or -NH-;
$L_2$ is a single bond, or $-CH_2-$;
$R_2$ is a group represented by the following formula:

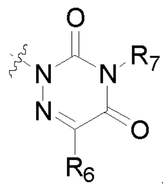
,
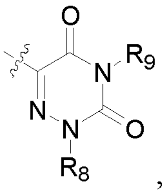
, or
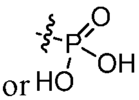
;

$R_6$, $R_7$, $R_8$ and $R_9$ are hydrogen, or $C_{1-6}$ alkyl, or $C_{3-8}$ cycloalkyl.

In some aspects, the compound of Formula I provided by the present application is shown in Formula III:

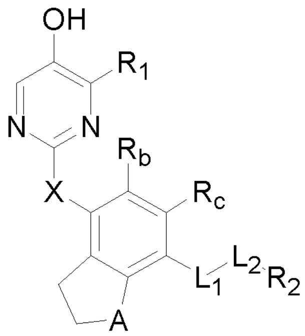

Formula III

wherein,

$R_b$ and $R_c$ are hydrogen, deuterium, halogen, $C_{1-6}$ linear or branched alkyl, or cycloalkyl; and
A is O, or methylene.

Other substituents are defined as in Formula I.

In other preferred embodiments, in the compound of Formula I provided by the present application, $R_1$ is selected from:

1) optionally substituted $C_{1-6}$ linear and branched alkyl;
2) optionally substituted $C_{3-8}$ cycloalkyl;
3) optionally substituted $C_{3-8}$ non-aromatic heterocyclyl containing 1 to 3 heteroatoms selected from nitrogen atom, oxygen atom and sulfur atom;
4) optionally substituted phenyl; or
5) optionally substituted $C_{5-6}$ heteroaryl containing 1 to 3 heteroatoms selected from nitrogen atom, oxygen atom and sulfur atom.

In other preferred embodiments, in the compound of Formula I provided by the present application, $R_1$ is selected from $-(CR_{11}R_{12})_mR_{13}$; $R_{11}$ and $R_{12}$ are selected from hydrogen, deuterium, halogen, hydroxyl, amino, carboxyl or optionally substituted $C_{1-4}$ alkyl; and $R_{13}$ is selected from:

1) hydrogen, or deuterium;
2) halogen;
3) hydroxyl;
4) amino;
5) carboxyl;
6) optionally substituted $C_{1-4}$ alkyl, or $C_{1-4}$ alkoxy;

7) optionally substituted $C_{3-8}$ cycloalkyl;
8) optionally substituted $C_{3-8}$ non-aromatic heterocyclyl containing 1 to 3 heteroatoms selected from nitrogen atom, oxygen atom and sulfur atom;
9) optionally substituted phenyl; or
10) optionally substituted $C_{5-6}$ heteroaryl containing 1 to 3 heteroatoms selected from nitrogen atom, oxygen atom and sulfur atom; and
m is 0, 1, 2, or 3.

In other preferred embodiments, in the compound of Formula I provided by the present application, $R_1$ is selected from $-COR_{10}$, wherein $R_{10}$ is selected from:

1) amino;
2) hydroxyl;
3) optionally substituted $C_{1-4}$ alkyl, or $C_{1-4}$ alkoxy;
4) optionally substituted $C_{3-8}$ cycloalkyl;
5) optionally substituted $C_{3-8}$ non-aromatic heterocyclyl containing 1 to 3 heteroatoms selected from nitrogen atom, oxygen atom and sulfur atom;
6) optionally substituted phenyl; or
7) optionally substituted $C_{5-6}$ heteroaryl containing 1 to 3 heteroatoms selected from nitrogen atom, oxygen atom and sulfur atom.

In other preferred embodiments, in the compound of Formula I, $R_1$ is hydrogen, $C_{1-10}$ alkyl (preferably $C_{1-5}$ alkyl), $C_{3-10}$ cycloalkyl (preferably $C_{3-8}$ cycloalkyl), $C_{3-10}$ cycloalkyl$C_{1-6}$ alkyl (preferably $C_{3-8}$ cycloalkyl$C_{1-4}$ alkyl), $C_{5-10}$ aryl (preferably $C_{5-8}$ aryl), $C_{5-10}$ aryl$C_{1-6}$ alkyl (preferably $C_{5-8}$ aryl$C_{1-4}$ alkyl), 5-10 membered heterocyclyl containing 1 to 3 heteroatoms selected from nitrogen atom, oxygen atom and sulfur atom, 5-10 membered heteroaryl containing 1 to 3 heteroatoms selected from nitrogen atom, oxygen atom and sulfur atom, amino, or $-COR_{10}$, and the $C_{1-10}$ alkyl (preferably the $C_{1-5}$ alkyl), the $C_{3-10}$ cycloalkyl (preferably the $C_{3-8}$ cycloalkyl), the $C_{3-10}$ cycloalkyl$C_{1-6}$ alkyl (preferably the $C_{3-8}$ cycloalkyl$C_{1-4}$ alkyl), the $C_{5-10}$ aryl (preferably the $C_{5-8}$ aryl), the $C_{5-10}$ aryl$C_{1-6}$ alkyl (preferably the $C_{5-8}$ aryl$C_{1-4}$ alkyl), the 5-10 membered heterocyclyl containing 1 to 3 heteroatoms selected from nitrogen atom, oxygen atom and sulfur atom, the 5-10 membered heteroaryl containing 1 to 3 heteroatoms selected from nitrogen atom, oxygen atom and sulfur atom, or the amino is unsubstituted, or is capable of being substituted by deuterium, tritium, $C_{1-6}$ alkyl, hydroxyl, halogen, or CN;

X is methylene, -O-, -S-, or $-SO_2-$;
$R^a$ is hydrogen, deuterium, halogen, $C_{1-6}$ linear or branched alkyl, or cycloalkyl; or two adjacent $R^a$ are bounded to form a 5-10 membered carbocyclic ring, or a 5-10 membered heterocyclic ring containing 1 to 3 heteroatoms selected from nitrogen atom, oxygen atom and sulfur atom;
$L_1$ is a single bond, methylene, -O-, -CO-, $-NR_3-$, $-NR_3CO-$, $-CONR_3-$, $-CH_2NR_3-$, or -S-;
$L_2$ is a single bond, or $C_{1-6}$ alkyl (preferably $C_{1-4}$ alkyl);
$R_2$ is a carboxyl, or a group represented by the following formula:

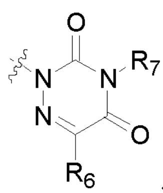 , 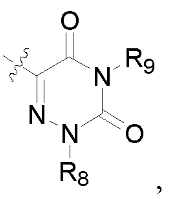 ,

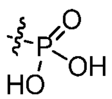 , 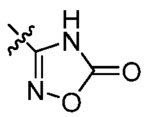 , or 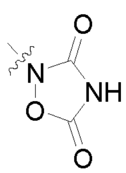 ;

$R_3$ is hydrogen, or $C_{1-6}$ alkyl;
$R_6$ is hydrogen, cyano, amino, COOH, $C_{1-6}$ alkyl, or $C_{1-6}$ haloalkyl;
$R_8$ is hydrogen, cyano, COOH, $C_{1-6}$ alkyl, or $C_{1-6}$ haloalkyl;
$R_7$ and $R_9$ are hydrogen, $C_{1-3}$ alkyl, or $C_{1-3}$ haloalkyl;

$R_{10}$ is $C_{3-10}$ cycloalkyl (preferably $C_{3-8}$ cycloalkyl), $C_{5-10}$ aryl (preferably $C_{5-8}$ aryl), 5-10 membered heterocyclyl containing 1 to 3 heteroatoms selected from nitrogen atom, oxygen atom and sulfur atom, or 5-10 membered heteroaryl containing 1 to 3 heteroatoms selected from nitrogen atom, oxygen atom and sulfur atom; and
n is 0, 1, 2, 3, or 4.

In other preferred embodiments, in the compound of Formula I, $R_1$ is $C_{1-8}$ alkyl (preferably $C_{1-5}$ alkyl), $C_{3-8}$ cycloalkyl (preferably $C_{3-6}$ cycloalkyl), $C_{3-8}$ cycloalkyl$C_{1-5}$ alkyl (preferably $C_{3-6}$ cycloalkyl$C_{1-3}$ alkyl), $C_{5-8}$ aryl (preferably $C_{5-6}$ aryl), $C_{5-8}$ aryl$C_{1-5}$ alkyl (preferably $C_{5-6}$ aryl-$C_{1-3}$ alkyl), 5-8 membered heterocyclyl containing 1 to 3 heteroatoms selected from nitrogen atom, oxygen atom and sulfur atom, 5-8 membered heteroaryl containing 1 to 3 heteroatoms selected from nitrogen atom, oxygen atom and sulfur atom, amino, or $-COR_{10}$, and the $C_{1-8}$ alkyl (preferably the $C_{1-5}$ alkyl), the $C_{3-8}$ cycloalkyl (preferably the $C_{3-6}$ cycloalkyl), the $C_{3-8}$ cycloalkyl$C_{1-5}$ alkyl (preferably the $C_{3-6}$ cycloalkyl$C_{1-3}$ alkyl), the $C_{5-8}$ aryl (preferably the $C_{5-6}$ aryl), the $C_{5-8}$ aryl$C_{1-5}$ alkyl (preferably the $C_{5-6}$ aryl$C_{1-3}$ alkyl), the 5-8 membered heterocyclyl containing 1 to 3 heteroatoms selected from nitrogen atom, oxygen atom and sulfur atom, the 5-8 membered heteroaryl containing 1 to 3 heteroatoms selected from nitrogen atom, oxygen atom and sulfur atom, or the amino is unsubstituted, or is capable of being substituted by deuterium, tritium, $C_{1-6}$ alkyl, hydroxyl, halogen, or CN;

X is methylene, -O-, -S-, or $-SO_2-$;
$R^a$ is halogen, or $C_{1-4}$ linear or branched alkyl; or two adjacent $R^a$ are bounded to form a 5-7 membered carbocyclic ring, or a 5-7 membered heterocyclic ring containing 1 to 3 heteroatoms selected from nitrogen atom, oxygen atom and sulfur atom;
$L_1$ is a single bond, -O-, $-NR_3-$, $-NR_3CO-$, $-CONR_3-$, $-CH_2NR_3-$, or -S-;
$L_2$ is a single bond, or $C_{1-5}$ alkyl (preferably $C_{1-3}$ alkyl);
$R_2$ is a carboxyl, or a group represented by the following formula:

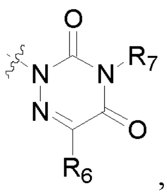
,
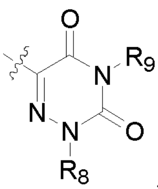
,

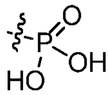
, or
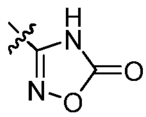
;

$R_3$ is hydrogen, or $C_{1-3}$ alkyl;
$R_6$ is hydrogen, cyano, COOH, or $C_{1-4}$ alkyl;
$R_8$ is hydrogen, or $C_{1-4}$ alkyl;
$R_7$ and $R_9$ are hydrogen, or $C_{1-3}$ alkyl;
$R_{10}$ is $C_{5-8}$ aryl (preferably $C_{5-6}$ aryl), or 5-8 membered heteroaryl (preferably 5-6 membered heteroaryl) containing 1 to 3 heteroatoms selected from nitrogen atom, oxygen atom and sulfur atom; and
n is 1, 2, or 3.

In other preferred embodiments, in the compound of Formula I, $R_1$ is methyl, ethyl, propyl, butyl, pentyl, cyclopropane, cyclobutane, cyclopentane, cyclohexane, cyclopropanemethyl, cyclobutanemethyl, cyclopentanemethyl, cyclohexanemethyl, phenyl, benzyl, or $-COR_{10}$, and the methyl, the ethyl, the propyl, the butyl, the pentyl, the cyclopropane, the cyclobutane, the cyclopentane, the cyclohexane, the cyclopropanemethyl, the cyclobutanemethyl, the cyclopentanemethyl, the cyclohexanemethyl, the phenyl, or the benzyl is unsubstituted, or capable of being substituted by deuterium, $C_{1-3}$ alkyl, hydroxyl, halogen, or CN;

X is methylene, -O-, -S-, or $-SO_2-$;
$R^a$ is halogen; or two adjacent $R^a$ are bonded to form a 5 membered carbocyclic ring, or a 5 membered heterocyclic ring containing 1 to 2 heteroatoms selected from nitrogen atom, oxygen atom and sulfur atom;
$L_1$ is a single bond, -O-, -NH-, -NHCO-, -CONH-, $-CH_2NH-$, or -S-;
$L_2$ is a single bond, methyl, ethyl, or propyl;
$R_2$ is a carboxyl, or a group represented by the following formula:

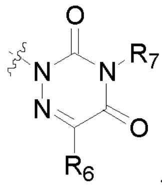, 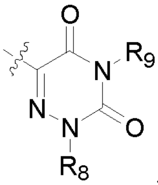,

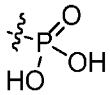, or 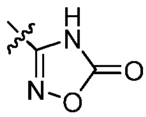;

$R_6$ is hydrogen, cyano, COOH, methyl, ethyl, or propyl;
$R_8$ is hydrogen, methyl, ethyl, or propyl;
$R_7$ and $R_9$ are hydrogen, or methyl;
$R_{10}$ is phenyl; and
n is 2, or 3.

In other preferred embodiments, in the compound of Formula I, $R_1$ is methyl, ethyl, propyl, butyl, pentyl, cyclopropane, cyclobutane, cyclopentane, cyclohexane, cyclopropanemethyl, cyclobutanemethyl, cyclopentanemethyl, cyclohexanemethyl, phenyl, or benzyl, and the methyl, the ethyl, the propyl, the butyl, the pentyl, the cyclopropane, the cyclobutane, the cyclopentane, the cyclohexane, the cyclopropanemethyl, the cyclobutanemethyl, the cyclopentanemethyl, the cyclohexanemethyl, the phenyl, or the benzyl is unsubstituted, or capable of being substituted by deuterium, $C_{1-3}$ alkyl, hydroxyl, F, Cl, Br, or CN;

X is methylene, -O-, or -S-;
$R^a$ is F, Cl, or Br; or two adjacent $R^a$ are bonded to form a 5 membered carbocyclic ring, or a 5 membered heterocyclic ring containing 1 to 2 heteroatoms selected from nitrogen atom, oxygen atom and sulfur atom;
$L_1$ is a single bond, -O-, -NH-, or -NHCO-;
$L_2$ is a single bond, methyl, ethyl, or propyl;
$R_2$ is a carboxyl, or a group represented by the following formula:

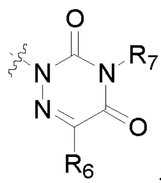, 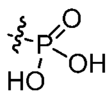,

or

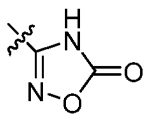;

$R_6$ is hydrogen, cyano, or methyl;
$R_7$ is hydrogen; and
n is 2, or 3.

In some specific embodiments, in the compound of Formula I provided by the present application, $R_1$ is selected from:

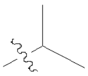, 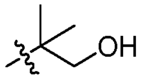, 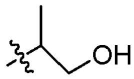, 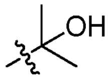, 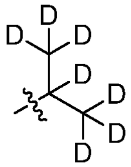,

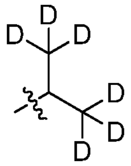, 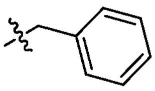, 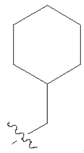, 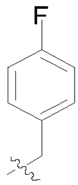, 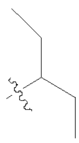, 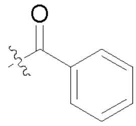, 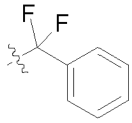, 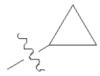, or 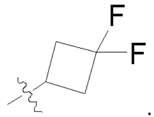.

In some specific embodiments, in the compound of Formula I provided by the present application, $R_b$, $R_c$, $R_d$ and $R_e$ are selected from hydrogen, deuterium, or halogen.

In some specific embodiments, in the compound of Formula I provided by the present application, $L_1$ is selected from a single bond, -O-, -NH-, -NHCO-, or $-NHCH_2-$.

In some specific embodiments, in the compound of Formula I provided by the present application, $L_2$ is selected from a single bond, or methylene ($-CH_2-$).

In some specific embodiments, in the compound of Formula I provided by the present application, $R_2$ is selected from: a carboxyl,

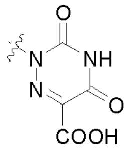, 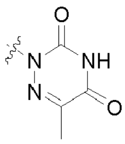, 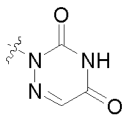, 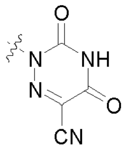,

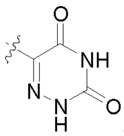, 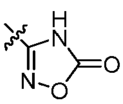, 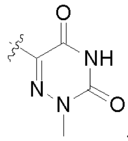, or 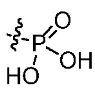.

In some specific embodiments, the compound of Formula I provided by the present application is selected from:

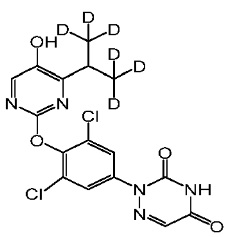, 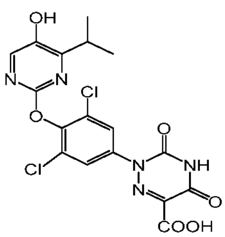, 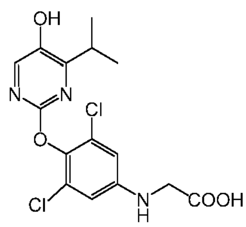, 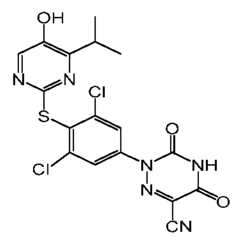,

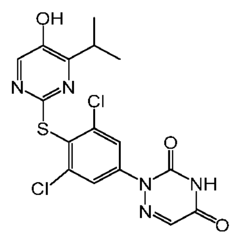, 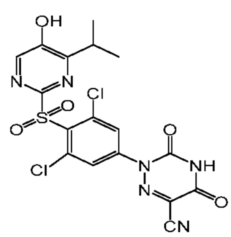, 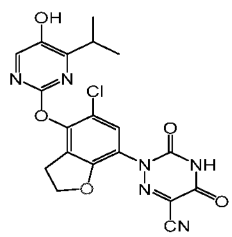, 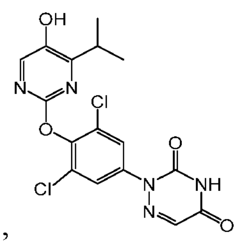,

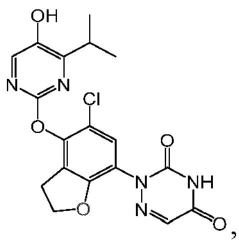 , ,
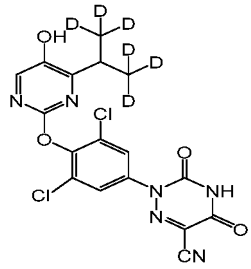 ,
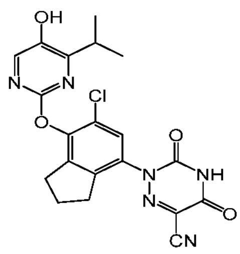 ,
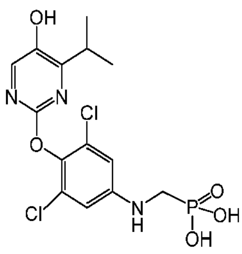 ,

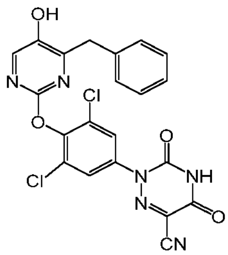 ,
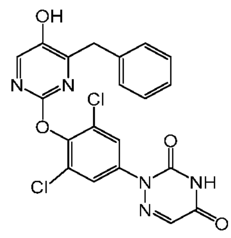 ,
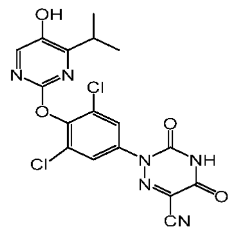 ,
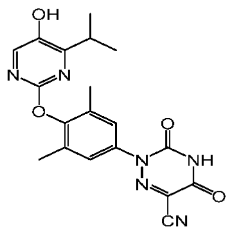 ,

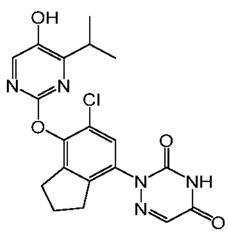 ,
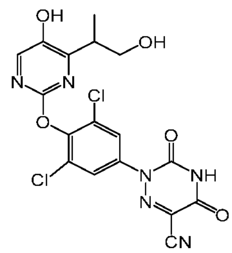 ,
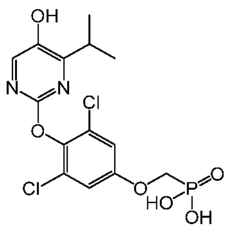 ,
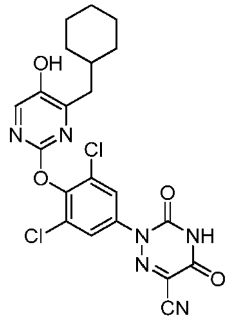 ,

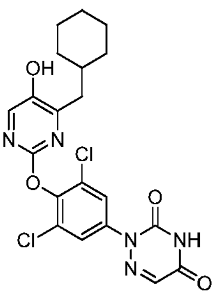 ,
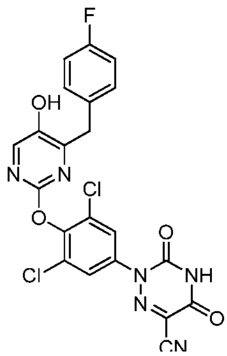 ,
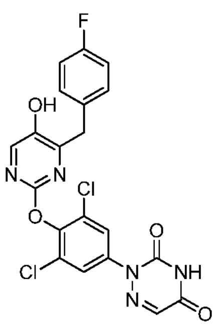 ,
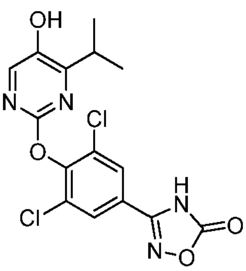 ,

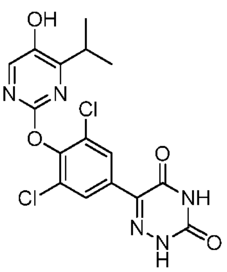 ,
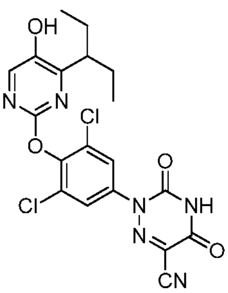 ,
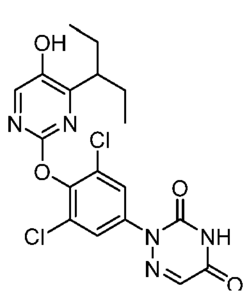 ,
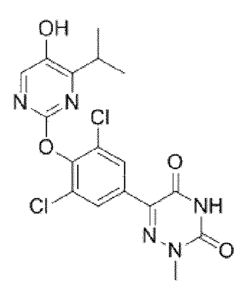 ,

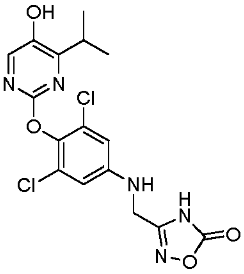, 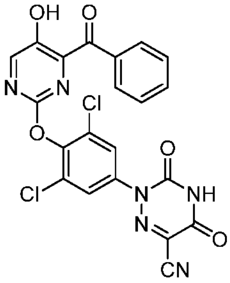, 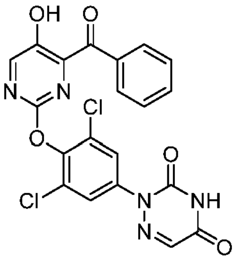, 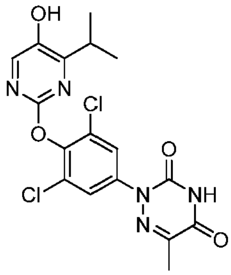,

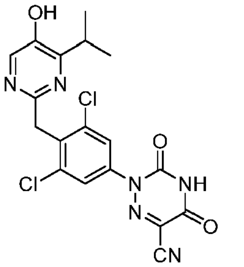, 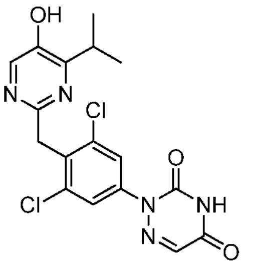, 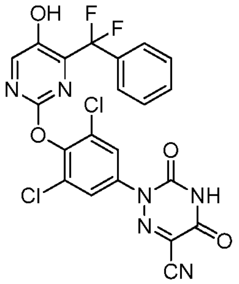, 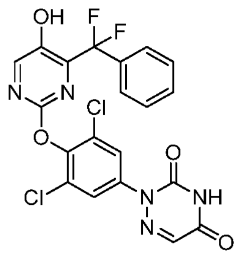,

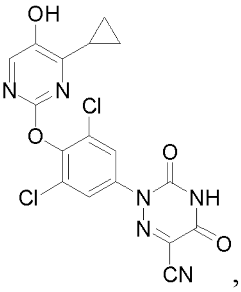, 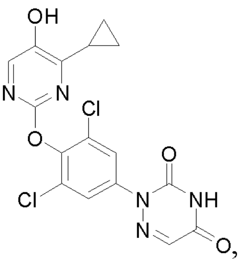, 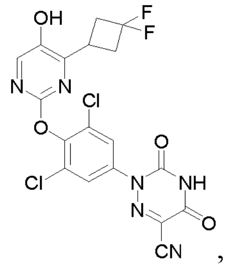, 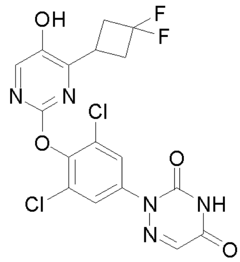,

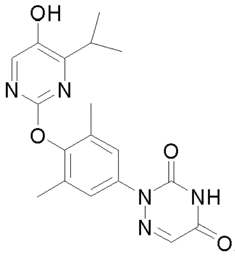, or 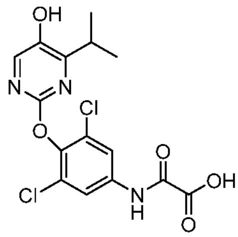.

Another aspect of the present application lies in providing a pharmaceutical composition, comprising the compound of Formula I of the present application, or the pharmaceutically acceptable salt thereof, and one or more pharmaceutically acceptable carriers.

Another aspect of the present application lies in providing use of the compound of the present invention, or the pharmaceutically acceptable salt thereof in preventing, or treating a disease related to a THR-β agonist action (such as obesity, hyperlipidemia, hypercholesterolemia, diabetes, steatohepatitis, non-alcoholic steatohepatitis, nonalcoholic fatty liver disease, atherosclerosis, thyroid cancer, hypothyroidism. Alternatively, the present application provides the compound, the pharmaceutically acceptable salt thereof, or the prodrug thereof above for preventing, or treating the disease related to the β receptor agonist action. Alternatively, the present application provides a compound for use in a method for preventing, or treating the disease related to THR β agonism, which comprises administering the compound, or the pharmaceutically acceptable salt thereof above to a subject in need thereof. Preferably, the disease related to the β receptor agonist action comprises, but is not limited to: hypercholesterolemia, hyperlipidemia, hypertriglyceridemia, familial hypercholesterolemia, dyslipidemia, thyroid cancer, hypothyroidism, underlying hypothyroidism, atherosclerosis, metabolic syndrome, obesity, diabetes, cardiovascular diseases, coronary artery diseases, myocardial infarction, ventricular insufficiency, heart failure, fatty liver, liver cirrhosis, non-alcoholic steatohepatitis (NASH), non-alcoholic fatty liver disease (NAFLD), depression, dementia, osteoporosis, alopecia, nail diseases, skin diseases, kidney diseases,

chronic renal failure and/or cancer, and the like, especially the hypercholesterolemia, the hyperlipidemia, the hypertriglyceridemia, the familial hypercholesterolemia, the dyslipidemia, the atherosclerosis, the hypothyroidism, and/or the underlying hypothyroidism.

Definition

Unless otherwise defined hereinafter, all technical terms and scientific terms used herein have the same meanings as those commonly understood by those skilled in the art. The technical intention used herein refers to the technology commonly understood in the art, comprising those technical changes obvious to those skilled in the art, or the equivalent technical substitutions. Although it is believed that the following terms are well understood by those skilled in the art, the following definitions are still set forth to better explain the present application.

As used herein, the terms "comprising", "including", "having", "containing", or "involving" and other variants thereof herein are inclusive, or open-ended, and other unlisted elements, or method steps are not excluded.

As used herein, the term "hydrogen" and the hydrogen in each group cover the naturally occurring isotope thereof, such as protium (P), deuterium (D), or tritium (T).

"Alkyl" is a linear or branched chain-typed organic group containing only carbon atom and hydrogen atom. Examples of alkyl comprise linear, or branched alkyl of $C_{1-10}$, preferably $C_{1-6}$, and more preferably $C_{1-4}$, such as $C_1$, $C_2$, $C_3$, $C_4$, $C_5$, $C_6$, $C_7$, $C_8$, $C_9$, or $C_{10}$ alkyl, and specifically, such as methyl, ethyl, propyl, isopropyl, butyl, isobutyl, 1-methylpropyl, pentyl, hexyl, and the like.

"Halogen" comprises a fluorine atom, a chlorine atom, a bromine atom and an iodine atom.

"Cycloalkyl" comprises a monocyclic, bicyclic, or tricyclic non-aromatic carbocyclic ring of $C_{3-14}$, preferably $C_{3-10}$, and more preferably $C_{6-10}$, which is partially or fully saturated optionally.

"Heterocyclyl" comprises a monocyclic, bicyclic, or tricyclic non-aromatic carbocyclic ring, or cycloalkanes containing one or more (such as 1 to 5, 1 to 4, 1 to 3, or 1 to 2) heteroatoms selected from phosphorus atom, nitrogen atom, oxygen atom and sulfur atom (especially the nitrogen atom, the oxygen atom and the sulfur atom). Exemplarily, the "heterocyclyl" comprises a 5-12 membered monocyclic, or bicyclic non-aromatic carbocyclic ring, or cycloalkanes containing 1 to 4 heteroatoms selected from nitrogen atom, oxygen atom and sulfur atom, which is partially, or fully saturated optionally.

"Aryl" refers to an all-carbon monocyclic, or fused ring polycyclic aromatic group with a conjugated $\pi$ electron system. For example, as used herein, the term "$C_{6-14}$ aryl" refers to an aromatic group containing 6 to 14 carbon atoms, such as phenyl, or naphthyl. The aryl is optionally substituted by one or more (such as 1 to 3) suitable substituents (such as halogen, -OH, -CN, $-NO_2$, $C_{1-6}$ alkyl, and the like).

"Heteroaryl" is an aromatic cyclic group containing at least one heteroatom (nitrogen, oxygen, or sulfur) and a carbon atom, and comprises a 5-, or 6-membered monocyclic compound, an 8-10 membered bicyclic group in which the same or different monocyclic heteroaromatic rings are fused, and an 8-10 membered bicyclic group in which a monocyclic heteroaromatic ring is fused with benzene. Specific examples of the heteroaryl comprise furyl, thienyl, pyrrolyl, imidazolyl, pyrazolyl, triazolyl, tetrazolyl, oxazolyl, thiazolyl, isoxazolyl, isothiazolyl, oxadiazolyl, thiadiazolyl, furazanyl, pyridyl, pyrazinyl, pyrimidinyl, pyridazinyl, triazinyl, indolyl, indazolyl, benzimidazolyl, purinyl, quinolyl, isoquinolyl, naphthyridinyl, quinoxalyl, quinazolinyl, cinnolinyl, benzofuranyl, benzothienyl, benzoxazolyl, benzothiazolyl, benzisoxazolyl, benzisothiazolyl and the like.

As used herein, the term "substitution" refers to that one or more (such as one, two, three, or four) hydrogens on a specified atom are substituted by choices from indicated groups, provided that a normal valence of the specified atom in a current situation is not exceeded and the substitution forms a stable compound. A combination of substituents and/or variables is allowed only when such combination forms the stable compound.

If the substituent is described as being "optionally substituted", the substituent may be (1) unsubstituted, or (2) substituted. If carbon of the substituent is described as being optionally substituted by one or more from a substituent list, one or more hydrogens on the carbon (to an extent of existence of any hydrogen) may be optionally substituted by separately and/or jointly independently selected substituents. If nitrogen of the substituent is described as being optionally substituted by one or more from the substituent list, one or more hydrogens on the nitrogen (to an extent of existence of any hydrogen) may be optionally substituted by respectively independently selected substituents.

The "optionally substituted" may refer to substitution with 1 to 5, preferably 1 to 3, substituents, and the substituents comprise (1) alkyl substituted by 1 to 3 substituents selected from halogen, hydroxyl, carboxyl, amino, aryl, heteroaryl, cycloalkyl and heterocyclyl, (2) carbocyclyl substituted by 1 to 3 substituents selected from alkyl, halogen, hydroxyl, carboxyl, haloalkyl, alkoxy, haloalkoxy, alkanoyl and cyano, (3) heterocyclyl substituted by 1 to 3 substituents selected from alkyl, halogen, hydroxyl, carboxyl, haloalkyl, alkoxy, haloalkoxy, alkanoyl and cyano, (4) aryl substituted by 1 to 3 substituents selected from alkyl, halogen, hydroxyl, carboxyl, haloalkyl, alkoxy, haloalkoxy, alkanoyl and cyano, (5) heteroaryl substituted by 1 to 3 substituents selected from alkyl, halogen, hydroxyl, carboxyl, haloalkyl, alkoxy, haloalkoxy, alkanoyl and cyano, (6) hydroxyl, (7) alkoxy, (8) halogen, (9) amino optionally substituted by 1, or 2 alkyl groups, and (10)

oxy.

The present application further comprises all pharmaceutically acceptable and isotopically labeled compounds, which are the same as the compound of the present application, except that one or more atoms are substituted by atoms with the same atomic number, but the atomic mass, or mass number different from that prevailing in nature. Examples of isotopes suitable for being contained in the compound of the present application comprise (but are not limited to) isotopes of hydrogen (such as deuterium ($^{2}H$) and tritium ($^{3}H$)); isotopes of carbon (such as $^{11}C$, $^{13}C$ and $^{14}C$); isotopes of chlorine (such as $^{36}Cl$); isotopes of fluorine (such as $^{18}F$); isotopes of iodine (such as $^{123}I$ and $^{125}I$); isotopes of nitrogen (such as $^{13}N$ and $^{15}N$); isotopes of oxygen (such as $^{15}O$, $^{17}O$ and $^{18}O$); isotopes of phosphorus (such as $^{32}P$); and isotopes of sulfur (such as $^{35}S$). Some isotopically labeled compounds of the present application (such as those doped with radioisotopes) may be used in drug and/or substrate tissue distribution research (such as analysis). Radioisotopes tritium ($^{3}H$) and carbon-14 ($^{14}C$) are especially useful for this purpose due to easy doping and easy detection. Substitution with positron emission isotopes (such as $^{11}C$, $^{18}F$, $^{15}O$ and $^{13}N$) may be used for examining occupancy of a substrate receptor in a position emission tomography (PET) research.

A structure described herein also refers to comprising all isomeric (such as enantiomeric, diastereomeric and geometric (or conformational)) forms of the structure, such as a R and S configuration of each asymmetric center, a (Z) and (E) double bond isomer, and a (Z) and (E) conformational isomer. Therefore, single stereochemical isomers and enantiotopic, diastereomeric and geometric (or conformational) mixtures of these compounds all belong to the scope of the present application. Unless otherwise specified, all tautomeric forms of the compound of the present application belong to the scope of the present application. In addition, unless otherwise specified, the structure described herein also refers to comprising all different compounds only in the existence of one or more isotope-enriched atoms.

All possible crystal forms or polymorphic substances of the compound of the present application are contained herein, which may be a single crystal form, or a mixture of more than one polymorphic substance in any proportion.

It should also be understood that some compounds of the present application may exist in free form for treatment, or exist in a form of pharmaceutically acceptable derivatives if appropriate. In the present application, the pharmaceutically acceptable derivatives comprise, but are not limited to, a pharmaceutically acceptable salt, a solvate, or a N-oxide, and after the derivatives are administered to patients in need thereof, the compound of the present application is directly or indirectly provided. Therefore, when "the compound of the present application" is mentioned herein, the above various derivative forms of the compound are also contained.

The pharmaceutically acceptable salt of the compound of the present application comprises an acid addition salt and a base addition salt thereof, wherein types of the salt are not particularly limited as long as the salt is physiologically acceptable. Examples of a suitable pharmaceutically acceptable acid addition salt comprise, but are not limited to, hydrochloride, hydrobromide, sulfate, nitrate, phosphate, acetate, trifluoroacetate, tartrate, fumarate, oxalate, maleate, citrate, succinate, methanesulfonate, benzenesulfonate, malate, aspartate, gluceptate, gluconate, orotate, palmitate and other similar salts. Examples of a suitable pharmaceutically acceptable base addition salt comprise, but are not limited to, a sodium salt, a potassium salt, an ammonium salt, a calcium salt, a magnesium salt, an aluminum salt, an iron salt, a histidine salt, an arginine salt, a choline salt and other similar salts.

The compound of the present application may exist in a form of a solvate (preferably a hydrate), wherein the compound of the present application contains a polar solvent as a structural element of a crystal lattice of the compound, such as water, methanol, or ethanol especially. An amount of the polar solvent, especially the water, may exist in a form of a stoichiometric or non-stoichiometric ratio.

Those skilled in the art may understand that, since nitrogen needs to be oxidized into an oxide with available lone pair electrons, not all nitrogen-containing heterocycles can form the N-oxide. Those skilled in the art may recognize the nitrogen-containing heterocycle capable of forming the N-oxide. Those skilled in the art may also recognize that tertiary amine can form the N-oxide. A synthetic method of the N-oxide for preparing the heterocycle and the tertiary amine is well known to those skilled in the art, and comprises oxidizing the heterocycle and the tertiary amine with peroxyacid such as peracetic acid and m-chloroperoxybenzoic acid (MCPBA), hydrogen peroxide, alkyl hydrogen peroxide such as tert-butyl hydroperoxide, sodium perborate and dioxirane such as dimethyl dioxirane.

The metabolite of the compound of the present application is a substance formed in vivo when the compound of the present application is administered. Such product may be produced by, for example, oxidation, reduction, hydrolysis, amidation, deamidation, esterification, enzymolysis, and the like of the compound administered. Therefore, the present application comprises the metabolite of the compound of the present application, and comprises a compound produced by a method of contacting the compound of the present application with a mammal for a time sufficient to produce the metabolite.

A "pharmaceutically acceptable carrier" in the present application refers to a pharmacologically and pharmaceutically acceptable additive administered together with an active ingredient, and an excipient, a disintegrant, an adhesive, a lubricant, a coating agent, a dye, a diluent, a base agent, an isotonic agent and the like may be used.

The dosage forms comprise, but are not limited to, a tablet, a capsule, a lozenge, a hard candy, a powder, a spray, a cream, an ointment, a drop, a suppository, a gel, a paste, a lotion, an aqueous suspension, an injectable solution, an elixir

and a syrup.

Examples of the dosage forms suitable for oral administration comprise tablet, capsule, powder, a fine granule, a granule, a liquid, syrup, and the like. Examples of the dosage forms suitable for non-oral administration comprise injection, drop, suppository, and the like.

In the text, unless otherwise stated, singular terms contain plural referents, and vice versa. Unless otherwise stated, the term "subject" may be used interchangeably with the terms "individual" and "patient", and comprises a vertebrate, such as birds, fishes and mammals, comprising but being not limited to mice, rats, guinea pigs, dogs, pigs, sheep, cattle, chickens, rabbits, monkeys (such as rhesus monkeys), human beings, and the like.

In the text, unless otherwise stated, all numbers used herein to indicate amounts of ingredients, measured values, or reaction conditions should be understood as being modified by the term "about" in all cases, so as to indicate possible measurement errors. For example, when connected with a percentage, the term "about" may refer to ±1%.

The compound of formula I of the present application shows a thyroid hormone β receptor agonist action, and can be a drug for preventing, or treating a disease regulated by receptor β, such as being used for preventing, reducing and/or treating the following diseases: hypercholesterolemia, hyperlipidemia, hypertriglyceridemia, familial hypercholesterolemia, dyslipidemia, thyroid cancer, hypothyroidism, underlying hypothyroidism, atherosclerosis, metabolic syndrome, obesity, diabetes, cardiovascular diseases, coronary artery diseases, myocardial infarction, ventricular insufficiency, heart failure, fatty liver, liver cirrhosis, non-alcoholic steatohepatitis (NASH), non-alcoholic fatty liver disease (NAFLD), depression, dementia, osteoporosis, alopecia, nail diseases, skin diseases, kidney diseases, chronic renal failure, and/or cancer, and the like, especially the hypercholesterolemia, the hyperlipidemia, the hypertriglyceridemia, the familial hypercholesterolemia, the dyslipidemia, the atherosclerosis, the hypothyroidism and/or underlying hypothyroidism, and the like.

## DESCRIPTION OF THE DRAWINGS

FIG. 1 shows results of fibrosis evaluation.
FIG. 2 shows results of NAS score

## EMBODIMENTS

In, order to make the objects and technical solutions of the present application clearer, the present application is further illustrated below with reference to the specific embodiments. It should be understood that these embodiments are only used to illustrate the present application and are not used to limit the scope of the present application. Further, the specific experimental methods not mentioned in the following embodiments are carried out according to the usual experimental methods.

### Example 1 Synthesis of key intermediate KH01

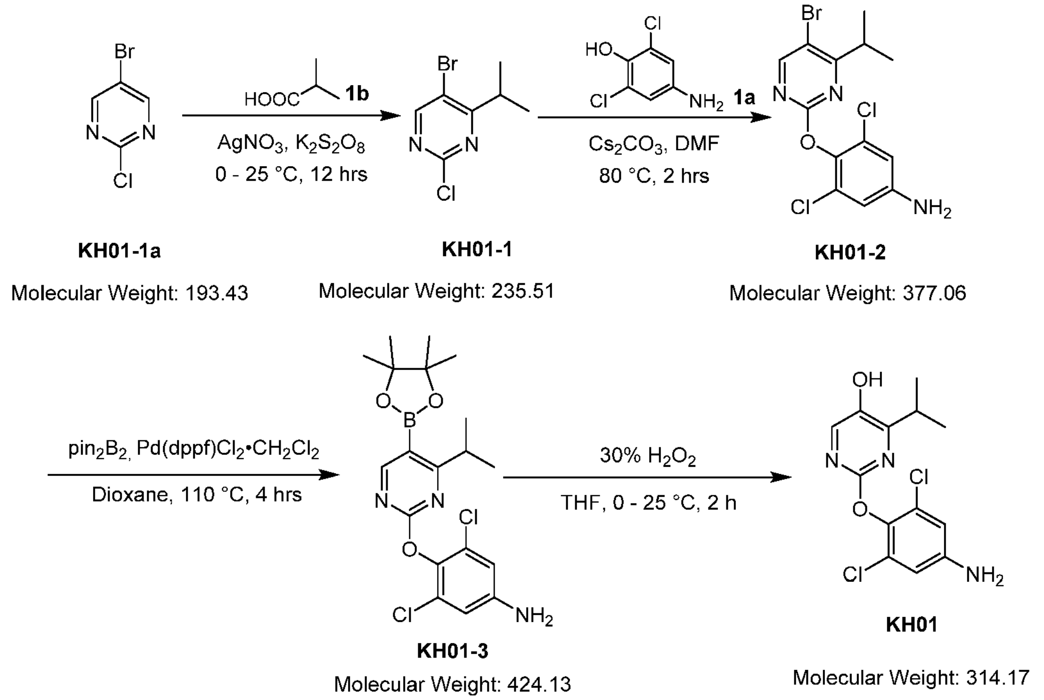


**KH01-1a** Molecular Weight: 193.43

**KH01-1** Molecular Weight: 235.51

**KH01-2** Molecular Weight: 377.06

**KH01-3** Molecular Weight: 424.13

**KH01** Molecular Weight: 314.17

**Compound KH01-1:** starting materials 2-chloro-5-bromopyrimidine (KH01-1a) (100 g, 516 mmol, 1.00 eq), isobutyric acid (1b) (36.4 g, 413 mmol, 38.3 mL, 0.80 eq), potassium persulfate (111 g, 413 mmol, 82.8 mL, 0.80 eq), and silver nitrate (17.5 g, 103 mmol, 0.20 eq) were added into a round-bottom flask at 0°C, then added with 1 L of dichloromethane and 1 L of water. After stirring evenly, some solids were un-dissolved, and then the mixture was stirred at room temperature (25°C) for 12 hours under the protection of nitrogen. TLC monitoring showed that the raw materials were completely reacted and a new spot was formed. The reaction mixture was filtered and the filter cake was washed with dichloromethane twice (2 × 1 L). The filtrate was collected and concentrated under reduced pressure; The residue was purified by silica gel (100-200 mesh) column chromatography (petroleum ether/ethyl acetate =100: 1) to afford 54.0 g of substance KH01-1 as an oil, yield:44.3%. LCMS:MS (ESI) $m/z$ = 236.9 [M+H]$^+$. $^1$H NMR (400MHz $CDCl_3$): δ 8.58 (s, 1H), 3.48 -3.41 (m, 1H), 1.29 (d, $J$ = 6.8 Hz, 6H).

**Compound KH01-2:** A reaction mixture of **KH01-1** (15.0 g, 63.6 mmol, 1.00 eq), 1a (11.3 g, 63.6 mmol, 1.00 eq), and cesium carbonate (62.2 g, 191 mmol, 3.00 eq) in N,N-dimethylformamide (150 mL) was subjected to nitrogen replacement thrice under stirring, then heated at an external temperature of 80°C for 2 hours under the protection of nitrogen. The reaction was monitored by TLC until it was complete. The reaction mixture was poured into 100 mL of water and stirred until a clear solution formed. The reaction mixture was extracted with ethyl acetate (2 × 100 mL). The ethyl acetate layers were combined, washed with saturated salti solution (2 × 100 mL), dried over anhydrous sodium sulfate, and filtered. The filtrate was concentrated and the residue was purified through silica gel (100-200 mesh) column chromatography (petroleum ether/ethyl acetate = 1: 2) to obtain 13.0 g of **KH01-2** as a yellow solid yield: 54.1%. LCMS: MS (ESI) $m/z$ = 378.0 [M+H]$^+$. $^1$H NMR (400MHz $CDCl_3$): δ 8.46 (s, 1H), 6.68 (s, 2H), 3.78 (s, 2H), 3.43 - 3.36 (m, 1H), 1.22 (d, $J$ = 6.8 Hz, 6H).

**Compound KH01-3:** A reaction mixture of **KH01-2** (10.0 g, 26.5 mmol, 1.00 eq), bis(pinacolato)diboron ($pin_2B_2$) (13.4 g, 53.0 mmol, 2.00 eq), 1,1'-bis(diphenylphosphino)ferrocene-palladium(II)dichloride dichloromethane complex (1.08 g, 1.33 mmol, 0.05 eq), and potassium acetate (5.21 g, 53.0 mmol, 2.00 eq) in dioxane (100 mL) was subjected to nitrogen replacement thrice, then heated at an external temperature of 110°C for 4 hours under the protection of nitrogen. TLC showed that the raw materials were completely reacted. The reaction mixture was added with 200 mL of water, and extracted with ethyl acetate (3 × 200 mL). The ethyl acetate layers were combined, washed with saturated salt solution (300 mL × 2), dried over anhydrous sodium sulfate, and filtered. The filtrate was concentrated to obtain 12.0 g of crude product as a brown oil, which was directly used in the next step without further purification. **LCMS:** MS (ESI) $m/z$ = 425.2 [M+H]$^+$.

**Compound KH01:** the crude **KH01-3** (12.0 g, 28.2 mmol, 1.00 eq) above and 30.0% hydrogen peroxide (6.74 g, 59.4 mmol, 5.71 mL, 2.10 eq) were dissolved in tetrahydrofuran (120 mL) at an external temperature of 0°C. The mixture was stirred at room temperature (25°C) for 2 hours under the protection of nitrogen. TLC indicated that the reaction was complete. The reaction mixture was quenched with 50 mL of 2M sodium sulfite solution, then extracted with dichloromethane (3× 5 mL). The combined organic layers were dried over anhydrous sodium sulfate, concentrated. The residue was purified by silica gel (100-200 mesh) column chromatography (petroleum ether/ethyl acetate = 1: 2) to obtain 4.80 g of

yellow solid yield: 53.4%. LCMS: MS (ESI) *m/z* = 314.1 $[M+H]^+$. $^1H$ NMR (400 MHz, DMSO-$d_6$): δ 9.85 (br s, 1H), 7.95 (s, 1H), 6.66 (s, 2H), 5.52 (s, 2H), 3.31 - 3.18 (m, 1H), 1.10 (d, *J* = 6.8 Hz, 6H).

**Example 2 Synthesis of compound KH02**

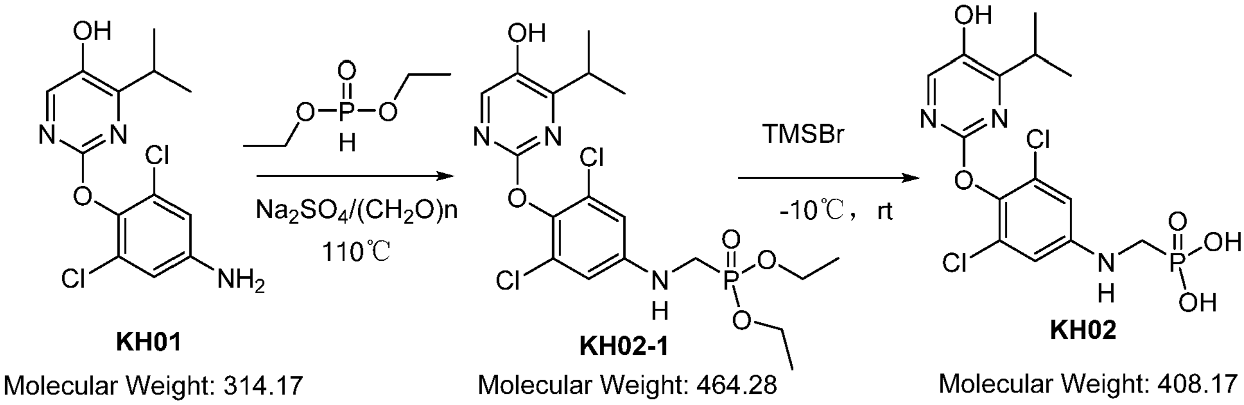


**KH01** Molecular Weight: 314.17
**KH02-1** Molecular Weight: 464.28
**KH02** Molecular Weight: 408.17

**Compound KH02-1:** To a mixture of **KH01** (0.152 g, 0.485 mmol), diethyl phosphite (0.104 g, 0.754 mmol), paraformaldehyde (0.095 g, 1.055 mmol) and sodium sulfate (0.156 g, 1.098 mmol) in a single-necked flask, was added toluene (8 mL) under the protection of $N_2$. The partially dissolved reaction mixture was heated at an external temperature of 110°C for 3 hours. TLC showed that the reaction was complete and a new spot with increased polarity was formed. The reaction mixture was added with 100 mL of water and extracted with ethyl acetate (3 × 50 mL). The organic phases were collected, dried over anhydrous sodium sulfate, filtered, and concentrated. The residue was purified by silica gel column chromatography (petroleum ether/ethyl acetate = 1: 2) to obtain **KH02-1** as a colorless oil. (142 mg, 63%). LC-MS (ESI, *m/z*): 465.3$[M+H]^+$.

**Compound KH02:** compound **KH02-1** (0.142 g, 0.306 mmol) was added into a single-necked flask, and dissolved with dichloromethane (10 mL) under the protection of $N_2$. At an external temperature of -10°C, trimethylbromosilane (3.2 mL) was slowly added dropwise to the reaction system, and reacted at this temperature for 30 minutes, and then the temperature was slowly raised to room temperature for a reaction overnight. LCMS showed that the raw materials were completely reacted. The reaction mixture was directly concentrated by rotary evaporation. The residue was purified by preparative HPLC to give compound **KH02** as a faint yellow solid after freeze-dried (55 mg, 44%). $^1H$ NMR (400 MHz, DMSO): δ 9.87 (s, 1H), 7.95 (s, 1H), 6.82 (s, 2H), 4.61 - 5.22 (m, 3H), 3.27 (m, 3H), 1.12 (d, J = 4 MHz, 6H). LC-MS (ESI, *m/z*): 408.0 $[M+1]^+$.

**Example 3 Synthesis of compound KH03**

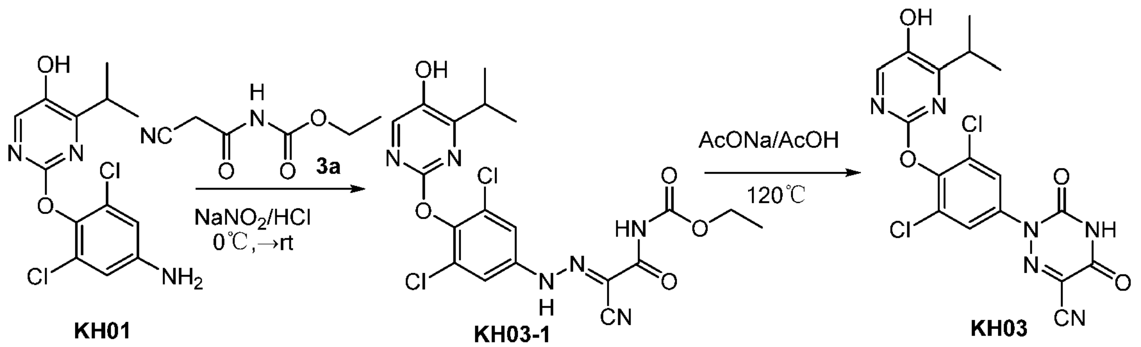


**KH01** Molecular Weight: 314.17
**KH03-1** Molecular Weight: 481.29
**KH03** Molecular Weight: 435.22

**Compound KH03-1:** compound **KH01** (0.265 g, 0.846 mmol) was added into 11.2 mL of water, added with 5.6 mL of concentrated hydrochloric acid at an external temperature of 0°C, then weighed sodium nitrite (0.072 g, 1.043 mmol)

was dissolved into 0.8 mL of water, slowly dropwise added into the reaction solution, and stirred at 0°C for 1.5 hours to obtain a mixture. Compound **3a** (0.148 g, 0.948 mmol) was additionally weighed and dissolved in 19.4 mL of water, added with 5.6 mL of pyridine at 0°C, stirred for 1.5 hours at this temperature, and then added quickly to the above-mentioned reaction solution at 0°C. In this case, tangerine solids were formed, and then the reaction solution was slowly heated to room temperature (25°C) and continuously reacted overnight. After TLC monitoring showed that the reaction was complete, the solids were directly filtered on a filter funnel and washed thrice with 50 mL of water and PE respectively. The tangerine solids **KH03-1** (380 mg, 93.8%) were collected and obtained. LC-MS (ESI, *m/z*): 482.3 $[M+1]^+$.

**Compound KH03:** compound **KH03-1** (0.380 g, 0.931 mmol) and sodium acetate (0.650 g, 7.926 mmol) were added into a single-necked flask, and dissolved with acetic acid (10 mL) under the protection of $N_2$. The reaction was carried out for 3 hours at an external temperature of 120°C. After TLC monitoring showed that the raw materials were completely reacted, the reaction was stopped. The reaction solution was cooled to 0°C and added with 100 mL of water, then a large amount of solids were precipitated, which were directly filtered through a filter funnel, and the solids were washed with of water and PE respectively (3 × 50 mL). Tangerine solids **KH03** were collected and obtained (230 mg, 66.9%). $^1$H NMR (400 MHz, DMSO) δ 10.09 (s, 1H), 8.01 (s, 1H), 7.75 (s, 2H), 3.27 - 3.28 (m, 1H), 1.13 (d, J = 4 MHz, 6H). LC-MS (ESI, *m/z*): 435.2 $[M+1]^+$.

**Example 4 Synthesis of compound KH04**

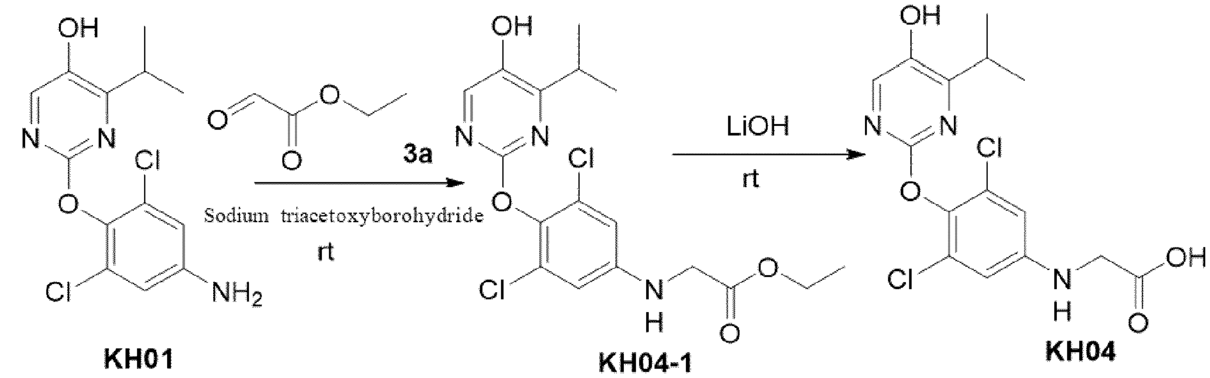


**KH01** Molecular Weight: 314.17

**KH04-1** Molecular Weight: 400.26

**KH04** Molecular Weight: 372.20

**Compound KH04-1:** compound KH01 (0.0512 g, 0.1635 mmol), ethyl glyoxylate (0.0274 g, 0.268 mmol) and sodium triacetoxyborohydride (0.1023 g, 0.483 mmol) were weighed and added into a single-necked flask, and then added with 1,2-dichloroethane (3 mL) for dissolution. The reaction was carried out at an external temperature of 75°C for 3 hours, TLC monitoring showed that the raw materials were reacted completely, and a new increased polarity spot was formed. Then, the reaction was stopped. The reaction solution was added with 50 mL of dichloromethane and 100 mL of water. After stirring for 10 minutes, organic phases were separated, aqueous phases were extracted with dichloromethane (3×50 mL), and then the, organic phases were combined, dried with anhydrous sodium sulfate, filtered and concentrated. The residue was purified by chromatoplate (petroleum ether/ethyl acetate = 1: 2) to obtain colorless oily substance **KH04-1** (50 mg, 78.5%). LC-MS (ESI, *m/z*): 401.3 $[M+1]^+$.

**Compound KH04:** compound **KH04-1** (50 mg, 0.125 mmol) and lithium hydroxide (35 mg, 1.458 mmol) were added into a single-necked flask, and added with tetrahydrofuran/methanol/water (4:1:1, 6 mL) for dissolution. The reaction was carried out at room temperature overnight. After TLC monitoring showed that the raw materials were completely reacted, the reaction was stopped. 20 mL of water was added to dilute the reaction solution, the, organic solvent was removed under reduced pressure, and the reaction PH was adjusted to 3-4 at 0°C. Dichloromethane (50 mL × 3) was added to the reaction solution for extraction, the organic phases were collected, dried with anhydrous $Na_2SO_4$, filtered and concentrated to remove the solvent. The residues were purified by chromatoplate (dichloromethane/methanol = 5: 1). The target product was collected, and freeze-dried to obtain white solids **KH04** (24 mg, 51.6%). $^1$H NMR (400 MHz, DMSO) δ 10.48 (s, 1H), 8.00 (s, 1H), 6.65 (s, 2H), 5.98 (s, 1H), 4.21 - 4.22 (m, 1H), 3.49 (s, 1H), 3.34 - 3.19 (m, 2H), 1.12 (d, *J* = 4 MHz, 6H). LC-MS (ESI, *m/z*): 372.1 $[M+1]^+$.

**Example 5 Synthesis of compound KH05**

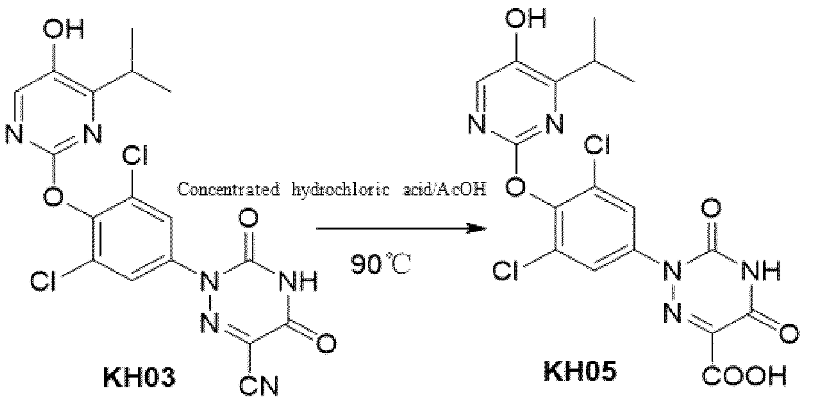


KH03 KH05

Molecular Weight: 435.22 Molecular Weight: 454.22

100 mg of compound **KH03** was dissolved in 5 mL of acetic acid, added with 1 mL of concentrated hydrochloric acid, and stirred at an external temperature of 90°C for 4 hours. After TLC monitoring showed that the raw materials were completely reacted, the reaction solution was decompressed and dried by rotary evaporation and adjust the pH to 9-10 with adding a saturated sodium carbonate solution. After the reaction solution was extracted with 50 mL of ethyl acetate, organic phases were discarded, aqueous phases were adjusted to a pH =3-4, and extracted with ethyl acetate (50 mL × 3), and then the, organic phases were combined, dried with anhydrous sodium sulfate, and concentrated to obtain 70 mg of white solids. The yield was 67.1%. LC-MS (ESI, *m*/*z*): 455.3 $[M+1]^+$.

**Example 6 Synthesis of compound KH06**

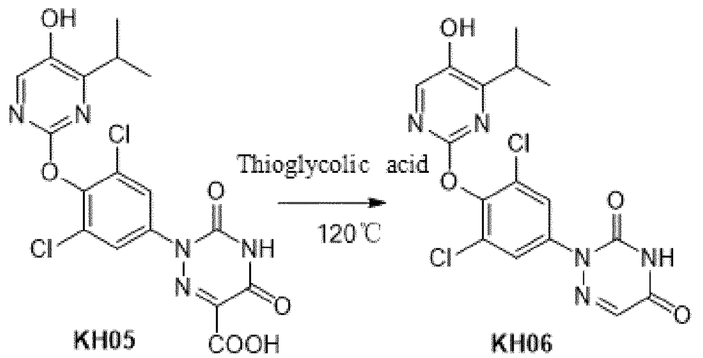


KH05 KH06

Molecular Weight: 454.22 Molecular Weight: 410.21

**KH05 (60mg)** was dissolved in 4 mL of thioglycolic acid, and stirred at an external temperature of 120°C under the protection of nitrogen for 6 hours, TLC monitoring showed that a low polarity spot was formed. Saturated sodium thiosulfate was added into the reaction solution to quench the reaction, and ethyl acetate was used for extraction (25 ml × 3), organic phases were combined, dried with anhydrous sodium sulfate, concentrated, and purified through chromatoplate (dichloromethane/methanol = 10: 1). The target product was collected, and freeze-dried to obtain about 1.5 mg of white solids KH06, yield: 27.8%. LC-MS (ESI, m/z): 410.1 $[M+1]^+$. $^1$H NMR (400 MHz, DMSO) δ: 12.49 (s, 1H), 10.08 (s, 1H), 8.01 (s, 2H), 7.71 (d, J = 4 MHz, 1H), 3.27-3.33 (m, 1H), 1.12 (d, J = 4MHz, 6H).

**Example 7 Synthesis of key intermediate KH07-10**

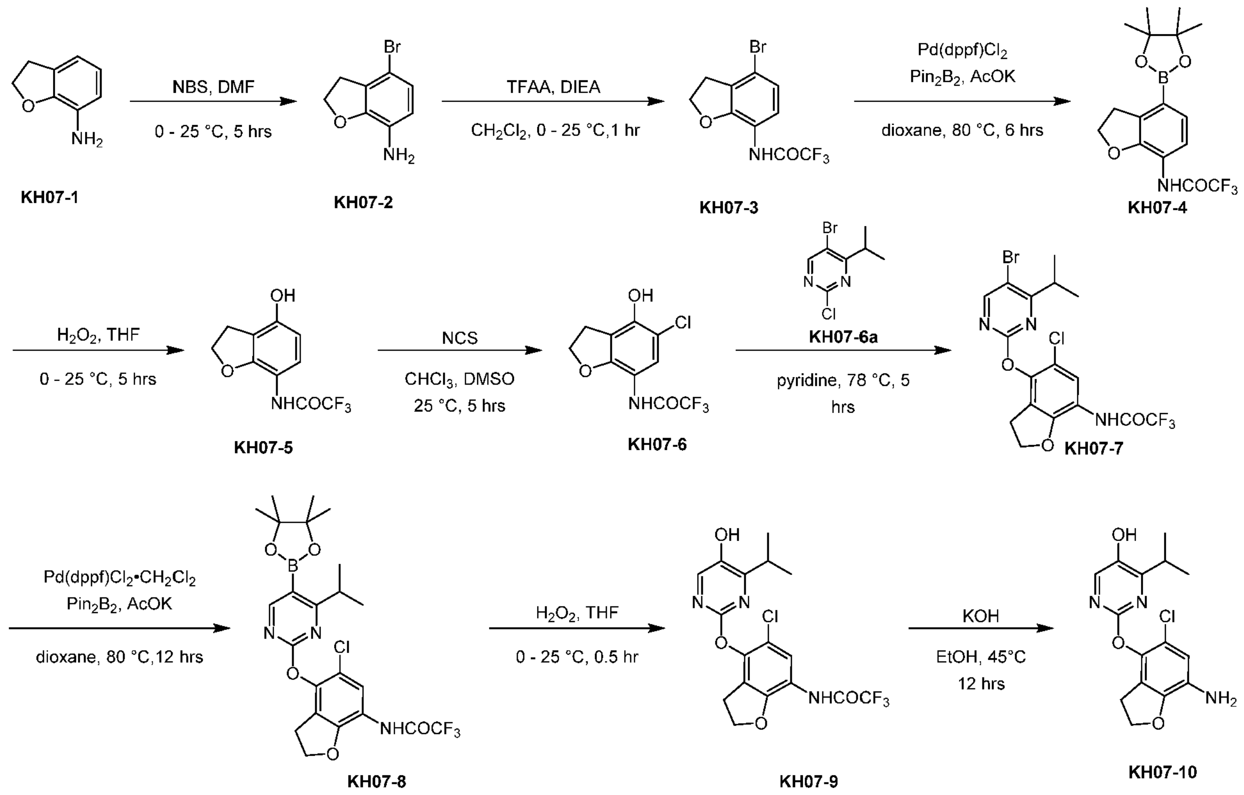


KH07-1 KH07-2 KH07-3 KH07-4 KH07-5 KH07-6 KH07-7 KH07-8 KH07-9 KH07-10

**Compound KH07-2:** the raw material **KH07-1** (25.0 g, 184 mmol, 1 eq) was dissolved in DMF (200 mL), slowly added with NBS (32.9 g, 184 mmol, 1 eq) at 0°C, and after the addition, stirred at an external temperature of 25°C for 5 hours. LCMS monitoring showed that the raw materials were complete reacted, and new spot (RT = 0.483) was formed. TLC (petroleum ether/ethyl acetate = 3/1) monitoring showed that two new spots were formed. The reaction solution was diluted with water (250 mL), and then added with ethyl acetate (250 mL × 2) for extraction, organic phases were combined, washed with saturated salt solution (250 mL × 2), dried with anhydrous sodium sulfate, filtered, concentrated, and purified through silica gel column chromatography ($SiO_2$, petroleum ether/ ethyl acetate = 50/1 to 3/1) to obtain yellow solids **KH07-2** (29.1 g, 135 mmol, yield: 73.5%). MS (ESI) *m/z*: 216.1 $[M+H]^+$. **$^1$H NMR** (DMSO-$d_6$, 400 MHz): δ 6.75 - 6.69 (m, 1H), 6.43 (d, *J* = 8.4 Hz, 1H), 4.76 (s, 2H), 4.58 - 4.49 (m, 2H), 3.15 - 3.08 (m, 2H).

**Compound KH07-3:** compound **KH07-2** (28.0 g, 130 mmol, 1 eq) and TFAA (32.9 g, 156 mmol, 21.8 mL, 1.2 eq) were dissolved in dichloromethane (280 mL), slowly added dropwise with DIEA (33.8 g, 261 mmol, 45.5 mL, 2 eq) at an external temperature of 0°C, and after the addition, the mixture was stirred at an external temperature of 25°C for 1 hour. TLC (petroleum ether/ethyl acetate = 5/1) monitoring showed that the raw materials were completely reacted. The reaction solution was poured into water (280 mL), and extracted with dichloromethane (300 mL × 3), organic layers were combined, washed with saturated salt solution (280 mL), dried with anhydrous sodium sulfate, filtered, concentrated, and purified through silica gel column chromatography ($SiO_2$, petroleum ether/ ethyl acetate= 50/1 to 3/1) to obtain yellow solids **KH07-3** (27.8 g, yield: 68.5%). MS (ESI) *m/z*: 309.9 $[M+H]^+$. **$^1$H NMR** (DMSO-$d_6$, 400 MHz): δ 11.26 - 10.86 (m, 1H), 7.15 - 7.10 (m, 1H), 7.09 - 7.03 (m, 1H), 4.71 - 4.62 (m, 1H), 4.66 (t, *J* = 8.8 Hz, 1H), 3.25 (t, *J* = 8.8 Hz, 2H).

**Compound KH07-4:** compound **KH07-3** (27.0 g, 87.0 mmol, 1 eq), $Pd(dppf)Cl_2{\bullet}CH_2Cl_2$ (3.56 g, 4.35 mmol, 0.05 eq), $Pin_2B_2$ (55.2 g, 217 mmol, 2.5 eq) and potassium acetate (25.6 g, 261 mmol, 3 eq) were added into dioxane (270 mL), and stirred at an external temperature of 80°C for 6 hours under the protection of nitrogen. After LCMS monitoring showed that the raw materials were completely reacted, the reaction was poured into water (500 mL) and extracted with ethyl acetate (500 mL × 3). Combined, organic phases were washed with saturated salt solution (500 mL × 3), dried with anhydrous sodium sulfate, filtered and concentrated to obtain brown solids **KH07-4** (33.0 g, crude product) which were used in the next step without purification. MS (ESI) *m/z*: 358.1 $[M+H]^+$.

**Compound KH07-5:** compound **KH07-4** (32.0 g, 89.6 mmol, 1 eq) was dissolved with tetrahydrofuran (300 mL), slowly added dropwise with $H_2O_2$ (30.4 g, 268 mmol, 25.8 mL, purity 30.0%, 3 eq) at an external temperature of 0°C, and after the addition, the mixture stirred at an external temperature of 25°C for 5 hours. After LCMS monitoring showed that the raw materials were complete reacted, the reaction solution was slowly poured into saturated sodium sulfite (400 mL) to terminate the reaction, and then extracted with ethyl acetate (200 mL × 2), organic phases were combined, washed with saturated salt solution (100 mL × 2), dried with anhydrous sodium sulfate, filtered, concentrated and purified through silica gel column chromatography ($SiO_2$, petroleum ether/ ethyl acetate = 20/1 to 5/1) to obtain white solids **KH07-5** (20.0 g, 80.9

mmol, yield: 90.3%). MS (ESI) *m/z*: 248.1 $[M+H]^+$. **$^1$H NMR** (DMSO-$d_6$, 400 MHz): δ 10.6 (s, 1H), 9.63 (s, 1H), 6.87 (d, *J* = 8.4 Hz, 1H), 6.31 (d, *J* = 8.8 Hz, 1H), 4.55 (t, *J* = 8.8 Hz, 2H), 3.09 (t, *J* = 8.8 Hz, 2H).

**Compound KH07-6:** compound **KH07-5** (10.0 g, 40.4 mmol, 1 eq) and NCS (6.48 g, 48.5 mmol, 1.20 eq) were added into a mixed solvent of chloroform (100 mL) and DMSO (25.0 mL), and stirred at an external temperature of 25°C for 5 hours. After LCMS monitoring showed that the raw materials were completely reacted, the reaction solution was directly concentrated and purified by preparative HPLC to obtain white solids **KH07-6** (4.50 g, 15.9 mmol, yield: 39.5%). MS (ESI) *m/z*: 282.0 $[M+H]^+$. **$^1$H NMR** ($CDCl_3$, 400 MHz): δ 8.00 - 7.88 (m, 1H), 7.76 (br s, 1H), 5.57 (br s, 1H), 4.65 (t, *J* = 8.8 Hz, 2H), 3.24 - 3.18 (m, 2H).

**Compound KH07-7:** compound **KH07-6** (2.50 g, 8.88 mmol, 1 eq) and **KH07-6a** were added to 25 mL of pyridine, and stirred at an external temperature of 78°C for 5 hours. TLC (petroleum ether/ethyl acetate = 5/1) monitoring showed that the raw materials were completely reacted. The reaction solution was poured into 60 mL of water, and extracted with ethyl acetate (100 mL × 3), organic phases were combined, washed with saturated salt solutoin (100 mL × 3), dried with anhydrous sodium sulfate, filtered, concentrated, and purified through silica gel column chromatography ($SiO_2$, petroleum ether/ ethyl acetate= 5/1 to 3/1) to obtain yellow solids **KH07-7** (1.20 g, 2.50 mmol, yield: 28.1%). MS (ESI) *m/z*: 482.0 $[M+H]^+$. **$^1$H NMR** (DMSO-$d_6$, 400 MHz): δ 11.20 - 11.11 (m, 1H), 8.73 (s, 1H), 7.47 - 7.35 (m, 1H), 4.68 - 4.61 (m, 2H), 3.39 - 3.34 (m, 1H), 3.15 - 3.06 (m, 2H), 1.15 (d, *J* = 6.8 Hz, 6H).

**Compound KH07-8:** compound **KH07-7** (1.05 g, 2.18 mmol, 1 eq), $Pin_2B_2$ (1.39 g, 5.46 mmol, 2.5 eq), AcOK (643 mg, 6.55 mmol, 3 eq) and $Pd(dppf)Cl_2 \cdot CH_2Cl_2$ (107 mg, 131 mmol, 0.06 eq) were added into 10 mL of dioxane, was subjected to nitrogen replacement thrice under stirring and then the mixture was continuously stirred at an external temperature of 80°C for 12 hours under the protection of nitrogen. LCMS monitoring showed that some raw materials were remained, and about 39.8% product was formed. The reaction solution was directly concentrated, and the residues were added into 100 mL of water, stirred, extracted with ethyl acetate (100 mL × 2), washed with saturated salt solution (100 mL × 2), dried with anhydrous sodium sulfate, filtered, and concentrated to obtain brown solids (1.00 g, crude product). MS (ESI) *m/z*: 528.1 $[M+H]^+$.

**Compound KH07-9:** the operation was the same as the synthesis of compound **KH07-5,** and the reaction solution was purified through silica gel column chromatography ($SiO_2$, petroleum ether/ethyl acetate = 1/0 to 3/1) to obtain white solids **KH07-9** (700 mg). MS (ESI) *m/z*: 418.0 $[M+H]^+$. **$^1$H NMR** (DMSO-$d_6$, 400 MHz): δ 11.12 (s, 1H), 10.00 (s, 1H), 8.00 (s, 1H), 7.36 (s, 1H), 4.62 (t, *J* = 8.8 Hz, 2H), 4.34 (t, *J* = 5.2 Hz, 1H), 3.30 - 3.26 (m, 1H), 3.02 (t, *J* = 8.8 Hz, 2H), 1.17 - 1.10 (m, 1H), 1.14 (d, *J* = 6.8 Hz, 6H).

**Compound KH07-10:** compound **KH07-9** (700 mg, 1.68 mmol, 1 eq) and potassium hydroxide (376 mg, 6.70 mmol, 4 eq) were added into a mixed solution of ethanol (4 mL) and water (3 mL) and stirred at an external temperature of 45°C for 12 hours. LCMS monitoring showed that the raw materials were completely reacted. The reaction solution was adjusted to a pH 7 approximately by 1N hydrochloric acid, then added with 50 mL of water, stirred for 10 minutes, and then extracted with ethyl acetate (50 mL × twice), organic phases were combined, washed with saturated salt solution (100 mL × 2), dried with anhydrous sodium sulfate, filtered, and purified by preparative HPLC to obtain faint yellow solids **KH07-10** (501.4mg, yield: 93.0%, purity 99.6%). MS (ESI) *m/z*: 322.2 $[M+H]^+$. **$^1$H NMR** (DMSO-$d_6$, 400 MHz): δ 9.81 (s, 1H), 7.95 (s, 1H), 6.71 - 6.35 (m, 1H), 5.75 (s, 1H), 4.78 (s, 2H), 4.50 (br t, *J* = 8.8 Hz, 2H), 3.31 - 3.21 (m, 1H), 2.90 (br t, *J* = 8.8 Hz, 2H), 1.12 (d, *J* = 6.8 Hz, 6H).

## Example 8 Synthesis of compound KH07

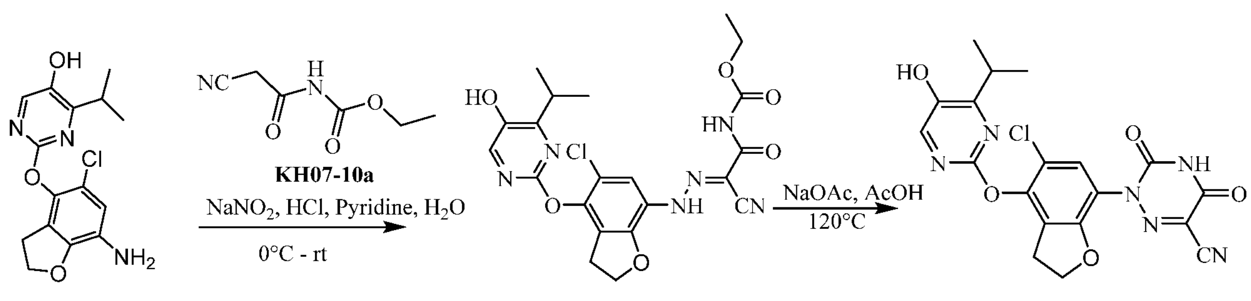


KH07-10 KH07-11 KH07

**Compound KH07-11:** reaction solution **A:** compound **KH07-10** (202.5 mg, 0.637 mmol) was added into 10 mL of water, and added with 5.6 mL of concentrated hydrochloric acid at 0°C. Weighed sodium nitrite (58.3 mg, 0.803 mmol) was dissolved in 1 mL of water, slowly dropwise added into the reaction solution, and stirred at 0°C for 1.5 hours to make the

sodium nitrite to obtain a solution; Reaction solution B: compound **KH07-10a** (108.8 mg, 0.7 mmol) was added into 20 mL of water, added with 5.6 mL of pyridine at 0°C, and then stirred at this temperature for 1.5 hours. Then, the reaction solution A was quickly poured into the reaction solution B at 0°C to form tangerine solids, and the temperature was slowly raised to room temperature to continue the reaction overnight. After TLC monitoring showed that the reaction was complete, the solids were directly filtered and washed with water and petroleum ether (25 mL × 3) respectively. Tangerine solids **KH07-11** (280 mg, yield: 89.9%, crude product) were obtained, which were directly used in the next step without purification. MS (ESI) m/z: 289.2 $[M+H]^+$.

**Compound KH07:** compound **KH07-11** (280 mg, 0.573 mmol) and sodium acetate (485.4 mg, 5.73 mmol) were added into in a single-necked flask, and dissolved with acetic acid (10 mL) under the protection of $N_2$. The reaction was carried out for 3 hours at an external temperature of 120°C. After TLC monitoring showed that the raw materials were completely reacted, the reaction was stopped. The reaction solution was cooled to 0°C and after the addition of 50 mL of water, a large amount of solids were precipitated which were directly filtered and washed with water (20 mL × 3) and petroleum ether (20 mL × 3) respectively. Tangerine solids (254 mg, crude product) were collected, and 50 mg of the crude product was purified by preparative HPLC to obtain off-white solid **compound KH07** (13.5 mg, 27.0%). MS (ESI) m/z: 443.0 $[M+H]^+$. **$^1$H NMR** (DMSO-$d_6$, 400 MHz): 10.06 (s, 1H), 7.40 (s, 1H), 4.65 (t, 2H, J = 8.0Hz), 3.21-3.32 (m, 1H), 3.05 (t, 2H, J = 8.0 Hz), 1.16 (d, 6H, J = 8.0Hz).

**Example 9 Synthesis of compound KH08**

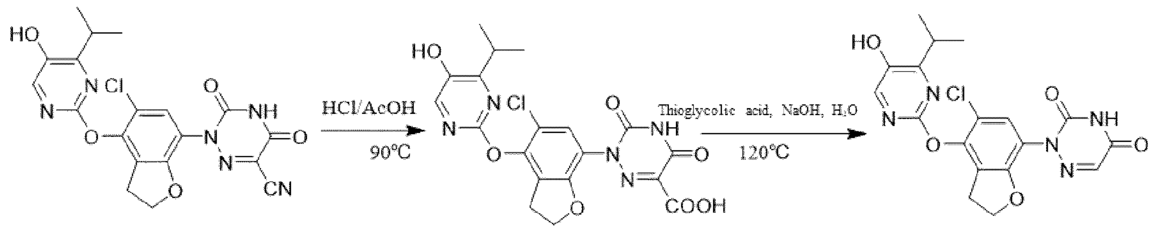


KH07 KH08-1 KH08

**Compound KH08-1:** crude compound **KH07** (200 mg, 0.452 mmol) was added into a single-necked flask, dissolved in acetic acid (7.5 mL), and then concentrated hydrochloric acid (2.5 mL) was added dropwise to the reaction. After a reaction for 4 hours at 90°C, TLC monitoring showed that the raw materials were reacted completely, a new increased polarity spot was formed, and the reaction was stopped. The reaction solution was directly dried by rotary evaporation and the reaction pH was adjusted to 9-10 with a saturated sodium carbonate solution, then the reaction solution was extracted twice with ethyl acetate (20 mL × 2). Aqueous phases were collected and the pH of the aqueous phases was adjusted to 3-4, then the aqueous phases were extracted with ethyl acetate (20 mL × 3), organic phases were collected, dried with anhydrous sodium sulfate, and filtered. The, organic phases were concentrated to obtain yellow solids **KH08-1** (186.5 mg, 89.4%), which were directly used in the next step without purification.

**Compound KH08:** compound **KH08-1** (150 mg, 0.325 mmol, 1 eq) and sodium hydroxide (52 mg, 1.3 mmol, 4 eq) were added into in a single-necked flask, and dissolved with water (20 mL), then thioglycolic acid (0.6 g, 6.5 mmol, 20 eq) was added to the reaction solution, and reacted at 120°C for 3 hours. After TLC monitoring showed that the raw materials were completely reacted, a reduced polarity spot was formed, and the reaction was stopped. Saturated sodium carbonate solution was added to adjust the pH of the reaction system to neutral, then the reaction system was extracted with ethyl acetate (20 mL × 3), dried with anhydrous sodium sulfate, filtered, dried by rotary evaporation and purified by preparative HPLC to obtain white solids **KH08** (36.8 mg, 27.1%). MS (ESI) m/z: 418.2 $[M+H]^+$.

**Example 10 Synthesis of key intermediate KH09-6**

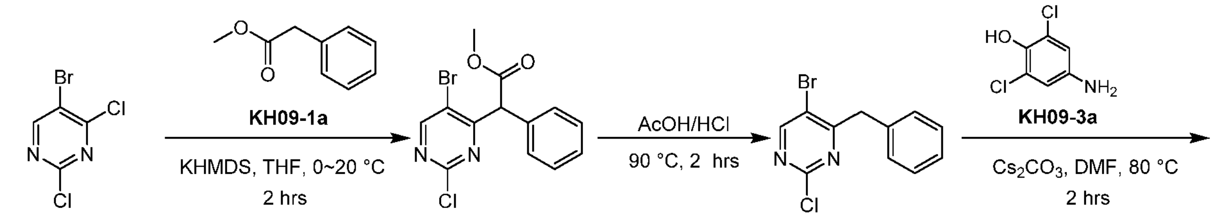


KH09-1 KH09-2 KH09-3

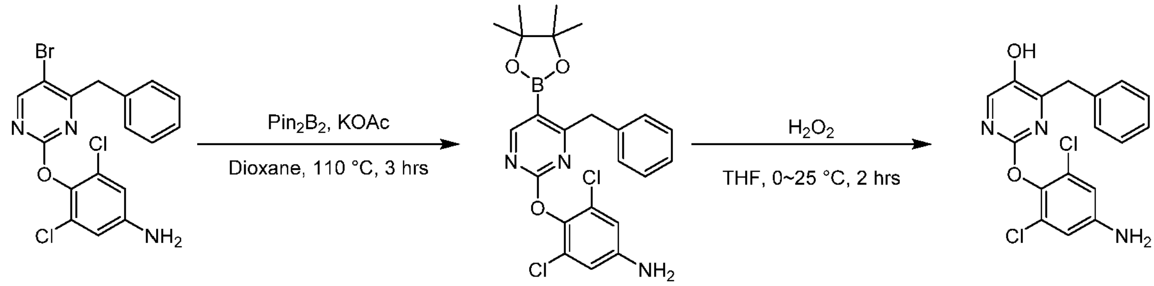


KH09-4 KH09-5 KH09-6

**Compound KH09-2:** raw **KH09-1** (50.0 g, 219 mmol, 28.0 mL, 1.00 eq) and **KH09-1a** (32.9 g, 219 mmol, 30.8 mL, 1.00 eq) were dissolved in tetrahydrofuran (200 mL), dropwise added with KHMDS (1.00M, 230 mL, 1.05 eq) at 0°C, after the addition, stirred at this temperature for 10 minutes, and then stirred at 25°C for 2 hours. After TLC (petroleum ether/ethyl acetate = 10: 1) monitoring showed that the raw materials were completely reacted, the reaction solution was poured slowly into ice water and extracted with dichloromethane (500 mL × 3), organic phases were combined, washed with saturated salt solution (300 mL), dried with anhydrous sodium sulfate, and concentrated to obtain yellow oily substance KH09-2 (75.0 g, crude product) which were directly used in the next step without purification. **$^1$H NMR** (DMSO-$d_6$, 400 MHz): δ 8.97 (s, 1H), 7.37 - 7.28 (m, 5H), 7.26 - 7.20 (m, 1H), 5.64 (s, 1H), 3.68 - 3.64 (m, 3H).

**Compound KH09-3: KH09-2** (75.0 g, 219 mmol, 1.00 *eq)* was dissolved in acetic acid solution (100 mL) of hydrogen chloride, and stirred at an external temperature of 90°C for 2 hours. After LCMS monitoring showed that the raw materials were completely reacted, the reaction solution was directly dried by rotary evaporation and purified through silica gel column chromatography ($SiO_2$, **petroleum ether/ ethyl acetate =100/1 to 20/1)** to obtain off-white solid **KH09-3** (23.0 g, 81.1 mmol, yield: 36.9%). MS (ESI) *m/z*: 282.9 $[M+H]^+$. **$^1$H NMR** (DMSO-$d_6$, 400 MHz): δ 8.93 (s, 1H), 7.37 - 7.28 (m, 2H), 7.28 - 7.21 (m, 3H), 4.23 (s, 2H).

**Compound KH09-4:** compound **KH09-3** (10.0 g, 35.2 mmol, 1.00 eq) and compound **KH09-3a** (6.28 g, 35.2 mmol, 1.00 eq) were dissolved in100 mL of DMF, then added with cesium carbonate (34.4 g, 105 mmol, 3.00 eq), and stirred at an external temperature of 80°C for 2 hours. TLC (petroleum ether/ethyl acetate = 100: 1) monitoring showed that the raw materials were completely reacted. The reaction solution was filtered, and the filter residues were washed with ethyl acetate (20 mL). The filtrate was collected, added with 50 mL of water, and stirred for 5 minutes, then, organic phases were separated, and aqueous phases were extracted with ethyl acetate (20 mL × 2). The organic phases were combined, washed with saturated salt solution (100 mL × 2), dried with anhydrous sodium sulfate, concentrated and purified through silica gel column chromatography ($SiO_2$, petroleum ether/ethyl acetate = 100/1 to 20/1) to obtain white solids **KH09-4** (12.0 g, 28.2 mmol, yield: 80.0%). **$^1$H NMR(**DMSO-$d_6$, 400 MHz): δ 8.75 (s, 1H), 7.41 - 7.12 (m, 5H), 6.68 (s, 2H), 5.64 (s, 2H), 4.14 (s, 2H).

**Compound KH09-5:** compound KH09-4 (5.00 g, 11.7 mmol, 1.00 eq), $Pin_2B_2$ (5.97 g, 23.5 mmol, 2.00 eq), Pd(dppf)$Cl_2$.$CH_2Cl_2$ (480 mg, 588. μmol, 0.05 eq) and potassium acetate (2.31 g, 23.5 mmol, 2.00 eq) were added into 50 mL of dioxane, the air in the reaction solution was replaced with nitrogen thrice, then the reaction solution was stirred at an external temperature of 110°C for 4 hours under the protection of nitrogen. After TLC monitoring showed that the raw materials were reacted completely, the reaction solution was filtered, and the filtrate was collected, dried by rotary evaporation directly and purified through silica gel column chromatography ($SiO_2$, **petroleum ether/ethyl acetate =100: 1 to 50: 1)** to obtain brown oily substance (5.00 g, crude product, boronic acid product present).

**Compound KH09-6:** compound KH09-5 (5.00 g, 10.5 mmol, 1.00 eq) was dissolved with tetrahydrofuran, added with $H_2O_2$ (2.46 g, 21.7 mmol, 2.08 mL, purity 30%, 2.05 eq) at 0°C and stirred for 30 minutes, then continuously added with $H_2O_2$ (4.80 g, 42.3 mmol, 4.07 mL, purity 30%, 4.00 eq), and stirred at room temperature for 2 hours. After TLC monitoring showed that the raw materials were reacted completely, the reaction solution was poured into 50 mL of saturated sodium sulfite solution and stirred for 15 minutes; and extracted with dichloromethane (200 mL × 3), organic phases were

combined, dried with anhydrous sodium sulfate, concentrated, and purified through silica gel column chromatography ($SiO_2$, petroleum ether/ethyl acetate = 100: 1 to 10: 1) to obtain faint yellow solids KH09-6 (1.01 g, 2.74 mmol, yield: 25.8%). MS (ESI) *m/z*: 362.0 $[M+H]^+$. **$^1$H NMR** (DMSO-$d_6$, 400 MHz): δ 10.03 (s, 1H), 8.01 (s, 1H), 7.38 - 7.09 (m, 5H), 6.66 (s, 2H), 5.54 (s, 2H), 3.97 (s, 2H), 1.98 (s, 1H).

**Example 11 Synthesis of compound KH09**

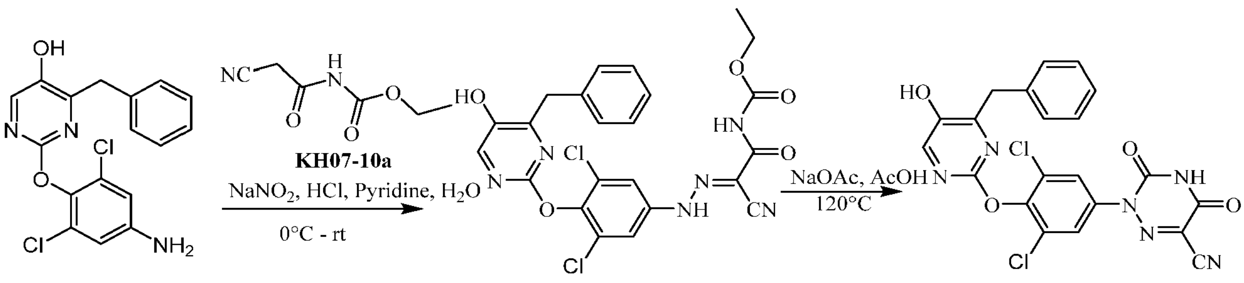


KH09-6 KH09-7 KH09

**Compound KH09-7:** the operation was the same as the synthesis of **KH07-11,** and tangerine solids **KH09-7** (323 mg, crude product) were obtained, which were directly used in the next step without purification.

**Compound KH09:** the operation was the same as the synthesis of **KH07,** and tangerine solids **KH09** (432 mg, crude product) were obtained. 50 mg of the solids were taken and purified by preparative HPLC to obtain 6.12 mg of white solids, yield: 7.65%. MS (ESI) m/z: 482.9 $[M+H]^+$.**$^1$H NMR(**DMSO-$d_6$, 400 MHz): δ 13.05 -13.10 (m, 1H), 10.26 (s, 1H), 8.07 (s, 1H), 7.75 (s, 2H), 7.18 - 7.30(m, 5H), 4.00 (s, 1H).

**Example 12 Synthesis of compound KH10**

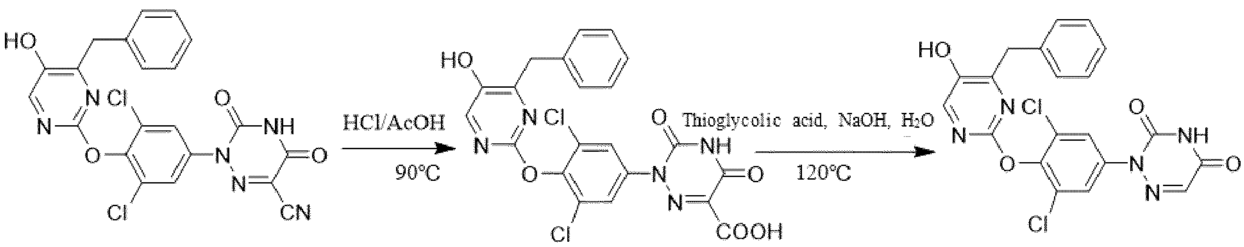


KH09 KH10-1 KH10

**Compound KH10-1:** the operation was the same as the synthesis of **KH08-1,** and yellow solids (255.7 mg, crude product) were obtained, which were directly reacted in the next step without purification. MS (ESI) m/z: 502.0 $[M+1]^+$.

**Compound KH10:** the operation was the same as the synthesis of KH08, and white solids **KH10** (51 mg, yield: 20.0%) were obtained. MS (ESI) m/z: 458.0 $[M+1]^+$. **$^1$H NMR(**DMSO-$d_6$, 400 MHz): 12.49(s, 1H), 10.25(s, 1H), 8.01(s, 1H), 7.77(s, 2H), 7.72(d, 1H, J=4.0Hz), 7.17-7.30(m, 5H), 4.0(s, 2H).

**Example 13 Synthesis of key intermediate KH11-3**

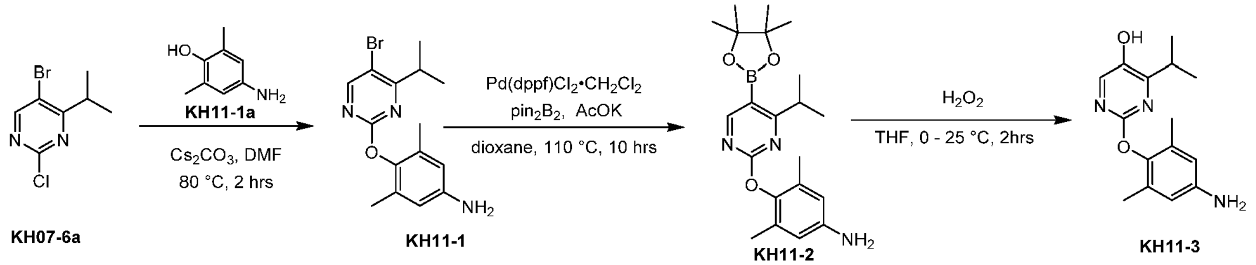


KH07-6a KH11-1 KH11-2 KH11-3

**Compound KH11-1:** the operation was the same as the synthesis of **KH09-4,** and tangerine solids **KH11-1** (9.00 g, 26.7 mmol, yield: 73.4%) were obtained by purification through silica gel column chromatography ($SiO_2$, petroleum ether/ethyl acetate = 1/0 to 1/2). MS (ESI) *m/z*: 338.1 $[M+H]^+$.**$^1$H NMR** (DMSO-$d_6$, 400 MHz): δ 8.42 (s, 1H), 6.43 (s, 2H), 3.52 (br s, 2H), 3.46 - 3.33 (m, 1H), 2.04 (s, 6H), 1.25 (d, *J* = 6.8 Hz, 6H).

**Compound KH11-2:** compound **KH11-1** (5.00 g, 14.8 mmol, 1.00 eq), pin$_2$B$_2$ (7.55 g, 29.7 mmol, 2.00 eq), Pd(dppf)$Cl_2$•$CH_2Cl_2$ (607 mg, 743 μmol, 0.05 eq) and potassium acetate (2.92 g, 29.7 mmol, 2.00 eq) were added into 50 mL of dioxane, the air in the reaction solution was replaced with nitrogen thrice, then the reaction solution was stirred at an external temperature of 110°C for 10 hours under the protection of nitrogen. After TLC monitoring showed that the raw materials were reacted completely, the reaction solution was dried by rotary evaporation directly, added with 50 mL of water and 50 mL of ethyl acetate, and stirred for 10 minutes, organic phases were separated, and aqueous phases were extracted with ethyl acetate (50 mL × 2). The, organic phases were combined, dried with anhydrous sodium sulfate, filtered, and concentrated to obtain black oily substance **KH11-2** (6.00 g, crude product) which was directly used in the next step without purification. MS (ESI) *m/z*: 384.5 $[M+H]^+$.

**Compound KH11-3:** the operation was the same as the synthesis of compound KH09-6, and off-white solids (1.11 g, 2.79 mmol, yield: 17.8%, TFA salt) were obtained by purification through preparative HPLC (0.1% TFA). MS (ESI) *m/z*: 274.2 $[M+H]^+$.**$^1$H NMR** (MeOD, 400 MHz): δ 7.93 (s, 1H), 7.12 (s, 2H), 3.40 - 3.33 (m, 1H), 2.13 (s, 6H), 1.11 (d, *J* = 6.8 Hz, 6H).

**Example 14 Synthesis of compound KH11**

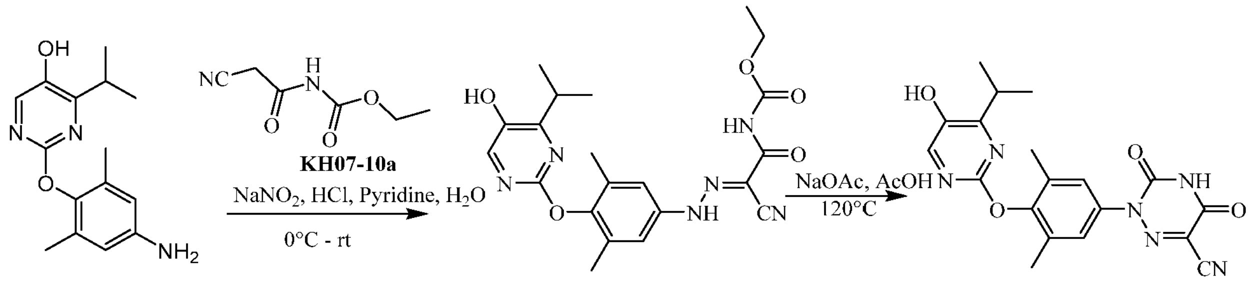


KH11-3 KH11-4 KH11

**Compound KH11-4:** the operation was the same as the synthesis of **KH07-11,** and tangerine solids **KH11-4** (368.4 mg, crude product) were obtained, which were directly used in the next step without purification. MS (ESI) m/z: 441.3$[M+H]^+$.

**Compound KH11:** the operation was the same as the synthesis of **KH07,** and tangerine solids **KH11** (342.3 mg, crude product) were obtained. 50 mg of the solids were taken and purified by preparative HPLC to obtain 11.2 mg of white solids, yield: 23.2%. MS (ESI) m/z: 395.2 $[M+H]^+$.

**Example 15 Synthesis of compound KH12**

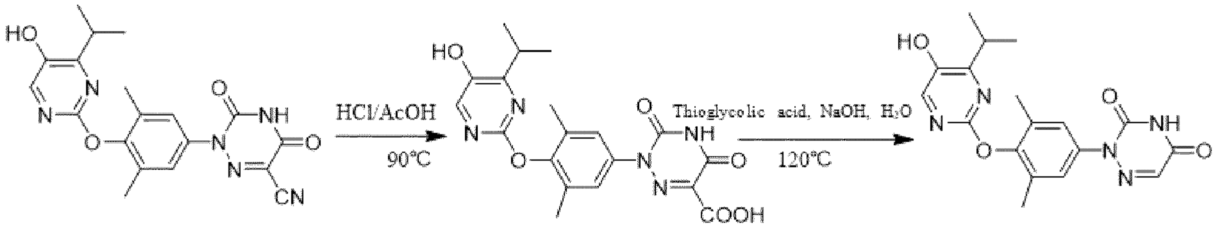


KH11 KH12-1 KH12

**Compound KH12-1:** the operation was the same as the synthesis of **KH08-1,** and yellow solids (286 mg, crude product) were obtained, which were directly used in the next step without purification.MS (ESI) m/z: 414.2 [M+H]+.

**Compound KH10:** the operation was the same as the synthesis of KH08, and white solids **KH10** (38.4 mg, yield: 15.2%) were obtained. MS (ESI) m/z: 370.1 [M+H]+.

**Embodiment 16 Synthesis of key intermediate KH13-7**

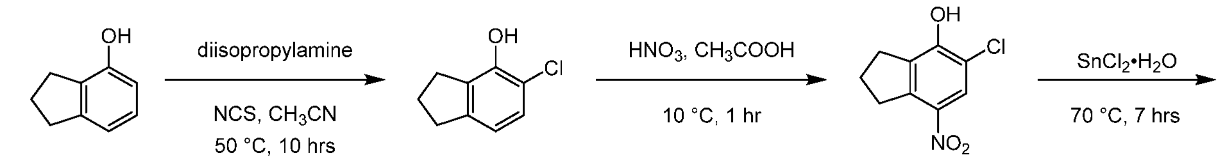


KH13-1 KH13-2 KH13-3

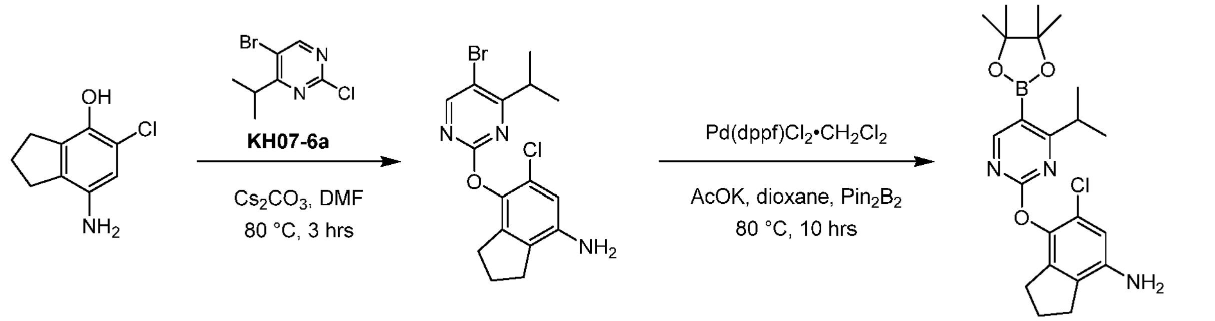


KH13-4 KH13-5 KH13-6

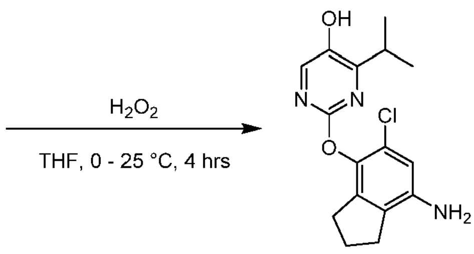


KH13-7

**Compound KH13-2:** N,N-diisopropylamine (1.89 g, 18.6 mmol, 2.63 mL, 0.1 eq) and raw **KH13-1** (25.0 g, 186 mmol, 1 eq) were dissolved in acetonitrile (250 mL), and then NCS (26.1 g, 195 mmol, 1.05 eq) was added into the mixture. The mixture was stirred at 50°C for 10 hours. TLC (petroleum ether/ethyl acetate = 10/1) monitoring showed that the reaction was complete. The reaction solution was dried by rotary evaporation and diluted with 100 mL of water, and extracted with ethyl acetate (100 mL × 3), organic phases were combined, dried with anhydrous sodium sulfate, filtered, dried by rotary evaporation and purified through silica gel column chromatography ($SiO_2$, petroleum ether/ethyl acetate =1/0 to 20/1) to obtain yellow solids **KH13-2** (5.00 g, 29.6 mmol, yield 15.9%). **$^1$H NMR**(DMSO-$d_6$, 400 MHz): δ 9.24 (s, 1H), 7.07 (d, *J* = 8.0 Hz, 1H), 6.69 (d, *J* = 8.0 Hz, 1H), 2.85 - 2.77 (m, 4H), 2.05 - 1.96 (m, 2H).

**Compound KH13-3:** compound **KH13-2** (3.70 g, 21.9 mmol, 1 eq) was dissolved in ethanol (37 mL), added with

nitric acid (2.42 g, 23.0 mmol, 1.73 mL, purity 60.0%, 1.05 eq) at 10°C, and continuously stirred at this temperature for 1 hour. TLC monitoring showed that the raw materials completely disappeared. The mixture was diluted with 100 mL of water, and then extracted with ethyl acetate (50 mL × 3), organic phases were collected, washed with saturated salt solution (100 mL), dried with anhydrous sodium sulfate, filtered, dried by rotary evaporation, and purified through silica gel column chromatography ($SiO_2$, petroleum ether/ethyl acetate = 1/0 to 10/1) to obtain yellow solids **KH13-3** (3.5.0 g, 16.3 mmol, yield: 74.6%). **$^1$H NMR(**DMSO-*d*$_6$, 400 MHz): δ 10.96 (br s, 1H), 7.98 (s, 1H), 3.25 (t, *J* = 7.6 Hz, 2H), 2.89 (t, *J* = 7.6 Hz, 2H), 2.50 (td, *J* = 1.6, 3.5 Hz, 1H), 2.06 (quin, *J* = 7.6 Hz, 2H).

**Compound KH13-4:** compound **KH13-3** (3.50 g, 16.3 mmol, 1 eq) and $SnCl_2$•$2H_2O$ (18.4 g, 81.9 mmol, 5 eq) were dissolved in methanol (10 mL) and stirred at 70°C for 7 hours. After LCMS monitoring showed that the raw materials were completely disappeared, the reaction solvent was directly dried by rotary evaporation, the residues were dissolved by ethyl acetate, and then saturated sodium bicarbonate solution was added, then solids were obtained. The reaction solution was filtered to remove the solids, and the filtrate was collected, organic phases were separated, washed with saturated salt solution (100 mL), dried with anhydrous sodium sulfate, and then filtered and dried by rotary evaporation. The residues were directly used in the next step without purification to obtain yellow solids **KH13-4** (2.70 g, 14.7 mmol, yield: 89.7%). **$^1$H NMR** (DMSO-*d*$_6$, 400 MHz): δ 8.12 (s, 1H), 6.39 (s, 1H), 4.48 (s, 2H), 2.75 (t, *J* = 7.6 Hz, 2H), 2.59 (t, *J* = 7.6 Hz, 2H), 2.04 - 1.89 (m, 2H).

**Compound KH13-5:** compound **KH13-4** (2.50 g, 13.6 mmol, 1 eq), compound **KH07-6a** (3.21 g, 13.6 mmol, 1 eq) and cesium carbonate (13.3 g, 40.8 mmol, 3 eq) were added into DMF (30 mL), and stirred at 80°C for 3 hours. TLC (petroleum ether/ethyl acetate = 3/1) monitoring showed that the raw materials completely disappeared. After the reaction solution was diluted with 100 mL of water, the reaction solution was extracted with ethyl acetate (50 mL × 3), and then, organic phases were combined, and washed with saturated salt solution, dried with anhydrous sodium sulfate, filtered, dried by rotary evaporation and purified through silica gel column chromatography ($SiO_2$, petroleum ether/ethyl acetate = 1/0 to 3/1) to obtain yellow solids **KH13-5** (3.70 g, 9.57 mmol, yield: 70.3%, purity: 99.0%). MS (ESI) m/z: 383.8 [M+H]$^+$. **$^1$H NMR(**DMSO-*d*$_6$, 400 MHz): δ 8.54 - 8.35 (m, 1H), 6.70 - 6.51 (m, 1H), 3.59 (s, 2H), 3.41 (spt, *J* = 6.8 Hz, 1H), 2.76 (td, *J* = 7.6, 17.5 Hz, 4H), 2.19 - 2.06 (m, 2H), 1.25 (d, *J* = 6.8 Hz, 6H).

**Compound KH13-6:** the operation was the same as the synthesis of compound **KH11-2,** and brown solids **KH13-6** (4.30 g, crude product) were obtained. MS (ESI) m/z: 430.3 [M+H]$^+$.

**Compound KH13-7:** the operation was the same as the synthesis of compound **KH11-3,** and faint yellow solids **KH13-7** (1.20 g, 3.72 mmol, yield: 38.0%) was obtained by purification through silica gel column chromatography ($SiO_2$, petroleum ether/ethyl acetate = 1/0 to 1/1). MS (ESI) m/z: 320.0 [M+H]$^+$. **$^1$H NMR** (DMSO-*d*$_6$, 400 MHz): δ 9.74 (s, 1H), 7.92 (s, 1H), 6.50 (s, 1H), 5.22 - 4.80 (m, 2H), 3.27 (spt, *J* = 6.8 Hz, 1H), 2.64 (br t, *J* = 7.6 Hz, 2H), 2.59 - 2.51 (m, 2H), 1.95 (br dd, *J* = 7.6, 15.1 Hz, 2H), 1.11 (d, *J* = 6.8 Hz, 6H).

**Example 17 Synthesis of compound KH13**

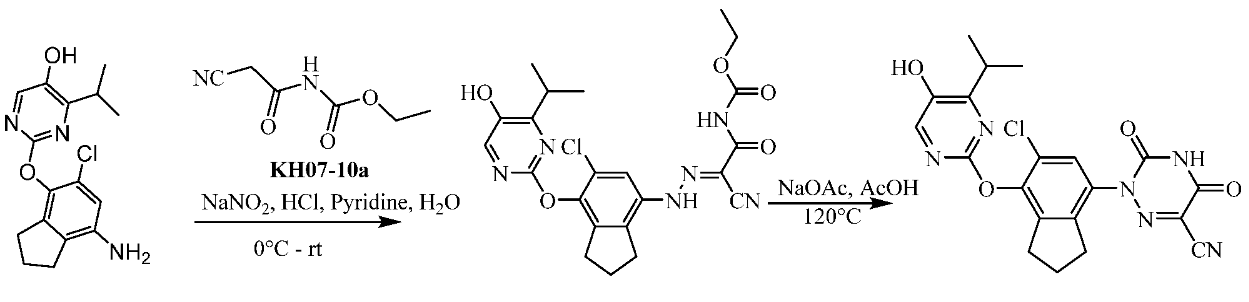


**KH13-7** **KH13-8** **KH13**

**Compound KH13-8:** the operation was the same as the synthesis of **KH07-11,** and tangerine solids (431.3 mg, crude product) were obtained, which were directly reacted in the next step without purification. MS (ESI) m/z: 487.2[M+H]$^+$.

**Compound KH13:** the operation was the same as the synthesis of **KH07,** and tangerine solids **KH13** (354.2 mg, crude product) were obtained. 120 mg of the solids were taken and purified by preparative HPLC to obtain 43 mg of white solids. MS (ESI) m/z: 441.2 [M+H]$^+$. **$^1$H NMR** (DMSO-*d*$_6$, 400 MHz): 13.07 (s, 1H), 10.06 (s, 1H), 7.98 (s, 1H), 7.38 (s, 1H), 3.25 - 3.34 (m, 1H), 2.84 (t, 2H, J = 8.0Hz), 2.68 (t, 2H, J = 8.0Hz), 2.01 (t, 2H, J = 8.0Hz), 1.15 (d, 6H, J = 4.0Hz).

### Example 18 Synthesis of compound KH14

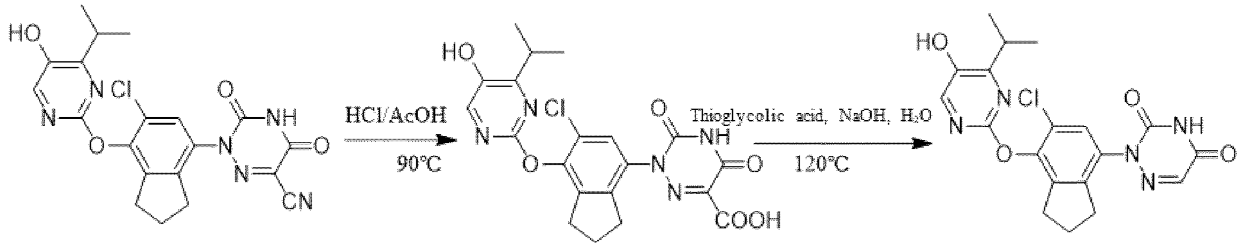


KH13 KH14-1 KH14

**Compound KH14-1:** the operation was the same as the synthesis of **KH08-1,** and yellow solids (251.4 mg, crude product) were obtained, which were directly reacted in the next step without purification. MS (ESI) m/z: 460.1 $[M+H]^+$.

**Compound KH14:** the operation was the same as the synthesis of **KH08,** and white solids **KH14** (6 mg, yield: 11.1%) were obtained. MS (ESI) m/z: 416.2 $[M+H]^+$. **$^1$H NMR** (DMSO-$d_6$, 400 MHz): 12.40 (s, 1H), 9.98 (s, 1H), 7.99 (s, 1H), 7.63 (s,1H), 7.46 (s, 1H), 3.27 - 3.30 (m, 1H), 2.83 (t, 2H, J=8.0Hz), 2.68 (t, 2H, J=8.0Hz), 2.0 (t, 2H, J=8.0Hz), 1.15 (d, 6H, J=4.0Hz).

### Example 19 Synthesis of key intermediate KH15-8

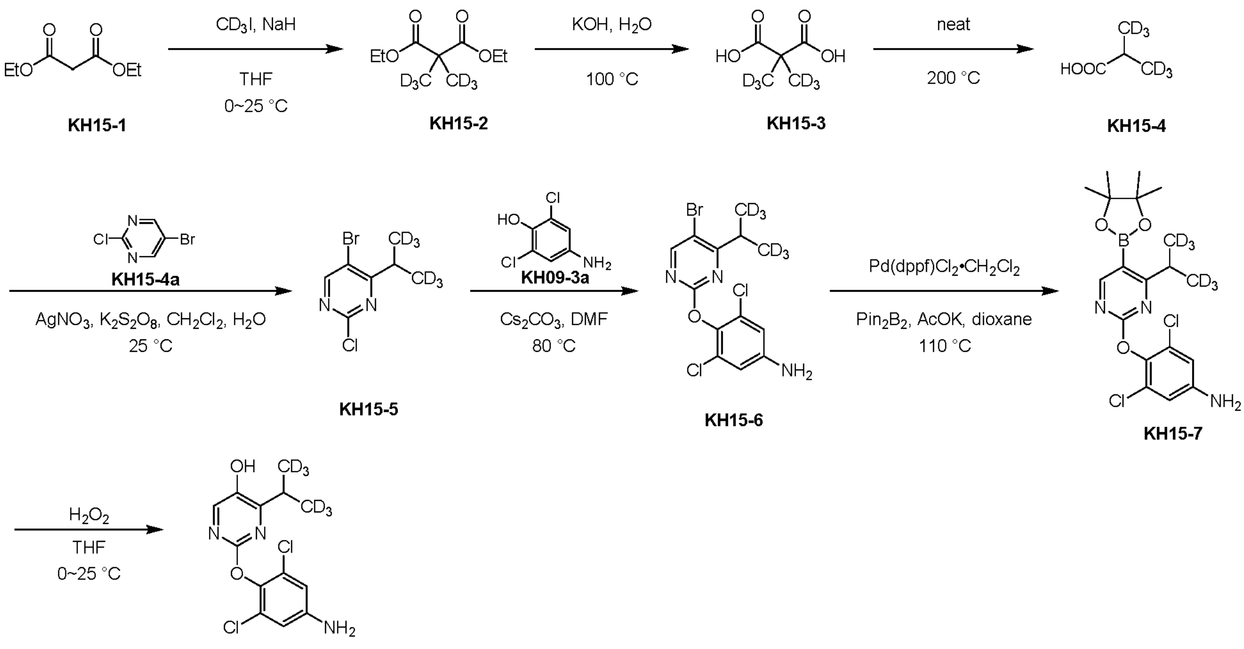


KH15-1 KH15-2 KH15-3 KH15-4 KH15-5 KH15-6 KH15-7 KH15-8

**Compound KH15-2:** sodium hydride (7.49 g, 187 mmol, purity 60.0%, 3.00 eq) was dissolved in tetrahydrofuran (100 mL), and **KH15-1** (10.0 g, 62.4 mmol, 9.43 ml, 1.00 eq) and $CD_3I$ (19.0 g) were added in the above reaction system at 0°C. The mixture was continuously stirred at 0°C for 0.5 hour, and then slowly heated to 25°C and stirred for 11.5 hours. TLC monitoring showed that the raw materials were reacted completely. The reaction solution was poured into 200 mL of ice water, and extracted with ethyl acetate (100 mL × 3), organic phases were collected, dried with anhydrous sodium sulfate, filtered, and dried by rotary evaporation to obtain yellow oily substance **KH15-2** (8.35 g, 42.9 mmol, yield: 68.8%). The solids were directly reacted in the next step without purification.

**Compound KH15-3:** compound **KH15-2** (8.95 g, 46.0 mmol, 1.00 eq) was dissolved in water (20 mL), and sodium hydroxide (6.27 g, 111 mmol, 2.42 eq) was added into the above-mentioned reaction solution. After the addition, a reaction was carried out for 3 hours at 100°C. TLC monitoring showed that a new spot was formed. The pH was adjusted to

2 with 6N hydrochloric acid at 0°C, and then the reaction solution was extracted with dichloromethane (100 mL × 2), organic phases were combined, washed with saturated salt solution (100 mL), dried with anhydrous sodium sulfate, filtered and dried by rotary evaporation. and directly used in the next step without purification to obtain colorless oil **KH15-3** (5.02 g, crude product).

**Compound KH15-4:** compound **KH15-3** (5.00 g, 36.1 mmol, 1.00 eq) was heated to 200°C and stirred for 0.5 hour. After TLC monitoring showed that a new compound was formed, a crude product was distilled (140°C, 1 atm) to obtain yellow oil **KH15-4** (2.52 g, 26.7 mmol, yield: 73.9%).

**Compound KH15-5:** compound **KH15-4** (2.50 g, 28.3 mmol, 1.00 eq), compound **KH15-4a** (5.49 g, 28.3 mmol, 1.00 eq), silver nitrate (1.64 g, 9.65 mmol, 0.34 eq), and $K_2S_2O_8$ (11.5 g, 42.5 mmol, 8.52 mL, 1.50 eq) were added into dichloromethane (50 mL) and water (50 mL), and stirred at an external temperature of 25°C for 12 hours under the protection of nitrogen. TLC (petroleum ether/ethyl acetate = 20/1) monitoring showed that a new spot was formed. The reaction solution was quenched with sodium sulfite solution and extracted with dichloromethane (100 mL × 2), organic phases were combined, washed with saturated salt solution (50 mL), dried with anhydrous sodium sulfate, filtered, dried by rotary evaporation and purified through silica gel column chromatography ($SiO_2$, petroleum ether/ethyl acetate = 1/0 to 10/1) to obtain a crude product, then the crude product is purified by preparative HPLC to obtain yellow oily substance **KH15-5** (2.60 g, 10.7 mmol, yield: 37.9%). MS (ESI) m/z: 243.1 $[M+H]^+$.

**Compound KH15-6:** compound **KH15-5** (2.60 g, 10.7 mmol, 1.00 eq), compound **KH09-3a** (1.92 g, 10.7 mmol, 1.00 eq) and cesium carbonate (10.5 g, 32.29 mmol, 3.00 eq) were added into DMF (26 mL), and stirred at an external temperature of 80°C for 10 hours under the protection of nitrogen. LCMS monitoring showed that the raw materials were already completely reacted. Ethyl acetate (100 mL) and water (100 mL) were added into the reaction solution for extraction and separation, organic phases were collected, washed with saturated salt solution (100 mL), dried with anhydrous sodium sulfate, filtered, dried by rotary evaporation and purified through silica gel column chromatography ($SiO_2$, petroleum ether/ethyl acetate 1/0 to 5/1) to obtain yellow oily substance **KH15-6** (2.7 0 g, 7.05 mmol, yield: 65.4%). MS (ESI) m/z: 384.1 $[M+H]^+$.

**Compound KH15-7:** the operation was the same as that of compound **KH11-2,** and the residue was purified through silica gel column chromatography ($SiO_2$, petroleum ether/ethyl acetate = 1/0 to 5/1) to obtain white solids **KH15-7** (3.00 g, 6.97 mmol, yield: 98.9%). MS (ESI) m/z = 429.9 $[M+H]^+$.

**Compound KH15-8:** compound **KH15-7** (3.00 g, 6.97 mmol, 1.00 eq) was dissolved in tetrahydrofuran (30 mL), and hydrogen peroxide (1.66 g, 14.6 mmol, 1.41 mL, purity 30.0%, 2.10 eq) was added into the reaction at 0°C, and after the addition, the mixture was stirred at room temperature for 8 hours. LCMS monitoring showed that the raw materials were completely reacted, and a new product MS was found. The reaction was terminated with saturated sodium sulfite solution (50 mL), extracted with dichloromethane (50 mL × 3), organic phases were combined, dried with anhydrous sodium sulfate, filtered, dried by rotary evaporation and purified through silica gel column chromatography ($SiO_2$, petroleum ether/ethyl acetate = 10/1 to 1/1) to obtain faint yellow solids **KH15-8** (1.20 g, 3.73 mmol, yield: 53.5%). MS (ESI) m/z: 320.1 $[M+H]^+$. **$^1$H NMR** (DMSO-$d_6$, 400 MHz): δ 9.91 - 9.78 (m, 1H), 8.01 - 7.90 (m, 1H), 6.70 - 6.56 (m, 2H), 5.56 - 5.42 (m, 2H), 3.25 - 3.19 (m, 1H).

**Example 20 Synthesis of compound KH15**

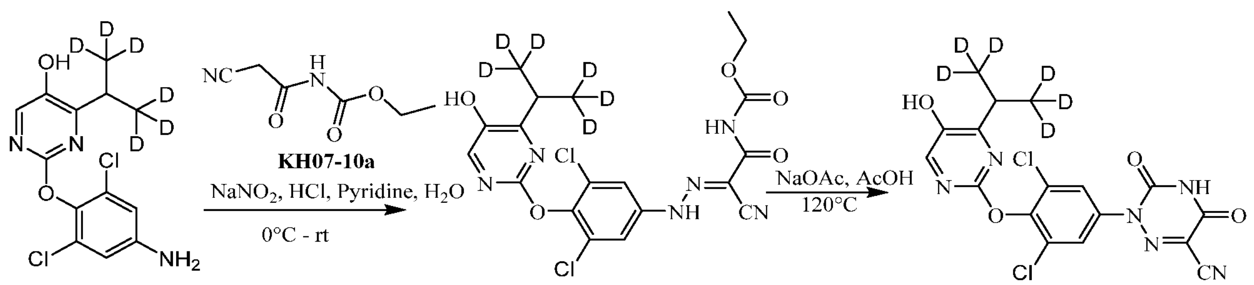


KH15-8 KH15-9 KH15

**Compound KH15-9:** the operation was the same as the synthesis of **KH07-11,** and tangerine solids (430 mg, crude product) were obtained, which were directly reacted in the next step without purification. MS (ESI) m/z: 487.2$[M+H]^+$.

**Compound KH15:** the operation was the same as the synthesis of **KH07,** and tangerine solids **KH15** (320 mg, crude product) were obtained. 100 mg of the solids were taken and purified by preparative HPLC to obtain 6 mg of white solids. MS (ESI) m/z: 441.2 $[M+H]^+$.

**Example 21 Synthesis of compound KH16**

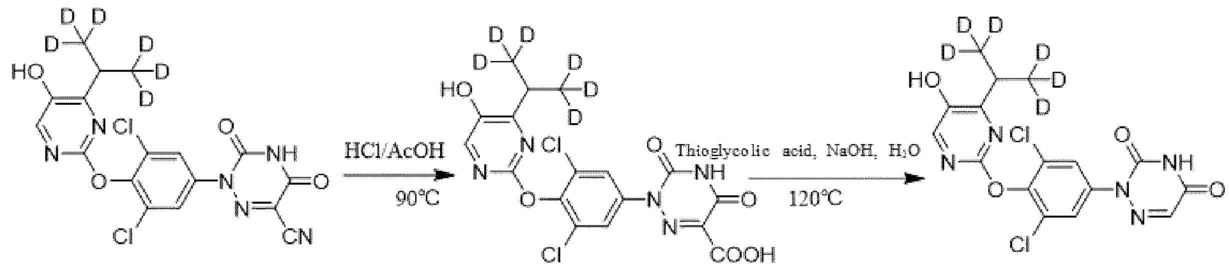


**KH15** **KH16-1** **KH16**

**Compound KH16-1:** the operation was the same as the synthesis of **KH08-1,** and yellow solids (200 mg, crude product) were obtained, which were directly reacted in the next step without purification. MS (ESI) m/z: 460.1 $[M+H]^+$.

**Compound KH16:** the operation was the same as the synthesis of **KH08,** and white solids **KH16** (11 mg, yield: 21.2%) were obtained. MS (ESI) m/z: 416.2 $[M+H]^+$.

**Example 22 Synthesis of key intermediate KH17-5**

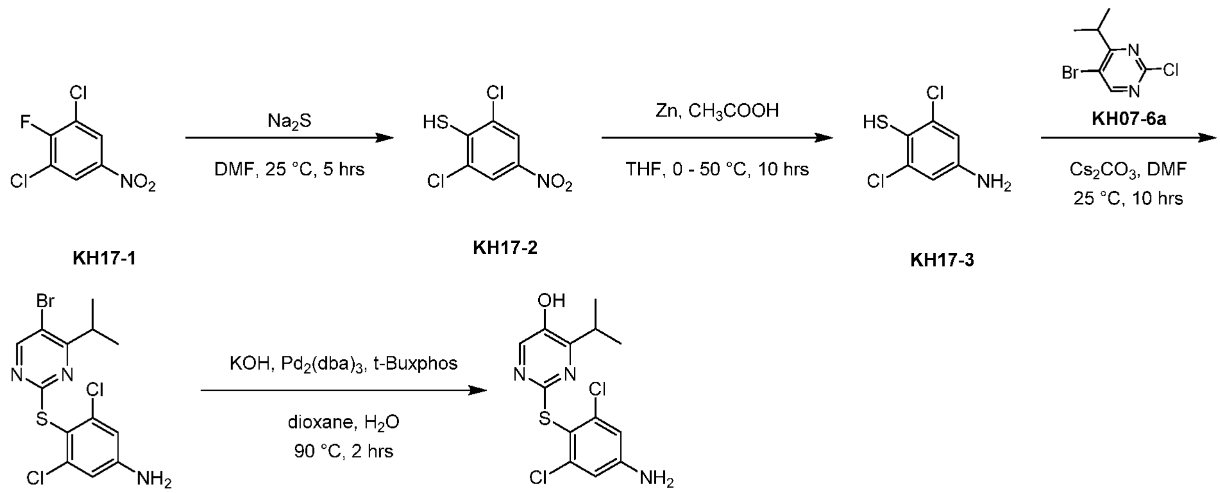


**KH17-1** **KH17-2** **KH17-3** **KH17-4** **KH17-5**

**Compound KH17-2:** compound **KH17-1** (50.0 g, 238 mmol, 1 eq) and sodium sulfide (27.8 g, 357 mmol, 14.9 mL, 1.5 eq) were added into DMF (500 mL), and stirred for 5 hours at 25°C. LCMS monitoring showed that compound **KH17-1** already disappeared. The reaction solution was poured into 500 mL of ice water, and adjusted pH to 5 with hydrochloric acid (2N). The reaction solution was extracted with ethyl acetate (200 mL × 2), organic phases were combined, washed with saturated salt solution (300 mL), dried with anhydrous sodium sulfate, filtered, and dried by rotary evaporation The residues were directly used in the next step without purification to obtain red solids **KH17-2** (43.0 g, crude product). MS (ESI) m/z: 224.1 $[M+H]^+$.

**Compound KH17-3:** compound **KH17-2** (40.0 g, 178 mmol, 1 eq) and Zn (58.3 g, 892 mmol, 5 eq) were added into tetrahydrofuran (1.5 L), dropwise added with acetic acid (21.4 g, 357 mmol, 20.4mL, 2 eq) at 0°C, and the mixture was stirred at 50°C for 10 hours. LCMS monitoring showed that the raw material completely disappeared. The reaction solution was directly filtered. The filtrate was collected and dried by rotary evaporation to obtain residue. The residues were pulped into solid powder with methyl tertbutyl ether to obtain white solid **KH17-3** (220 g, 108 mmol, yield: 60.8%, purity: 95.8%). MS (ESI) m/z: 194.0 $[M+H]^+$.

**Compound KH17-4:** compound **KH17-3** (16.0 g, 82.4 mmol, 1 eq), compound **KH07-6a** (16.1 g, 57.7 mmol, 0.7 eq) and cesium carbonate (40.2 g, 123 mmol, 1.5 eq) were added into DMF (200 mL), and stirred for 10 hours at 25°C. LCMS monitoring showed that the raw materials were already completely reacted. The reaction solution was added into

200 mL of water and extracted with ethyl acetate (200 mL × 2), organic phases were collected, washed with saturated salt solution (200 mL), dried with anhydrous sodium sulfate, filtered, and dried by rotary evaporation to obtain residues. The residue were pulped into solid powder with methyl tertbutyl ether to obtain yellow solid **KH17-4** (2.60 g, 10.7 mmol, yield: 37.9%). MS (ESI) m/z: 394.0 [M+H]+.

**Compound KH17-5:** after compound **KH17-4** (5.00 g, 12.7 mmol, 1 eq) was dissolved in water (25.0 mL) and dioxane (100 mL), potassium hydroxide (2.85 g, 50.8 mmol, 4 eq), $Pd_2(dba)_3$ (1.16 g, 1.27 mmol, 0.1 eq) and t-Buxphos (540 mg, 1.27 mmol, 0.1 eq) were added into the mixture and stirred at 90°C for 2 hours under the protection of nitrogen. After LCMS monitoring showed that the raw materials already completely disappeared, the reaction solution was adjusted pH to 5,with 1M diluted hydrochloric acid . then added with 100 mL of water, and extracted with ethyl acetate (100 mL × 2), organic phases were collected, washed with saturated salt solution (100 mL), dried with anhydrous sodium sulfate, filtered, dried by rotary evaporation, and purified through silica gel column chromatography ( $SiO_2$, petroleum ether/ethyl acetate = 1/0 to 3/1) to obtain off-white solids **KH17-5** (1.20 g, 3.48 mmol, yield: 27.3%). MS (ESI) m/z: 330.0 [M+H]+. **$^1$H NMR** (DMSO-$d_6$, 400 MHz): δ 9.99 (br s, 1H), 8.10 - 7.90 (m, 1H), 6.83 - 6.64 (m, 2H), 6.14 - 5.88 (m, 2H), 3.22 (spt, *J* = 6.8 Hz, 1H), 1.04 (d, *J* = 6.8 Hz, 6H).

**Example 23 Synthesis of compound KH17**

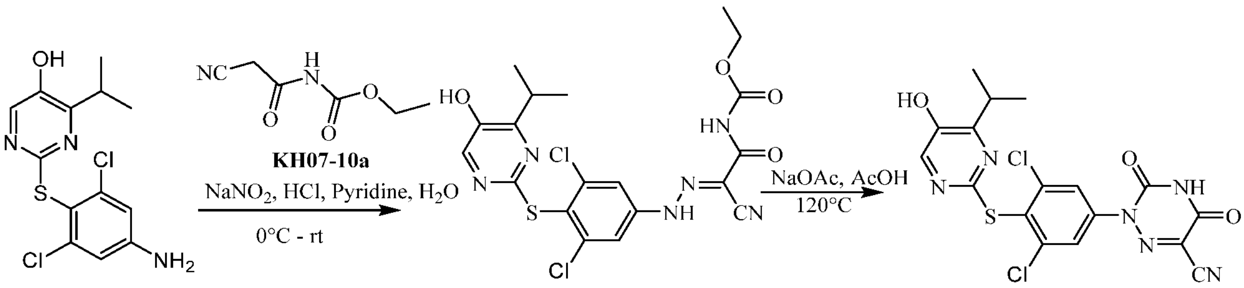


KH17-5 KH17-6 KH17

**Compound KH17-6:** the operation was the same as the synthesis of **KH07-11,** and tangerine solids (390 mg, crude product) were obtained, which were directly reacted in the next step without purification. MS (ESI) m/z: 497.1[M+H]+.

**Compound KH17:** the operation was the same as the synthesis of **KH07,** and tangerine solids **KH17** (140 mg, crude product) were obtained. 40 mg of the solids were taken and purified by preparative HPLC to obtain 5 mg of faint yellow solids, yield: about 12.5%. MS (ESI) m/z: 451.1 [M+H]+.

**Example 24 Synthesis of compound KH18**

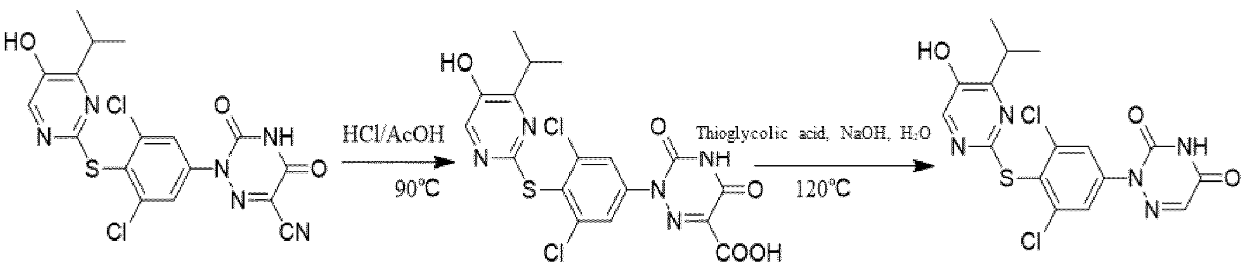


KH17 KH18-1 KH18

**Compound KH18-1:** the operation was the same as the synthesis of **KH08-1,** and brown solids (102.4 mg, crude product) were obtained, which were directly reacted in the next step without purification. MS (ESI) m/z: 470.1 [M+H]+.

**Compound KH18:** the operation was the same as the synthesis of **KH08,** and white solids **KH18** (18 mg, yield: 19.8%) were obtained. MS (ESI) m/z: 426.0 [M+H]+. **$^1$H NMR** (DMSO-$d_6$, 400 MHz): 12.50 (s, 1H), 10.21 (s, 1H), 8.05 (s, 1H), 7.94 (s, 2H), 7.73 (s, 1H), 3.20-3.27 (m, 1H), 1.04 (d, 6H, J=4.0Hz).

**Example 25 Synthesis of compound KHE001**

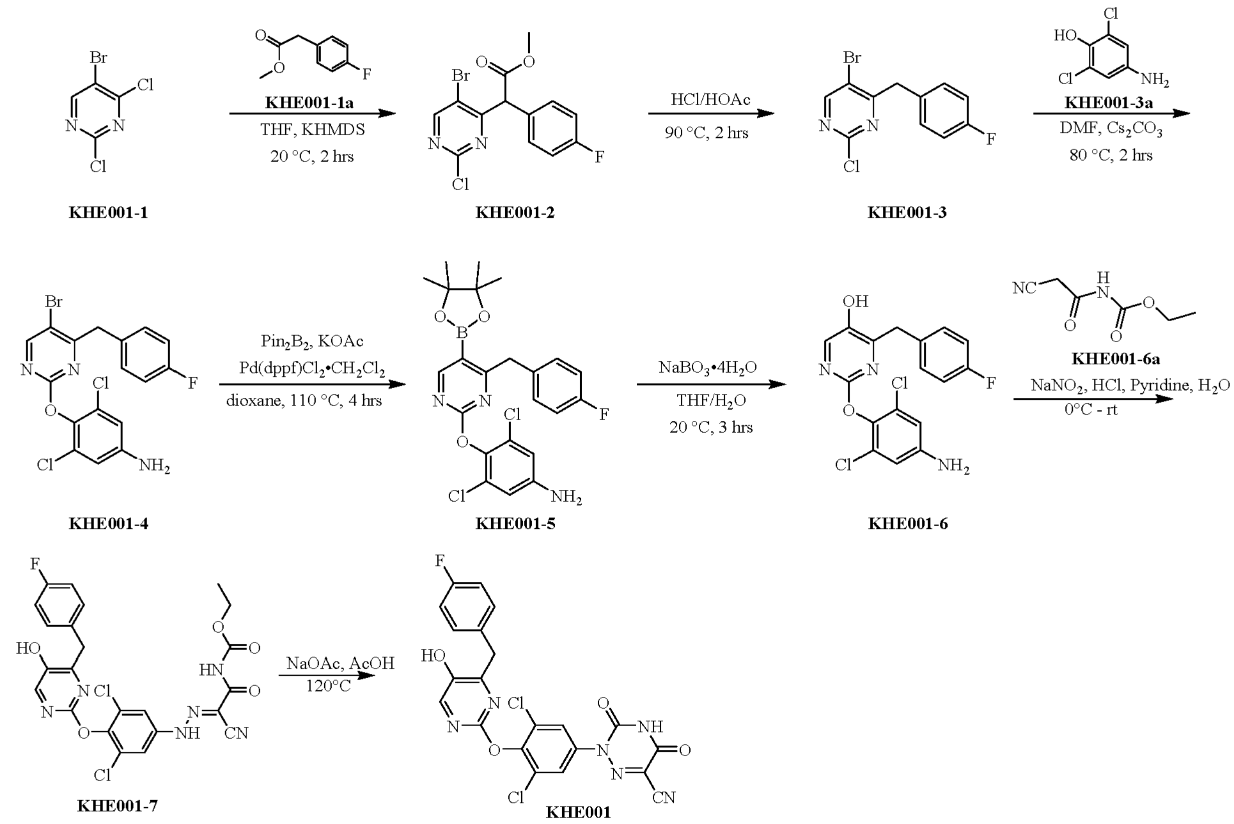


KHE001-1 KHE001-2 KHE001-3 KHE001-4 KHE001-5 KHE001-6 KHE001-7 KHE001

**Compound KHE001-2:** compounds **KHE001-1** (20.0 g, 87.8 mmol, 11.2 mL, 1.00 eq) and **KHE001-1a** (14.8 g, 87.8 mmol, 1.00 eq) were dissolved in toluene (8 mL), and slowly added dropwise with potassium bis(trimethylsilyl)amide (KHMDS) (1 M, 92.2 mL, 1.05 eq) at 0°C. After the mixture was stirred at 20°C for 2 hours, TLC (petroleum ether/ethyl acetate = 10/1) monitoring showed that the remaining amount of the raw materials was less than 5%, and a new increased polarity spot was formed. The reaction was quenched in ice water (500 mL), and then extracted with ethyl acetate (50 mL × 3). Organic phases were collected, washed with saturated salt solution (100 mL), dried with anhydrous sodium sulfate, filtered, concentrated, and purified through silica gel column chromatography ($SiO_2$, petroleum ether/ethyl acetate = 30/1 to 5/1) to obtain yellow oily substance **KHE001-2** (17.1 g, yield: 54.2%).

**Compound KHE001-3:** compound **KHE001-2** (17.0 g, 47.3 mmol, 1.00 eq) was dissolved in hydrochloric acid (20 mL, purity 36%) and acetic acid (80 mL), and stirred at 90°C for 2 hours. TLC (petroleum ether/ethyl acetate = 10/1) monitoring showed that the raw materials were completely reacted, and a new spot ($R_f$ = 0.60) was formed. The reaction mixture was directly dried by rotary evaporation under reduced pressure to obtain a crude product. The crude product was dissolved in ethyl acetate and washed with saturated sodium bicarbonate solution (50 mL × 2). Organic phases were collected, dried with anhydrous sodium sulfate, filtered, concentrated to remove the solvent, and purified by silica gel column chromatography ($SiO_2$, petroleum ether/ethyl acetate = 10/1) to obtain white solids **KHE001-3** (9.70 g, yield: 68.0%). **$^1$H NMR** (DMSO-*d6*, 400MHz): δ 8.62 (s, 1H), 7.31 (dd, *J* = 5.6, 8.8 Hz, 2H), 7.04 - 6.97 (m, 2H), 4.22 (s, 2H).

**Compound KHE001-4:** compounds **KHE001-3** (9.00 g, 29.9 mmol, 1.00 eq), **KHE001-3a** (6.38 g, 35.8 mmol, 1.20 eq) and cesium carbonate (29.2 g, 89.5 mmol, 3.00 eq) were dissolved in dimethylformamide (90 mL). The mixture was stirred for 2 hours at 80°C under the protection of nitrogen. LCMS monitoring showed that the raw material **KHE001-3** was completely reacted, and a target product signal is the main peak. Ethyl acetate (300 mL) and water (100 mL) were added to the reaction solution for extraction and separation. Organic phases were collected, washed with saturated salt solution, dried with anhydrous sodium sulfate, and concentrated to remove the solvent to obtain a crude product, which was purified by silica gel column chromatography ($SiO_2$, petroleum ether/ethyl acetate = 30/1 to 5/1) to obtain yellow solids **KHE001-4** (4.50 g, yield: 34.0%). MS (ESI) m/z: 443.8 $[M+H]^+$.

**Compound KHE001-5:** compound **KHE001-4** (4.50 g, 10.1 mmol, 1.0 eq), $Pin_2B_2$ (5.16 g, 20.3 mmol, 2.00 eq), $Pd(dppf)Cl_2{\bullet}CH_2Cl_2$ (415 mg, 508 μmol, 0.05 eq) and potassium acetate (1.99 g, 20.3 mmol, 2.00 eq) were dissolved in dioxane (45 mL). The reaction system was under the protection of nitrogen, and stirred for 4 hours at 110°C. LCMS

monitoring showed that the raw material **KHE001-4** was completely reacted, and a target product signal is the main peak. The reaction system was directly dried by rotary evaporation to obtain brown oily substance **KHE001-5** (14.0 g, mixture), which was directly used in the next step without purification. MS (ESI) m/z: 490.1 $[M+H]^+$.

**Compound KHE001-6:** compound **KHE001-5** (14.0 g, 28.6 mmol, 1.00 eq) was dissolved in tetrahydrofuran (70 mL) and water (70 mL), and sodium perborate ($NaBO_3 \cdot 4H_2O$) (13.2 g, 85.7 mmol, 3.00 eq) was added in the reaction. The mixture was stirred at 20°C for 3 hours, and TLC (petroleum ether/ethyl acetate = 2/1, $R_f$ = 0.15) monitoring showed that the reaction was already completed. The reaction was diluted with water (150 mL) and stirred at 25°C for 10 minutes. The reaction solution was filtered, and the filter cake was washed with ethyl acetate (20 mL × 2). The combined filtrates were extracted with ethyl acetate (100 mL × 2). Organic phases were collected and washed with saturated salt solution (300 mL × 2), dried with anhydrous sodium sulfate, concentrated to remove the solvent to obtain a crude product, which was purified through silica gel column chromatography ($SiO_2$, petroleum ether/ethyl acetate = 50/1 to 0/1) to obtain brown solids **KHE001-6** (1.11 g, yield: 28.8%, purity: 94.2%).MS (ESI) m/z: 380.0 $[M+H]^+$.**$^1$H NMR** (DMSO-*d*6, 400MHz): δ 10.10-10.05 (m, 1H), 8.02 (s, 1H), 7.30-7.20 (m, 2H), 7.14-7.02 (m, 2H), 6.66 (s, 2H), 5.54 (br s, 2H), 3.99-3.91 (m, 2H).

**Compound KHE001-7:** reaction solution A: compound **KHE001-6** (0.5014 g, 1.322 mmol) was added into 26 mL of water, and added with 14 mL of concentrated hydrochloric acid at 0°C. Sodium nitrite (0.1218 g, 1.765 mmol) was dissolved in 4 mL of water, and slowly dropwise added into the reaction solution, and stirred at 0°C for 1.5 hours to become a solution. Reaction solution B: Compound **KHE001-6a** (0.2381 g, 1.526 mmol) was added into 40 mL of water, added with 14 mL of pyridine at 0°C, and continuously stirred at this temperature for 1.5 hours. Then, the reaction solution A was quickly poured into the reaction solution B at 0°C to form tangerine solids, and the temperature was slowly raised to room temperature to continue the reaction overnight. After TLC monitoring showed that the reaction was complete, the solids were filtered and washed with 50 mL of water and petroleum ether thrice respectively. Tangerine solids **KHE001-7** (600 mg, yield: 82.9%, crude product) were obtained, which were directly used for the next step without purification. MS (ESI) m/z: 547.1 $[M+H]^+$.

**Compound KHE001:** compound **KHE001-7** (600 mg, 1.097 mmol) and sodium acetate (0.9035 g, 11.018 mmol) were put into a single-necked bottle and dissolved with acetic acid (12 mL) under the protection of $N_2$. The reaction was carried out for 3 hours at a temperature of 120°C. After TLC monitoring showed that the raw materials were completely reacted, the reaction was stopped. The reaction solution was cooled to 0°C and added with 100 mL of water, then a large amount of solids were precipitated, which were directly filtered, and the solids were washed thrice with 50 mL of water and petroleum ether respectively. Tangerine solids (560 mg, crude product) were collected, and 100 mg of the crude product was purified by preparative HPLC to obtain off-white solid **compound KHE001** (21.8 mg, 23.8%). MS (ESI, *m*/*z*): 500.9 $[M+1]^+$.**$^1$H NMR (DMSO-d6, 400 MHz):** δ 13.25 (s, 1H), 10.28 (s, 1H), 8.08 (s, 2H), 7.74 (s, 2H), 7.22-7.24 (m, 2H), 7.07-7.11 (m, 2H), 3.39 (s, 2H).

**Example 26 Synthesis of compound KHE002**

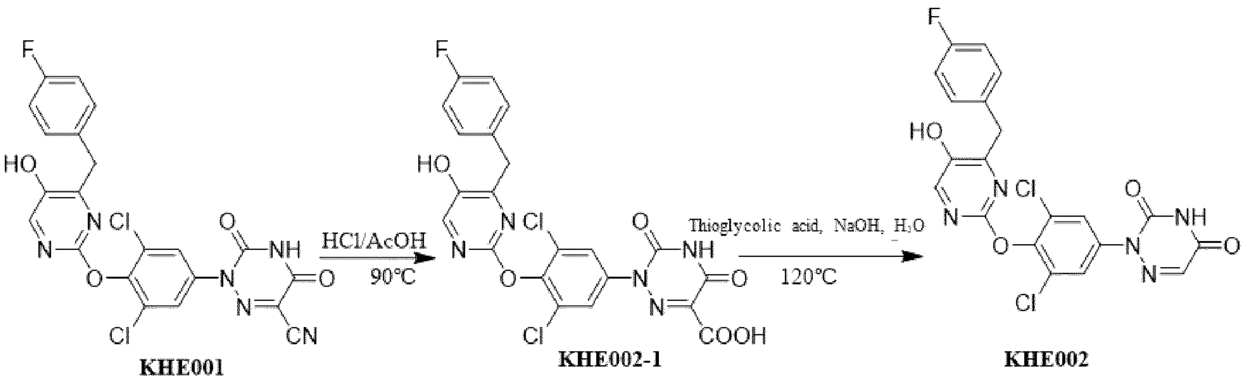


KHE001 KHE002-1 KHE002

**Compound KHE002-1:** crude compound **KHE001** (460 mg, 0.918mmol) was added into a single-necked flask and dissolved by acetic acid (15 mL), and then concentrated hydrochloric acid (5 mL) was added dropwise to the reaction. After reaction for 4 hours at 90°C, TLC monitoring showed that the raw materials were reacted completely, a new increased polarity spot was formed, and the reaction was stopped. The reaction solution was directly dried by rotary evaporation, and the reaction pH was adjusted to 9-10 with a saturated sodium carbonate solution, then the reaction solution was extracted with ethyl acetate (20 mL × 2). Aqueous phases were collected and the pH of the aqueous phases was adjusted to 3-4, then the aqueous phases were extracted with ethyl acetate (20 mL × 3). Organic phases were collected, dried with anhydrous sodium sulfate, and filtered. The organic phases were concentrated to obtain yellow solids **KHE002-1** (300 mg,

62.8%), which were directly used in the next step without purification.

**Compound KHE002:** compound **KHE002-1** (300 mg, 0.577 mmol) and sodium hydroxide (0.0985 g, 2.462 mmol) were added into a single-necked flask, and dissolved with water (40 mL), and then the reaction solution was added with thioglycolic acid (1.0831 g, 11.773 mmol) and reacted at 120°C for 3 hours. After TLC monitoring showed that the raw materials were completely reacted, a reduced polarity spot was formed, and the reaction was stopped. Saturated sodium carbonate solution was added to adjust the pH of the reaction system to neutral, then the reaction system was extracted with ethyl acetate (20 mL × 3), dried with anhydrous sodium sulfate, filtered, dried by rotary evaporation, and purified by preparative HPLC to obtain white solids **KHE002** (46.7 mg, 17.0%). MS (ESI, *m/z*): 476.3$[M+1]^+$. **$^1$H NMR (DMSO-*d6*, 400 MHz):** δ 12.49 (s, 1H), 10.25 (s, 1H), 8.08 (s, 1H), 7.69 (s, 2H), 7.60 (s, 1H), 7.23-7.24 (m, 2H), 7.07-7.12 (m, 1H), 3.98 (m, 2H).

**Example 27 Synthesis of compound KHE003**

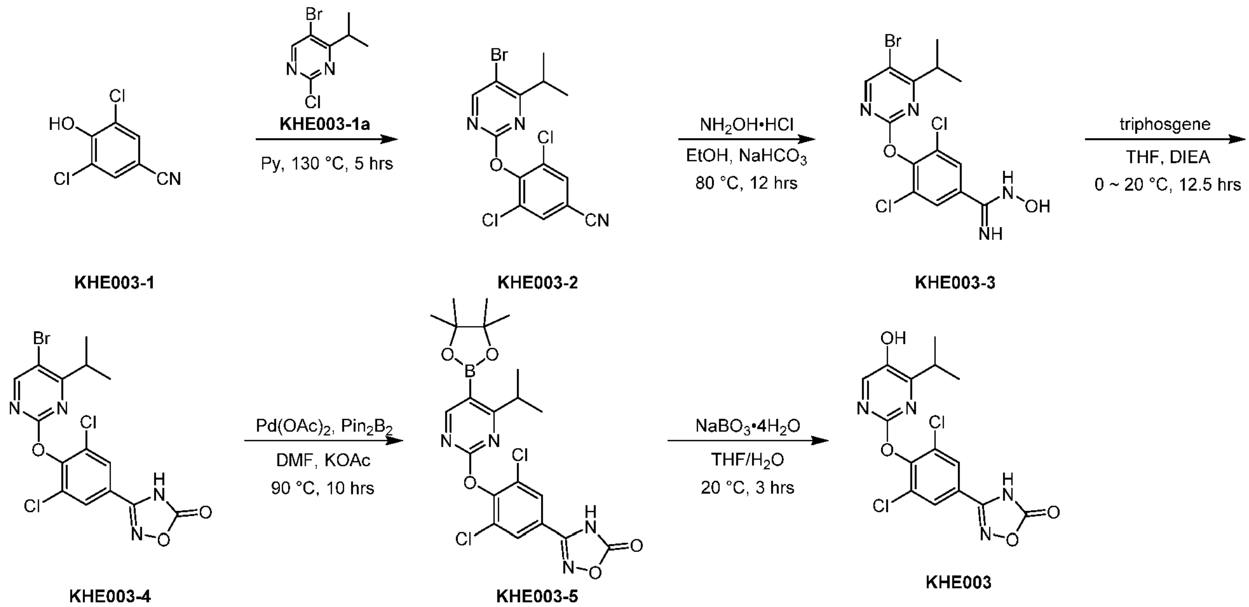


KHE003-1 KHE003-2 KHE003-3

KHE003-4 KHE003-5 KHE003

**Compound KHE003-2: KHE003-1a** (10.0 g, 42.5 mmol, 1.60 eq) and compound KHE003-1 (5.00 g, 26.6 mmol, 1.00 eq) were dissolved in pyridine (50 mL), and stirred at 130°C for 5 hours. TLC (petroleum ether/ethyl acetate= 10/1, $R_f$ = 0.52) monitoring showed that the reaction was complete. The reaction was cooled to 20°C and diluted with water (500 mL), and extracted with ethyl acetate (200 mL × 2). Combined organic phases were washed with saturated salt solution (300 mL × 2), dried with anhydrous sodium sulfate, filtered, and concentrated to remove the solvent to obtain a crude product, which was purified by silica gel column chromatography ($SiO_2$, petroleum ether/ethyl acetate = 50/1 to 5/1) to obtain yellow oily substance **KHE003-2** (2.40 g, yield: 23.2%).**$^1$H NMR** (DMSO-*d6*, 400MHz): δ 8.51 - 8.47 (m, 1H), 7.73 - 7.70 (m, 2H), 3.41 (spt, *J* = 6.8 Hz, 1H), 1.19 (d, *J* = 6.8 Hz, 6H).

**Compound KHE003-3:** hydroxylamine hydrochloride (377 mg, 5.43 mmol, 1.50 eq), compound **KHE003-2** (1.40 g, 3.62 mmol, 1.00 eq) and sodium bicarbonate (304 mg, 3.62 mmol, 1.00 eq) were added into ethanol (20 mL), and stirred at 80°C for 12 hours. TLC (petroleum ether/ethyl acetate= 2/1, $R_f$ = 0.11) monitoring showed that the reaction was complete. The reaction was directly dried by rotary evaporation to obtain a crude product, which was purified by silica gel column chromatography ($SiO_2$, petroleum ether/ethyl acetate = 1/0 to 20/1) to obtain yellow oily substance **KHE003-3** (1.00 g, yield: 65.8%).

**Compound KHE003-4:** triphosgene (1.61 g, 5.43 mmol, 1.20 eq), compound **KHE017-3** (1.90 g, 4.52 mmol, 1.00 eq) and DIEA (2.92 g, 22.6 mmol, 3.94 mL, 5.00 eq) were dissolved in tetrahydrofuran (20 mL) under the protection of nitrogen at 0°C and continuously stirred at 0°C for 0.5 hour, then heated to 20°C and stirred for 12 hours. TLC (petroleum ether/ethyl acetate= 1/1, $R_f$ = 0.22) monitoring showed that the reaction was complete. The reaction solution was diluted with water (500 mL) and then ethyl acetate (300 mL) and water (150 mL) were added to the reaction solution for extraction and separation. Organic phases were collected, washed with saturated salt solution, dried with anhydrous sodium sulfate, and concentrated to remove the solvent to obtain a crude product, which was purified by silica gel column chromatography ($SiO_2$, petroleum ether/ethyl acetate = 50/1 to 0/1) to obtain yellow solids **KHE003-4** (1.30 g, yield: 64.4%). **$^1$H NMR** (DMSO-d6, 400MHz): δ 13.02 (br s, 1H), 8.79 (s, 1H), 8.05 (s, 2H), 3.42-3.40 (m, 1H), 1.13 (d, *J* = 6.8 Hz, 6H).

**Compound KHE003-5:** palladium acetate (60.4 mg, 269 μmol, 0.10 eq), compound **KHE003-4** (1.20 g, 2.69 mmol, 1 eq), $Pin_2B_2$ (6.83 g, 26.9 mmol, 10.0 eq) and potassium acetate (792 mg, 8.07 mmol, 3.00 eq) were added into DMF (10 mL) under the protection of nitrogen and stirred at 90°C for 10 hours. LCMS monitoring showed that the reaction was complete. After cooling to 20°C, the reaction solution was slowly added with water (100 mL) and extracted with ethyl acetate (30 mL × 2). Organic phases were collected, washed with saturated salt solution (100 mL × 2), dried with anhydrous sodium sulfate and filtered. Organic solvent was dried by rotary evaporation to obtain yellow oily substance **KHE003-5** (2.00 g, mixture), which was used in the next step without purification. MS (ESI) m/z = 493.0 $[M+1]^+$.

**Compound KHE003:** compound **KHE003-5** (2.00 g, 4.06 mmol, 1.00 eq) was dissolved in tetrahydrofuran (10 mL) and water (10 mL), and then sodium perborate ($NaBO_3•4H_2O$) (1.87 g, 12.2 mmol, 3.00 eq) was added into the reaction. The reaction was stirred at 20°C for 3 hours, and LCMS monitoring showed that the reaction was complete. The reaction solution was extracted and separated with ethyl acetate (20 mL) and water (100 mL). Organic phases were collected, washed with saturated salt solution, dried with anhydrous sodium sulfate and filtered. Then the organic phases were dried by rotary evaporation to obtain residues. The residues were purified with preparative HPLC to obtain yellow solids **KHE003** (154 mg, yield: 14.9%, and purity 97.0%). MS (ESI) m/z: 383.1 $[M+1]^+$. **$^1$H NMR** (DMSO-*d6*, 400MHz): δ 10.12 (br s, 1H), 8.00 (s, 1H), 7.97 (s, 2H), 3.27 (br s, 1H), 1.10 (d, *J* = 6.8 Hz, 6H).

**Example 28 Synthesis of compound KHE004**

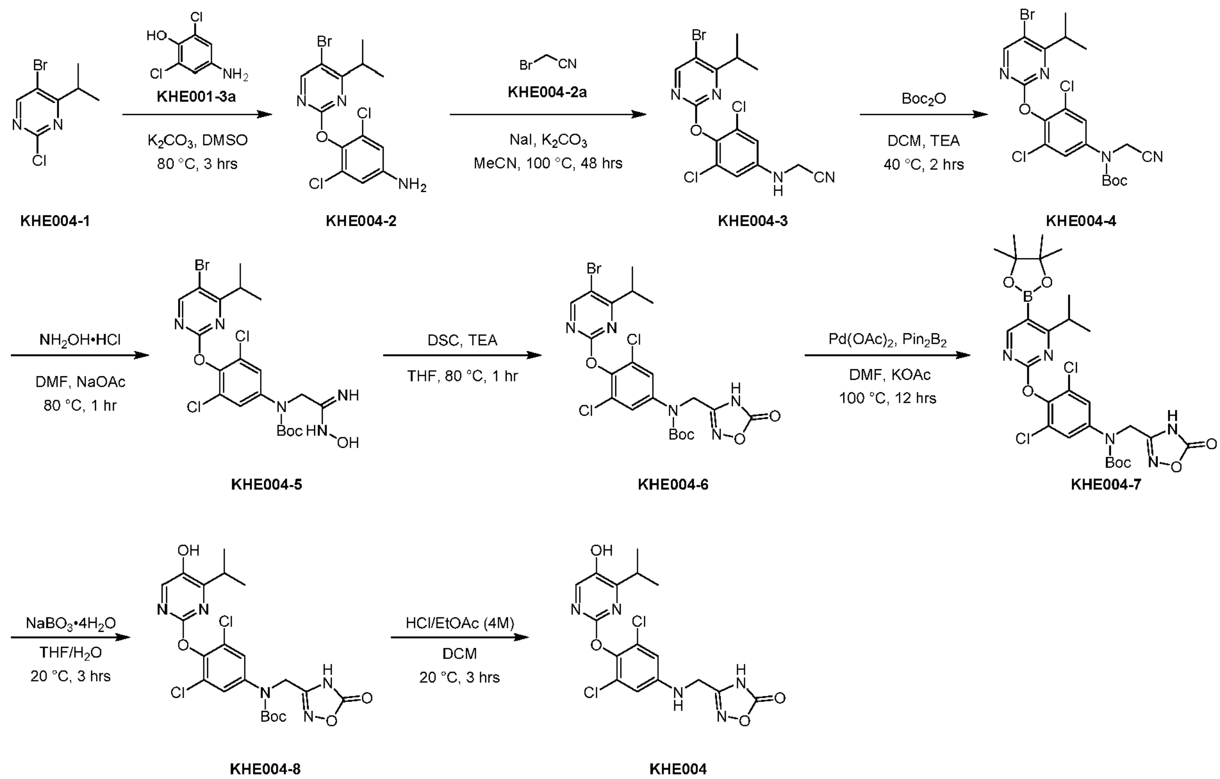


KHE004-1 KHE004-2 KHE004-3 KHE004-4 KHE004-5 KHE004-6 KHE004-7 KHE004-8 KHE004

**Compound KHE004-2: KHE001-3a** (7.56 g, 42.5 mmol, 1.00 eq), compound **KHE004-1** (10.0 g, 42.5 mmol, 1.00 eq) and potassium carbonate (11.7 g, 84.9 mmol, 2.00 eq) were added to DMF (100mL) and stirred at 80°C for 3 hours under the protection of nitrogen. LCMS monitoring showed that the reaction was complete. After cooling to 20°C, the reaction solution was diluted with water (200 mL) and extracted with ethyl acetate (100 mL × 2). Organic phases were collected, washed with saturated salt solution, dried with anhydrous sodium sulfate, filtered, dried by rotary evaporation, and purified through silica gel column chromatography ($SiO_2$, petroleum ether/ethyl acetate = 1/0 to 2/1) to obtain yellow solids **KHE004-2** (12.0 g, yield: 75.0%). MS (ESI) m/z = 378.0 $[M+1]^+$.

**Compound KHE004-3:** compound **KHE004-2** (5.00 g, 13.3 mmol) and bromoacetonitrile **(KHE004-2a)** (11.9 g, 99.5 mmol) were dissolved in MeCN (50 mL), and then added with sodium iodide (5.96 g, 39.8 mmol) and potassium carbonate (5.50 g, 39.8 mmol). The mixture was heated to 100°C in a 100mL sealed tube and stirred for 48 hours. TLC (petroleum ether/ethyl acetate = 5/1, $R_f$ = 0.22) monitoring showed that the reaction was complete. The reaction solution was directly dried by rotary evaporation, and the residues were purified through silica gel column chromatography ($SiO_2$,

petroleum ether/ethyl acetate = 50/1 to 2/1) to obtain yellow solids **KHE004-3** (4.00 g, yield: 72.5%). **$^1$H NMR** (DMSO-*d6*, 400MHz): δ 8.72 (s, 1H), 6.92 (s, 2H), 6.77 (br t, *J* = 6.8 Hz, 1H), 4.41 - 4.34 (m, 1H), 4.38 (d, *J* = 6.6 Hz, 2H), 3.39 - 3.34 (m, 1H), 3.39 - 3.34 (m, 1H), 1.14 (d, *J* = 6.8 Hz, 6H).

**Compound KHE004-4:** compound **KHE004-3** (4.00 g, 9.61 mmol) and triethylamine (1.02 g, 10.1 mmol, 1.40 mL) were dissolved in dichloromethane (40 mL) and then $Boc_2O$ (2.20 g, 10.1 mmol, 2.32 mL) was added. The mixture was stirred at 40°C for 2 hours. TLC (petroleum ether/ethyl acetate = 5/1, $R_f$ = 0.62) monitoring showed that the reaction was complete. The reaction solution was cooled to 20°C, slowly added with water (200 mL), and extracted with dichloromethane (40 mL × 2). Organic phases were collected and washed with saturated salt solution (200 mL × 2), dried with anhydrous sodium sulfate, filtered and dried by rotary evaporation to obtain yellow oily substance **KHE004-4** (4.80 g, yield: 96.7%). **$^1$H NMR** (DMSO-*d6*, 400MHz): δ 8.78 (s, 1H), 7.65 (s, 2H), 4.81 (s, 2H), 3.38 - 3.33 (m, 1H), 1.46 - 1.41 (m, 10H), 1.10 (d, *J* = 6.8 Hz, 6H).

**Compound KHE004-5:** compound **KHE004-4** (4.80 g, 9.30 mmol) and sodium acetate (6.10 g, 74.4 mmol) were dissolved in DMF (40 mL), and added with hydroxylamine hydrochloride (5.17 g, 74.4 mmol). The mixture was stirred at 80°C for 1 hour. TLC (petroleum ether/ethyl acetate = 1/1, $R_f$ = 0.39) monitoring showed that the reaction was complete. After cooling to room temperature, the reaction solution was extracted with ethyl acetate (100 mL) and water (250 mL). Organic phases were collected, dried with anhydrous sodium sulfate, filtered, dried by rotary evaporation and purified through silica gel column chromatography ($SiO_2$, petroleum ether/ethyl acetate = 50/1 to 0/1) to obtain yellow solids **KHE004-5** (4.60 g, yield: 90.1 %). **$^1$H NMR** (DMSO-*d6*, 400MHz): δ 8.78 (s, 1H), 7.65 (s, 2H), 4.81 (s, 2H), 3.38 - 3.33 (m, 1H), 1.46 - 1.41 (m, 9H), 1.10 (d, J = 6.8 Hz, 6H).

**Compound KHE004-6:** N,N'-disuccinimidyl carbonate (DSC) (2.67 g, 10.4 mmol), compound **KHE004-5** (4.40 g, 8.01 mmol) and triethylamine (3.05 g, 30.1 mmol, 4.19 mL) were dissolved in DMF (10 mL), and stirred at 80°C for 1 hour under the protection of nitrogen,. TLC (petroleum ether/ethyl acetate = 2/1, $R_f$ = 0.07) monitoring showed that the reaction was complete. **KHE004-6** signal was monitored by LCMS and the reaction solution was separated with ethyl acetate (50 mL) and water (50 mL). Organic phases were collected, washed with saturated salt solution, dried with anhydrous sodium sulfate, filtered, and dried by rotary evaporation to obtain yellow solids **KHE004-6** (4.20 g, mixture) which were directly used for the next reaction. MS (ESI) m/z: 576.0 $[M+1]^+$.

**Compound KHE004-7:** palladium acetate (39.0 mg, 174 μmol, 0.10 eq), compound **KHE004-6** (1.00 g, 1.74 mmol, 1.00 eq), potassium acetate (512 mg, 5.22 mmol, 3.00 eq) and $Pin_2B_2$ (4.41 g, 17.4 mmol, 10.0 eq) were dissolved in DMF (15 mL), and stirred at 100°C for 12 hours under the protection of nitrogen. The target product **KHE004-7** was monitored by LCMS. The reaction solution was extracted and separated with ethyl acetate (20 mL) and water (20 mL). Organic phases were collected, dried with anhydrous sodium sulfate, filtered and dried by rotary evaporation to obtain yellow solids **KHE004-7** (4.00 g, crude product). The crude product was directly used for the next reaction. MS (ESI) m/z: 622.3 $[M+1]^+$.

**Compound KHE004-8:** compound **KHE004-7** (4.00 g, 6.43 mmol, 1.00 eq) was dissolved in tetrahydrofuran (20 mL) and water (20 mL), then sodium perborate ($NaBO_3 \cdot 4H_2O$) (2.97 g, 19.3 mmol, 3.00 eq) was added to the reaction and stirred at 20°C for 3 hours. LC-MS monitoring showed that the reaction was complete. The reaction solution was extracted and separated with ethyl acetate (20 mL) and water (100 mL). Organic phases were washed with saturated salt solution, dried with anhydrous sodium sulfate, filtered, and dried by rotary evaporation to obtain yellow solids **KHE004-8** (2.00 g, crude product). The crude product was directly used for the next reaction. MS (ESI) m/z: 514.0 $[M+1]^+$.

**Compound KHE004: KHE004-8** (2.00 g, 3.90 mmol, 1.00 eq) was dissolved in dichloromethane (20 mL), ethyl acetate solution (4 M, 10 mL, 10.3 eq) of hydrogen chloride was added into the reaction solution, and stirred at 20°C for 3 hours. LC-MS monitoring showed that the reaction was complete. The reaction solution was directly dried by rotary evaporation and purified with preparative HPLC to obtain brown solids **KHE004** (19.5 mg, yield: 1.16%, purity 96.3%). MS (ESI) m/z: 412.0 $[M+1]^+$. **$^1$H NMR** (DMSO-*d6*, 400MHz): δ 12.42 (br s, 1H), 9.89 (br s, 1H), 7.95 (s, 1H), 6.79 (s, 1H), 6.68 - 6.55 (m, 1H), 4.29 (s, 2H), 3.32 - 3.23 (m, 2H), 1.11 (s, 6H).

**Example 29 Synthesis of compound KHE005**

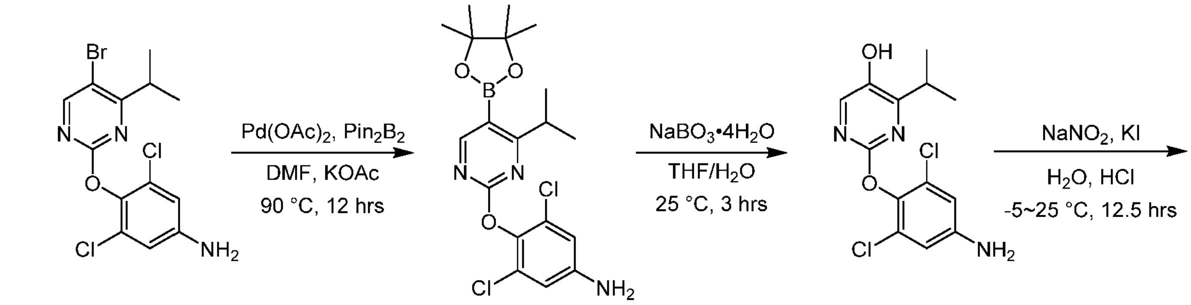


**KHE004-2** **KHE005-1** **KHE005-2**

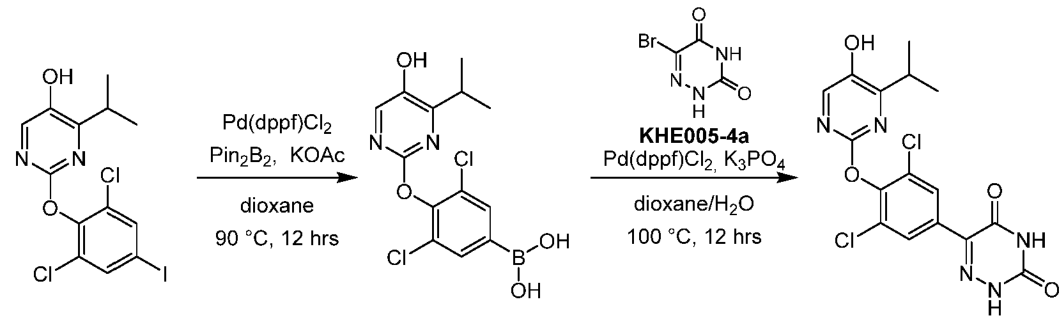


**KHE005-3** **KHE005-4** **KHE005**

**Compound KHE005-1:** palladium acetate (238 mg, 1.06 mmol, 0.10 eq),compound **KHE004-2** (4.00 g, 10.6 mmol, 1.00 eq), $Pin_2B_2$ (27.0 g, 106 mmol, 10.0 eq) and potassium acetate (3.12 g, 31.8 mmol, 3.00 eq) were dissolved in DMF (100 mL), and stirred at 90°C for 12 hours under the protection of nitrogen. LCMS monitoring showed that the reaction was complete and a target peak was apparent. The reaction solution was extracted and separated with ethyl acetate (50 mL) and water (50 mL). Organic phases were collected, dried with anhydrous sodium sulfate, filtered, and dried by rotary evaporation to obtain yellow solids **KHE005-1** (4.00 g, 9.43 mmol, crude product). The crude product was directly used for the next reaction. MS (ESI) m/z: 424.1 $[M+H]^+$.

**Compound KHE005-2:** compound **KHE005-1** (4.00 g, 9.43 mmol, 1.00 eq) was dissolved in tetrahydrofuran (10 mL) and water (10 mL), and then sodium perborate ($NaBO_3$•$4H_2O$) (4.35 g, 28.3 mmol, 3.00 eq) was added into the reaction, and the mixture was stirred at 20°C for 3 hours. LCMS monitoring showed that the reaction was complete and a target peak was apparent. The reaction solution was extracted and separated with ethyl acetate (20 mL) and water (20 mL). Organic phases were collected, washed with saturated salt solution, dried with anhydrous sodium sulfate, filtered, dried by rotary evaporation, and purified through silica gel column chromatography ($SiO_2$, petroleum ether/ethyl acetate= 20/1 to 5:/1) to obtain purple oily substance **KHE005-2** (1.40 g, yield: 42.0%). MS (ESI) m/z: 314.0 $[M+H]^+$. **$^1$H NMR** (DMSO-*d6*, 400MHz): δ 10.04 (s, 1 H), 7.97-7.99 (m, 3 H), 3.27 (d, *J* = 6.8 Hz, 1 H), 1.11 (s, 3 H), 1.09 (s, 3 H).

**Compound KHE005-3:** compound **KHE005-2** (800 mg, 2.55 mmol, 1.00 eq) was added to concentrated hydrochloric acid (12.0 M, 20 mL, 94.3 eq), and the reaction was cooled to -5°C. Sodium nitrite (1.05 g, 15.3 mmol, 6.00 eq) was dissolved in water (4.00 mL), and then slowly added dropwise into the foregoing solution, and stirred vigorously, and the reaction temperature was kept between -5°C and 0°C. After reaction for 0.5 hour, potassium iodide (6.34 g, 38.2 mmol, 15.0 eq) was dissolved in water (6.00 mL), and then slowly added dropwise into the foregoing solution, and stirred vigorously, and the reaction temperature was continuously kept between -5°C and 0°C. After the addition, the reaction solution was heated to 25°C and stirred for 12 hours under the protection of nitrogen. LCMS monitoring showed that the reaction was complete. The reaction solution was extracted and separated with ethyl acetate (20 mL) and water (100 mL). Organic phases were collected, washed with saturated sodium sulfite solution, dried with anhydrous sodium sulfate, filtered, dried by rotary evaporation, and purified through silica gel column chromatography ($SiO_2$, petroleum ether/ethyl acetate = 20/1 to 0/1) to obtain yellow solids **KHE005-3** (546 mg, yield: 50.5%). MS (ESI) m/z: 424.9 $[M+H]^+$. **$^1$H NMR** (DMSO-*d6*, 400MHz): δ 10.05 (br s, 1 H) 7.98 (s, 2 H) 7.98 (s, 1 H) 3.27 (d, *J* = 6.8 Hz, 1 H) 1.11 (s, 3 H) 1.10 (s, 3 H).

**Compound KHE005-4:** under the protection of nitrogen, $Pd(dppf)Cl_2$ (93 mg, 0.127 mmol, 0.10 eq), compound **KHE005-3** (540 mg, 1.27 mmol, 1.00 eq), $Pin_2B_2$ (3.227 g, 12.7 mmol, 10.0 eq) and potassium acetate (374 mg, 3.81 mmol, 3.00 eq) were dissolved in dioxane (7 mL), and stirred at 90°C for 12 hours. LCMS monitoring showed that the reaction was complete. The reaction solution was directly dried by rotary evaporation and purified through silica gel column chromatography ($SiO_2$, dichloromethane/methanol = 20/1) to obtain a crude product. The crude product was purified with preparative HPLC to obtain red solid boronic acid product **KHE005-4** (400 mg, yield: 92.0%). MS (ESI) m/z: 343.0 $[M+H]^+$. **$^1$H NMR** (DMSO-*d6*, 400MHz): δ 10.05 (s, 1 H), 8.47 (s, 1 H), 7.96 (s, 1 H), 7.86 (s, 1 H), 3.27 (d, *J* = 6.8 Hz, 1 H), 1.10 (s, 3

H),1.08 (s, 3 H).

**Compound KHE005:** under the protection of nitrogen, Pd(dppf)$Cl_2$ (51.2 mg, 0.07 mmol, 0.06 eq), compound **KHE005-4** (400 mg, 1.17 mmol, 1.00 eq), **KHE005-4a** (224 mg, 1.17 mmol, 1.00 eq) and potassium phosphate (495 mg, 2.33 mmol, 2.00 eq) were added into a system of dioxane (2.5 mL) and water (2.5 mL), and stirred at 100°C for 12 hours. LCMS monitoring showed that the reaction was complete. The reaction solution was extracted and separated with ethyl acetate (10 mL) and water (80 mL). Organic phases were collected, washed with saturated salt solution, dried with anhydrous sodium sulfate, filtered, dried by rotary evaporation, and purified through silica gel column chromatography ($SiO_2$, dichloromethane/methanol = 20/1) to obtain a crude product, which was purified with preparative HPLC to obtain yellow solids **KHE005-5** (101 mg, yield: 21.0%, purity 99.5%). MS (ESI) m/z: 410.1 $[M+1]^+$. **$^1$H NMR** (DMSO-*d6*, 400MHz): δ 12.69 (d, *J* = 1.2 Hz, 1H), 12.23 (s, 1H), 10.07 (br s, 1H), 8.04-7.97 (m, 3H), 3.36-3.21 (m, 1H), 1.12 (d, *J* = 6.8 Hz, 6H).

**Example 30 Synthesis of compound KHE006**

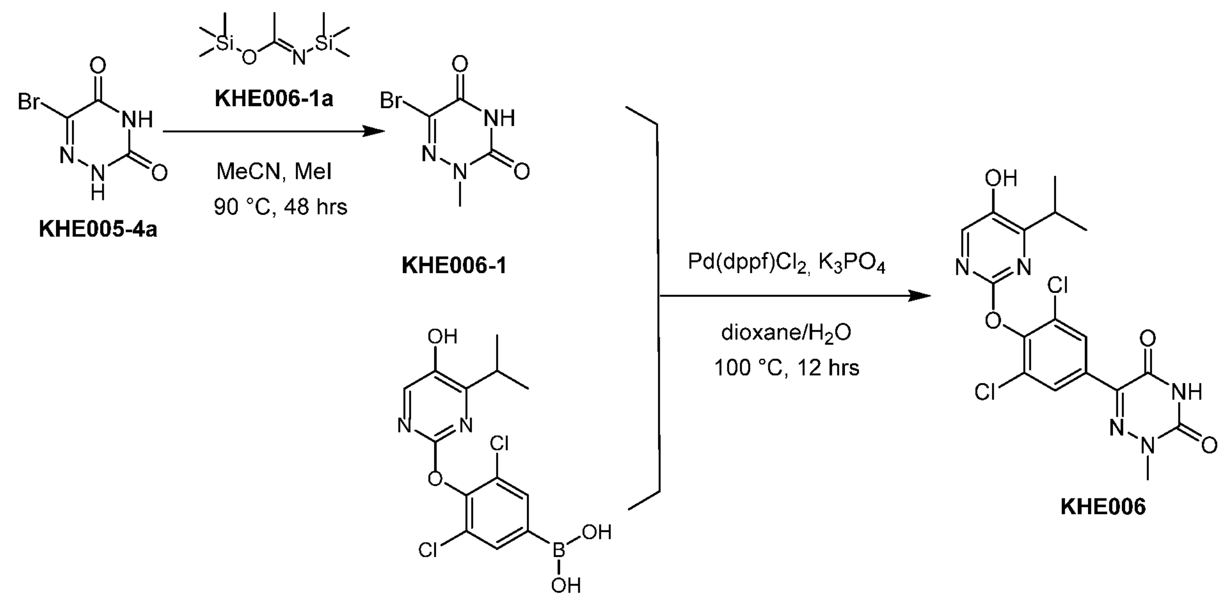


KHE005-4

**Compound KHE006-1:** compound **KHE005-4a** (3.00 g, 15.6 mmol, 1.00 eq) was dissolved in acetonitrile (45 mL), then the reaction was added with **KHE006-1a** (8.14 g, 40.0 mmol, 9.89 mL, 2.56 eq) and stirred at 82°C for 3 hours. Then, methyl iodide (2.71 g, 19.1 mmol, 1.19 mL, 1.22 eq) was added into the above reaction system, stirred for 24 hours, and then the reaction was added with methyl iodide (1.11 g, 7.81 mmol, 486 μL, 0.50 eq) continuously, and stirred for 24 hours. TLC (petroleum ether/ethyl acetate = 2/1, $R_f$ = 0.55) showed that the reaction was complete. The reaction solution was extracted with ethyl acetate (50 mL) and water (50 mL). Organic phases were collected, dried with anhydrous sodium sulfate and filtered. The filtrate was concentrated and purified through silica gel column chromatography ($SiO_2$, petroleum ether/ethyl acetate = 20/1 to 0/1) to obtain yellow solids **KHE006-1** (2.00 g, yield: 62.1%). **$^1$H NMR** (DMSO-*d6*, 400MHz): δ 3.44 (s, 3 H), 12.49 (br s, 1 H).

**Compound KHE006:** under the protection of nitrogen, Pd(dppf)$Cl_2$ (89.6 mg, 0.122 mmol, 0.06 eq), **KHE005-4** (700 mg, 2.04 mmol, 1.00 eq), **KHE006-1** (631 mg, 3.06 mmol, 1.50 eq) and potassium phosphate (866 mg, 4.08 mmol, 2.00 eq) were dissolved in a system of dioxane (2.5 mL) and water (2.5 mL), and stirred at 100°C for 12 hours. LCMS monitoring showed that the reaction was complete. The reaction solution was extracted and separated with ethyl acetate (10 mL) and water (80 mL). Organic phases were collected, washed with saturated salt solution, dried with anhydrous sodium sulfate, filtered, dried by rotary evaporation and purified with preparative HPLC to obtain faint yellow solids **KHE006** (10.4 mg, yield: 1.11%, purity 91.9%). MS (ESI) m/z: 424.1 $[M+1]^+$.

**Example 31 Synthesis of compound KHE007**

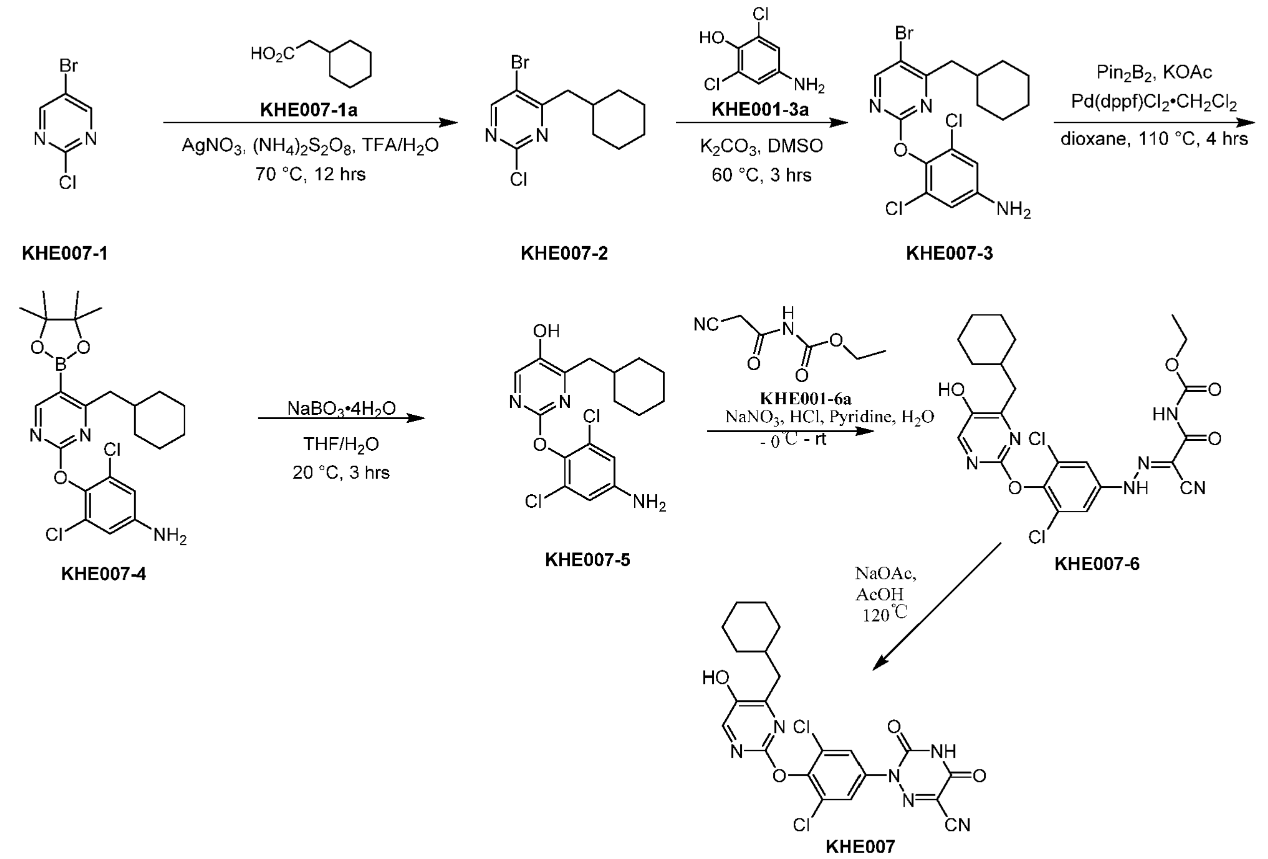


**Compound KHE007-2:** compound **KHE007-1** (30.0 g, 155 mmol, 1.00 eq) was added into water (300 mL), **KHE007-1a** (26.5 g, 186 mmol, 26.2 mL, 1.20 eq), silver nitrate (5.27 g, 31.0 mmol, 0.20 eq) and trifluoroacetic acid (8.84 g, 77.6 mmol, 5.74 mL, 0.50 eq) were added into the above reaction system. The mixture was heated to 70°C and stirred, and then slowly added with ammonium persulfate $(NH_4)_2S_2O_8$ (70.8 g, 310 mmol, 2.00 eq). After the addition, the reaction was continued for 12 hours. TLC (petroleum ether/ethyl acetate = 1/0, $R_f$ = 0.12) showed that the reaction was complete. The reaction solution was cooled to 20°C, and extracted with ethyl acetate (20 mL) and water (100 mL). Organic phases were collected, washed with saturated sodium chloride solution, dried with anhydrous sodium sulfate, filtered, dried by rotary evaporation and purified with silica gel column chromatography ($SiO_2$, petroleum ether/ethyl acetate = 1/0 to 10/1) to obtain colorless oily substance **KHE007-2** (24.4 g, 84.3 mmol, yield: 54.3%). MS (ESI) m/z: 291.2 $[M+H]^+$. **$^1$H NMR** (DMSO-*d6*, 400MHz): δ 8.88 (s, 1H), 2.74 (d, *J* = 7.2 Hz, 2H), 1.81 (dt, *J* = 3.6, 7.2 Hz, 1H), 1.69-1.54 (m, 5H), 1.27-1.12 (m, 3H), 1.09-0.96 (m, 2H).

**Compound KHE007-3:** compound **KHE007-2** (24.4 g, 84.3 mmol, 1.00 eq) and $K_2CO_3$ (23.3 g, 169 mmol, 2.00 eq) were added into DMSO (200 mL), and meanwhile, **KHE001-3a** (15.0 g, 84.3 mmol, 1.00 eq) was added into the above reaction system. The reaction solution was heated to 60°C and stirred for 3 hours under the protection of nitrogen. TLC (petroleum ether/ethyl acetate = 5/1, $R_f$ = 0.34) showed that the reaction was complete. The reaction solution was extracted and separated with ethyl acetate (20 mL) and water (100 mL). Organic phases were collected, washed with saturated salt solution, dried with anhydrous sodium sulfate, filtered, dried by rotary evaporation and purified with silica gel column chromatography ($SiO_2$, petroleum ether/ethyl acetate = 50/1 to 1/1) to obtain colorless oily substance **KHE007-3** (28.0 g, 64.9 mmol, yield: 77.1%). MS (ESI) m/z: 432.1 $[M+H]^+$. **$^1$H NMR** (DMSO-*d6*, 400MHz): δ 8.71 (s, 1H), 6.68 (s, 2H), 5.62 (s, 2H), 2.68-2.63 (m, 2H), 1.83-1.69 (m, 1H), 1.66-1.51 (m, 5H), 1.15-1.05 (m, 3H), 1.02-0.88 (m, 2H).

**Compound KHE007-4:** compound **KHE007-3** (2.00 g, 4.64 mmol, 1.00 eq), potassium acetate (911 mg, 9.28 mmol, 2.00 eq) and $Pin_2B_2$ (2.36 g, 9.28 mmol, 2.00 eq) were added into dioxane (10 mL), and then, $Pd(dppf)Cl_2 \cdot CH_2Cl_2$ (189 mg, 0.232 mmol, 0.05 eq) was added into the above reaction system. The reaction solution was heated to 110°C and stirred for 4 hours under the protection of nitrogen. LCMS monitoring showed that the reaction was complete. The reaction solution was extracted with ethyl acetate (20 mL) and water (100 mL). Organic phases were collected, washed with saturated salt solution, dried with anhydrous sodium sulfate, filtered, and dried by rotary evaporation to obtain brown solids KHE026-4 (4.00 g, crude product) which were directly reacted in the next step without purification. MS (ESI) m/z: 478.1 $[M+H]^+$.

**Compound KHE007-5:** crude product of compound **KHE007-4** (4.00 g, 8.36 mmol, 1.00 eq) was dissolved in tetrahydrofuran (20 mL) and water (20 mL), and then sodium perborate $NaBO_3 \cdot 4H_2O$ (3.86 g, 25.1 mmol, 3.00 eq) was

added into the foregoing solution. The mixture was carried out for 3 hours at 20°C. TLC (petroleum ether/ethyl acetate = 2/1, $R_f$ = 0.15) showed that the reaction was complete. The reaction solution was filtered, the filter cake was washed with ethyl acetate, and the filtrate was collected and extracted with ethyl acetate (50 mL × 2). Organic phases were collected, washed with saturated salt solution, dried with anhydrous sodium sulfate, filtered, dried by rotary evaporation and purified with silica gel column chromatography ($SiO_2$, petroleum ether/ethyl acetate = 50/1 to 0/1) to obtain pale yellow oily substance (1.40 g) which was purified with preparative HPLC to obtain grey solids **KHE007-5** (1.23 g, 3.27 mmol, yield: 72.0%, purity: 97.8%). MS (ESI) m/z: 368.1 $[M+H]^+$. **$^1$H NMR** (DMSO-*d6*, 400MHz): δ 9.74 (s, 1H), 8.00 (s, 1H), 6.65 (s, 2H), 5.51 (s, 2H), 2.94 (s, 1H), 1.62-1.41 (m, 4H), 0.68 (t, *J* = 7.2 Hz, 6H).

**Compound KHE007-6: reaction solution A:** compound **KHE007-5** (0.5038 g, 1.369 mmol) was dissolved in water (26 mL), added with 14 mL of concentrated hydrochloric acid at 0°C, weighed sodium nitrite (0.1234 g, 1.788 mmol) was dissolved in 4 mL of water, slowly dropwise added into the reaction solution, and the reaction temperature was maintained at 0-5°C. After the addition, the reaction solution was continuously stirred at 0°C for 1.5 hours to obtain the solution. **Reaction solution B:** compound **KHE001-6a** (0.2381 g, 1.526 mmol) was added into 40 mL of water, added with 14 mL of pyridine at 0°C, and stirred for 1.5 hours at this temperature. Then, **the reaction solution A** was quickly poured into **the reaction solution B** at 0°C to form tangerine solids, and the temperature was slowly raised to room temperature to continue the reaction overnight. After TLC monitoring showed that the reaction was complete, the solids were directly filtered and washed thrice with 50 mL of water and petroleum ether respectively. The solids were collected to obtain tangerine solids **KHE007-6** (700 mg, 95.6%, crude product). The crude product was directly reacted in the next step without purification. MS (ESI) m/z: 535.1 $[M+H]^+$.

**Compound KHE007:** compound **KHE007-6** (700 mg, 1.310 mmol) and NaOAc (1.075 g, 13.110 mmol) were added into a single-necked flask and dissolved with acetic acid (12 mL) under the protection of nitrogen. The reaction was carried out for 3 hours at a temperature of 120°C. After TLC monitoring showed that the raw materials were completely reacted, the reaction was stopped. The reaction solution was cooled to 0°C and added with 100 mL of water, then a large amount of solids were precipitated, which were directly filtered and washed thrice with 50 mL of water and petroleum ether respectively. Tangerine solids (620 mg, crude product) were collected and obtained, and 120 mg of the crude product was purified with preparative HPLC to obtain faint yellow solid **compound KHE007** (26.9 mg, yield: 22.4%). MS (ESI) m/z: 489.3 $[M+1]^+$. **$^1$H NMR** (DMSO-*d6*, 400 MHz): δ 13.25 (s, 1H), 10.02 (s, 1H), 8.03 (s, 2H), 7.75 (s, 1H), 2.49 - 2.50 (m, 2H), 1.55-1.71 (m, 6H), 1.04 - 1.13 (m, 4H), 0.92 - 0.97 (m, 2H).

**Example 32 Synthesis of compound KHE008**

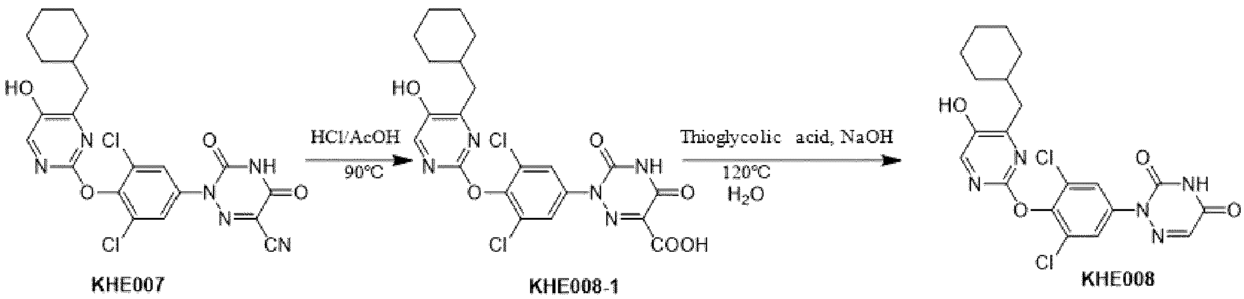


KHE007 KHE008-1 KHE008

**Compound KHE008-1:** compound KHE007 (503.8 mg, 1.030 mmol) was added into a single-necked flask, dissolved with acetic acid (15 mL), and then concentrated hydrochloric acid (5 mL) was dropwise added in the reaction. After the addition, the reaction was carried out for 4 hours at 90 °C, TLC monitoring showed that the raw materials were reacted completely, a new increased polarity spot was formed, and the reaction was stopped. The reaction solution was directly dried by rotary evaporation, pH was adjusted to 9-10 with a saturated sodium carbonate solution. The reaction solution was extracted with ethyl acetate (20 mL × 2), aqueous phases were collected and the aqueous phases were adjusted pH to 3-4, and extracted with ethyl acetate (20 mL × 2), and then, organic phases were collected, dried with anhydrous sodium sulfate and filtered. The organic phases were concentrated to remove the solvent and obtain yellow solids **KHE008-1** (260 mg, 49.7%) which were directly used in the next step without purification.

**Compound KHE008:** compound **KHE008-1** (260 mg, 0.581 mmol) and sodium hydroxide (0.0891 g, 2.227 mmol) were added into a single-necked flask, dissolved with water (40 mL), and then thioglycolic acid (1.0125 g, 10.331 mmol) was added in the reaction. The reaction was carried out for 3 hours at 120°C. After TLC monitoring showed that the raw materials were completely reacted, a reduced polarity spot was formed, and the reaction was stopped. Saturated sodium carbonate solution was added to adjust the pH of the reaction system to neutral, then the reaction system was

extracted with ethyl acetate (20 mL × 2), dried with anhydrous sodium sulfate, filtered, concentrated and purified by preparative HPLC to obtain white solids **KHE008** (96.9 mg, 40.9%). MS (ESI) m/z: 464.3[M+1]$^+$. **$^1$H NMR** (DMSO-*d6*, 400 MHz): δ 12.46 (s, 1H), 9.99 (s, 1H), 8.03 (s, 2H), 7.77 (s, 1H), 7.69 (s, 1H), 2.49 - 2.50 (m, 2H), 1.60 - 1.71 (m, 1H), 1.56 - 1.59 (m, 5H), 1.10 - 1.13 (m, 3H), 0.92 - 0.97 (m, 2H).

**Example 33 Synthesis of compound KHE009**

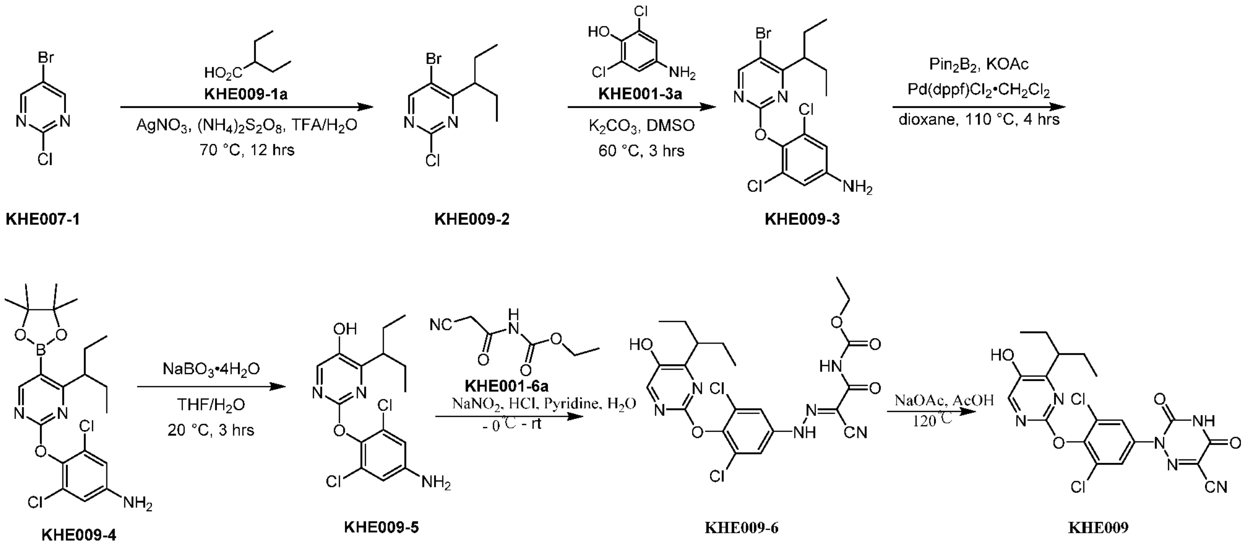


KHE007-1 KHE009-2 KHE009-3 KHE009-4 KHE009-5 KHE009-6 KHE009

**Compound KHE009-2:** the operation was the same as the synthesis of compound **KHE007-2,** and the crude product was purified through silica gel column chromatography ($SiO_2$, petroleum ether/ethyl acetate = 1/0 to 10/1) to obtain yellow oil **KHE009-2** (45.5 g, 173 mmol, yield: 66.8%). MS (ESI) m/z: 263.0[M+1]$^+$. **$^1$H NMR** ($CDCl_3$, 400MHz): δ 8.93 (s, 1H), 3.15 - 3.05 (m, 1H), 1.73 - 1.59 (m, 4H), 0.74 (t, J = 7.6 Hz, 6H).

**Compound KHE009-3:** the operation was the same as the synthesis of compound **KHE007-3,** and the crude product was purified through silica gel column chromatography ($SiO_2$, petroleum ether/ethyl acetate = 50/1 to 0/1) to obtain white solids **KHE009-3** (11.8 g, 29.1 mmol, yield: 38.4%). MS (ESI) m/z: 404.0[M+1]$^+$. **$^1$H NMR** (DMSO-*d6*, 400MHz): δ 8.77 (s, 1H), 6.68 (s, 2H), 5.61 (s, 2H), 3.04 (t, *J* = 7.0 Hz, 1H), 1.54 (quin, *J* = 7.2 Hz, 4H), 0.69 (t, *J* = 7.2 Hz, 6H).

**Compound KHE009-4:** the operation was the same as the synthesis of compound **KHE007-4** to obtain brown solids **KHE009-4** (22.0 g, crude product). The crude product was directly reacted in the next step without purification. MS (ESI) m/z: 452.2 [M+H]$^+$.

**Compound KHE009-5:** the operation was the same as the synthesis of compound **KHE007-5** to obtain white solids **KHE009-5** (4.44 g, 12.5 mmol, yield: 48.7%, purity 96.5%). MS (ESI) m/z: 342.0 [M+H]$^+$.

**$^1$H NMR** (DMSO-*d6*, 400MHz): δ 9.74 (s, 1H), 8.00 (s, 1H), 6.65 (s, 2H), 5.51 (s, 2H), 2.82 - 3.01 (m, 1H), 1.62 - 1.41 (m, 4H), 0.68 (t, *J* = 7.2 Hz, 6H).

**Compound KHE009-6:** the operation was the same as the synthesis of compound **KHE007-6,** and tangerine solids **KHE009-6** (650 mg, 86.7%, crude product) were obtained, which were directly reacted in next step without purification. MS (ESI) m/z: 509.2 [M+H]$^+$.

**Compound KHE009:** the operation was the same as the synthesis of compound **KHE007,** and tangerine solids (570 mg, crude product) were obtained. 100 mg of the crude product was purified with preparative HPLC to obtain faint yellow solids **KHE009** (18 mg, yield: 18.2%). MS (ESI) m/z: 463.0 [M+1]$^+$. **$^1$H NMR** (DMSO-*d6*, 400 MHz): δ 13.26 (s, 1H), 10.00 (s, 1H), 8.06 (s, 1H), 7.75 (s, 2H), 2.98 - 3.02 (m, 1H), 1.52-1.55 (m, 4H), 1.24 (s, 1H), 0.68 - 0.72 (m, 6H).

**Example 34 Synthesis of compound KHE010**

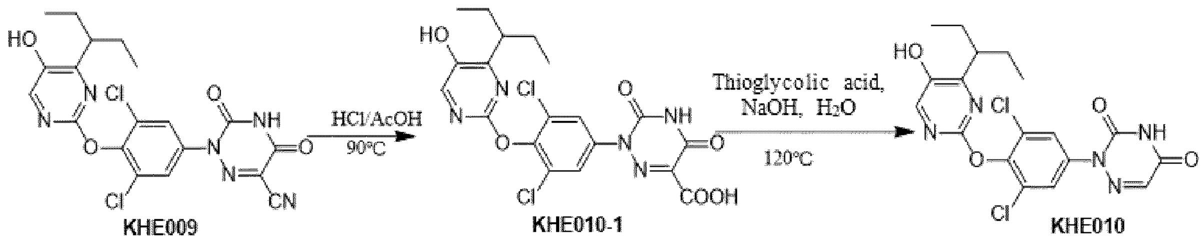


KHE009 KHE010-1 KHE010

**Compound KHE010-1:** the operation was the same as the synthesis of compound **KHE008-1,** and yellow solids **KH010-1** (280 mg, 57.4%) were obtained, which were directly reacted in the next step without purification. MS (ESI) m/z: 482.0 [M+1]$^+$.

**Compound KHE010:** the operation was the same as the synthesis of compound **KHE008,** and white solids **KHE010** (113.6 mg, 44.7%) were obtained after purification by preparative HPLC. MS (ESI) m/z: 438.2 [M+1]$^+$. **$^1$H NMR** (DMSO-*d6*, 400 MHz): 12.47(s, 1H), 9.98(s, 1H), 8.06(s, 1H), 7.78(s, 2H), 7.70(s, 1H), 2.94-3.01(m, 1H), 1.48-1.61(m, 4H), 0.70(t, 6H, J=8.0Hz).

**Example 35 Synthesis of compound KHE011**

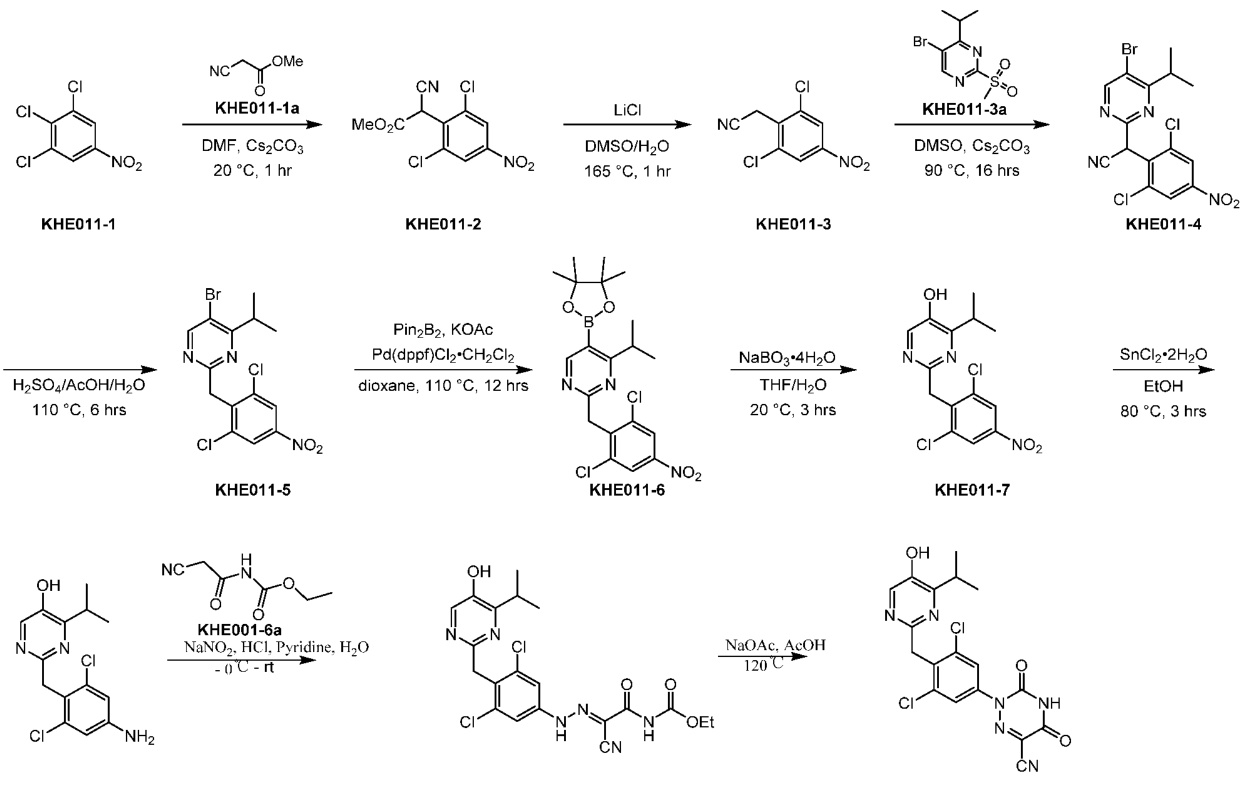


KHE011-1 KHE011-2 KHE011-3 KHE011-4 KHE011-5 KHE011-6 KHE011-7 KHE011-8 KHE011-9 KHE011

**Compound KHE011-2:** compound **KHE011-1** (50.0 g, 221 mmol, 1.00 *eq*), **KHE011-1a** (30.0 g, 265 mmol, 28.3 mL, 1.20 *eq*) and $Cs_2CO_3$ (144 g, 442 mmol, 2.00 *eq*) were added into DMF (250 mL), and stirred at 20°C for 1 hour under the protection of nitrogen.

TLC (petroleum ether/ethyl acetate = 10/1) monitoring showed the raw materials were completely reacted, and a new spot was formed ($R_f$ = 20). The reaction solution was poured into 1N HCl (1 L). Yellow solids were precipitated and filtered. The filter cake was washed with water (200 mL × 3), and then the filter cake was collected, dissolved in ethyl acetate (1 L), dried with anhydrous sodium sulfate and filtered. The filtrate was concentrated to obtain a yellow solid compound KHE011-2 (60.0 g, 198 mmol, yield: 89.7%), which was directly reacted in the next step without purification. MS (ESI) m/z: 289.0 [M+1]$^+$.

**Compound KHE011-3:** compound **KHE001-2** (60.0 g, 198 mmol, 1.00 *eq*) and LiCl (12.6 g, 297 mmol, 1.50 *eq*)

were added into DMSO (150 mL) and water (50 mL), subjected to nitrogen replacement thrice, and reacted at 165°C for 1 hour under the protection of nitrogen. TLC (petroleum ether/ethyl acetate = 5/1) monitoring showed the raw materials were completely reacted, and a new spot was formed ($R_f$ = 0.50). The reaction solution was poured into ice water (1 L), and extracted with ethyl acetate (500 mL × 3). Organic phases were collected, washed with saturated salt solution (200 mL × 3), dried with anhydrous sodium sulfate, filtered and the filtrate was concentrated. The concentrated filtrate was purified through silica gel column chromatography ($SiO_2$, petroleum ether/ethyl acetate = 30/1 to 5/1) to obtain yellow solids **KHE011-3** (22.1 g, 95.7 mmol, yield: 48.3%). MS (ESI) m/z: 231.0 $[M+1]^+$.

**Compound KHE011-4:** compound **KHE011-3** (7.50 g, 32.5 mmol, 1.00 *eq*), **KHE011-3a** (9.06 g, 32.5 mmol, 1.00 *eq*) and $Cs_2CO_3$ (21.2 g, 64.9 mmol, 2.00 *eq*) were added into DMSO (70 mL), and stirred at 90°C for 16 hours. TLC (petroleum ether/ethyl acetate = 5/1) monitoring showed that the raw materials were completely reacted, and a new compound with small polarity ($R_f$ = 0.70) was formed. The reaction solution was poured into saturated salt solution (500 mL), and extracted with ethyl acetate (500 mL × 3). Organic phases were combined, washed with saturated salt solution (200 mL × 3), dried with anhydrous sodium sulfate, filtered, concentrated, and purified through silica gel column chromatography ($SiO_2$, petroleum ether/ethyl acetate = 50/1 to 1/1) to obtain dark grey solids **KHE011-4** (3.95 g, 9.18 mmol, yield: 28.3%). MS (ESI) m/z: 429.0 $[M+1]^+$. **$^1$H NMR** ($CDCl_3$, 400MHz): δ 8.66 (s, 1H), 8.29 (s, 2H), 6.39 (s, 1H), 3.43 (d, *J* = 6.7 Hz, 1H), 1.24 - 1.13 (m, 6H).

**Compound KHE011-5: compound KHE011-4** (2.90 g, 6.74 mmol, 1.0 *eq*) was added into a system of water (12 mL), acetic acid (12 mL) and concentrated sulfuric acid (12 mL), and stirred at 110°C for 6 hours. TLC (petroleum ether/ethyl acetate = 5/1) monitoring showed the raw materials were completely reacted, and a new spot was formed ($R_f$ = 0.47). The reaction solution was diluted with water (200 mL), and extracted with ethyl acetate (80 mL × 3). Organic phases were combined, washed with saturated salt solution (200 mL × 3), dried with anhydrous sodium sulfate, filtered, concentrated, and purified through silica gel column chromatography ($SiO_2$, petroleum ether/ethyl acetate = 50/1 to 1/1) to obtain yellow solids **KHE011-5** (2.50 g, 6.17 mmol, yield: 91.5%). MS (ESI) m/z: 404.0 $[M+1]^+$. **$^1$H NMR** (DMSO-$d_6$, 400MHz): δ 8.79 (s, 1H), 8.35 (s, 2H), 4.62 (s, 2H), 3.38 - 3.33 (m, 1H), 1.11 (d, *J* = 6.8 Hz, 6H).

**Compound KHE011-6:** the operation was the same as the synthesis of compound **KHE007-4** to obtain brown oily substance **KHE011-6** (4.50 g, crude product) which wwas directly reacted in the next step without purification. MS (ESI) m/z: 452.2 $[M+1]^+$.

**Compound KHE011-7:** the operation was the same as the synthesis of compound **KHE007-5** to obtain grey solids **KHE011-7** (680 mg, 1.94 mmol, yield: 31.5%, purity 97.8%) after purification with preparative HPLC. MS (ESI) m/z: 342.0 $[M+1]^+$.

**Compound KHE011-8:** compound **KHE011-7** (680 mg, 1.95 mmol, purity 97.9%, 1.00 *eq*) was dissolved in ethanol (10 mL), added with stannous chloride ($SnCl_2.2H_2O$) (1.32 g, 5.84 mmol, 3.00 *eq*), and stirred at 80°C for 3 hours. LCMS monitoring showed that the raw materials were complete reacted. The reaction solution was added into a system of ethyl acetate (30 mL) and water (80 mL) in batches to separate organic phases. Aqueous phases were extracted with ethyl acetate (30 mL × 2). The organic phases were combined, washed with saturated salt solution (100 mL × 2), dried with anhydrous sodium sulfate, concentrated, and purified with preparative HPLC to obtain yellow solids **KHE011-8** (254 mg, 788 μmol, yield: 40.5%, purity 96.8%). MS (ESI) m/z: 312.0 $[M+1]^+$. **$^1$H NMR** (DMSO-$d_6$, 400MHz): δ 10.19 (s, 1H), 8.08 (s, 1H), 6.80 - 6.68 (m, 2H), 4.24 (s, 2H), 3.31 - 3.23 (m, 1H), 2.08 - 2.06 (m, 1H), 1.10 (d, *J* = 6.8 Hz, 6H).

**Compound KHE011-9:** the operation was the same as the synthesis of compound **KHE007-6,** and yellow solids **KHE011-9** (350 mg, crude product) were obtained, which were directly reacted in the next step without purification. MS (ESI) m/z: 479.0 $[M+1]^+$.

**Compound KHE011:** the operation was the same as the synthesis of compound **KHE007,** and yellow solids **KHE011** (194 mg, 433 μmol,yield: 63.9%, purity 96.6%) were obtained after purification with preparative HPLC. MS (ESI) m/z: 433.0 $[M+1]^+$. **$^1$H NMR** (DMSO-*d6*, 400MHz): δ 13.21 (s, 1H), 10.06 (s, 1H), 8.07 (s, 1H), 7.63 (s, 2H), 4.46 (s, 2H), 3.30 - 3.26 (m, 1H), 1.11 (d, *J* = 6.8 Hz, 6H).

**Example 36 Synthesis of compound KHE012**

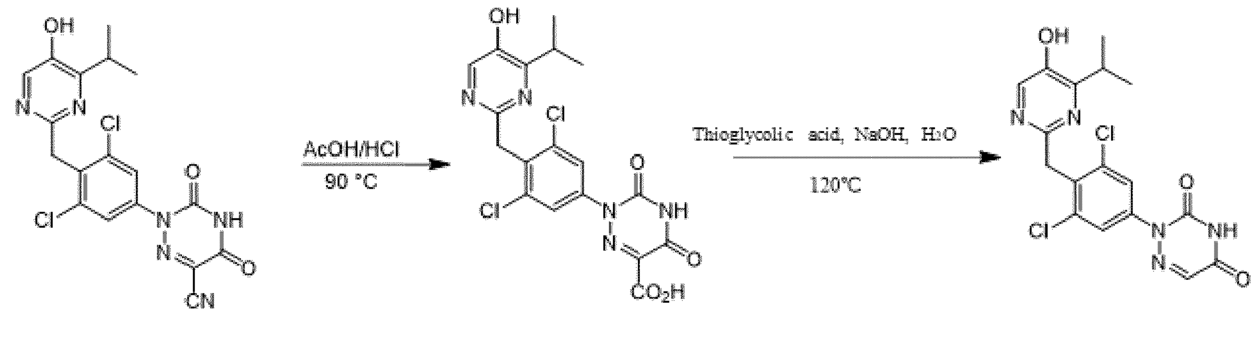


KHE011 KHE012-1 KHE012

**Compound KHE012-1:** the operation was the same as the synthesis of compound **KHE008-1,** and yellow solids **KHE012-1** (120 mg, crude product) were obtained, which were directly reacted in the next step without purification. MS (ESI) m/z: 451.9 [M+1]$^+$.

**Compound KHE012:** the operation was the same as the synthesis of compound **KHE008,** and faint yellow solids **KHE012** (58.0 mg, yield: 51.2%, purity: 97.9%) were obtained after purification with preparative HPLC. MS (ESI) m/z: 408.2 [M+1]+. **$^1$H NMR** (DMSO-$d_6$, 400MHz): δ 12.43 (s, 1H), 10.05 (m, 1H), 8.07 (s, 1H), 7.69 (d, J = 2.0 Hz, 1H), 7.66 (s, 2H), 4.44 (s, 2H), 3.35 - 3.21 (m, 1H), 1.11 (d, J = 6.8 Hz, 6H).

**Example 37 Synthesis of compound KHE013**

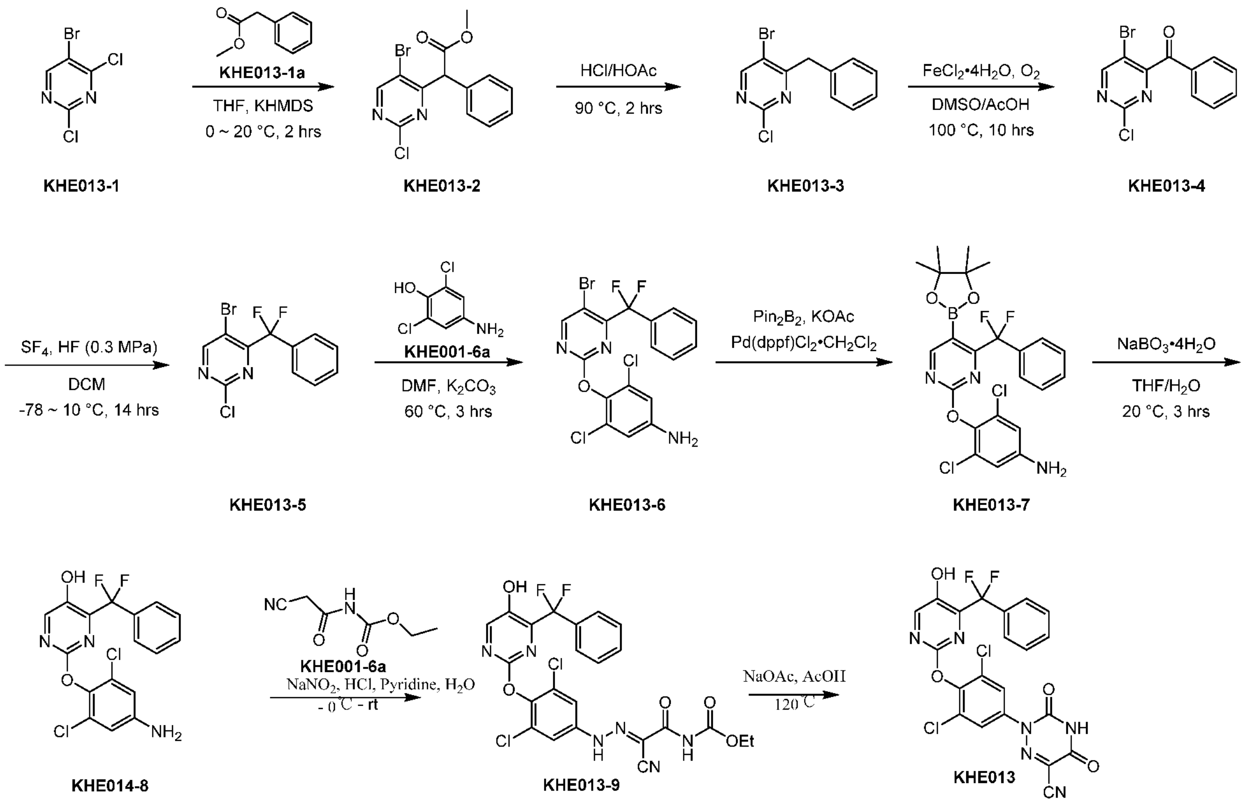


KHE013-1 KHE013-2 KHE013-3 KHE013-4

KHE013-5 KHE013-6 KHE013-7

KHE014-8 KHE013-9 KHE013

**Compound KHE013-2: KHE013-1** (250 g, 1.10 mol, 140 mL, 1.00 eq) and **KHE013-1a** (165 g, 1.10 mol, 154 mL, 1.00 eq) were dissolved in tetrahydrofuran (2L), and slowly added dropwise with KHMDS (1.0 M, 1.15 L, 1.05 eq) at an external temperature of 0°C. After the addition, the reaction solution was heated to 20°C and stirred for 2 hours. TLC (petroleum ether/ethyl acetate = 20/1) monitoring showed the raw materials were completely reacted, and a new spot was formed ($R_f$ = 0.33). The reaction solution was slowly poured into saturated ammonium chloride solution (1 L), and then added with ethyl acetate for extraction (600 mL × 3). Organic phases were combined, washed with saturated salt solution, dried with anhydrous sodium sulfate and concentrated to obtain brown solids **KHE013-2** (360 g, crude product) which were

directly reacted in the next step without purification. MS (ESI) *m/z:* 343.0 [M+H]$^+$.

**Compound KHE013-3: KHE013-2** (330 g, 966 mmol, 1.00 *eq)* was added into a solution system of concentrated hydrochloric acid (165 mL, purity 37%) and acetic acid (660 mL), and stirred for 2 hours at an external temperature of 90°C. TLC (petroleum ether/ethyl acetate = 20/1) monitoring showed that the raw materials were completely reacted, and a new compound was formed ($R_f$ = 0.48). The solvent was removed by evaporation under reduced pressure and the residual oily substance was purified directly by silica gel column chromatography ($SiO_2$, petroleum ether/ethyl acetate = 50/1 to 2/1) to obtain white solids **KHE013-3** (117 g, 412 mmol, yield: 37.6%). **$^1$H NMR** (DMSO-$d_6$, 400MHz): δ 8.93 (br d, *J* = 6.0 Hz, 1H), 7.37 - 7.21 (m, 5H), 4.23 (br d, *J* = 2.4 Hz, 2H).

**Compound KHE013-4: KHE013-3** (30.0 g, 106 mmol, 1.00 *eq*) was dissolved in a mixed solution of DMSO (300 mL) and acetic acid (150 mL), added with $FeCl_2$•$4H_2O$ (2.10 g, 10.6 mmol, 0.10 *eq*), and stirred at an external temperature of 100°C for 10 hours in oxygen. TLC (petroleum ether/ethyl acetate = 20/1) monitoring showed the raw materials were completely reacted, and a new spot was formed ($R_f$ = 0.24). The reaction solution was slowly poured into ethyl acetate (200 mL) and water (800 mL), ethyl acetate layers were separated, and aqueous layers were extracted with ethyl acetate (150 mL × 2). Organic layers were combined, washed with saturated salt solution (500 mL × 2), dried with anhydrous sodium sulfate, filtered, concentrated, and purified by silica gel column chromatography ($SiO_2$, petroleum ether/ethyl acetate = 50/1 to 2/1) to obtain yellow solids **KHE013-4** (16.0 g, 53.8 mmol, yield: 50.8%).

**Compound KHE013-5:** $SF_4$ (14.2 g, 131 mmol, 3.00 *eq*) and HF (8.74 g, 437 mmol, 7.95 mL, 10.0 *eq*) were added to a solution of **KHE013-4** (13.0 g, 43.7 mmol, 1.00 *eq*) in dichloromethane (50 mL) at an external temperature of -78°C. After the addition, the reaction solution was stirred at an external temperature of 10°C and 0.30 MPa for 14 hours. TLC (petroleum ether/ethyl acetate = 5/1) monitoring showed that the reaction was complete, and a new compound was formed ($R_f$ = 0.70). The reaction solution was poured into ethyl acetate (100 mL), and adjusted pH to 7-8.with saturated sodium bicarbonate (300 mL). Organic layers were separated, and aqueous layers were extracted with ethyl acetate (100 mL × 3). The organic layers were combined, washed with saturated salt solution (200 mL × 2), dried with anhydrous sodium sulfate, filtered, concentrated and purified through silica gel column chromatography ($SiO_2$, petroleum ether/ethyl acetate = 1/0 to 1/1) to obtain colorless oily substance **KHE013-5** (10.0 g, 31.3 mmol, yield: 71.6%). MS (ESI) *m*/*z*: 320.8 [M+H]$^+$.**$^1$H NMR:** ($CDCl_3$, 400MHz): δ 8.78 (s, 1H), 7.63 (dd, *J* = 1.2, 7.2 Hz, 2H), 7.54 - 7.41 (m, 3H).**$^{19}$F NMR:** ($CDCl_3$, 400MHz): δ -96.31.

**Compound KHE013-6:** the operation was the same as that of compound **KHE007-3,** and the reaction solution was purified through silica gel column chromatography ($SiO_2$, petroleum ether/ethyl acetate = 50/1 to 2/1) to obtain yellow solids **KHE013-6** (10.2 g, 22.1 mmol, yield: 88.4%). MS (ESI) *m*/*z*: 460.0 [M+H]$^+$.**$^1$H NMR**(DMSO-$d_6$, 400MHz): δ 9.03 (s, 1H), 7.64 - 7.44 (m, 5H), 6.69 (s, 2H), 5.70 (s, 2H).

**Compound KHE013-7:** the operation was the same as the synthesis of compound **KHE007-4** to obtain brown oily substance **KHE013-7** (25.0 g, crude product). The crude product was directly reacted in the next step without purification. MS (ESI) *m*/*z*: 508.2 [M+H]$^+$.

**Compound KHE013-8:** the operation was the same as the synthesis of compound **KHE007-5** to obtain off-white solids **KHE013-8** (3.28 g, 8.09 mmol, yield: 33.3%, purity: 98.2%) after purification with preparative HPLC. MS (ESI) *m*/*z*: 380.0 [M+H]$^+$.**$^1$H NMR**(DMSO-$d_6$, 400MHz):δ 10.65 (s, 1H), 8.29 (s, 1H), 7.57 - 7.43 (m, 5H), 6.67 (s, 2H).**$^{19}$F NMR** (DMSO-$d_6$, 400MHz): δ -95.89.

**Compound KHE013-9:** the operation was the same as the synthesis of compound **KHE007-6,** and yellow solids **KHE013-9** (512 mg, crude product) were obtained, which were directly reacted in the next step without purification. MS (ESI) *m*/*z*: 565.0 [M+H]$^+$.

**Compound KHE013:** the operation was the same as the synthesis of compound **KHE007,** and off-white solids **KHE013** (134 mg, yield: 65.2%, purity: 98.1%) were obtained after purification with preparative HPLC. MS (ESI) *m/z:* 519.1 [M+H]$^+$.

**Example 38 Synthesis of compound KHE014**

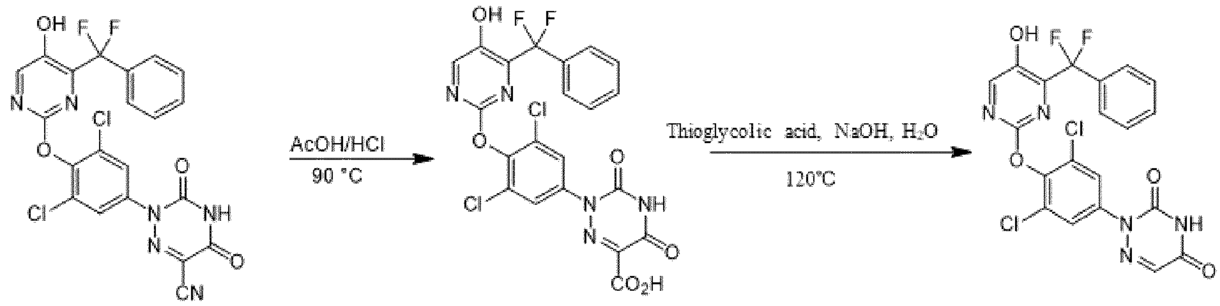


KHE013 KHE014-1 KHE014

**Compound KHE014-1:** the operation was the same as the synthesis of compound **KHE008-1,** and yellow solids **KHE014-1** (63 mg, crude product) were obtained, which were directly reacted in the next step without purification. MS (ESI) m/z: 538.0 [M+1]$^+$.

**Compound KHE014:** the operation was the same as the synthesis of compound **KHE008,** and faint yellow solids **KHE014** (24.0 mg, yield: 23.2% in two steps, and purity: 96.8%) were obtained after purification with preparative HPLC. MS (ESI) m/z: 494.1 [M+1]$^+$.

### Example 39 Synthesis of compound KHE015

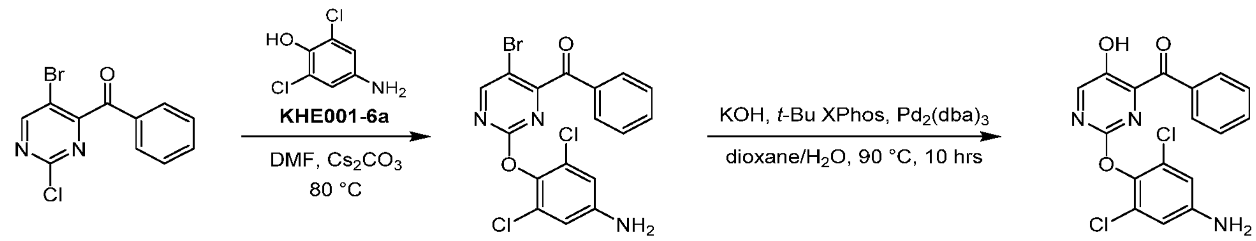


KHE013-4 KHE015-1 KHE015-2

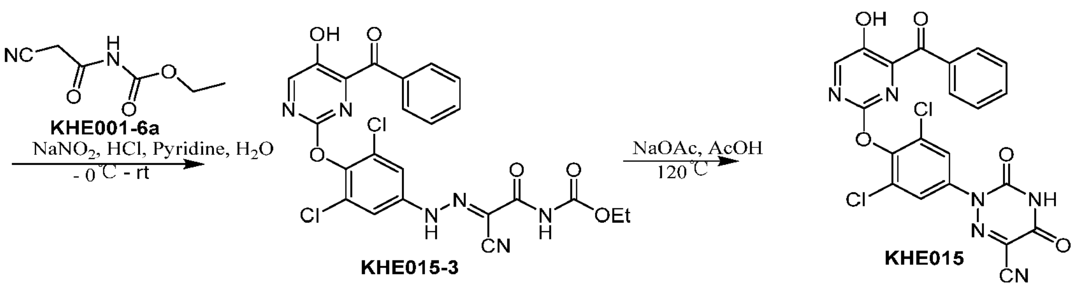


KHE015-3 KHE015

**Compound KHE015-1:** the operation was the same as the synthesis of compound **KHE007-3,** and yellow solids **KHE015-1** (6.00 g, 13.7 mmol, yield: 56.5%) were obtained after purification through silica gel column chromatography ($SiO_2$, petroleum ether/ethyl acetate = 50/1 to 1/1) . **$^1$H NMR** (DMSO-$d_6$, 400MHz): δ 9.09 (s, 1H), 7.85 - 7.75 (m, 3H), 7.62 (t, J = 7.7 Hz, 2H), 6.66 (s, 2H), 5.66 (br s, 2H).

**Compound KHE015-2: compound KHE015-1** (6.00 g, 13.7 mmol, 1.00 *eq),* potassium hydroxide (3.07 g, 54.7 mmol, 4.00 *eq)* and t-Bu Xphos (580 mg, 1.37 mmol, 0.10 *eq)* were added into a system of dioxane (60 mL) and water (15 mL), then added with $Pd_2(dba)_3$ (1.25 g, 1.37 mmol, 0.10 *eq*). After the addition, the solution was placed at an external temperature of 90°C, and stirred for 10 hours. TLC (petroleum ether/ethyl acetate = 3/1) monitoring showed that the reaction was complete, and a new spot was formed ($R_f$ = 0.22). The reaction solution was poured into ethyl acetate (50 mL) and water (200 mL), and stirred for 10 minutes. Organic phases were separated, and aqueous phases were extracted with ethyl acetate (50 mL × 2). The organic phases were combined, washed with saturated salt solution (100 mL × 2), dried with anhydrous sodium sulfate, filtered, concentrated and purified with preparative HPLC to obtain yellow solids **KHE015-2**

(2.45 g, 6.46 mmol, yield: 47.3%, 99.2%). MS (ESI) *m*/*z*: 376.0 [M+H]+. **$^1$H NMR** (DMSO-*$d_6$*, 400MHz): δ 10.62 (s, 1H), 8.44 (s, 1H), 7.83 - 7.77 (m, 2H), 7.72 (s, 1H), 7.56 (s, 2H), 6.64 (s, 2H), 5.84 - 5.29 (m, 2H).

**Compound KHE015-3:** the operation was the same as the synthesis of compound **KHE007-6,** and faint yellow solids **KHE015-3** (625.7 mg, crude product) were obtained, which were directly reacted in the next step without purification. MS (ESI) *m*/*z*: 543.0 [M+H]+.

**Compound KHE015:** the operation was the same as the synthesis of compound **KHE007,** and off-white solids **KHE015** (278.4 g, yield in two steps: 31.1%, 95.9%) were obtained after purification with preparative HPLC. **$^1$H NMR** (DMSO-*$d_6$*, 400MHz): 10.87(s, 1H), 8.49(s, 1H), 7.82(s, 1H), 7.80(d, 1H, J=4.0Hz), 7.77(s, 2H), 7.71-7.74(m, 1H), 7.55-7.59(m, 2H).

**Example 40 Synthesis of compound KHE016**

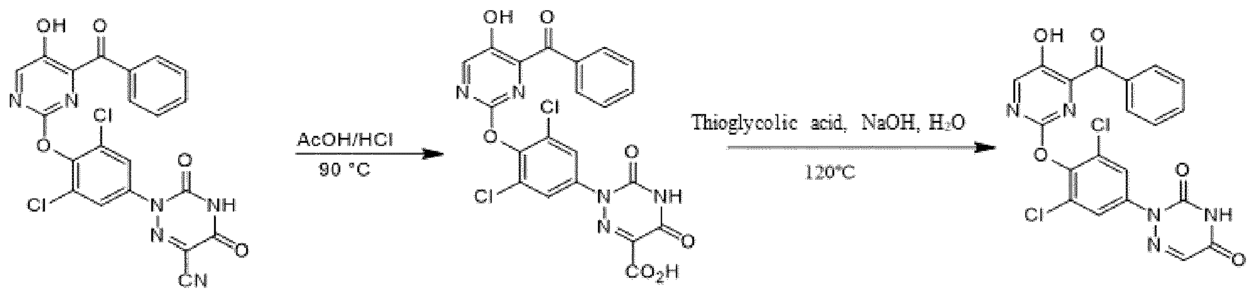


KHE015 KHE016-1 KHE016

**Compound KHE016-1:** the operation was the same as the synthesis of compound **KHE008-1,** and yellow solids **KHE016-1** (74.5 mg, crude product) were obtained, which were directly reacted in the next step without purification. MS (ESI) m/z: 516.0 [M+1]+.

**Compound KHE016:** the operation was the same as the synthesis of compound **KHE008,** and off-white solids **KHE016** (31.2 mg, yield: 26.3% in two steps, and purity 97.2%) were obtained after purification with preparative HPLC. MS (ESI) m/z: 472.0 [M+1]+.

**Example 41 Synthesis of compound KHE017**

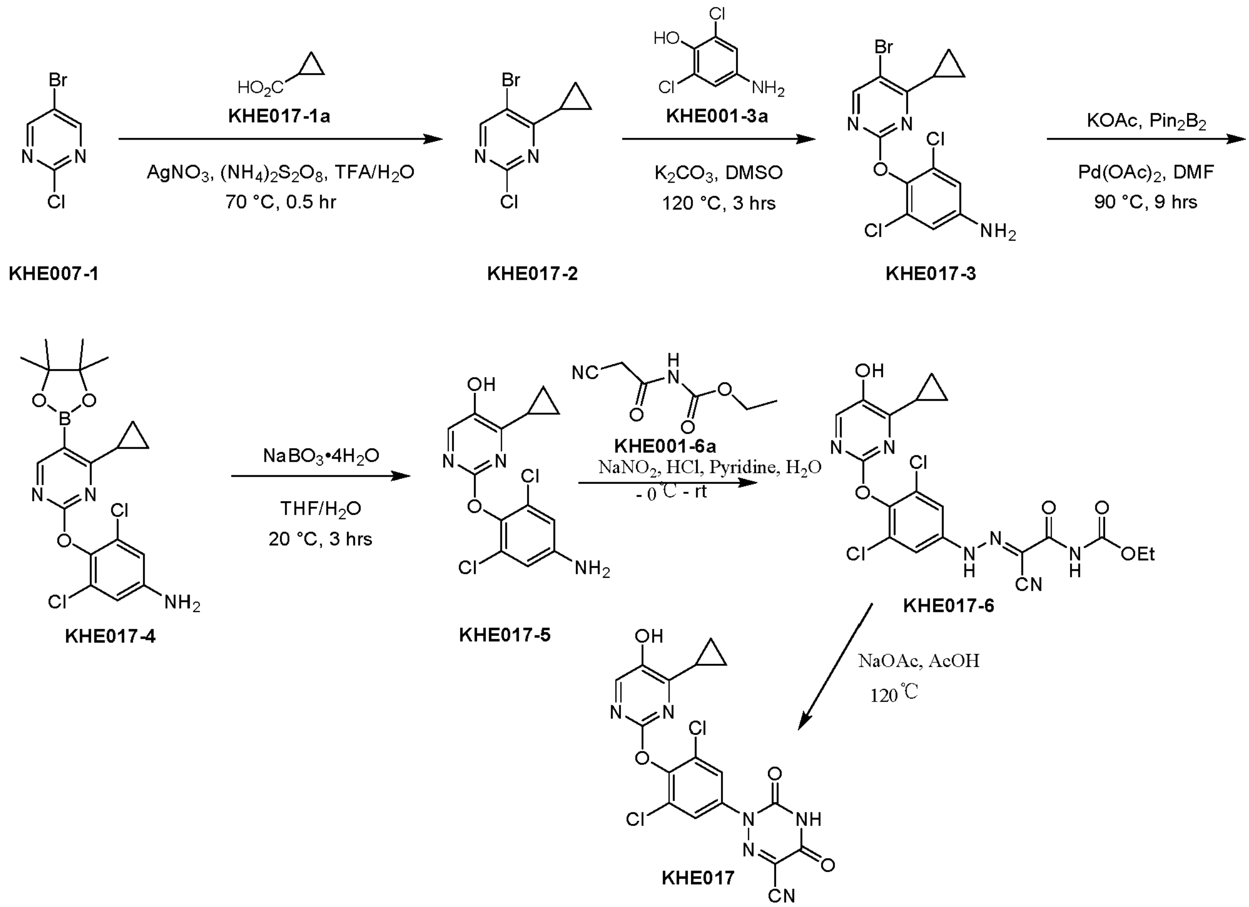


KHE007-1 KHE017-2 KHE017-3 KHE017-4 KHE017-5 KHE017-6 KHE017

**Compound KHE017-2:** the operation was the same as that of compound **KHE007-2,** and the reaction solution was purified through silica gel column chromatography ($SiO_2$, petroleum ether/ethyl acetate = 1/0 to 5/1) to obtain colorless oily substance **KHE017-2** (9.50 g, 40.7 mmol, yield: 78.7%). **$^1$H NMR** ($CDCl_3$, 400MHz): δ 8.46 - 8.39 (m, 1H), 2.39 (tt, *J* = 4.8, 8.0 Hz, 1H), 1.25 - 1.20 (m, 1H), 1.25 - 1.20 (m, 1H), 1.18 - 1.11 (m, 2H).

**Compound KHE017-3:** the operation was the same as that of compound **KHE007-3,** and grey solids **KHE017-3** (13.0 g, crude product) were obtained, which were directly reacted in the next step without purification. MS (ESI) *m/z:* 375.9 $[M+H]^+$.

**Compound KHE017-4:** the operation was the same as that of compound **KHE007-4,** and yellow solids **KHE017-4** (25.0 g, crude product) were obtained, which were directly reacted in the next step without purification. MS (ESI) *m/z*: 422.3 $[M+H]^+$.

**Compound KHE017-5:** the operation was the same as that of compound **KHE007-5,** and yellow solids **KHE017-5** (439 mg, 1.40 mmol, yield: 13.1%, purity: 99.4%) were obtained after purification with preparative HPLC. MS (ESI) m/z: 312.0 $[M+H]^+$.

**$^1$H NMR** (DMSO-$d_6$, 400MHz): δ 9.89 (s, 1H), 7.89 (s, 1H), 6.64 (s, 1H), 6.70 - 6.60 (s, 1H), 5.52 (s, 2H), 2.39 - 2.25 (m, 1H), 1.06 - 0.93 (m, 2H), 0.88 - 0.76 (m, 2H).

**Compound KHE017-6:** the operation was the same as that of compound **KHE007-6,** and about 451.6 mg of tangerine solids **KHE017-6** were obtained, which were directly reacted in the next step without purification. MS (ESI) m/z: 479.1 $[M+H]^+$.

**Compound KHE017:** the operation was the same as that of compound **KHE007,** and 200 mg were taken to obtain about 56.2 mg of white solids **KHE017** by purification with preparative HPLC. MS (ESI) m/z: 433.0 $[M+H]^+$. **$^1$H NMR** (DMSO-$d_6$, 400MHz): δ13.27 (s, 1H), 10.12 (s, 1H), 7.94 (s, 1H), 7.74 (s, 2H), 2.28 (m, 1H), 1.04 (m, 2H), 0.86 (m, 2H).

**Example 42 Synthesis of compound KHE018**

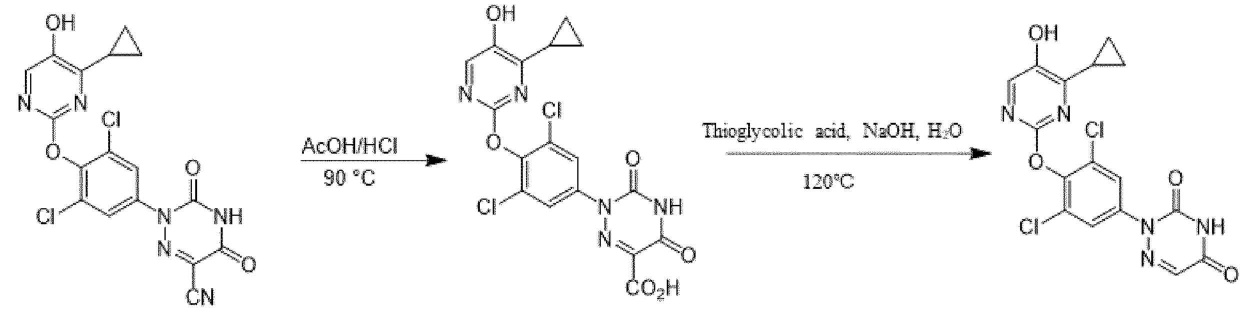


KHE017 KHE018-1 KHE018

**Compound KHE018-1:** the operation was the same as the synthesis of compound **KHE008-1,** and yellow-brown solids **KHE018-1** (205.7 mg, crude product) were obtained, which were directly reacted in the next step without purification. MS (ESI) m/z: 452.0 $[M+H]^+$.

**Compound KHE018:** the operation was the same as the synthesis of compound **KHE008,** and off-white solids **KHE018** (20 mg, purity 96.81%) were obtained after purification with preparative HPLC. MS (ESI) m/z: 472.0 $[M+H]^+$. **$^1$H NMR** (DMSO-$d_6$, 400MHz): 12.46(s, 1H), 10.09(s, 1H), 8.25(s, 1H), 7.75(s, 2H), 7.68(s, 1H), 2.32-2.39(m, 1H), 1.02-1.07(m, 2H), 0.85-0.90(m, 2H).

**Example 43 Synthesis of compound KHE019**

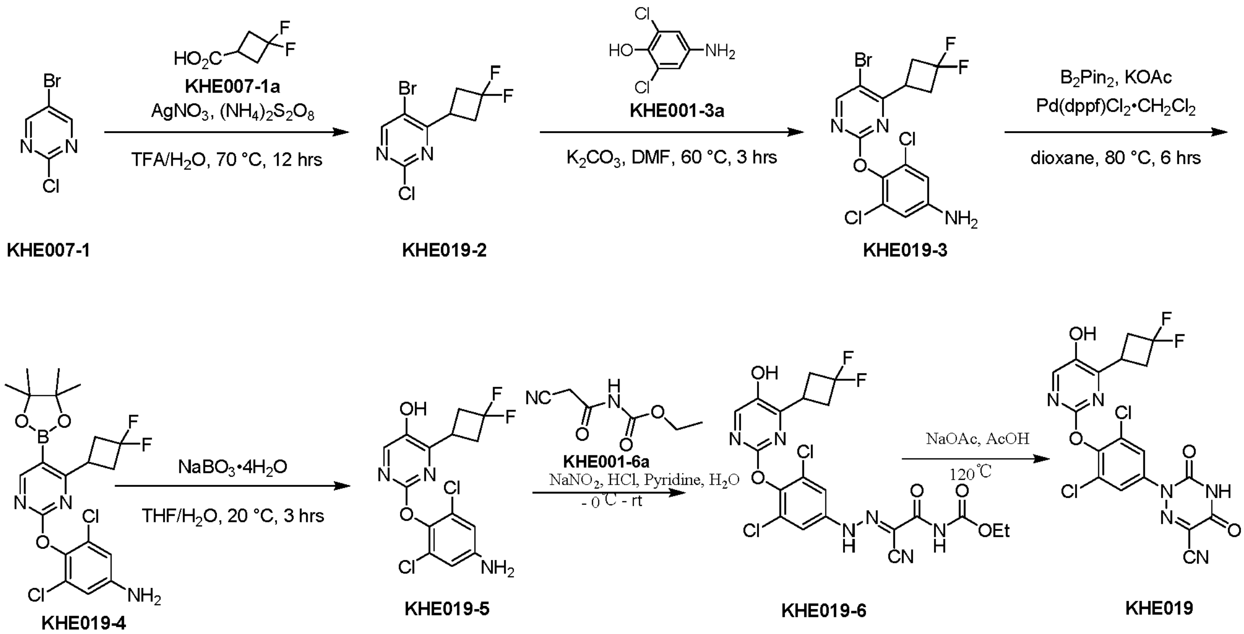


KHE019-4 KHE019-5 KHE019-6 KHE019

**Compound KHE019-2:** the operation was the same as that of **KHE007-2,** and yellow solids **KHE019-2** (9.00 g, 31.7 mmol, yield: 68.2%) were obtained after purification with silica gel column chromatography ($SiO_2$, petroleum ether/ethyl acetate = 50/1 to 10/1). MS (ESI) m/z: 283.0 $[M+H]^+$. **$^1$H NMR** ($CDCl_3$, 400 MHz): δ 8.62 (s, 1H), 3.88 - 3.60 (m, 1H), 3.11 - 2.90 (m, 4H).

**Compound KHE019-3:** the operation was the same as that of **KHE007-3,** and yellow solids **KHE019-3** (5.20 g, 12.2 mmol, yield: 86.7%) were obtained after purification with silica gel column chromatography ($SiO_2$, petroleum ether/ethyl acetate = 50/1 to 10/1). MS (ESI) *m/z*: 424.0$[M+H]^+$. **$^1$H NMR** (CDCl3, 400MHz): δ 8.50 (s, 1H), 6.69 (s, 2H), 4.06 - 3.75 (m, 2H), 3.75 - 3.64 (m, 1H), 2.91 (td, *J* = 8.4, 16.4 Hz, 4H).

**Compound KHE019-4:** the operation was the same as that of **KHE007-4,** and brown oily substances **KHE019-4** (5.80 g, crude product) were obtained, which were directly reacted in the next step without purification. MS (ESI) *m/z*: 472.1 $[M+H]^+$.

**Compound KHE019-5:** the operation was the same as **KHE007-5,** and white solids **KHE019-5** (1.1 g, 2.96

mmol, yield: 24.0%, purity 97.4%) were obtained after purification with preparative HPLC. MS (ESI) m/z: 362.0 [M+H]+. **$^1$H NMR** (DMSO-$d_6$, 400MHz): δ 10.13 (s, 1H), 8.01 (s, 1H), 6.67 (s, 2H), 5.55 (s, 2H), 3.62 (dquin, *J* = 2.8, 8.8 Hz, 1H), 2.91 - 2.73 (m, 4H). **$^{19}$F NMR** (DMSO-$d_6$, 400MHz): δ -80.48 (d, *J* = 12.8 Hz, 1F), -95.69 (d, *J* = 12.8 Hz, 1F).

**Compound KHE019-6:** the operation was the same as that of compound **KHE007-6,** and about 324 mg of tangerine solids **KHE019-6** were obtained, which were directly reacted in the next step without purification. MS (ESI) m/z: 529.0 [M+H]+.

**Compound KHE019:** the operation was the same as that of compound **KHE007,** and 120 mg were taken to obtain about 43.7 mg of white solids **KHE019** by purification with preparative HPLC. MS (ESI) m/z: 483.0 [M+H]+. **$^1$H NMR** (DMSO-$d_6$, 400MHz): δ 13.25 (s, 1H), 10.377 (s, 1H), 8.073 (s, 1H), 7.779 (s, 2H), 3.653-3.675 (m, 1H), 2.836 - 2.948 (m, 4H).

**Example 44 Synthesis of compound KHE020**

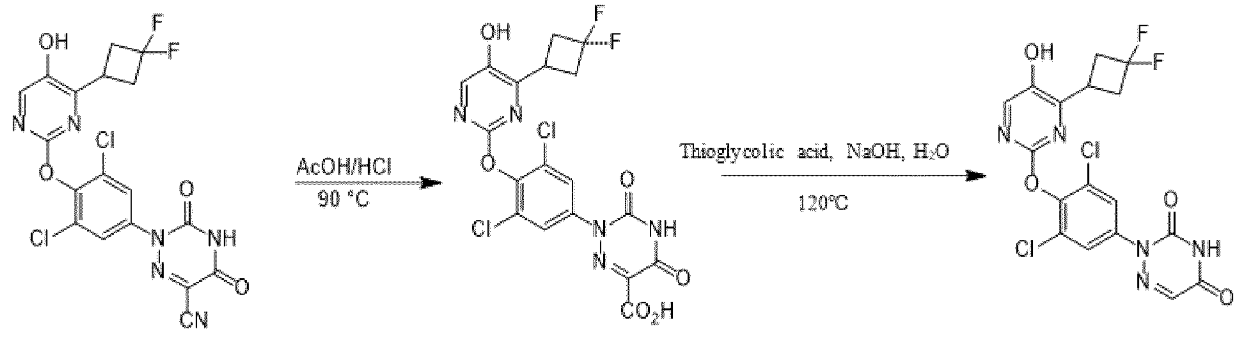


KHE019 KHE020-1 KHE020

**Compound KHE020-1:** the operation was the same as the synthesis of compound **KHE008-1,** and brown solids **KHE020-1** (157 mg, crude product) were obtained, which were directly reacted in the next step without purification. MS (ESI) m/z: 502.0 [M+H]+.

**Compound KHE020:** the operation was the same as the synthesis of compound **KHE008,** and off-white solids **KHE020** (15.4 mg, purity 97.23%) were obtained after purification with preparative HPLC. MS (ESI) m/z: 458.0 [M+H]+. **$^1$H NMR** (DMSO-$d_6$, 400MHz): δ12.480 (s, 1H), 10.341 (s, 1H), 8.073 (s, 1H), 7.722 (s, 2H), 7.718 (s, 1H), 3.343 - 3.665 (m, 1H), 2.841-2.946 (m, 4H).

**Example 45 Synthesis of compound KHE021**

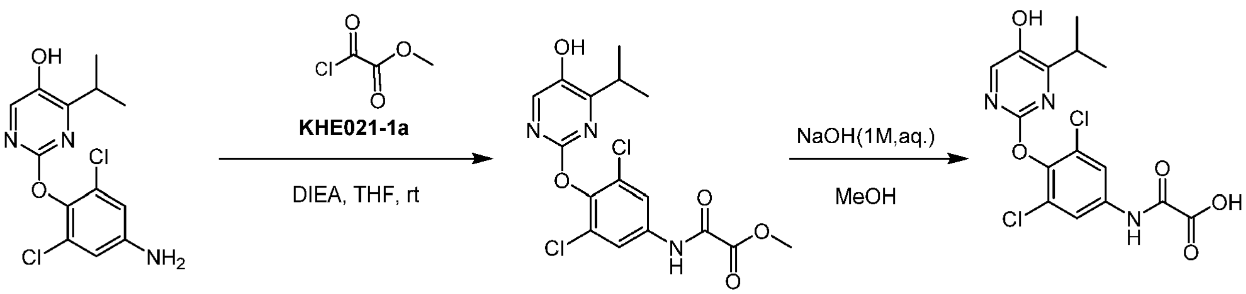


**KHE005-2** **KHE021-1** **KHE021**

**Compound KHE021-1:** compound **KHE005-2** (0.1095 g, 3.498 mmol, 1.00 eq) was dissolved in THF (10 ml), then **KHE021-1a** (0.4736 g, 3.882 mmol, 12 eq) and DIEA (0.9965 g, 7.665 mmol, 24 eq) were added in the reaction system, and stirred at room temperature overnight. TLC monitoring showed that a new spot was formed. The reaction solution was diluted with water and extracted with ethyl acetate (20 mL × 3). Organic phases were combined, dried with anhydrous sodium sulfate, filtered, and dried by rotary evaporation to obtain residues. The residues were purified by TLC plate to obtain yellow solids **KHE021-1** (80 mg, yield: 57.3%), and sampled and use LCMS for determining product signal.

MS (ESI) m/z: 400.0 $[M+H]^+$.

**Compound KHE021:** compound **KHE021-1** (80 mg, 0.2 mmol) was dissolved in methanol (6 mL), and added with sodium hydroxide solution (1 ml, l M, aq). The reaction solution was stirred overnight at room temperature. TLC monitoring showed that the reaction was complete. The reaction solution was diluted with 10 ml of water, and the organic solvent in the reaction was directly dried by rotary evaporation. The pH was adjusted to 3-4, and then the reaction solution was extracted with ethyl acetate (20 mL × 3). Organic phases were combined, dried with anhydrous sodium sulfate and dried by rotary evaporation to obtain residues. The residues were purified with chromatoplate to obtain white solid **compound KHE021** (12 mg, yield: 15.5%). MS (ESI) m/z: 386.0 $[M+H]^+$. **$^1$H NMR** (DMSO-$d_6$, 400MHz): δ 9.87 (s, 1H), 7.94 (s, 1H), 6.65 (s, 2H), 5.53 (s, 2H), 3.23 - 3.28 (m, 1H), 1.11 (d, *J* = 6.8 Hz, 6H).

**Example 46 Synthesis of compound KHE022**

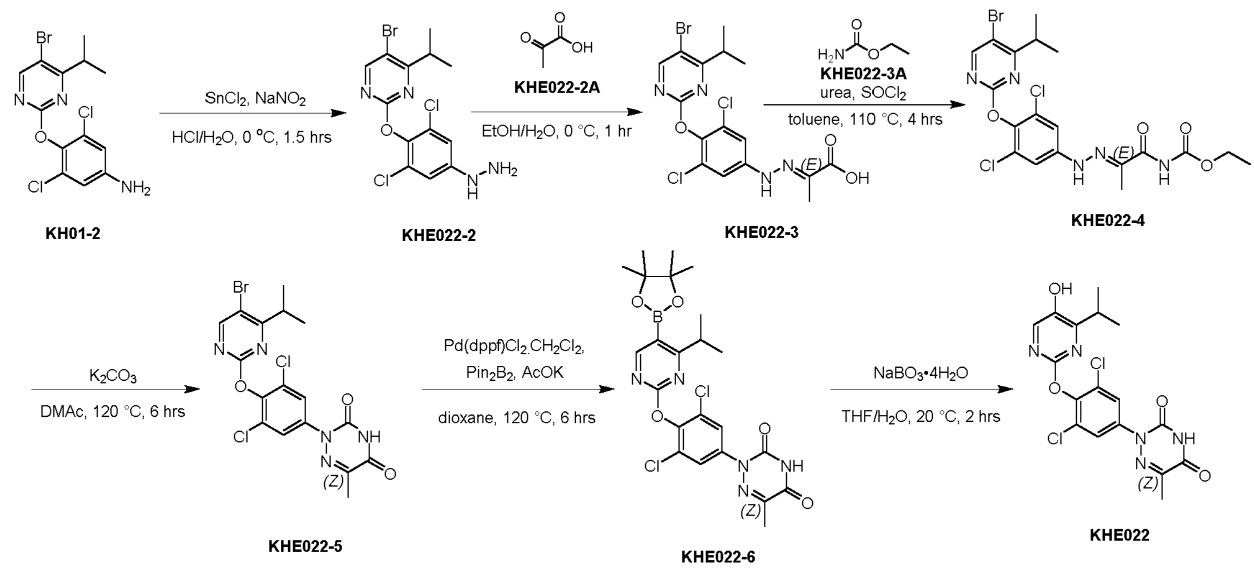


**Compound KHE022-2: KH01-2** (120 g, 318 mmol, 1.0 eq) was added into hydrochloric acid solution (12 M, 358 mL, 13.5 eq), stirred for 30 minutes, and cooled to 0°C. Sodium nitrite (24.1 g, 350 mmol, 1.1 eq) was dissolved in 50 mL of water, slowly added dropwise into the foregoing solution, and after the addition, continuously stirred at 0°C for 1 hour. TLC monitoring (petroleum ether/ethyl acetate = 5/1) showed that the raw materials were completely reacted, and a new spot was formed ($R_f$= 0.80). Stannous chloride (215 g, 954 mmol, 3.0 eq) was dissolved in hydrochloric acid (12 M, 493 mL, 18.6 eq), dropwise added in the above-mentioned reaction solution at 0°C, and after the addition, continuously stirred at this temperature for 1 hour. LCMS monitoring showed that the raw materials were completely reacted, and a new target product was formed. The reaction solution was filter, and the filter cake was collected to obtain crude product of yellow solids **KHE022-2** (80.0 g, 204 mmol, yield: 64.1%) without further purification. MS (ESI) m/z = 392.9 $[M+1]^+$.

**Compound KHE022-3:** compound **KHE022-2** (80.0 g, 204 mmol, 1.0 eq) and **KHE022-2A** (32.3 g, 367 mmol, 25.8 mL, 1.8 eq) were added into 480 mL of ethanol and 1.5 L of water, and stirred at 0°C for 1 hour. LC-MS monitoring showed that **KHE022-2** was completely reacted, a target product was formed. The reaction solution was extracted twice with ethyl acetate (2L × 2); ethyl acetate layers were combined, and washed with 500 mL of saturated salt solution, dried with anhydrous sodium sulfate, filtered, and concentrated to obtain yellow solids **KHE022-3** (26.5 g, 57.34 mmol, yield: 28.1%), which were directly reacted in the next step without purification. MS (ESI) m/z = 462.9 $[M+1]^+$. $^1$H NMR (DMSO-d6, 400MHz): δ 1H NMR: DMSO-d6, 400MHz δ 12.16 (s, 1H), 10.04 (s, 1H), 8.75 (s, 1H), 7.56 (s, 2H), 2.06 (s, 3H), 1.24 - 1.14 (m, 6H).

**Compound KHE022-4:** compound **KHE022-3** (10.0 g, 21.64 mmol, 1 eq) was added into 2.0 L of toluene, added with thionyl chloride (7.72 g, 64.92 mmol, 4.71 mL, 3.0 eq) at room temperature, then heated to 110°C, and stirred for 2 hours. The reaction solution was dried by rotary evaporation to remove excess thionyl chloride, then added with 2.0 L of toluene for dissolution, and then added with urea (5.78 g, 64.9 mmol, 3.0 eq) and **KHE022-3A** (2.89 g, 32.4 mmol, 1.5 eq), and stirred at 110°C for 2 hours. LC-MS monitoring showed that the raw materials were completely reacted, and a target product was formed. The reaction solution was dried by rotary evaporation, added with 1 L of water and 1 L of ethyl acetate, and stirred at room temperature for 30 minutes. After that, organic phases were separated, and aqueous phases were extracted once with 1 L of ethyl acetate. The organic phases were combined, dried with anhydrous sodium sulfate, filtered

and concentrated. The residues were purified with reversed-phase flash (0.1% TFA) to obtain yellow solids **KHE022-4** (1.00 g, 1.45 mmol, yield: 3.35%, purity 71.4%). MS (ESI) m/z = 534.0 [M+1]+.

**Compound KHE022-5: KHE022-4** (1.00 g, 1.88 mmol, 1.0 eq) was dissolved in N,N-dimethylacetamide (15 mL), added with potassium carbonate (777 mg, 5.63 mmol, 3.0 eq), and stirred at 120°C for 6 hours. LC-MS monitoring showed that the raw materials were completely reacted, and a target product was formed. The reaction solution was added with 30 mL of water, and extracted with ethyl acetate (30 mL × 2). Organic phases were combined, dried with anhydrous sodium sulfate, filtered and concentrated. The residues were purified with HPLC to obtain yellow solids **KHE022-5** (400 mg, 821 μmol,yield: 43.7%). MS (ESI) m/z = 487.9 [M+1]+.

**Compound KHE022-6: KHE022-5** (400 mg, 821 μmol, 1.0 eq) and $Pin_2B_2$ (521 mg, 2.05 mmol, 2.5 eq) were dissolved in dioxane (4.0 mL), added with potassium acetate (241 mg, 2.46 mmol, 3.0 eq) and $Pd(dppf)Cl_2$•$CH_2Cl_2$ (33.5 mg, 41.0 μmol, 0.05 eq), and stirred at 120°C for 6 hours. LC-MS monitoring showed that the raw materials were completely reacted. The reaction solution was filtered. The filtrate was collected, added with 5 mL of ethyl acetate, and then washed with 5 mL of water and 5 mL of saturated salt solution in turn. Organic phases were collected, dried with anhydrous sodium sulfate, filtered and concentrated. The residues were purified with preparative HPLC to obtain yellow solids **KHE022-6** (100 mg, 187 μmol, yield: 22.8%). MS (ESI) m/z = 534.1 [M+1]+.

**Compound KHE022: KHE022-6** (90.0 mg, 168 μmol, 1 eq) was added into a mixed solution of tetrahydrofuran (1.0 mL) and water (1.0 mL), added with sodium perborate ($NaBO_3$•$4H_2O$) (150 mg, 673.91 μmol, 4.0 eq), and stirred at room temperature for 2 hours. LC-MS monitoring showed that the raw materials were completely reacted, and a target product was formed. The reaction solution was added with 5 mL of water, and extracted with ethyl acetate (5.0 mL × 2). Organic phases were combined, dried with anhydrous sodium sulfate, filtered and concentrated. The residues were purified with preparative HPLC to obtain yellow solids **KHE022** (18.0 mg, 42. 17μmol, yield: 25.0%, purity 99.4%). MS (ESI) m/z = 424.0 [M+1]+. $^1$H NMR (DMSO-d6, 400MHz): δ 12.40 (s 1H), 10.07 (s, 1H), 8.01 (s, 1H), 7.78 (s, 2H), 3.32 - 3.26 (m, 1H), 2.17 (s, 3H), 1.16 - 1.10 (m, 6H).

## Example 47 Synthesis of compound KHE023

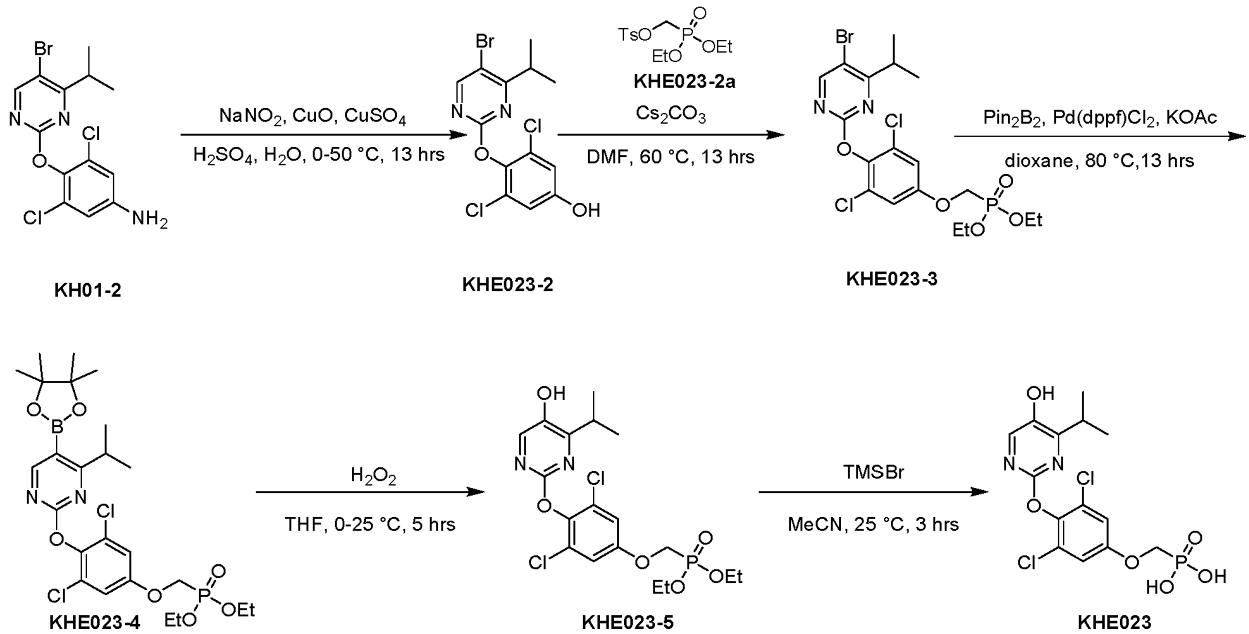

KH01-2 KHE023-2 KHE023-3
KHE023-4 KHE023-5 KHE023

**Compound KHE023-2: KH01-2** (40.0 g, 106 mmol, 1.00 eq) was added in concentrated sulfuric acid (60.0 mL) and water (25.0 mL), dropwise added with sodium nitrite solution (7.32 g, 106 mmol, 1.00 eq, dissolved in 150 mL of water) at 0°C, and after the addition, continuously stirred at this temperature for 1 hour. Copper sulfate solution (253 g, 1.59 mol, 244 mL, 15.0 eq, dissolved in 150 mL of water) was dropwise added in the above-mentioned reaction solution, and meanwhile, added with cupric oxide (8.44 g, 106.08 mmol, 1.34 mL, 1.00 eq). The reaction solution was heated to 50°C, and stirred for 2 hours. LC-MS monitoring showed that a target product was formed. The reaction solution was filtered. The filtrate was added with 800 mL of ethyl acetate, and washed with 200 mL of saturated salt solution. Organic phases were collected, dried with anhydrous sodium sulfate, filtered, concentrated, and purified with reversed-phase HPLC to obtain yellow solids **KHE023-2** (7.60 g, 20.0 mmol, yield: 18.96%). MS (ESI) m/z = 378.9 [M+1]+. $^1$H NMR (DMSO-d6, 400MHz): δ

10.36 (s, 1H), 8.73 (s, 1H), 6.96 (s, 2H), 3.40 - 3.36 (m, 1H),, 1.12 (d, J = 6.8 Hz, 6H).

**Compound KHE023-3: KHE023-2** (4.50 g, 11.9 mmol, 1.00 eq) was dissolved in DMF (50.0 mL), added with KHE023-2a (4.60 g, 14.2 mmol, 3.68 mL, 1.20 eq) and cesium carbonate (4.65 g, 14.2 mmol, 1.20 eq), and stirred at 60°C for 13 hours. LC-MS monitoring showed that a target product was formed. The reactant was poured into 50 mL of water, and extracted with ethyl acetate (50 mL × 2). Organic phases were combined, washed with saturated salt solution (50 mL × 2), dried with anhydrous sodium sulfate, filtered and concentrated. The residues were purified through column chromatography (petroleum ether/ethyl acetate = 50/1 to 2/1) to obtain yellow oily substances **KHE023-3** (3.80 g, 7.19 mmol, yield: 60.4%). MS (ESI) m/z = 529.0 $[M+1]^+$. $^1H$ NMR (DMSO-d6, 400MHz): δ 8.73 (s, 1H), 7.36 (s, 2H), 4.57 (d, J = 9.6 Hz, 2H), 4.18 - 4.05 (m, 4H), 3.37 (m, 1H), 1.25 (t, J = 7.2 Hz, 6H), 1.12 (d, J = 6.8 Hz, 6H).

**Compound KHE023-4: KHE023-3** (3.50 g, 6.63 mmol, 1.00 eq) was dissolved in dioxane (85.0 mL), added with potassium acetate (1.30 g, 13.2 mmol, 2.00 eq), $Pin_2B_2$ (3.37 g, 13.2 mmol, 2.00 eq) and $Pd(dppf)Cl_2$ (484 mg, 662 μmol, 0.10 eq), and stirred at 80°C for 13 hours under the protection of nitrogen. LC-MS monitoring showed that a target product was formed. The reaction solution was poured into 10 mL of water, and extracted with ethyl acetate (10 mL × 2). Organic phases were combined, washed with saturated salt solution (20 mL), dried with anhydrous sodium sulfate, filtered and concentrated to obtain crude product of yellow oily substances **KHE023-4** (2.00 g), which were directly reacted in the next step without purification. MS (ESI) m/z = 575.1 $[M+1]^+$.

**Compound KHE023-5: KHE023-4** (2.00 g, 3.48 mmol, 1.00 eq) was dissolved with tetrahydrofuran (40.0 mL), added with hydrogen peroxide (3.94 g, 34.7 mmol, 3.34 mL, purity 30.0%, 10.0 eq) at 0°C, and then heated to room temperature and stirred for 5 hours. LC-MS monitoring showed that the raw materials were completely reacted, and a target product was formed. The reaction solution was poured into saturated sodium sulphite (50 ml), stirred for 10 minutes, and then extracted with ethyl acetate (50 mL × 2). Organic phases were collected, washed with saturated salt solution (50 mL), dried with anhydrous sodium sulfate, filtered and concentrated. The residues were purified with preparative HPLC to obtain brown oily substances KHE023-5 (0.80 g, 1.72 mmol, yield: 49.4%). MS (ESI) m/z = 465.2 $[M+1]^+$. $^1H$ NMR (DMSO-d6, 400MHz): δ 10.0 (br s, 1H), 7.98 (s, 1H), 7.32 (s, 2H), 4.57 (d, J = 9.6 Hz, 2H), 4.13 (quin, J = 7.2 Hz, 4H), 3.31 - 3.25 (m, 1H), 1.27 (t, J = 7.2 Hz, 6H), 1.11 (d, J = 6.8 Hz, 6H).

**Compound KHE023: KHE023-5** (0.65g, 1.40 mmol, 1.00 eq) was dissolved in 20 mL of acetonitrile, added with trimethylbromosilane (2.6g, 16.8mmol, 2.2 mL, 12.0 eq), and stirred at room temperature for 3 hours. LC-MS monitoring showed that the raw materials disappeared, and a target product was formed. The reactant was poured into 20 mL of methanol, stirred for 30 minutes, and dried by rotary evaporation under reduced pressure. The residues were purified with preparative HPLC to obtain white solids **KHE023** (0.5 g, 1.22 mmol, yield: 87.4%). MS (ESI) m/z = 409.0 $[M+H]^+$. $^1H$ NMR (DMSO-d6, 400MHz): δ 9.96 (br s, 1H), 7.97 (s, 1H), 7.25 (s, 2H), 4.21 (d, J = 10.0 Hz, 2H), 3.30 (br d, J = 6.8 Hz, 1H), 1.11 (d, J = 6.8 Hz, 6H).

**Example 48 In vitro binding experiment of TRα, or TRβ**

In vitro analysis of the compound agonism on TRα or TRβ was performed by peptide recruitment experiment based on time-resolved fluorescence resonance energy transfer assay. In the experiment, a Europium-anti-GST antibody (Cisbio, 61GSTKLB), a biotin-SRC2-2 co-activated peptide (Sangon Biotech), streptavidin-d2 (Cisbio, 610SADAB), RXRα (Pharmaron), and TRα-LBD (Invitrogen, PV4762), or TRβ-LBD (Invitrogen, PV4764) with a GST tag were used. The Europium-anti-GST antibody indirectly labeled the TRα-LBD, or the TRβ-LBD by binding to the GST tag. The Streptavidin-d2 (Cisbio, 610SADAB) indirectly labeled the SRC2-2 co-activated peptide by binding to a biotin tag. In the case of existence of the RXRα, the TRα-LBD, or the TRβ-LBD could form a heterodimer TRα-LBD/RXRα, or TRβ-LBD/RXRα with the RXRα respectively. An agonis bound to the TRα-LBD/RXRα, or the TRβ-LBD/RXRα and led to a conformational change of the TRα-LBD, or the TRβ-LBD, thus improving a recruitment ability of the heterodimer to the SRC2-2 co-activated peptide. Meanwhile, since a distance between the d2-labeled SRC2-2 co-activated peptide and the Europium-anti-GST antibody was reduced, a TR-FRET signal was increased.
Depending on the effect of the compound on TRα or TRβ activity at different concentrations, the agonism of the compound can be evaluated

Operation steps:

a. 6 mM solution of the positive reference compound (MGL-3196) and compounds to be tested (100X) were prepared in dimethyl sulfoxide (DMSO), and 100% DMSO was used as the negative control
b. The 6 mM (100X) solution of the positive reference compound (MGL-3196) or the compound to be tested is diluted sequentially with 100% dimethyl sulfoxide at a 1:3 ratio for a total of 10 concentrations, and transferred to a 96-well plate
c. A 4X compound subjected to concentration gradient dilution was prepared with a 1X reaction buffer (50 mM HEPES (pH7.0), 50 mM KF, 1 mM DTT, 0.05% NP-40, 0.2% BSA).
d. 5 μl of 4X compound subjected to concentration gradient dilution was added into a 384-well experimental plate.

e. 4X TRαLBD and 4X RXRα were prepared with the 1X reaction buffer (50 mM HEPES (pH7.0), 50 mM KF, 1 mM DTT, 0.05% NP-40, 0.2% BSA).
f. 5 μl of 4X TRαLBD and 4X RXRα were added into the 384-well experimental plate.
g. a mixture solution of the 2X biotin-SRC2-2, the 2X Europium-anti-GST and the 2X streptavidin-d2 was prepared with the 1X reaction buffer (50 mM HEPES (pH7.0), 50 mM KF, 1 mM DTT, 0.05% NP-40, 0.2% BSA).
h. 10 μl of 2X mixture solution (in step g) was added into the 384-well experimental plate.
i. Incubation was carried out at room temperature in the dark for 4 hours.
j. Fluorescence signal values at 665 nm and 615 nm wavelengths in each well of the 384-well experimental plate were recorded by an Envision 2104 (PerkinElmer) microplate reader, and the fluorescence signal ratio of 665 nm/615 nm was calculated.

Data analysis:

The relative ratio of each well was calculated: (a ratio of 665 nm/615 nm - a ratio blank), and the activity percentage (% Activity) was calculated as follows:

$$\%\ Activity = \left[\frac{Ratio_{cmpd} - \overline{Ratio}_{Vehicle}}{\overline{Ratio}_{Positive} - \overline{Ratio}_{Vehicle}}\right] * 100$$

wherein:

$Ratio_{positive}$ was a relative ratio of the positive control in the whole plate;
$Ratio_{vehicle}$ was a relative ratio of the negative control in the whole plate; and
$Ratio_{empd}$ was a relative ratio of the compound in the whole plate.

$EC_{50}$ was calculated by fitting % activity values and log of compound concentrations to nonlinear regression with Graphpad 8.0.

$$Y = Bottom + (Top - Bottom)/(1 + 10\text{^}((LogEC_{50} - X) \times HillSlope))$$

X: Log of compound concentration; Y: % Activity.
Specific test data are shown in Table 1.

Table 1 Results of binding experiment of TRα, or TRβ in vitro

| Compound No. | TRα ($EC_{50}$ μM) | TRβ ($EC_{50}$ μM) |
|---|---|---|
| **MGL3196** | 2.223 | 0.163 |
| **KH02** | - | 1.056 |
| **KH03** | 0.144 | 0.031 |
| **KH04** | 0.133 | 0.113 |
| **KH06** | 0.094 | 0.007 |
| **KH07** | 0.31 | 0.048 |
| **KH09** | 1.884 | 0.066 |
| **KH10** | 1.47 | 0.054 |
| **KH13** | 0.132 | 0.054 |
| **KH14** | 0.102 | 0.105 |
| **KH15** | 0.194 | 0.014 |
| **KH16** | 0.106 | 0.012 |
| **KH17** | 0.385 | 0.021 |
| **KH18** | 0.188 | 0.011 |
| **KHE001** | 0.418 | 0.007 |

(continued)

| Compound No. | TRα ($EC_{50}$ μM) | TRβ ($EC_{50}$ μM) |
|---|---|---|
| **KHE002** | 0.272 | 0.003 |
| **KHE003** | 0.573 | 0.746 |
| **KHE007** | 5.123 | 0.089 |
| **KHE008** | 4.545 | 0.067 |
| **KHE009** | 0.258 | 0.04 |
| **KHE010** | 0.22 | 0.029 |
| **KHE011** | 0.014 | 0.001 |
| **KHE017** | 0.737 | 0.077 |
| **KHE018** | 0.346 | 0.04 |
| **KHE019** | 2.375 | 0.254 |
| **KHE021** | 0.008 | 0.001 |

The positive compound MGL3196 was prepared according to the method described in CN101228135B.

**Example 49 In vitro TRα, or TRβ cell transfection experiment**

The experiment was designed to evaluate the agonistic effect of the compound on TRα or the TRβ. Coding sequences of TRα-LBD, or TRβ-LBD and RXRα-LBD were inserted into a pBIND plasmid (Promega, E1581). Two expression vectors and reporter vectors (pGL4.35 carried with a stably integrated luciferase reporter gene driven by a GAL4 promoter) (Promega, E1370) were co-expressed in host cells. When the agonist bound to the corresponding chimeric receptor, the chimeric receptor bound to the GAL4 binding site on the reporter gene vector and stimulated reporter gene expression. The agonistic activity of a compound against TRα or TRβ can be evaluated by the intensity of the luminescence signal

Detailed steps:

Preparation of working solution

a) A 30 mM solution of the reference compound (MGL-3196), or a compound to-be-tested was prepared in dimethyl sulfoxide (DMSO).
b) All compounds were subjected to 3-times gradient dilution with the DMSO starting with an initial concentration of 30 mM for a total of 10 concentration gradients.
c) ) the positive control of 50 μM T3 (triiodothyronine prepared by dissolving in the DMSO) and the negative control (100% DMSO) were prepared.
d) The compound plate was sealed and shaken for 5 minutes.

Preparation of cell suspension

a) All cells were cultured according to an ATCC standard, and the HEK293T assay was performed during its exponential growth phase
b) Gently discard the cell culture medium supernatant. Wash the cells twice with PBS.
c) A TrypLE™ trypsin digestion solution (Gibco) was added to digest the cells, and the digestion was terminated with a complete culture medium (Gibco).
d) The cells were collected and counted. The experiment could only be carried out when cell viability was greater than 90%
e) $2.5 \times 10^6$ HEK293T cells were inoculated into each 60 mm cell culture dish respectively.
f) The culture dish inoculated with the cells was cultured in a 5% $CO_2$ incubator at 37°C overnight.

Cell transfection

a) A Fugene6 transfection reagent (Promega, E2691) was placed at room temperature.
b) 30 μl of Fugene6 reagent was added into an Opti-MEM™ culture medium (Gibco, 11058-021), avoiding contact with the tube wall.
c) The mixture was mixed evenly by a pipette, and was allowed to settle at room temperature for 5 minutes.
d) 6 μg of plasmids (the pBIND plasmid (Pharmaron) inserted with the coding sequences of the TRα-LBD, or the TRβ-LBD and the RXRα-LBD, and the pGL4.35 reporter gene plasmid (Promega, E1370)) were added into the diluted transfection reagent. The mixture was mixed evenly by a pipette, and allowed to settle for 20 minutes at room temperature
e) The transfection reagent mixed with plasmid DNA was added into the 60 mm cell culture dish inoculated with the cells.
f) The culture dish was cultured in a 5% $CO_2$ incubator at 37°C for 5 hours.

Compound processing

a) The diluted compound solutions, the positive control and the negative control were transferred into a 384-well cell culture plate (PerkinElmer, 6007680-50) by Echo550 (Labcyte, 550).
b) The cells were inoculated into the 384-well cell culture plate, with 15,000 cells per well, and 25 μl of culture medium containing 5% fetal bovine serum (Gibco, 16000-044) was added.
c) The cells were cultured in a 5% $CO_2$ incubator at 37°C overnight.

Compound detection

a) A Steady-Glo™ (Promega, E2520) detection reagent was placed at room temperature.
b) The 384-well cell culture plate was placed at room temperature.
c) 25 μl of Steady-Glo™ detection reagent was added into each well of the cell culture plate.
d) The culture plate was placed on an oscillator to shake in the dark for 5 minutes.

A luminescence value was detected by Envision 2104 (PerkinElmer, Envision HTS).

Data analysis:

Calculate the RLU fluorescence signal (Signal) for each well, followed by the percentage of activity (% activity) as shown below

$$\% \text{ Activity} = (\text{Signal}_{cmpd} - \text{Signal}_{Ave_VC}) / (\text{Signal}_{Ave_PC} - \text{Signal}_{Ave_VC}) \times 100.$$

wherein:

$\text{Signal}_{ave_pc}$ was an average RLU fluorescence signal of the positive control in the whole plate;
$\text{Signal}_{ave_vc}$ was an average RLU fluorescence signal of the negative control in the whole plate; and
$\text{Signa}_{cmpd}$ was an average RLU fluorescence signal of the compound in the whole plate.

$EC_{50}$ was calculated by fitting % activity values and log of compound concentrations to nonlinear regression with Graphpad 8.0.

$$Y = \text{Bottom} + (\text{Top} - \text{Bottom}) / (1 + 10\hat{}((\text{LogEC}_{50} - X) \times \text{HillSlope}))$$

X: Log of compound concentration; Y: % Activity.
Specific test data are shown in Table 2 below.

Table 2 Results of cell transfection experiment of TRα, or TRβ

| Compound No. | TRα ($EC_{50}$ μM) | TRβ ($EC_{50}$ μM) |
|---|---|---|
| **T3** | 0.0023 | 0.002 |
| **MGL3196** | 2.564 | 1.083 |
| **KH03** | 0.826 | 0.089 |
| **KH04** | 2.486 | 1.283 |
| **KH06** | 0.037 | 0.009 |
| **KH07** | 0.964 | 0.208 |
| **KH10** | 0.269 | 0.032 |
| **KH13** | 2.74 | 1.13 |
| **KH14** | 0.383 | 0.148 |
| **KH15** | 1.457 | 0.205 |
| **KH16** | 0.138 | 0.019 |
| **KH18** | 0.028 | 0.007 |
| **KHE001** | 0.563 | 0.258 |
| **KHE002** | 0.039 | 0.004 |
| **KHE007** | 0.528 | 0.227 |
| **KHE008** | 0.049 | 0.008 |
| **KHE009** | 0.507 | 0.074 |
| **KHE011** | 0.07 | 0.02 |
| **KHE017** | 3.313 | 0.88 |
| **KHE018** | 0.407 | 0.102 |
| **KHE021** | 1.172 | 0.5 |

**Example 50 Hepatotoxicity detection in vitro**

Information of primary hepatocyte

| Donor | Article number | Batch number | Sex | Age | Race | Supplier |
|---|---|---|---|---|---|---|
| 1 | M00995-P | ZSE | Male | 60 | Caucasian | BioreclamationIVT |

Experiment:

Step 1: a test substance was prepared into a 200 mM DMSO stock solution, and then subjected to 3-times gradient continuous dilution by 7 concentrations, then 1.5 μL of each of the solutions of the 8 concentrations was added into 498.5 μL of incubation culture medium (with a composition referring to Table 5, directly mixed evenly) to prepare a working solution, and the incubation culture medium was preheated at 37°C before preparation. DMSO was used as a solvent control. DMSO contents in the working solution and the solvent control were both 0.3 vol%. Concentrations of compounds were as follows:

| Compound | Concentration of working solution (μM) |
|---|---|
| **KH03** | 600, 200, 66.7, 22.2, 7.14, 2.47, 0.823 and 0.274 |
| **KH06** | 600, 200, 66.7, 22.2, 7.14, 2.47, 0.823 and 0.274 |
| **KH10** | 600, 200, 66.7, 22.2, 7.14, 2.47, 0.823 and 0.274 |

Step 2: plate inoculation and cell culture

Second step: plate inoculation and culture of cells

1) A tube of hepatocytes of the donor used in the experiment preserved at ultra-low temperature was taken, and the hepatocytes were ensured to be still frozen at low temperature before resuscitation. The hepatocytes were quickly placed in a water bath at 37°C and gently shaken until all ice crystals were dispersed, and were transferred to a biosafety cabinet after spraying 70 vol% ethanol.
2) Contents of hepatocyte tubules were poured into a 50 mL centrifuge tube containing 50 mL of resuscitation culture medium (with a composition referring to Table 3, directly mixed evenly), and centrifuged at 80 g for 8 minutes. After centrifugation, the resuscitation culture medium was sucked out and an inoculation culture medium was added (with a composition referring to Table 4, directly mixed evenly). The cells were counted by AO/PI staining to obtain a cell suspension with a cell density of $0.2 \times 10^6$ cells per milliliter.
3) The cell suspension above was inoculated into a 96-well plate coated with collagen I, with 100 μL per well. The culture plate was placed in a 5% $CO_2$ incubator with 95% relative humidity to culture for 4 hours to 6 hours.
4) After incubation for 4 hours to 6 hours, thestate of the cells was observed under microscope. The culture plate was shaken gently, the inoculation culture medium was sucked out, and 100 μL of incubation culture medium was added into each well (with a composition referring to Table 5). A toxicity test could be carried out after culturing in the incubator for 18 hours to 20 hours.
5) Before administration, the morphology of the cells was observed under microscope. The culture medium in the culture plate was sucked out, and 100 μL of solvent control (DMSO), or test substance working solution was added into each well. Three parallel samples were tested under each condition.
6) The newly prepared working solution or solvent control was used for solution exchange every 24 hours after dosing.

After the working solution acted for 48 hours, the morphology of the cells was observed under microscope for later use.

step 3: cytotoxicity detection

1) A CellTiter-Glo (Promega, article number G9243) reagent stored at -20°C was melted in a water bath at 37°C.
2) After the cell culture plate obtained above was incubated for 48 hours, 50 μL of CellTiter-Glo solution was directly added into each experimental well.
3) The cell culture plate was vortexed at 400 rpm for 10 minutes, and incubated at room temperature to stabilize a luminescence signal.

After 10 minutes 100 μL of reaction solution was sucked from each well and transferred to a new white nontransparent-bottom flat plate (Corning 96-well plate Cat No. 3917). The chemiluminescence value of each well was read by a microplate reader (a luminescence value of the white nontransparent-bottom flat plate was recorded as "a luminescence $value_{blank}$"; and a luminescence value of the solvent control was recorded as "a luminescence valuesolvent").

step 4: data processing

Cell viability (%) = [(luminescence $value_{to\text{-}be\text{-}tested\ compound}$ - luminescence $value_{blank}$)/(luminescence $value_{solvent}$ - luminescence $value_{blank}$)] × 100%

A curve of the cell viability (%) vs the concentration of the compound was plotted and $IC_{50}$ of the compound was calculated by fitting the cell viability (%)to the concentration of the compound with GraphPad Prism 8.0.2.

$$Y = bottom + (top - bottom)/(1 + 10^{\wedge}((LogIC_{50} - X) \times hillslope))$$

X was the concentration of the compound; and Y was the cell vitality (%).

Specific test data are shown in Table 6.

Table 3 Resuscitation culture medium

| Reagent | Source | Article number | Volume |
|---|---|---|---|
| William's E culture medium (containing phenol red) | Gibco | 12551-032 | 30 mL |
| Isotonic percoll | GE Healthcare | 17-0891-09 | 13.5 mL |

(continued)

| Reagent | Source | Article number | Volume |
|---|---|---|---|
| Phosphate buffer (10X) | Gibco | 14200-075 | 1.5 mL |
| glutaMAX culture medium | Gibco | 35050-061 | 500 μL |
| N-2 hydroxyethyl piperazine-N-2-ethanesulfonic acid buffer 1 M (HEPES) | Gibco | 15630-080 | 750 μL |
| Fetal calf serum (FBS) | Corning | 35-076-CVR | 2.5 mL |
| Human-insulin | Gibco | 12585-014 | 50 μL |
| Dexamethasone 1 M | Dr. Ehrenstorfer | C12170400 | 5 μL |

Table 4 Inoculation culture medium

| Reagent | Source | Article number | Volume |
|---|---|---|---|
| William's E culture medium (without containing phenol red) | Sigma | W1878 | 46 mL |
| glutaMAX culture medium | Gibco | 35050-061 | 500 μL |
| N-2 hydroxyethyl piperazine-N-2-ethanesulfonic acid buffer 1 M (HEPES) | Gibco | 15630-080 | 750 μL |
| Fetal calf serum (FBS) | Corning | 35-076-CVR | 2.5 mL |
| Human-insulin | Gibco | 12585-014 | 50 μL |
| Dexamethasone 10 mM | Dr. Ehrenstorfer | C12170400 | 5 μL |
| Penicillin/Streptomycin mixed solution (100×) | Solarbio | P1400-100 | 500 μL |

Table 5 Incubation culture medium

| Reagent | Source | Article number | Volume |
|---|---|---|---|
| William's E culture medium (without containing phenol red) | Sigma | W1878 | 46 mL |
| glutaMAX culture medium | Gibco | 35050-061 | 500 μL |
| N-2 hydroxyethyl piperazine-N-2-ethanesulfonic acid buffer 1 M (HEPES) | Gibco | 15630-080 | 750 μL |
| ITS liquid culture medium supplement (100×) | Sigma | I3146 | 500 μL |
| Dexamethasone 10 mM | Dr. Ehrenstorfer | C12170400 | 0.5 μL |
| Penicillin/Streptomycin mixed solution (100×) | Solarbio | P1400-100 | 500 μL |

Table 6 Results of hepatotoxicity detection

| Compound | $IC_{50}$ (μM) | Cell activity at the highest concentration (%) |
|---|---|---|
| **KH03** | 264 | 0.0421 |
| **KH06** | 207 | 4.50 |
| **KH10** | 160 | 0.0142 |

**Example 51 PK experiment of cynomolgus monkeys**

The purpose of this experiment was to evaluate the pharmacokinetic behavior of the compound to be tested after single intravenous and intragastric administration, and to study the bioavailability after intragastric administration, providing animal experimental data for clinical studies.

Preparation of solutions for intravenous injection and intragastric administration: an appropriate amount of compound to be tested was accurately weighed, and mixed with a suitable solvent (5 vol% DMSO +10 vol% polyethylene glycol - 15-hydroxystearate + 85 vol% normal saline). After vortex, or ultrasound processing, a clear and transparent solution (the solution for intravenous injection administration), or a uniform suspension was obtained. The solution

administrated by intravenous injection needed to be filtered through a 0.22 μm filter membrane

Experimental design: before the first dose the cynomolgus monkeys were divided into 2 groups by body weight with 3 male cynomolgus monkeys in each group, where animals in group 1were given the compound to be tested (1 mg/kg) by single intravenous injection (IV); and animals in the 2nd group were administrated with the compound to be tested (5 mg/kg) by single intragastric administration (PO). The animals were weighed before administration, and dosages were calculated based on the weights of the animals.

Sample collection: a whole blood sample (about 0.2 mL) was collected at a specified time by venipuncture of an upper limb (or other suitable blood collection sites), and the actual blood collection time was recorded in test records. The acceptable error for one acquisition time point within 1 hour after administration is ±1 minute, and the acceptable error for other time points is ±5% of the theoretical time. All blood samples were immediately transferred to a labeled commercial centrifuge tube containing K2-EDTA. After blood sample collection, the blood sample was centrifuged at 4°C and 3,200 g for 10 minutes,the supernatant plasma was sucked, quickly put in dry ice, and kept in a refrigerator at -70 ± 10°C for LC-MS/MS analysis. The collection time of both groups was 0.083 hour, 0.25 hour, 0.5 hour, 1 hour, 2 hours, 4 hours, 8 hours and 24 hours after administration.

Data processing: Area under the curve ($AUC_{(0-t)}$ and $AUC_{(0-\infty)}$), elimination half-life (T1/2), peak concentration (Cmax), time to reach maximum plasma concentration (Tmax), etc., were calculated by the non-compartment analysis module in Phoenix WinNonlin 7.0.

Bioavailability (F) = area under the curve $AUC_{(0-t)}$ in the case of PO administration × $dosage_{IV}$/(area under the curve $AUC_{(0-t)}$ in the case of IV administration × dosagero) × 100%

Specific data are shown in Table 7.

Table 7 Results of PK experiment of cynomolgus monkeys

| Name of to-be-tested compound | Administration mode | Administration dosage | Half-life period ($T_{1/2}$) | Peak concentration $C_{max}$ | Area under the curve $AUC_{(0-t)}$ in the case of administration | Bioavailability (F) |
|---|---|---|---|---|---|---|
| | | mg/kg | h | ng/mL | h*ng/mL | % |
| MGL3196 | IV | 1 | 1.45 | 11171.33 | 3896.26 | 8.47 |
| | PO | 5 | NA | 429.61 | 1649.25 | |
| KH06 | IV | 1 | 9.00 | 20683.02 | 48771.23 | 73.00 |
| | PO | 5 | 16.68 | 18293.47 | 178010.66 | |

**Example 52 In vivo pharmacological experiment**

In the early stages of this experiment, mice induced by DIO (diet-induced obesity) were fed with a high-fat diet, followed by intraperitoneal injection of CCl4 during high-fat diet feeding to induce the NASH model. In this model, the anti-NASH efficacy of the compound to be tested was evaluated

Animal information: C57BL/6J male mice (18 weeks old) were used, comprising 32 DIO mice + 8 normal mice, wherein the weight of DIO mice > 38 g.

Table 8 Administration information

| Name of to-be-tested compound | Administration concentration (mg/kg) | Compound concentration (mg/ml) | Solvent | Number of animals in each group | Administration frequency | Number of days of administration |
|---|---|---|---|---|---|---|
| MGL3196 | 3 | 0.6 | 1% HPMC (hydroxy-propyl methylcellu-lose) | 8 | Once per day | Everyday administration from day 0 to day 27 |

(continued)

| Name of to-be-tested compound | Administration concentration (mg/kg) | Compound concentration (mg/ml) | Solvent | Number of animals in each group | Administration frequency | Number of days of administration |
|---|---|---|---|---|---|---|
| KH06 | 1 | 0.2 | 40% PEG400/10% Solutol (polyethylene glycol-15-hydrox ystearate)/50% water | 8 | Once per day | Everyday administration from day 0 to day 27 |
| KH06 | 3 | 0.6 | | 8 | Once per day | Everyday administration from day 0 to day 27 |

Table 9 Pharmacodynamic animal grouping information

| Starting time of grouping | | | | (Day -1) | | |
|---|---|---|---|---|---|---|
| Number of animals in each cage | | | | 4 | | |
| Grouping requirement | | | | DIO mice were randomly grouped according to the principle of minimum weight difference between groups, and at the same time try to meet the principles of the same nest, the same cage and the same group, so as to avoid fights between mice with different nests. | | |
| Group | Number of animals in each group | $CCl_4$ (carbon tetrachloride) | Concentration of $CCl_4$ (%) | Feedstuff | Administration and processing conditions of each group | Solvent |
| 11 | 8 | N | NA | Normal | Solvent, oral administration, once per day | 40% PEG400 (polyethylene glycol 400)/10% Solutol (polyethylene glycol-15-hydroxystearate)/50% water |
| 22 | 8 | Y | 25 | High fat | Solvent, oral administration, once per day | |
| 33 | 8 | Y | 25 | High fat | MGL-3196, 3 mg/kg, oral administration, once per day | 1% HPMC (Hydroxypropyl methylcellulose) |
| 44 | 8 | Y | 25 | High fat | KH06, 1 mg/kg, oral administration, once per day | 40% PEG400 (polyethylene glycol 400)/10% Solutol (polyethylene glycol-15-hydroxystearate)/50% water |
| 55 | 8 | Y | 25 | High fat | KH06, 3mg/kg, oral administration, once per day | |

Induction with $CCl_4$: A 25% (volume ratio) of CCl4 solution was prepared by placing 1 part CCl4 and 3 parts olive oil in a glass bottle. The solution was mixed well and used right after it was ready Eight mice in the first group were intraperitoneally injected with normal saline solution as normal controls The mice in the 2[rd] to 5th groups were intraperitoneally injected with 25% $CCl_4$ solution twice a week. 25% (volume ratio) $CCl_4$ was administered by body weight, 0.5 ml/kg. The interval between the injection time of $CCl_4$ and the time of administration on the day should be more than 4 hours

Histopathological analysis

All liver samples were dehydrated by a dehydrating instrument (Leica HistoCore Pearl-0348) and then embedded by a paraffin embedding machine (HistoCoreArcadia) . The embedded liver sample was then sliced using a Lecia RM2235 machine.

NAS score

NAS scores were performed on HE (hematoxylin-eosin) stained slices as the sum of steatosis, balloon degeneration, and lobular inflammation. The slices were scored by a pathologist according to standards shown in Table 10 below.

Table 10 NAS evaluation method

| NASH | | |
|---|---|---|
| Pathological manifestation | Evaluation | Score (NAS) |
| Ballooning degeneration of hepatocytes | None<br>A few of balloon-like cells<br>A lot of balloon-like cells | 0<br>1<br>2 |
| Lobular inflammation Overall evaluation of all inflammatory focuses | None<br><2 focuses/200 times field of vision<br>2 - 4 focuses/200 times field of vision<br>>4 focuses/200 times field of vision | 0<br>1<br>2<br>3 |
| Steatosis | <5%<br>5%-33%<br>>33%-66%<br>>66% | 0<br>1<br>2<br>3 |

Criteria for assessment of lesions

(1)Ballooning degeneration of hepatocytes: a pathological change similar to a vacuole was observed in the hepatocytes. Due to a vacuole-like change, the size of the hepatocytes was increased, and hepatocyte nuclei concentrated or deviated.
(2) Infiltration of inflammatory cells: A large number of aggregated inflammatory cells, mainly neutrophils and macrophages, were found around the portal vein area, abdominal vein area, or around the hepatic lobules.
(3) Steatosis regular round vacuoles were observed in the hepatocytes of different sizes, and the nuclei of the hepatocytes were located at the edges.

Percentage of fibrosis

All slices stained with Sirius red were scanned by a Leica Aperio AT2 Brightfield scanner, and then a percentage of Sirius red positive staining area was calculated by the HALO AI system to evaluate the percentage area of the Sirius red in the total liver area scanned.

Results were shown in FIG. 1 and FIG. 2.

FIG. 1 showed the results of fibrosis evaluation, where the efficacy of KH06 was dose-dependent, and they all achieved the effect of reducing the proportion of fibrosis. The efficacy of KH06 at a dosage of 1 mg/kg was better than that of MGL3196 at a dosage of 3 mg/kg.

FIG. 2 showed the results of NAS score, wherein the vertical coordinate of the NAS score was the sum of the score of steatosis, ballooning degeneration and lobular inflammation. Compared with the model group, KH06 was dose-dependent, and all achieved a significant reduction in score Meanwhile, KH06 at 1 mg/kg had the same efficacy as MGL3196 at 3 mg/kg.

**Claims**

**1.** A compound of Formula I or a pharmaceutically acceptable salt thereof:

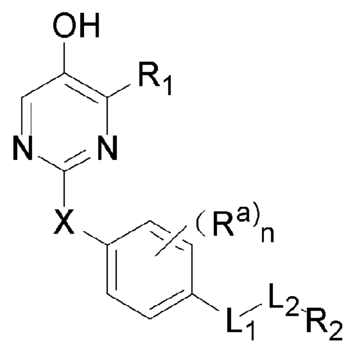

Formula I

wherein,

$R_1$ is hydrogen, optionally substituted alkyl, optionally substituted cycloalkyl, optionally substituted aryl, optionally substituted heterocyclyl, optionally substituted heteroaryl, optionally substituted amino, optionally substituted carbamoyl, or $-COR_{10}$;
X is optionally substituted methylene, -O-, -S-, or $-SO_2-$;
$R^a$ is selected from hydrogen, halogen, $C_{1-6}$ linear and branched alkyl, or cycloalkyl; or two adjacent $R^a$ are bonded to form a carbocyclic ring, or heterocyclic ring;
$L_1$ is a single bond, methylene, -CH=CH-, -O-, -CO-, $-NR_3-$, $-NR_3CO-$, $-CONR_3-$, $-CH_2NR_3-$, or -S-;
$L_2$ is a single bond, or $-(CR_4R_5)_p$;
$R_2$ is a carboxyl, or a group represented by the following formula:

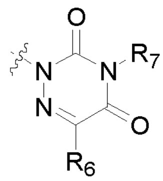
,

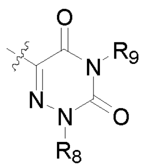
,
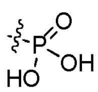
,
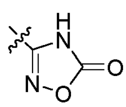
,
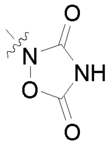
,
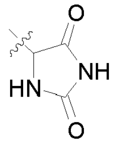
,
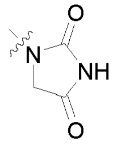
,
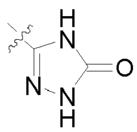
,

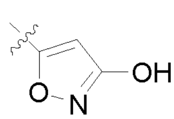
,
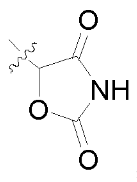
, or
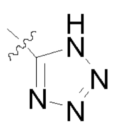
;

$R_3$ is hydrogen, or optionally substituted alkyl;
$R_4$ and $R_5$ are each independently selected from hydrogen, halogen, or optionally substituted alkyl, or $R_4$ and $R_5$ are bonded to form a cycloalkyl;
$R_6$ is hydrogen, cyano, amino, COOH, $C_{1-6}$ alkyl, $C_{1-6}$ haloalkyl, $C_{3-6}$ cycloalkyl, or $C_{3-6}$ halocycloalkyl;
$R_8$ is hydrogen, cyano, COOH, $C_{1-6}$ alkyl, $C_{1-6}$ haloalkyl, $C_{3-6}$ cycloalkyl, or $C_{3-6}$ halocycloalkyl;
$R_7$ and $R_9$ are hydrogen, $C_{1-3}$ alkyl, or $C_{1-3}$ haloalkyl;

$R_{10}$ is optionally substituted alkyl, amino, hydroxyl, optionally substituted cycloalkyl, optionally substituted aryl, optionally substituted heterocyclyl, or optionally substituted heteroaryl;
n is 0, 1, 2, 3, or 4; and
p is 0, 1, or 2.

**2.** The compound, or the pharmaceutically acceptable salt thereof according to claim 1, wherein the compound of Formula I is shown in Formula II:

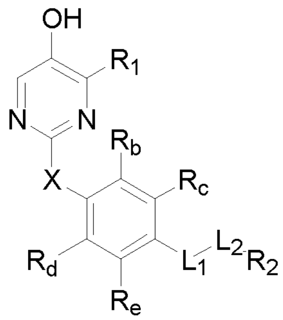

Formula II

wherein, $R_b$, $R_c$, $R_d$ and $R_e$ are hydrogen, deuterium, halogen, $C_{1-6}$ linear or branched alkyl, or cycloalkyl; or, $R_b$ and $R_c$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom; or, $R_d$ and $R_e$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom.

**3.** The compound or the pharmaceutically acceptable salt thereof according to claim 1 or 2, wherein the compound of Formula I is shown in Formula III:

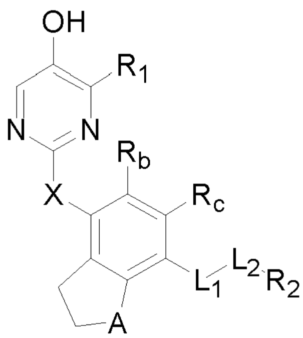

Formula III

wherein,

$R_b$ and $R_c$ are hydrogen, deuterium, halogen, $C_{1-6}$ linear or branched alkyl, or cycloalkyl; and
A is O, or methylene.

**4.** The compound or the pharmaceutically acceptable salt thereof according to any preceding claim, wherein $R_1$ is selected from:

1) optionally substituted $C_{1-6}$ linear and branched alkyl;
2) optionally substituted $C_{3-8}$ cycloalkyl;
3) optionally substituted $C_{3-8}$ non-aromatic heterocyclyl containing 1 to 3 heteroatoms selected from nitrogen atom, oxygen atom and sulfur atom;
4) optionally substituted phenyl; or
5) optionally substituted $C_{5-6}$ heteroaryl containing 1 to 3 heteroatoms selected from nitrogen atom, oxygen atom

and sulfur atom.

**5.** The compound or the pharmaceutically acceptable salt thereof according to any preceding claim, wherein $R_1$ is selected from $-(CR_{11}R_{12})_mR_{13}$; $R_{11}$ and $R_{12}$ are selected from hydrogen, deuterium, halogen, hydroxyl, amino, or optionally substituted $C_{1-4}$ alkyl; and $R_{13}$ is selected from:

1) hydrogen, or deuterium;
2) halogen;
3) hydroxyl;
4) amino;
5) carboxyl;
6) optionally substituted $C_{1-4}$ alkyl, or $C_{1-4}$ alkoxy;
7) optionally substituted $C_{3-8}$ cycloalkyl;
8) optionally substituted $C_{3-8}$ non-aromatic heterocyclyl containing 1 to 3 heteroatoms selected from nitrogen atom, oxygen atom and sulfur atom;
9) optionally substituted phenyl; or
10) optionally substituted $C_{5-6}$ heteroaryl containing 1 to 3 heteroatoms selected from nitrogen atom, oxygen atom and sulfur atom; and
m is 0, 1, 2, or 3.

**6.** The compound or the pharmaceutically acceptable salt thereof according to any preceding claim, wherein $R_1$ is selected from $-COR_{10}$, and $R_{10}$ is selected from:

1) amino;
2) hydroxyl;
3) optionally substituted $C_{1-4}$ alkyl, or $C_{1-4}$ alkoxy;
4) optionally substituted $C_{3-8}$ cycloalkyl;
5) optionally substituted $C_{3-8}$ non-aromatic heterocyclyl containing 1 to 3 heteroatoms selected from nitrogen atom, oxygen atom and sulfur atom;
6) optionally substituted phenyl; or
7) optionally substituted $C_{5-6}$ heteroaryl containing 1 to 3 heteroatoms selected from nitrogen atom, oxygen atom and sulfur atom.

**7.** The compound or the pharmaceutically acceptable salt thereof according to any preceding claim, wherein $R_1$ is selected from:

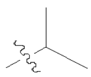, 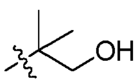, 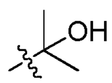, 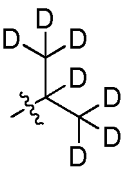,

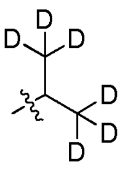, 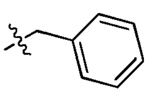, 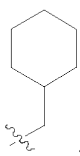, 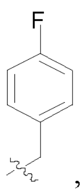, 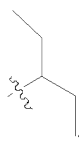, 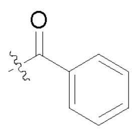, 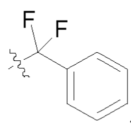, 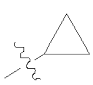, or 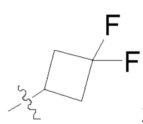;

preferably, $R_b$, $R_c$, $R_d$ and $R_e$ are selected from hydrogen, deuterium, or halogen;
preferably, $L_1$ is selected from a single bond, -O-, -NH-, or -NHCO-;
preferably, $L_2$ is selected from a single bond, or methylene; and
preferably, $R_2$ is selected from:

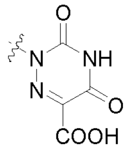, 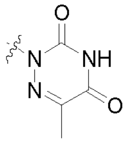, 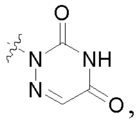, 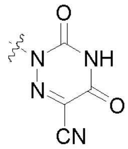, 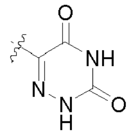,

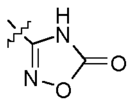, 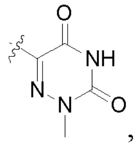, or 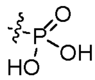.

8. The compound or the pharmaceutically acceptable salt thereof according to claim 2, wherein $R_1$ is optionally substituted $C_{1-6}$ linear or branched alkyl, or $C_{3-8}$ cycloalkyl;

X is O, S, or $-CH_2-$;
$R_b$, $R_c$, $R_d$ and $R_e$ are hydrogen, deuterium, halogen, $C_{1-6}$ linear or branched alkyl, or cycloalkyl; or $R_b$ and $R_c$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom; or, $R_d$ and $R_e$ are bonded to form a 5- or 6-membered cycloalkyl, or a 5-or 6-membered non-aromatic heterocyclic ring containing 1, or 2 heteroatoms selected from nitrogen atom, oxygen atom and sulfur atom;
$L_1$ is a single bond, $-NR_3-$, -O, or -S-;
$L_2$ is a single bond, or $-CH_2-$;
$R_2$ is selected from a group represented by the following formula :

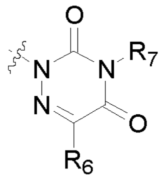,

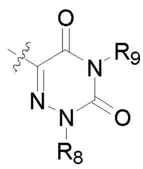, 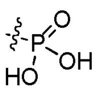, 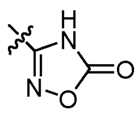, 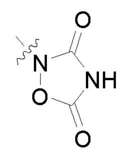, 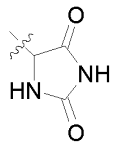, 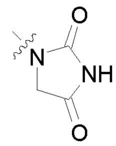, 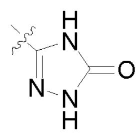,

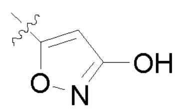, 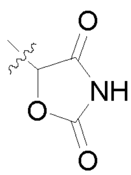, or 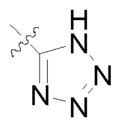;

$R_3$ is hydrogen, or optionally substituted $C_{1-6}$ alkyl;
$R_6$ is hydrogen, cyano, amino, COOH, $C_{1-6}$ alkyl, $C_{1-6}$ haloalkyl, $C_{3-6}$ cycloalkyl, or $C_{3-6}$ halocycloalkyl;
$R_8$ is hydrogen, cyano, COOH, $C_{1-6}$ alkyl, $C_{1-6}$ haloalkyl, $C_{3-6}$ cycloalkyl, or $C_{3-6}$ halocycloalkyl; and
$R_7$ and $R_9$ are hydrogen, $C_{1-3}$ alkyl, or $C_{1-3}$ haloalkyl.

9. The compound or the pharmaceutically acceptable salt thereof according to claim 2, or 8, wherein $R_1$ is optionally substituted $C_{1-6}$ linear or branched alkyl;

$R_b$, $R_c$, $R_d$ and $R_e$ are hydrogen, deuterium, halogen, $C_{1-6}$ linear, branched alkyl, or cycloalkyl; or $R_b$ and $R_c$ are bonded to form a 5-or 6-membered cycloalkyl, or a 5-or 6-membered non-aromatic heterocyclic ring containing 1, or 2 heteroatoms selected from nitrogen atom, oxygen atom and sulfur atom; or, $R_d$ and $R_e$ are bonded to form a 5- or 6-membered cycloalkyl, or a 5-or 6-membered non-aromatic heterocyclic ring containing 1, or 2 heteroatoms

selected from nitrogen atom, oxygen atom and sulfur atom;
X is O, S, or $-CH_2-$;
$L_1$ is a single bond, -O, -S-, or -NH-;
$L_2$ is a single bond;
$R_2$ is selected from a group represented by the following formula:

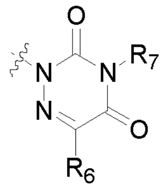
,

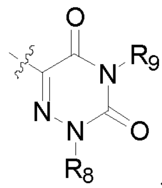
, or
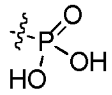
;

$R_6$ is hydrogen, cyano, $C_{1-6}$ alkyl, or $C_{1-6}$ haloalkyl;
$R_8$ is hydrogen, cyano, $C_{1-6}$ alkyl, or $C_{1-6}$ haloalkyl; and
$R_7$ and $R_9$ are hydrogen, $C_{1-3}$ alkyl, or $C_{1-3}$ haloalkyl.

**10.** The compound or the pharmaceutically acceptable salt thereof according to any one of claims 2, 8 and 9, wherein $R_1$ is $C_{1-6}$ linear, or branched alkyl, benzyl, or $C_{5-6}$ cycloalkylmethylene optionally substituted by hydrogen, deuterium, tritium, $C_{1-6}$ alkyl, hydroxyl, halogen, or CN, and further preferably isopropyl, or benzyl;

$R_b$ and $R_d$ are halogen, $R_c$ and $R_e$ are hydrogen, and $R_b$ and $R_d$ are further preferably chlorine;
X is O, S, or $-CH_2-$;
$L_1$ is a single bond, -O, -S-, or -NH-;
$L_2$ is a single bond, or $-CH_2-$;
$R_2$ is selected from a group represented by the following formula:

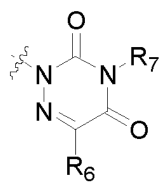
,

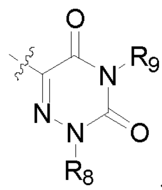
, or
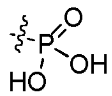
;

and
$R_6$, $R_7$, $R_8$ and $R_9$ are hydrogen, or $C_{1-6}$ alkyl, or $C_{3-8}$ cycloalkyl.

**11.** The compound or the pharmaceutically acceptable salt thereof according to claim 1, wherein $R_1$ is hydrogen, $C_{1-10}$ alkyl, $C_{3-10}$ cycloalkyl, $C_{3-10}$ cycloalkyl$C_{1-6}$ alkyl, $C_{5-10}$ aryl, $C_{5-10}$ aryl$C_{1-6}$ alkyl, 5-10 membered heterocyclyl containing 1 to 3 heteroatoms selected from nitrogen atom, oxygen atom and sulfur atom, 5-10 membered heteroaryl containing 1 to 3 heteroatoms selected from nitrogen atom, oxygen atom and sulfur atom, amino, or $-COR_{10}$, and the $C_{1-10}$ alkyl, the $C_{3-10}$ cycloalkyl, the $C_{3-10}$ cycloalkyl$C_{1-6}$ alkyl, the $C_{5-10}$ aryl, the $C_{5-10}$ aryl$C_{1-6}$ alkyl, the 5-10 membered heterocyclyl containing 1 to 3 heteroatoms selected from nitrogen atom, oxygen atom and sulfur atom, the

5-10 membered heteroaryl containing 1 to 3 heteroatoms selected from nitrogen atom, oxygen atom and sulfur atom, or the amino is unsubstituted, or is capable of being substituted by deuterium, tritium, $C_{1-6}$ alkyl, hydroxyl, halogen, or CN;

X is methylene, -O-, -S-, or -$SO_2$-;
$R^a$ is hydrogen, deuterium, halogen, $C_{1-6}$ linear or branched alkyl, or cycloalkyl; or two adjacent $R^a$ are bounded to form a 5-10 membered carbocyclic ring, or a 5-10 membered heterocyclic ring containing 1 to 3 heteroatoms selected from nitrogen atom, oxygen atom and sulfur atom;
$L_1$ is a single bond, methylene, -O-, -CO-, -$NR_3$-, -$NR_3$CO-, -$CONR_3$-, -$CH_2NR_3$-, or -S-;
$L_2$ is a single bond, or $C_{1-6}$ alkyl;
$R_2$ is a carboxyl, or a group represented by the following formula:

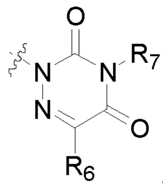 , 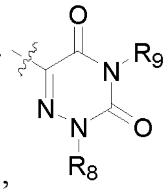 ,

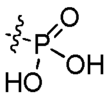 , 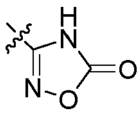 , or 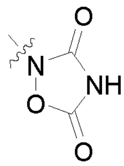 ;

$R_3$ is hydrogen, or $C_{1-6}$ alkyl;
$R_6$ is hydrogen, cyano, amino, COOH, $C_{1-6}$ alkyl, or $C_{1-6}$ haloalkyl;
$R_8$ is hydrogen, cyano, COOH, $C_{1-6}$ alkyl, or $C_{1-6}$ haloalkyl;
$R_7$ and $R_9$ are hydrogen, $C_{1-3}$ alkyl, or $C_{1-3}$ haloalkyl;
$R_{10}$ is $C_{3-10}$ cycloalkyl, $C_{5-10}$ aryl, 5-10 membered heterocyclyl containing 1 to 3 heteroatoms selected from nitrogen atom, oxygen atom and sulfur atom, or 5-10 membered heteroaryl containing 1 to 3 heteroatoms selected from nitrogen atom, oxygen atom and sulfur atom; and
n is 0, 1, 2, 3, or 4; and
preferably,
$R_1$ is methyl, ethyl, propyl, butyl, pentyl, cyclopropane, cyclobutane, cyclopentane, cyclohexane, cyclopropanemethyl, cyclobutanemethyl, cyclopentanemethyl, cyclohexanemethyl, phenyl, benzyl, or -$COR_{10}$, and the methyl, the ethyl, the propyl, the butyl, the pentyl, the cyclopropane, the cyclobutane, the cyclopentane, the cyclohexane, the cyclopropanemethyl, the cyclobutanemethyl, the cyclopentanemethyl, the cyclohexanemethyl, the phenyl, or the benzyl is unsubstituted, or capable of being substituted by deuterium, $C_{1-3}$ alkyl, hydroxyl, halogen, or CN;
X is methylene, -O-, -S-, or -$SO_2$-;
$R^a$ is halogen; or two adjacent $R^a$ are bonded to form a 5 membered carbocyclic ring, or a 5 membered heterocyclic ring containing 1 to 2 heteroatoms selected from nitrogen atom, oxygen atom and sulfur atom;
$L_1$ is a single bond, -O-, -NH-, -NHCO-, -CONH-, -$CH_2$NH-, or -S-;
$L_2$ is a single bond, methyl, ethyl, or propyl;
$R_2$ is a carboxyl, or a group represented by the following formula:

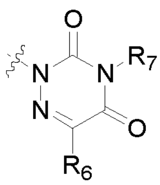 ,

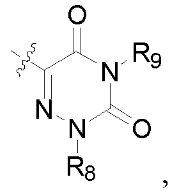 , 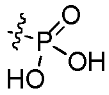 , or 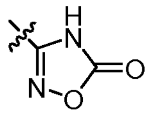 ;

$R_6$ is hydrogen, cyano, COOH, methyl, ethyl, or propyl;
$R_8$ is hydrogen, methyl, ethyl, or propyl;
$R_7$ and $R_9$ are hydrogen, or methyl;
$R_{10}$ is phenyl; and
n is 2, or 3;
preferably,
$R_1$ is methyl, ethyl, propyl, butyl, pentyl, cyclopropane, cyclobutane, cyclopentane, cyclohexane, cyclopropanemethyl, cyclobutanemethyl, cyclopentanemethyl, cyclohexanemethyl, phenyl, or benzyl, and the methyl, the ethyl, the propyl, the butyl, the pentyl, the cyclopropane, the cyclobutane, the cyclopentane, the cyclohexane, the cyclopropanemethyl, the cyclobutanemethyl, the cyclopentanemethyl, the cyclohexanemethyl, the phenyl, or the benzyl is unsubstituted, or capable of being substituted by deuterium, $C_{1-3}$ alkyl, hydroxyl, F, Cl, Br, or CN;
X is methylene, -O-, or -S-;
$R^a$ is F, Cl, or Br; or two adjacent $R^a$ are bonded to form a 5 membered carbocyclic ring, or a 5 membered heterocyclic ring containing 1 to 2 heteroatoms selected from nitrogen atom, oxygen atom and sulfur atom;
$L_1$ is a single bond, -O-, -NH-, or -NHCO-;
$L_2$ is a single bond, methyl, ethyl, or propyl;
$R_2$ is a carboxyl, or a group represented by the following formula:

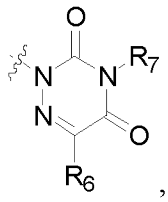 , 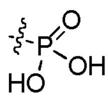 ,

or

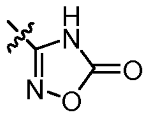 ;

$R_6$ is hydrogen, cyano, or methyl;
$R_7$ is hydrogen; and
n is 2, or 3.

**12.** A compound of the following formula or a pharmaceutically acceptable salt thereof:

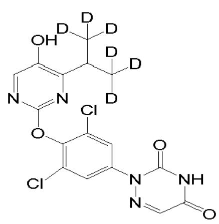 , 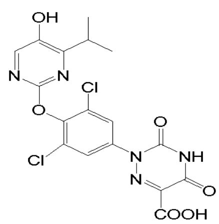 , 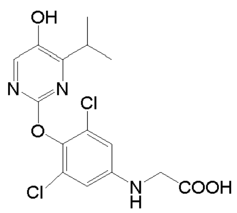 , 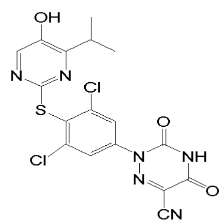 , 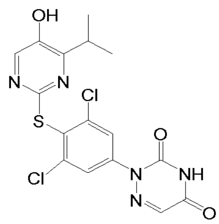 ,

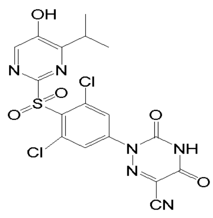 , 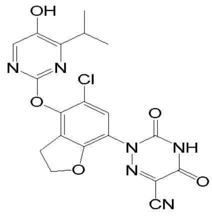 , 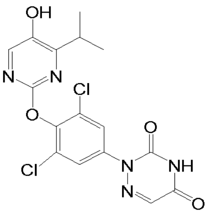 , 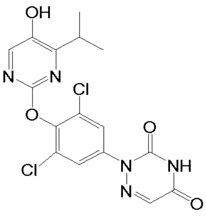 , 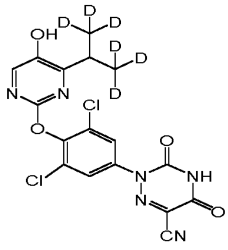 ,

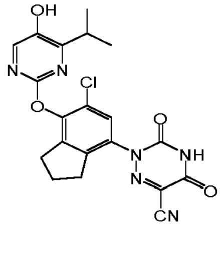 , 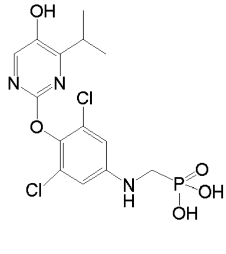 , 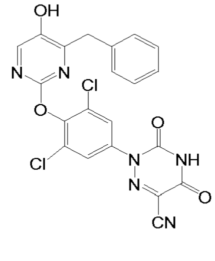 , 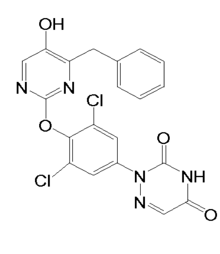 , 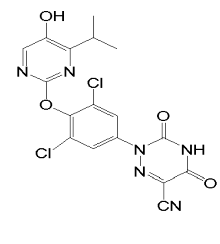 ,

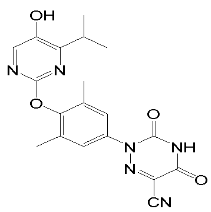 , 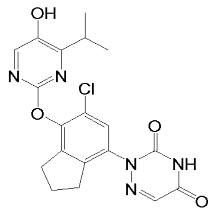 , 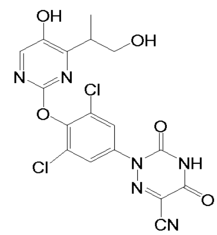 , 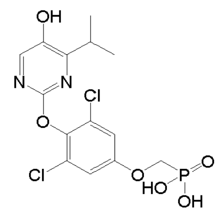 , 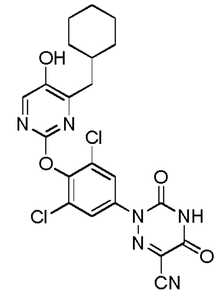 ,

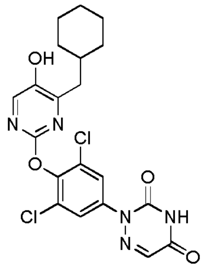 , 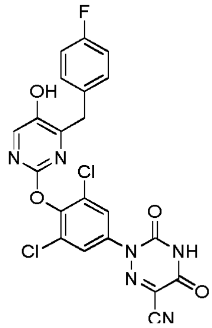 , 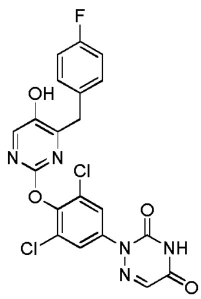 , 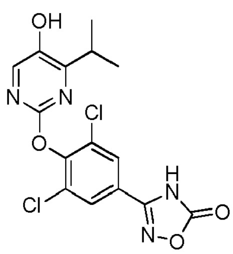 , 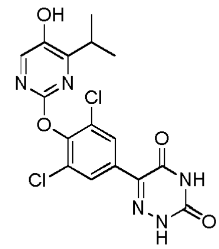 ,

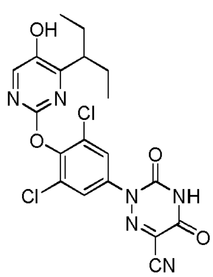 , 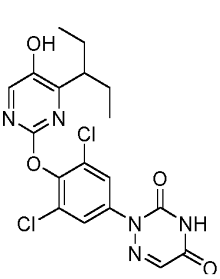 , 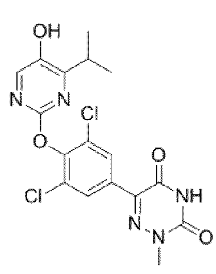 , 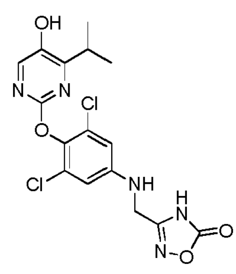 , 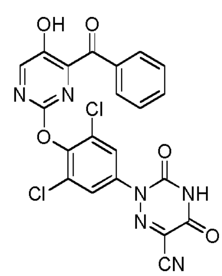 ,

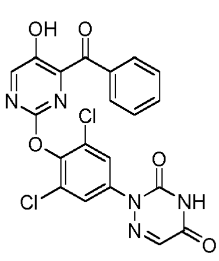,
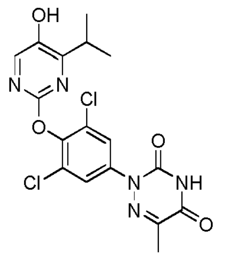,
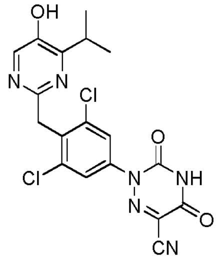,
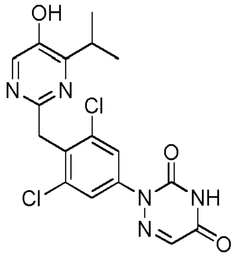,
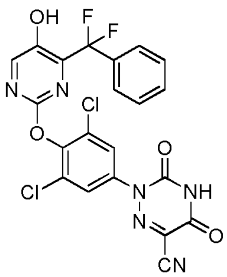,
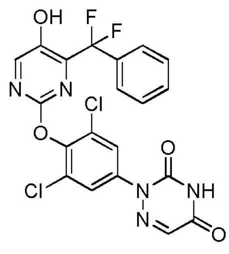,
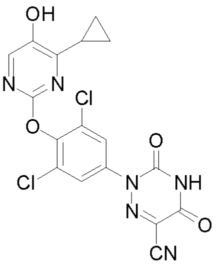,
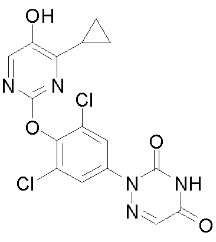,
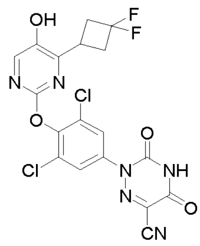,
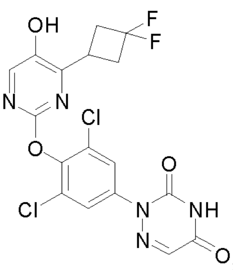,
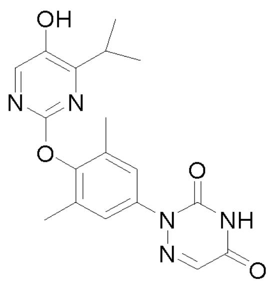, or
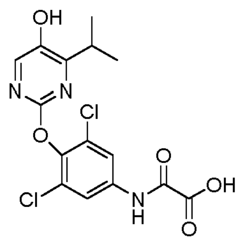.

**13.** A pharmaceutical composition, comprising the compound or the pharmaceutically acceptable salt thereof according to any one of claims 1 to 12, and one or more pharmaceutically acceptable carriers.

**14.** The compound or the pharmaceutically acceptable salt thereof according to any one of claims 1 to 12, or the pharmaceutical composition according to claim 13 for use as a medicament.

**15.** The compound or the pharmaceutically acceptable salt thereof according to any one of claims 1 to 12, or the pharmaceutical composition according to claim 13 for use in the prevention or treatment of a disease related to a β receptor agonist action, wherein preferably, the disease related to the β receptor agonist action is obesity, hyperlipidemia, hypercholesterolemia, hypertriglyceridemia, dyslipidemia, thyroid cancer, metabolic syndrome, cardiovascular disease, coronary artery disease, myocardial infarction, ventricular insufficiency, heart failure, fatty liver, cirrhosis, diabetes, steatohepatitis, non-alcoholic steatohepatitis, non-alcoholic fatty liver disease, atherosclerosis, or hypothyroidism disease, or disorder.

**Patentansprüche**

**1.** Verbindung der Formel I oder ein pharmazeutisch annehmbares Salz davon:

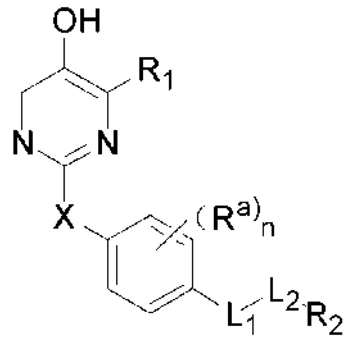

Formel I

wobei

$R_1$ Wasserstoff, gegebenenfalls substituiertes Alkyl, gegebenenfalls substituiertes Cycloalkyl, gegebenenfalls substituiertes Aryl, gegebenenfalls substituiertes Heterocyclyl, gegebenenfalls substituiertes Heteroaryl, gegebenenfalls substituiertes eine Aminosäure, gegebenenfalls substituiertes Carbamoyl oder $-COR_{10}$ ist;
X gegebenenfalls substituiertes Methylen, -O-, -S- oder $-SO_2-$ ist;
$R^a$ ausgewählt ist aus Wasserstoff, Halogen, $C_{1-6}$ linearem und verzweigtem Alkyl oder Cycloalkyl; oder wobei zwei benachbarte $R^a$ zu einem carbocyclischen Ring oder heterocyclischen Ring gebunden sind;
$L_1$ eine Einfachbindung, Methylen, -CH=CH-, -O-, -CO-, $-NR_3-$, $-NR_3CO-$, $-CONR_3-$, $-CH_2NR_3-$, oder -S- ist;
$L_2$ eine Einfachbindung oder - $(CR_4R_5)_P$ist;
$R_2$ eine Carboxylgruppe oder eine durch die folgende Formel dargestellte Gruppe ist:

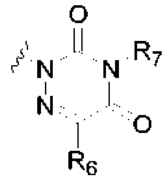
,

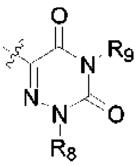
,
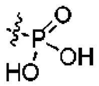
,
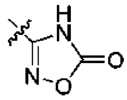
,
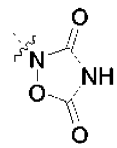
,
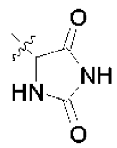
,
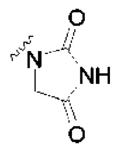
,
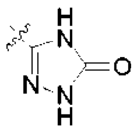
,

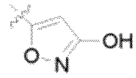
,
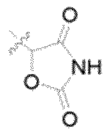
oder
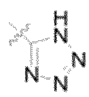
;

$R_3$ Wasserstoff oder gegebenenfalls substituiertes Alkyl ist;
$R_4$ und $R_5$ unabhängig voneinander ausgewählt sind aus Wasserstoff, Halogen oder gegebenenfalls substituiertem Alkyl, oder $R_4$ und $R_5$ zu einem Cycloalkyl gebunden sind;
$R_6$ Wasserstoff, Cyano, Aminosäure, COOH, $C_{1-6}$ Alkyl, $C_{1-6}$ Halogenalkyl, $C_{3-6}$ Cycloalkyl oder $C_{3-6}$ Halogencycloalkyl ist;
$R_8$ Wasserstoff, Cyano, COOH, $C_{1-6}$ Alkyl, $C_{1-6}$ Halogenalkyl, $C_{3-6}$ Cycloalkyl oder $C_{3-6}$ Halogencycloalkyl ist;
$R_7$ und $R_9$ Wasserstoff, $C_{1-3}$ Alkyl oder $C_{1-3}$ Halogenalkyl sind;
$R_{10}$ gegebenenfalls substituiertes Alkyl, eine Aminosäure, Hydroxy, gegebenenfalls substituiertes Cycloalkyl, gegebenenfalls substituiertes Aryl, gegebenenfalls substituiertes Heterocyclyl oder gegebenenfalls substituiertes Heteroaryl ist;
n für 0, 1, 2, 3 oder 4 steht; und
p für 0, 1 oder 2 steht.

**2.** Verbindung, oder pharmazeutisch verträgliches Salz davon nach Anspruch 1, wobei die Verbindung der Formel I in Formel II gezeigt ist:

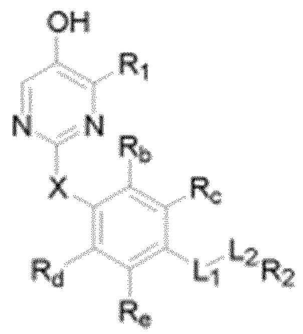

Formel II

wobei $R_b$, $R_c$, $R_d$ und $R_e$ Wasserstoff, Deuterium, Halogen, $C_{1-6}$ , lineares oder verzweigtes Alkyl oder Cycloalkyl sind; oder wobei $R_b$ und $R_c$ zu einem 5- oder 6-gliedrigen Cycloalkyl oder einem 5- oder 6-gliedrigen nichtaromatischen heterocyclischen Ring mit 1 oder 2 Heteroatomen gebunden sind, ausgewählt aus einem Stickstoffatom, einem Sauerstoffatom und einem Schwefelatom; oder wobei $R_d$ und $R_e$ zu einem 5- oder 6-gliedrigen Cycloalkyl oder einem 5- oder 6-gliedrigen nichtaromatischen heterocyclischen Ring mit 1 oder 2 Heteroatomen gebunden sind, ausgewählt aus einem Stickstoffatom, einem Sauerstoffatom und einem Schwefelatom.

**3.** Verbindung oder pharmazeutisch verträgliches Salz davon nach Anspruch 1 oder 2, wobei die Verbindung der Formel I in Formel III gezeigt ist:

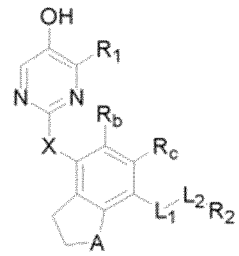

Formel III

wobei

$R_b$ und $R_c$ Wasserstoff, Deuterium, Halogen, $C_{1-6}$ lineares oder verzweigtes Alkyl oder Cycloalkyl sind; und A für O oder Methylen steht.

**4.** Verbindung oder pharmazeutisch verträgliches Salz davon nach einem der vorherigen Ansprüche, wobei $R_1$ ausgewählt ist aus Folgendem:

1) gegebenenfalls substituiertes $C_{1-6}$ lineares und verzweigtes Alkyl;
2) gegebenenfalls substituiertes $C_{3-8}$ Cycloalkyl;
3) gegebenenfalls substituiertes $C_{3-8}$ nichtaromatisches Heterocyclyl mit 1 bis 3 Heteroatomen, ausgewählt aus Stickstoffatom, Sauerstoffatom und Schwefelatom;
4) gegebenenfalls substituiertes Phenyl; oder
5) gegebenenfalls substituiertes $C_{5-6}$ Heteroaryl mit 1 bis 3 Heteroatomen, ausgewählt aus einem Stickstoffatom, einem Sauerstoffatom und einem Schwefelatom.

**5.** Verbindung oder pharmazeutisch verträgliches Salz davon nach einem der vorherigen Ansprüche, wobei $R_1$ ausgewählt ist aus $-(CR_{11}R_{12})_mR_{13}$; $R_{11}$ und $R_{12}$ ausgewählt sind aus Wasserstoff, Deuterium, Halogen, Hydroxy, Aminosäure oder gegebenenfalls substituiertem $C_{1-4}$ Alkyl; und $R_{13}$ ausgewählt ist aus:

1) Wasserstoff oder Deuterium;
2) Halogen;
3) Hydroxyl;
4) Aminosäure;
5) Carboxyl;
6) gegebenenfalls substituiertem $C_{1-4}$ Alkyl oder $C_{1-4}$ Alkoxy;
7) gegebenenfalls substituiertem $C_{3-8}$ Cycloalkyl;
8) gegebenenfalls substituiertem $C_{3-8}$ nichtaromatischem Heterocyclyl mit 1 bis 3 Heteroatomen ausgewählt aus Stickstoffatom, Sauerstoffatom und Schwefelatom;
9) gegebenenfalls substituiertem Phenyl; oder
10) gegebenenfalls substituiertem $C_{5-6}$ heteroaryl enthaltend 1 bis 3 Heteroatome ausgewählt aus Stickstoffatom, Sauerstoffatom und Schwefelatom; und

m für 0, 1, 2 oder 3 steht.

**6.** Verbindung oder pharmazeutisch verträgliches Salz davon nach einem der vorherigen Ansprüche, wobei $R_1$ ist ausgewählt aus $-COR_{10}$, und $R_{10}$ ausgewählt ist aus:

1) Aminosäure;
2) Hydroxyl;
3) gegebenenfalls substituiertem $C_{1-4}$ Alkyl oder $C_{1-4}$ Alkoxy;
4) gegebenenfalls substituiertem $C_{3-8}$ Cycloalkyl;
5) gegebenenfalls substituiertem $C_{3-8}$ nichtaromatischem Heterocyclyl mit 1 bis 3 Heteroatomen ausgewählt aus Stickstoffatom, Sauerstoffatom und Schwefelatom;
6) gegebenenfalls substituiertem Phenyl; oder
7) gegebenenfalls substituiertem $C_{5-6}$ Heteroaryl mit 1 bis 3 Heteroatomen, ausgewählt aus einem Stickstoffatom, einem Sauerstoffatom und einem Schwefelatom.

**7.** Verbindung oder pharmazeutisch verträgliches Salz davon gemäß einem der vorherigen Ansprüche, wobei $R_1$ ausgewählt ist aus Folgendem:

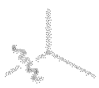, 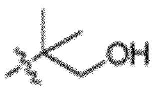, 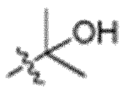, 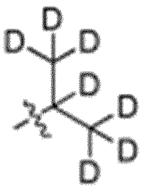,

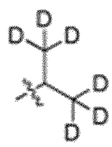, 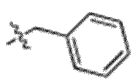, 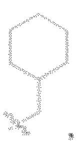, 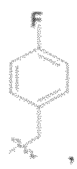, 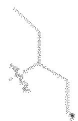, 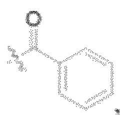, 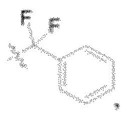, 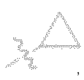, oder 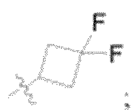;

vorzugsweise $R_b$, $R_c$, $R_d$ und $R_e$ ausgewählt sind aus Wasserstoff, Deuterium oder Halogen;
vorzugsweise $L_1$ ausgewählt ist aus einer Einfachbindung, -O-, -NH- oder -NHCO-;
vorzugsweise $L_2$ ausgewählt ist aus einer Einfachbindung oder Methylen; und

vorzugsweise $R_2$ ausgewählt ist aus:

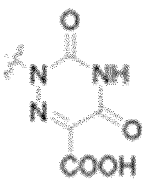, 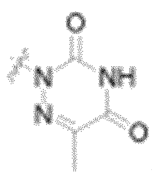, 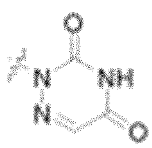, 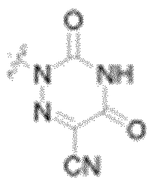, 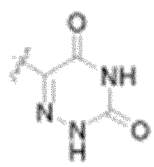,

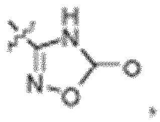, 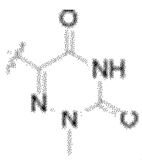, oder 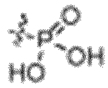

8. Verbindung oder pharmazeutisch verträgliches Salz davon nach Anspruch 2, wobei $R_1$ gegebenenfalls substituiertes $C_{1-6}$ lineares oder verzweigtes Alkyl oder $C_{3-8}$ Cycloalkyl ist;

X für O, S oder $-CH_2-$ steht;
$R_b$, $R_c$, $R_d$ und $R_e$ Wasserstoff, Deuterium, Halogen, $C_{1-6}$ lineares oder verzweigtes Alkyl oder Cycloalkyl sind; oder $R_b$ und $R_c$ zu einem 5- oder 6-gliedrigen Cycloalkyl oder einem 5- oder 6-gliedrigen nichtaromatischen heterocyclischen Ring mit 1 oder 2 Heteroatomen gebunden sind, ausgewählt aus einem Stickstoffatom, einem Sauerstoffatom und einem Schwefelatom; oder, $R_d$ und $R_e$ zu einem 5- oder 6-gliedrigen Cycloalkyl oder einem 5- oder 6-gliedrigen nichtaromatischen heterocyclischen Ring mit 1 oder 2 Heteroatomen gebunden sind, ausgewählt aus einem Stickstoffatom, einem Sauerstoffatom und einem Schwefelatom;
$L_1$ eine Einfachbindung, $-NR_3-$, -O oder -S- ist;
$L_2$ eine Einfachbindung oder $-CH_2-$ ist;
$R_2$ ausgewählt ist aus einer Gruppe, die durch die folgende Formel dargestellt wird :

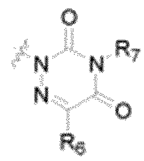,

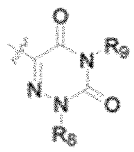, 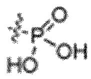, 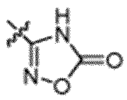, 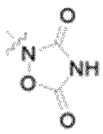, 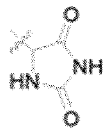, 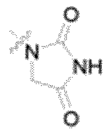, 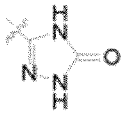,

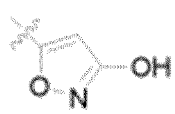, 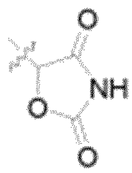, oder 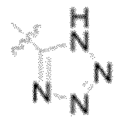;

$R_3$ Wasserstoff oder gegebenenfalls substituiertes $C_{1-6}$ Alkyl ist;
$R_6$ Wasserstoff, Cyano, Aminosäure, COOH, $C_{1-6}$ Alkyl, $C_{1-6}$ Halogenalkyl, $C_{3-6}$ Cycloalkyl oder $C_{3-6}$ Halogencycloalkyl ist;
$R_8$ Wasserstoff, Cyano, COOH, $C_{1-6}$ Alkyl, $C_{1-6}$ Halogenalkyl, $C_{3-6}$ Cycloalkyl oder $C_{3-6}$ Halogencycloalkyl ist; und
$R_7$ und $R_9$ Wasserstoff, $C_{1-3}$ Alkyl oder $C_{1-3}$ Halogenalkyl sind.

9. Verbindung oder pharmazeutisch verträgliches Salz davon nach Anspruch 2 oder 8, wobei $R_1$ gegebenenfalls substituiertes $C_{1-6}$ lineares oder verzweigtes Alkyl ist;

$R_b$, $R_c$, Rd und $R_e$ Wasserstoff, Deuterium, Halogen, $C_{1-6}$ lineares, verzweigtes Alkyl oder Cycloalkyl sind; oder $R_b$ und $R_c$ zu einem 5- oder 6-gliedrigen Cycloalkyl oder einem 5- oder 6-gliedrigen nichtaromatischen heterocyclischen Ring mit 1 oder 2 Heteroatomen gebunden sind, ausgewählt aus einem Stickstoffatom, einem Sauerstoffatom und einem Schwefelatom; oder, $R_d$ und $R_e$ zu einem 5- oder 6-gliedrigen Cycloalkyl oder einem 5- oder 6-gliedrigen nichtaromatischen heterocyclischen Ring mit 1 oder 2 Heteroatomen gebunden sind, ausgewählt aus einem Stickstoffatom, einem Sauerstoffatom und einem Schwefelatom;
X für O, S oder $-CH_2-$ steht;
$L_1$ eine Einfachbindung, -O, -S- oder -NH- ist;

$L_2$ eine Einfachbindung ist;
$R_2$ ausgewählt ist aus einer Gruppe, die durch die folgende Formel dargestellt wird:

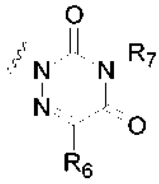
,

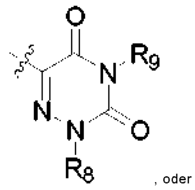
, oder
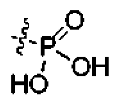
;

wobei $R_6$ Wasserstoff, Cyano, $C_{1-6}$ Alkyl oder $C_{1-6}$ Halogenalkyl ist;
$R_8$ Wasserstoff, Cyano, $C_{1-6}$ Alkyl oder $C_{1-6}$ Halogenalkyl ist; und
$R_7$ und $R_9$ Wasserstoff, $C_{1-3}$ Alkyl oder $C_{1-3}$ Halogenalkyl sind.

**10.** Verbindung oder pharmazeutisch verträgliches Salz davon nach einem der Ansprüche 2, 8 und 9, wobei $R_1$ $C_{1-6}$ lineares oder verzweigtes Alkyl, Benzyl oder $C_{5-6}$ Cycloalkylmethylen ist, gegebenenfalls substituiert mit Wasserstoff, Deuterium, Tritium, $C_{1-6}$ Alkyl, Hydroxy, Halogen oder CN und weiter bevorzugt Isopropyl oder Benzyl;

wobei $R_b$ und $R_d$ Halogen sind, $R_c$ und $R_e$ Wasserstoff sind, und $R_b$ und $R_d$ weiterhin bevorzugt Chlor sind;
X für O, S oder $-CH_2-$ steht;
$L_1$ eine Einfachbindung, -O, -S- oder -NH- ist;
$L_2$ eine Einfachbindung oder $-CH_2-$ ist;
$R_2$ ausgewählt ist aus einer Gruppe, die durch die folgende Formel dargestellt wird:

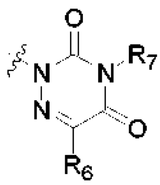
,

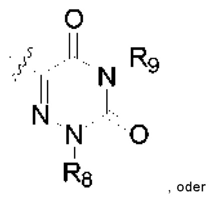
, oder
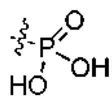
;

und
$R_6$, $R_7$, $R_8$ und $R_9$ Wasserstoff oder $C_{1-6}$ Alkyl oder $C_{3-8}$ Cycloalkyl sind.

**11.** Verbindung oder pharmazeutisch verträgliches Salz davon nach Anspruch 1, wobei $R_1$ Wasserstoff, $C_{1-10}$ Alkyl, $C_{3-10}$ Cycloalkyl, $C_{3-10}$ Cycloalkyl $C_{1-6}$ Alkyl, $C_{5-10}$ Aryl, $C_{5-10}$ Aryl $C_{1-6}$ Alkyl, 5-10 gliedriges Heterocyclyl mit 1 bis 3 Heteroatomen sind, ausgewählt aus einem Stickstoffatom, einem Sauerstoffatom und einem Schwefelatom,

5-10 gliedriges Heteroaryl mit 1 bis 3 Heteroatomen, ausgewählt aus einem Stickstoffatom, einem Sauerstoffatom und einem Schwefelatom, eine Aminosäure oder $-COR_{10}$, und das $C_{1-10}$ Alkyl, das $C_{3-10}$ Cycloalkyl, das $C_{3-10}$ Cycloalkyl $C_{1-6}$ Alkyl, das $C_{5-10}$ Aryl, das $C_{5-10}$ arylc$_{1-6}$ Alkyl, wobei das 5-10 gliedrige Heterocyclyl 1 bis 3 Heteroatome enthält, ausgewählt aus einem Stickstoffatom,
einem Sauerstoffatom und einem Schwefelatom, wobei das 5-10 gliedrige Heteroaryl 1 bis 3 Heteroatome

enthält, die ausgewählt sind aus einem Stickstoffatom, einem Sauerstoffatom und einem Schwefelatom, oder die Aminosäure unsubstituiert ist oder substituiert sein kann durch Deuterium, Tritium, $C_{1-6}$ Alkyl, Hydroxyl, Halogen oder CN;

X für Methylen, -O-, -S- oder $-SO_2-$ steht;

$R^a$ Wasserstoff, Deuterium, Halogen, $C_{1-6}$ lineares oder verzweigtes Alkyl oder Cycloalkyl ist; oder zwei benachbarte $R^a$ zu einem 5-10 gliedrigen carbocyclischen Ring oder einem 5-10-gliedrigen heterocyclischen Ring mit 1 bis 3 Heteroatomen, ausgewählt aus einem Stickstoffatom, einem Sauerstoffatom und einem Schwefelatom, gebunden sind;

$L_1$ eine Einfachbindung, Methylen, -O-, -CO-, $-NR_3-$, $-NR_3CO-$, $-CONR_3-$, $-CH_2NR_3-$, oder -S- ist;

$L_2$ eine Einfachbindung oder $C_{1-6}$ Alkyl ist;

$R_2$ eine Carboxylgruppe oder eine durch die folgende Formel dargestellte Gruppe ist:

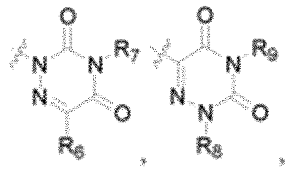
, ,

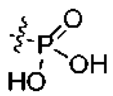
,
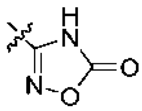
, oder
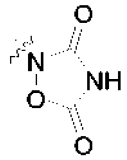
;

wobei $R_3$ Wasserstoff oder $C_{1-6}$ Alkyl ist;

$R_6$ Wasserstoff, Cyano, Aminosäure, COOH, $C_{1-6}$ Alkyl oder $C_{1-6}$ Halogenalkyl ist;

$R_8$ Wasserstoff, Cyano, COOH, $C_{1-6}$ Alkyl oder $C_{1-6}$ Halogenalkyl ist;

$R_7$ und $R_9$ Wasserstoff, $C_{1-3}$ Alkyl oder $C_{1-3}$ Halogenalkyl sind;

$R_{10}$ $C_{3-10}$ Cycloalkyl, $C_{5-10}$ Aryl, 5-10 gliedriges Heterocyclyl mit 1 bis 3 Heteroatomen ist, ausgewählt aus einem Stickstoffatom, einem Sauerstoffatom und einem Schwefelatom, oder 5-10 gliedriges Heteroaryl mit 1 bis 3 Heteroatomen, ausgewählt aus Stickstoffatom, Sauerstoffatom und Schwefelatom; und

n für 0, 1, 2, 3 oder 4 steht; und

wobei vorzugsweise,

$R_1$ Methyl, Ethyl, Propyl, Butyl, Pentyl, Cyclopropan, Cyclobutan, Cyclopentan, Cyclohexan, Cyclopropanmethyl, Cyclobutanmethyl, Cyclopentanmethyl, Cyclohexanmethyl, Phenyl, Benzyl oder $-COR_{10}$ ist, und das Methyl, das Ethyl, das Propyl, das Butyl, das Pentyl, das Cyclopropan, das Cyclobutan, das Cyclopentan, das Cyclohexan, das Cyclopropanmethyl, das Cyclobutanmethyl,

das Cyclopentanmethyl, das Cyclohexanmethyl, das Phenyl oder das Benzyl unsubstituiert ist oder durch Deuterium substituiert sein kann, $C_{1-3}$ Alkyl, Hydroxyl, Halogen oder CN ist;

X für Methylen, -O-, -S- oder $-SO_2-$ steht;

$R^a$ Halogen; oder zwei benachbarte $R^a$ zu einem 5-gliedrigen carbocyclischen Ring oder einem 5-gliedrigen heterocyclischen Ring mit 1 bis 2 Heteroatomen, ausgewählt aus Stickstoffatom, Sauerstoffatom und Schwefelatom, gebunden sind;

$L_1$ eine Einfachbindung, -O-, -NH-, -NHCO-, -CONH-, $-CH_2NH-$, oder -S- ist;

$L_2$ eine Einfachbindung, Methyl, Ethyl oder Propyl ist;

$R_2$ eine Carboxylgruppe oder eine durch die folgende Formel dargestellte Gruppe ist:

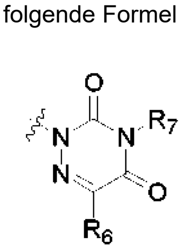
,

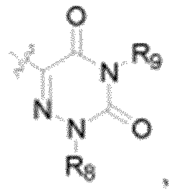 , 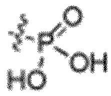 , oder 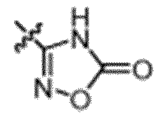 ;

$R_6$ Wasserstoff, Cyano, COOH, Methyl, Ethyl oder Propyl ist;
$R_8$ Wasserstoff, Methyl, Ethyl oder Propyl ist;
$R_7$ und $R_9$ Wasserstoff oder Methyl sind;
$R_{10}$ Phenyl ist; und
n für 2 oder 3 steht;
wobei vorzugsweise,
$R_1$ Methyl, Ethyl, Propyl, Butyl, Pentyl, Cyclopropan, Cyclobutan, Cyclopentan, Cyclohexan, Cyclopropanmethyl, Cyclobutanmethyl, Cyclopentanmethyl, Cyclohexanmethyl, Phenyl oder Benzyl ist, und das Methyl, das Ethyl, das Propyl,

das Butyl, das Pentyl, das Cyclopropan, das Cyclobutan,
das Cyclopentan, das Cyclohexan, das Cyclopropanmethyl, das Cyclobutanmethyl, das Cyclopentanmethyl, das Cyclohexanmethyl, das Phenyl oder das Benzyl unsubstituiert ist oder durch Deuterium substituiert sein kann, $C_{1-3}$ Alkyl, Hydroxyl, F, Cl, Br oder CN;

X für Methylen, -O- oder -S steht-;
$R^a$ für F, Cl oder Br steht; oder zwei benachbarte $R^a$ zu einem 5-gliedrigen carbocyclischen Ring oder einem 5-gliedrigen heterocyclischen Ring mit 1 bis 2 Heteroatomen gebunden sind, ausgewählt aus einem Stickstoffatom, einem Sauerstoffatom und einem Schwefelatom;
$L_1$ eine Einfachbindung, -O-, -NH- oder -NHCO- ist;
$L_2$ eine Einfachbindung, Methyl, Ethyl oder Propyl ist;
$R_2$ eine Carboxylgruppe oder eine durch die folgende Formel dargestellte Gruppe ist:

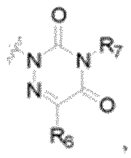 , 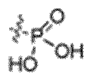 , oder 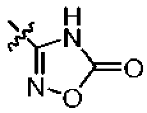 ;

$R_6$ Wasserstoff, Cyano oder Methyl ist;
$R_7$ Wasserstoff ist; und

n für 2 oder 3 steht.

**12.** Verbindung der folgenden Formel oder ein pharmazeutisch verträgliches Salz davon:

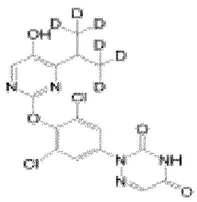 , 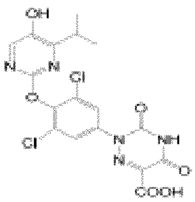 , 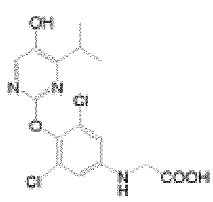 , 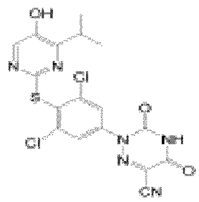 , 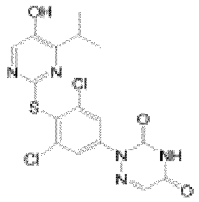 ,

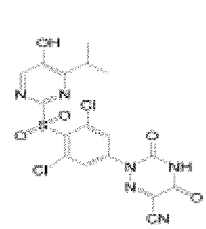 ,
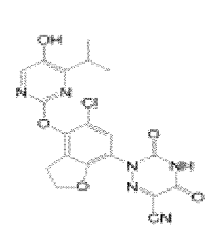 ,
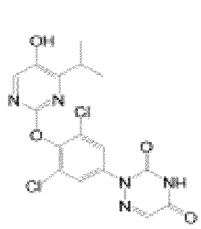 ,
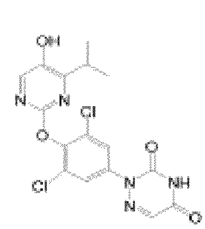 ,
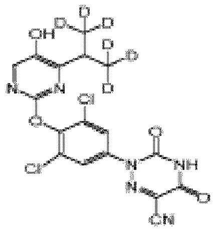 ,

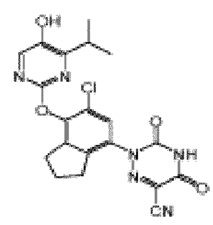 ,
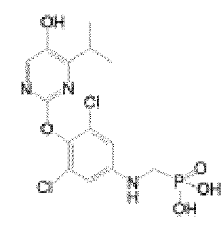 ,
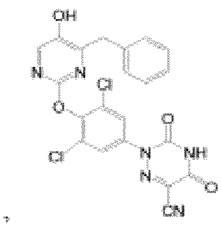 ,
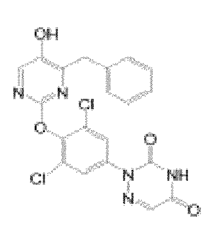 ,
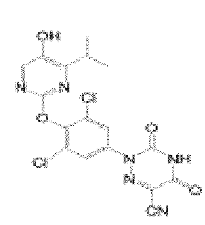 ,

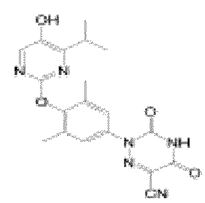 ,
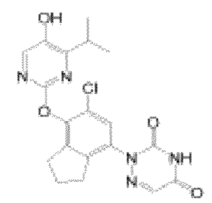 ,
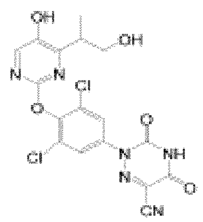 ,
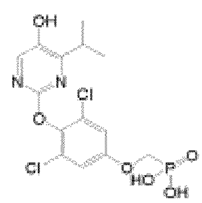 ,
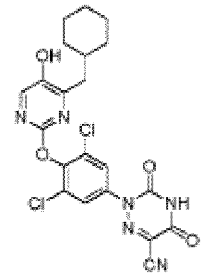 ,

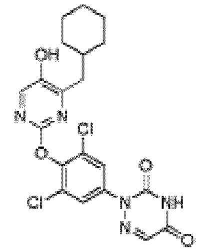 ,
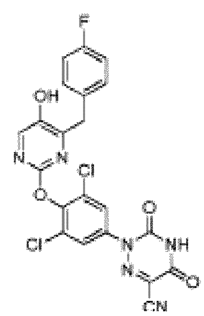 ,
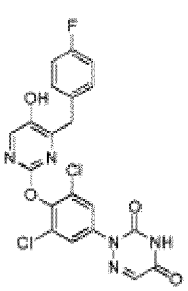 ,
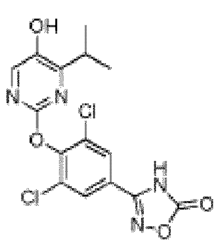 ,
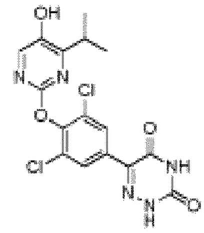 ,

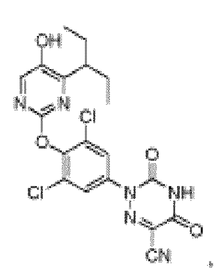 ,
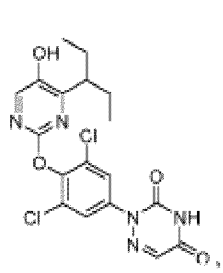 ,
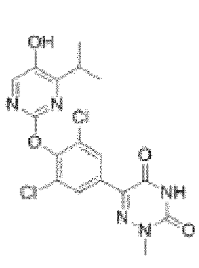 ,
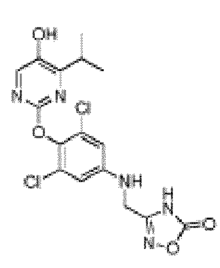 ,
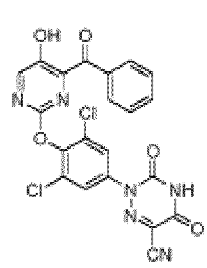 ,

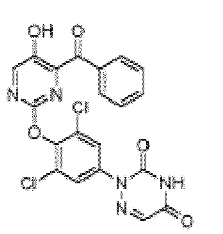 ,
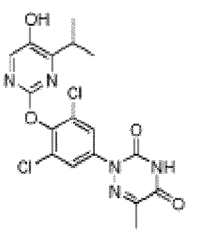 ,
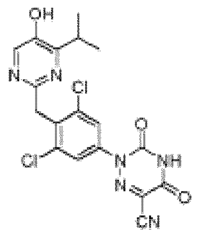 ,
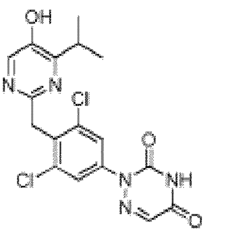 ,

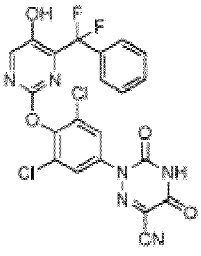,
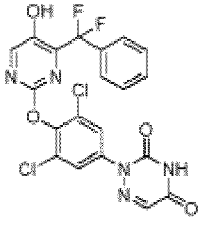,
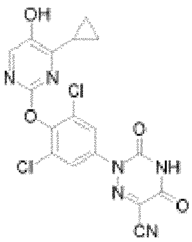,
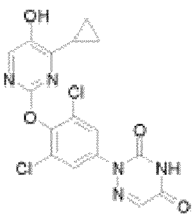,
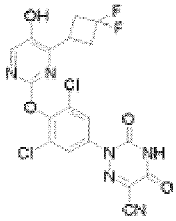,

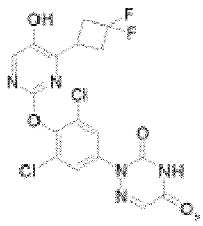,
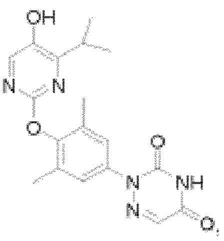, oder
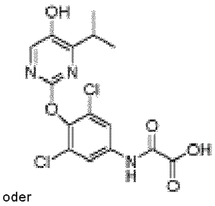

**13.** Pharmazeutische Zusammensetzung, umfassend die Verbindung oder das pharmazeutisch verträgliche Salz davon nach einem der Ansprüche 1 bis 12, und einen oder mehrere pharmazeutisch akzeptable Träger.

**14.** Verbindung oder pharmazeutisch verträgliches Salz davon nach einem der Ansprüche 1 bis 12, oder pharmazeutische Zusammensetzung nach Anspruch 13 zur Verwendung als Medikament.

**15.** Verbindung oder pharmazeutisch verträgliches Salz davon nach einem der Ansprüche 1 bis 12, oder pharmazeutische Zusammensetzung nach Anspruch 13 zur Verwendung bei der Vorbeugung oder Behandlung einer Krankheit, die mit einer β-Rezeptoragonistenwirkung zusammenhängt,

wobei vorzugsweise die Krankheit, die mit der β-Rezeptor-Agonistenwirkung zusammenhängt, Fettleibigkeit, Hyperlipidämie, Hypercholesterinämie, Hypertriglyceridämie, Dyslipidämie, Schilddrüsenkrebs, metabolisches Syndrom, Herz-Kreislauf-Erkrankung, koronare Herzkrankheit, Myokardinfarkt, ventrikuläre Insuffizienz, Herzinsuffizienz,
Fettleber, Zirrhose, Diabetes, Steatohepatitis, nichtalkoholische Steatohepatitis, nichtalkoholische Fettlebererkrankung, Atherosklerose oder Hypothyreose-Erkrankung oder -Störung ist.

## Revendications

**1.** Composé de formule I ou sel pharmaceutiquement acceptable de celui-ci :

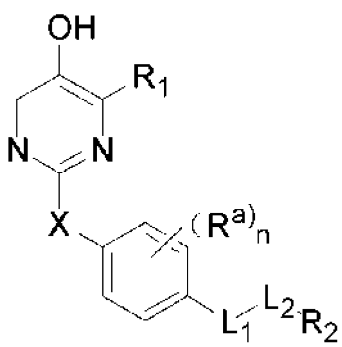

Formule I

dans lequel,

$R_1$ est un hydrogène, un alkyle facultativement substitué, un cycloalkyle facultativement substitué, un aryle

facultativement substitué, un hétérocyclyle facultativement substitué, un hétéroaryle facultativement substitué, un amino facultativement substitué, un carbamoyle facultativement substitué, ou $-COR_{10}$ ;

X est facultativement un méthylène substitué, -O-, -S- ou $-SO_2-$ ;

$R^a$ est sélectionné parmi un hydrogène, un halogène, un alkyle linéaire et ramifié en $C_{1-6}$, ou un cycloalkyle ; ou deux $R^a$ adjacents sont liés pour former un cycle carbocyclique, ou un cycle hétérocyclique ;

$L_1$ est une liaison simple, un méthylène, -CH=CH-, -O-, -CO-, $-NR_3-$, $-NR_3CO-$, $-CONR_3-$, $-CH_2NR_3$ -, ou -S- ;

$L_2$ est une liaison simple, ou $-(CR_4R_5)_P$ ;

$R_2$ est un carboxyle, ou un groupe représenté par la formule suivante :

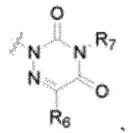
,

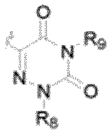
, 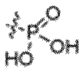
, 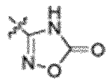
, 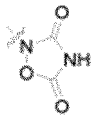
, 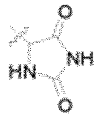
, 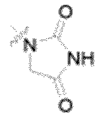
, 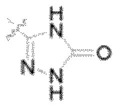
;

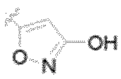
, 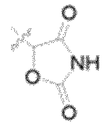
, ou 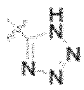
;

$R_3$ est un hydrogène, ou un alkyle facultativement substitué ;

$R_4$ et $R_5$ sont chacun indépendamment sélectionnés parmi un hydrogène, un halogène ou un alkyle facultativement substitué, ou $R_4$ et $R_5$ sont liés pour former un cycloalkyle ;

$R_6$ est un hydrogène, un cyano, un amino, COOH, un alkyle en $C_{1-6}$, un haloalkyle en $C_{1-6}$, un cycloalkyle en $C_{3-6}$ ou un halocycloalkyle en $C_{3-6}$ ;

$R_8$ est un hydrogène, un cyano, COOH, un alkyle en $C_{1-6}$, un haloalkyle en $C_{1-6}$, un cycloalkyle en $C_{3-6}$ ou un halocycloalkyle en $C_{3-6}$ ;

$R_7$ et $R_9$ sont un hydrogène, un alkyle en $C_{1-3}$ ou un haloalkyle en $C_{1-3}$ ;

$R_{10}$ est un alkyle facultativement substitué, un amino, un hydroxyle, un cycloalkyle facultativement substitué, un aryle facultativement substitué, un hétérocyclyle facultativement substitué ou un hétéroaryle facultativement substitué ;

n est 0, 1, 2, 3 ou 4 ; et

p est 0, 1 ou 2.

**2.** Composé, ou sel pharmaceutiquement acceptable de celui-ci selon la revendication 1, dans lequel le composé de formule I est représenté dans la formule II :

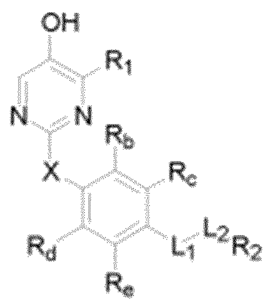

Formule II

dans lequel, $R_b$, $R_c$, $R_d$ et $R_e$ sont un hydrogène, un deutérium, un halogène, un alkyle linéaire ou ramifié en $C_{1-6}$, ou un cycloalkyle ; ou, $R_b$ et $R_c$ sont liés pour former un cycloalkyle à 5 ou 6 chaînons, ou un cycle hétérocyclique non

aromatique à 5 ou 6 chaînons contenant 1 ou 2 hétéroatomes sélectionnés parmi un atome d'azote, un atome d'oxygène et un atome de soufre ; ou, $R_d$ et $R_e$ sont liés pour former un cycloalkyle à 5 ou 6 chaînons, ou un cycle hétérocyclique non aromatique à 5 ou 6 chaînons contenant 1 ou 2 hétéroatomes sélectionnés parmi un atome d'azote, un atome d'oxygène et un atome de soufre.

**3.** Composé ou sel pharmaceutiquement acceptable de celui-ci selon la revendication 1 ou la revendication 2, dans lequel le composé de formule I est représenté dans la formule III :

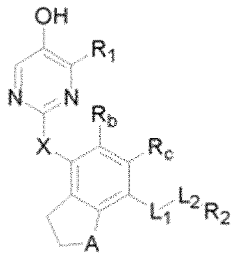

Formule (III)

dans lequel,

$R_b$ et $R_c$ sont un hydrogène, un deutérium, un halogène, un alkyle linéaire ou ramifié en $C_{1-6}$ ou un cycloalkyle ; et A est O, ou un méthylène.

**4.** Composé ou sel pharmaceutiquement acceptable de celui-ci selon une quelconque revendication précédente, dans lequel $R_1$ est sélectionné parmi :

1) un alkyle linéaire et ramifié en $C_{1-6}$ facultativement substitué ;
2) un cycloalkyle en $C_{3-8}$ facultativement substitué ;
3) un hétérocyclyle non aromatique en $C_{3-8}$ facultativement substitué contenant 1 à 3 hétéroatomes sélectionnés parmi un atome d'azote, un atome d'oxygène et un atome de soufre ;
4) un phényle facultativement substitué ; ou
5) un hétéroaryle en $C_{5-6}$ facultativement substitué contenant 1 à 3 hétéroatomes sélectionnés parmi un atome d'azote, un atome d'oxygène et un atome de soufre.

**5.** Composé ou sel pharmaceutiquement acceptable de celui-ci selon une quelconque revendication précédente, dans lequel $R_1$ est sélectionné parmi $-(CR_{11}R_{12})_mR_{13}$ ; $R_{11}$ et $R_{12}$ sont sélectionnés parmi un hydrogène, un deutérium, un halogène, un hydroxyle, un amino, ou un alkyle en $C_{1-4}$ facultativement substitué ; et $R_{13}$ est sélectionné parmi :

1) un hydrogène ou un deutérium ;
2) un halogène ;
3) un hydroxyle ;
4) un amino ;
5) un carboxyle ;
6) un alkyle en $C_{1-4}$ facultativement substitué, ou un alcoxy en $C_{1-4}$ ;
7) un cycloalkyle en $C_{3-8}$ facultativement substitué ;
8) un hétérocyclyle non aromatique en $C_{3-8}$ facultativement substitué contenant 1 à 3 hétéroatomes sélectionnés parmi un atome d'azote, un atome d'oxygène et un atome de soufre ;
9) un phényle facultativement substitué ; ou
10) un hétéroaryle en $C_{5-6}$ facultativement substitué contenant 1 à 3 hétéroatomes sélectionnés parmi un atome d'azote, un atome d'oxygène et un atome de soufre ; et m est 0, 1, 2 ou 3.

**6.** Composé ou sel pharmaceutiquement acceptable de celui-ci selon une quelconque revendication précédente, dans lequel $R_1$ est sélectionné parmi $-COR_{10}$, et $R_{10}$ est sélectionné parmi :

1) un amino ;
2) un hydroxyle ;
3) un alkyle en $C_{1-4}$ facultativement substitué, ou un alcoxy en $C_{1-4}$ ;

4) un cycloalkyle en $C_{3-8}$ facultativement substitué ;
5) un hétérocyclyle non aromatique en $C_{3-8}$ facultativement substitué contenant 1 à 3 hétéroatomes sélectionnés parmi un atome d'azote, un atome d'oxygène et un atome de soufre ;
6) un phényle facultativement substitué ; ou
7) un hétéroaryle en $C_{5-6}$ facultativement substitué contenant 1 à 3 hétéroatomes sélectionnés parmi un atome d'azote, un atome d'oxygène et un atome de soufre.

**7.** Composé ou sel pharmaceutiquement acceptable de celui-ci selon une quelconque revendication précédente, dans lequel $R_1$ est sélectionné parmi :

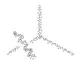, 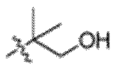, 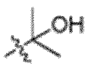, 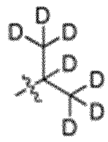,

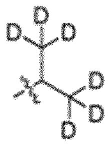, 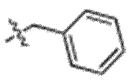, 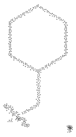, 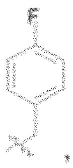, 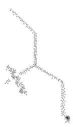, 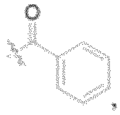, 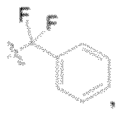, 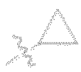, ou 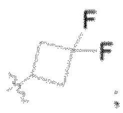 ;

de préférence, $R_b$, $R_c$, $R_d$ et $R_e$ sont sélectionnés parmi un hydrogène, un deutérium ou un halogène ;
de préférence, $L_1$ est sélectionné parmi une liaison simple, -O-, -NH-, ou -NHCO- ;
de préférence, $L_2$ est sélectionné parmi une liaison simple, ou un méthylène ; et
de préférence, $R_2$ est sélectionné parmi :

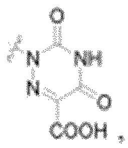, 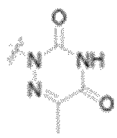, 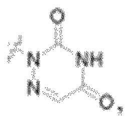, 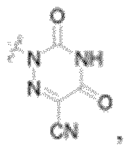, 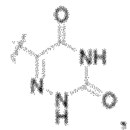,

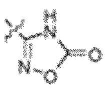, 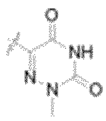, ou 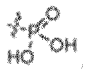

**8.** Composé ou sel pharmaceutiquement acceptable de celui-ci selon la revendication 2, dans lequel $R_1$ est un alkyle linéaire ou ramifié en $C_{1-6}$ facultativement substitué, ou un cycloalkyle en $C_{3-8}$ ;

X est O, S, ou $-CH_2-$ ;
$R_b$, $R_c$, $R_d$ et $R_e$ sont un hydrogène, un deutérium, un halogène, un alkyle linéaire ou ramifié en $C_{1-6}$, ou un cycloalkyle ; ou $R_b$ et $R_c$ sont liés pour former un cycloalkyle à 5 ou 6 chaînons, ou un cycle hétérocyclique non aromatique à 5 ou 6 chaînons contenant 1 ou 2 hétéroatomes sélectionnés parmi un atome d'azote, un atome d'oxygène et un atome de soufre ; ou, $R_d$ et $R_e$ sont liés pour former un cycloalkyle à 5 ou 6 chaînons, ou un cycle hétérocyclique non aromatique à 5 ou 6 chaînons contenant 1 ou 2 hétéroatomes sélectionnés parmi un atome d'azote, un atome d'oxygène et un atome de soufre ;
$L_1$ est une liaison simple, $-NR_3-$, -O, ou -S- ;
$L_2$ est une liaison simple, ou $-CH_2-$ ;
$R_2$ est sélectionné dans un groupe représenté par la formule suivante :

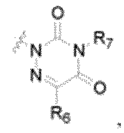,

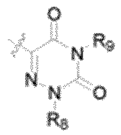, 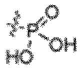, 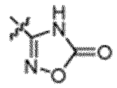, 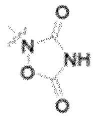, 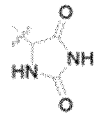, 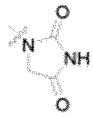, 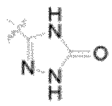,

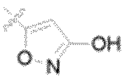, 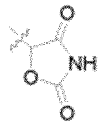, ou 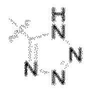 ;

$R_3$ est un hydrogène, ou un alkyle en $C_{1-6}$ facultativement substitué ;
$R_6$ est un hydrogène, un cyano, un amino, COOH, un alkyle en $C_{1-6}$, un haloalkyle en $C_{1-6}$, un cycloalkyle en $C_{3-6}$ ou un halocycloalkyle en $C_{3-6}$ ;
$R_8$ est un hydrogène, un cyano, COOH, un alkyle en $C_{1-6}$, un haloalkyle en $C_{1-6}$, un cycloalkyle en $C_{3-6}$ ou un halocycloalkyle en $C_{3-6}$ ; et
$R_7$ et $R_9$ sont un hydrogène, un alkyle en $C_{1-3}$ ou un haloalkyle en $C_{1-3}$.

**9.** Composé ou sel pharmaceutiquement acceptable de celui-ci selon la revendication 2 ou la revendication 8, dans lequel $R_1$ est un alkyle linéaire ou ramifié en $C_{1-6}$ facultativement substitué ;

$R_b$, $R_c$, Rd et $R_e$ sont un hydrogène, un deutérium, un halogène, un alkyle linéaire, ramifié en $C_{1-6}$, ou un cycloalkyle ; ou $R_b$ et $R_c$ sont liés pour former un cycloalkyle à 5 ou 6 chaînons, ou un cycle hétérocyclique non aromatique à 5 ou 6 chaînons contenant 1 ou 2 hétéroatomes sélectionnés parmi un atome d'azote, un atome d'oxygène et un atome de soufre ; ou, $R_d$ et $R_e$ sont liés pour former un cycloalkyle à 5 ou 6 chaînons, ou un cycle hétérocyclique non aromatique à 5 ou 6 chaînons contenant 1 ou 2 hétéroatomes sélectionnés parmi un atome d'azote, un atome d'oxygène et un atome de soufre ;
X est O, S ou $-CH_2-$ ;
$L_1$ est une liaison simple, -O, -S-, ou -NH- ;
$L_2$ est une liaison simple ;
$R_2$ est sélectionné dans un groupe représenté par la formule suivante :

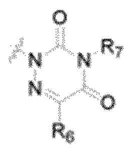,

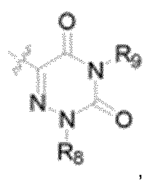, ou 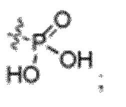 ;

$R_6$ est un hydrogène, un cyano, un alkyle en $C_{1-6}$ ou un haloalkyle en $C_{1-6}$ ;
$R_8$ est un hydrogène, un cyano, un alkyle en $C_{1-6}$ ou un haloalkyle en $C_{1-6}$ ; et
$R_7$ et $R_9$ sont un hydrogène, un alkyle en $C_{1-3}$ ou un haloalkyle en $C_{1-3}$.

**10.** Composé ou sel pharmaceutiquement acceptable de celui-ci selon l'une quelconque des revendications 2, 8 et 9, dans lequel $R_1$ est un alkyle linéaire ou ramifié en $C_{1-6}$, un benzyle, ou un cycloalkylméthylène en $C_{5-6}$ facultativement substitué avec un hydrogène, un deutérium, un tritium, un alkyle en $C_{1-6}$, un hydroxyle, un halogène, ou CN, et en

outre de préférence un isopropyle, ou un benzyle ;

$R_b$ et $R_d$ sont un halogène, $R_c$ et $R_e$ sont un hydrogène, et $R_b$ et $R_d$ sont en outre de préférence un chlore ;
X est O, S ou $-CH_2-$ ;
$L_1$ est une liaison simple, -O, -S-, ou -NH- ;
$L_2$ est une liaison simple, ou $-CH_2-$ ;
$R_2$ est sélectionné dans un groupe représenté par la formule suivante :

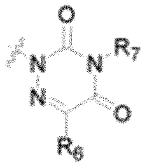
,

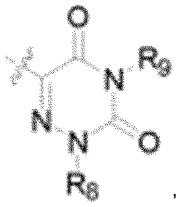
, ou
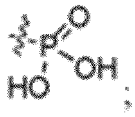
;

et
$R_6$, $R_7$, $R_8$ et $R_9$ sont un hydrogène, ou un alkyle en $C_{1-6}$, ou un cycloalkyle en $C_{3-8}$.

**11.** Composé ou sel pharmaceutiquement acceptable de celui-ci selon la revendication 1, dans lequel $R_1$ est un hydrogène, un alkyle en $C_{1-10}$, un cycloalkyle en $C_{3-10}$, un cycloalkyle en $C_{3-10}$-alkyle en $C_{1-6}$, un aryle en $C_{5-10}$, un aryle en $C_{5-10}$-alkyle en $C_{1-6}$, un hétérocyclyle de 5 à 10 chaînons contenant 1 à 3 hétéroatomes sélectionnés parmi un atome d'azote, un atome d'oxygène et un atome de soufre, un hétéroaryle de 5 à 10 chaînons contenant 1 à 3 hétéroatomes sélectionnés parmi un atome d'azote, un atome d'oxygène et un atome de soufre, un amino, ou $-COR_{10}$, et l'alkyle en $C_{1-10}$, le cycloalkyle en $C_{3-10}$, le cycloalkyle en $C_{3-10}$-alkyle en $C_{1-6}$, l'aryle en $C_{5-10}$, l'aryle en $C_{5-10}$-alkyle en $C_{1-6}$, l'hétérocyclyle de 5 à 10 chaînons contenant 1 à 3 hétéroatomes sélectionnés parmi un atome d'azote, un atome d'oxygène et un atome de soufre, l'hétéroaryle de 5 à 10 chaînons contenant 1 à 3 hétéroatomes sélectionnés parmi un atome d'azote, un atome d'oxygène et un atome de soufre, ou l'amino est non substitué, ou peut être substitué avec un deutérium, un tritium, un alkyle en $C_{1-6}$, un hydroxyle, un halogène, ou CN ;

X est un méthylène, -O-, -S-, ou $-SO_2-$ ;
$R^a$ est un hydrogène, un deutérium, un halogène, un alkyle linéaire ou ramifié en $C_{1-6}$, ou un cycloalkyle ; ou deux $R^a$ adjacents sont liés pour former un cycle carbocyclique de 5 à 10 chaînons, ou un cycle hétérocyclique de 5 à 10 chaînons contenant 1 à 3 hétéroatomes sélectionnés parmi un atome d'azote, un atome d'oxygène et un atome de soufre ;
$L_1$ est une liaison simple, un méthylène, -O-, -CO-, $-NR_3-$, $-NR_3CO-$, $-CONR_3-$, $-CH_2NR_3-$, ou -S- ;
$L_2$ est une liaison simple, ou un alkyle en $C_{1-6}$ ;
$R_2$ est un carboxyle, ou un groupe représenté par la formule suivante :

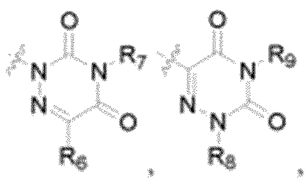
, ,

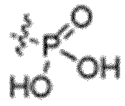, 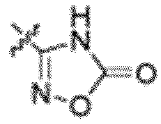, ou 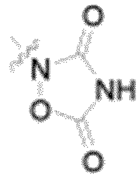;

$R_3$ est un hydrogène, ou un alkyle en $C_{1-6}$ ;
$R_6$ est un hydrogène, un cyano, un amino, COOH, un alkyle en $C_{1-6}$, ou un haloalkyle en $C_{1-6}$;
$R_8$ est un hydrogène, un cyano, COOH, un alkyle en $C_{1-6}$ ou un haloalkyle en $C_{1-6}$ ;
$R_7$ et $R_9$ sont un hydrogène, un alkyle en $C_{1-3}$, ou un haloalkyle en $C_{1-3}$ ;
$R_{10}$ est un cycloalkyle en $C_{3-10}$, un aryle en $C_{5-10}$, un hétérocyclyle de 5 à 10 chaînons contenant 1 à 3 hétéroatomes sélectionnés parmi un atome d'azote, un atome d'oxygène et un atome de soufre, ou un hétéroaryle de 5 à 10 chaînons contenant 1 à 3 hétéroatomes sélectionnés parmi un atome d'azote, un atome d'oxygène et un atome de soufre ; et
n est 0, 1, 2, 3 ou 4 ; et
de préférence,
$R_1$ est un méthyle, un éthyle, un propyle, un butyle, un pentyle, un cyclopropane, un cyclobutane, un cyclopentane, un cyclohexane, un cyclopropaneméthyle, un cyclobutaneméthyle, un cyclopentaneméthyle, un cyclohexaneméthyle, un phényle, un benzyle, ou $-COR_{10}$, et le méthyle, l'éthyle, le propyle, le butyle, le pentyle, le cyclopropane, le cyclobutane, le cyclopentane, le cyclohexane, le cyclopropaneméthyle, le cyclobutaneméthyle, le cyclopentaneméthyle, le cyclohexaneméthyle, le phényle ou le benzyle est non substitué, ou peut être substitué avec un deutérium, un alkyle en $C_{1-3}$, un hydroxyle, un halogène, ou CN ;
X est un méthylène, -O-, -S-, ou $-SO_2-$ ;
$R^a$ est un halogène ; ou deux $R^a$ adjacents sont liés pour former un cycle carbocyclique à 5 chaînons, ou un cycle hétérocyclique à 5 chaînons contenant 1 à 2 hétéroatomes sélectionnés parmi un atome d'azote, un atome d'oxygène et un atome de soufre ;
$L_1$ est une liaison simple, -O-, -NH-, -NHCO-, -CONH-, $-CH_2NH-$, ou -S- ;
$L_2$ est une liaison simple, un méthyle, un éthyle, ou un propyle ;
$R_2$ est un carboxyle, ou un groupe représenté par la formule suivante :

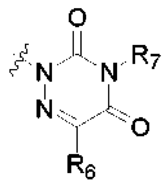,

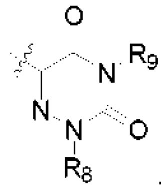, 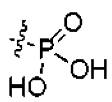, ou 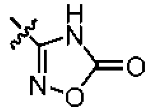;

$R_6$ est un hydrogène, un cyano, COOH, un méthyle, un éthyle, ou un propyle ;
$R_8$ est un hydrogène, un méthyle, un éthyle, ou un propyle ;
$R_7$ et $R_9$ sont un hydrogène, ou un méthyle ;
$R_{10}$ est un phényle ; et
n est 2 ou 3 ;
de préférence,
$R_1$ est un méthyle, un éthyle, un propyle, un butyle, un pentyle, un cyclopropane, un cyclobutane, un cyclopentane, un cyclohexane, un cyclopropaneméthyle, un cyclobutaneméthyle, un cyclopentaneméthyle, un cyclohexaneméthyle, un phényle, ou un benzyle, et le méthyle, l'éthyle, le propyle, le butyle, le pentyle, le cyclopropane, le cyclobutane, le cyclopentane, le cyclohexane, le cyclopropaneméthyle, le cyclobutaneméthyle, le cyclopentaneméthyle, le cyclohexaneméthyle, le phényle ou le benzyle est non substitué, ou peut être substitué avec un deutérium, un alkyle en $C_{1-3}$, un hydroxyle, F, Cl, Br ou CN ;

X est un méthylène, -O- ou -S- ;

$R^a$ est F, Cl ou Br ; ou deux $R^a$ adjacents sont liés pour former un cycle carbocyclique à 5 chaînons, ou un cycle hétérocyclique à 5 chaînons contenant 1 à 2 hétéroatomes sélectionnés parmi un atome d'azote, un atome d'oxygène et un atome de soufre ;

$L_1$ est une liaison simple, -O-, -NH-, ou -NHCO- ;

$L_2$ est une liaison simple, un méthyle, un éthyle, ou un propyle ;

$R_2$ est un carboxyle, ou un groupe représenté par la formule suivante :

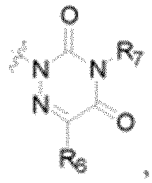

,

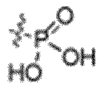

,

ou

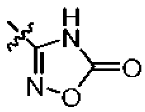

;

$R_6$ est un hydrogène, un cyano, ou un méthyle ;

$R_7$ est un hydrogène ; et

n est 2 ou 3.

**12.** Composé de formule suivante ou sel pharmaceutiquement acceptable de celui-ci :

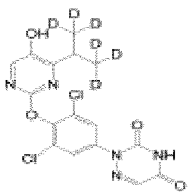
,
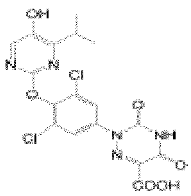
,
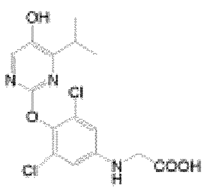
,
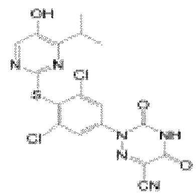
,
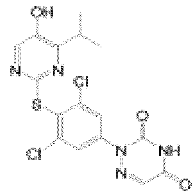
,
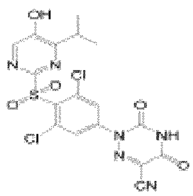
,
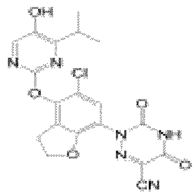
,
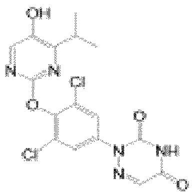
,
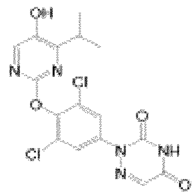
,
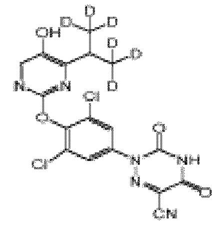
,
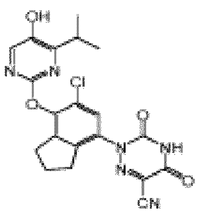
,
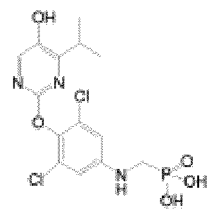
,
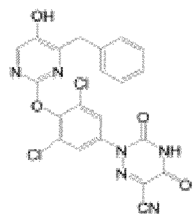
,
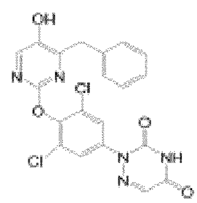
,
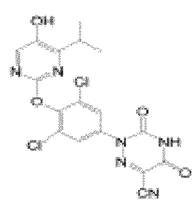
,

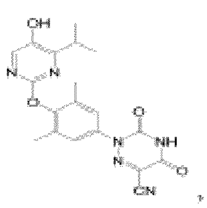 , 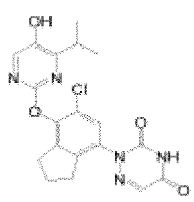 , 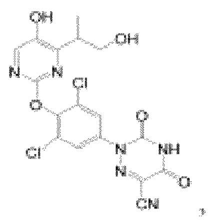 , 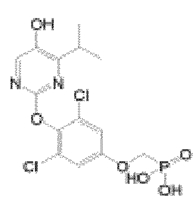 , 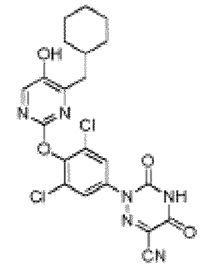 ,

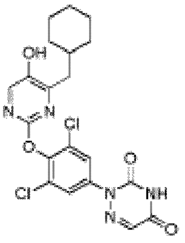 , 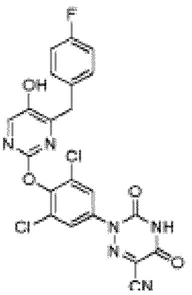 , 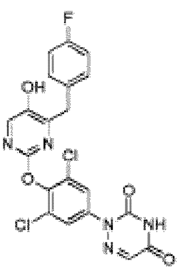 , 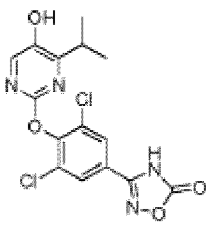 , 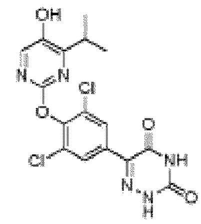 ,

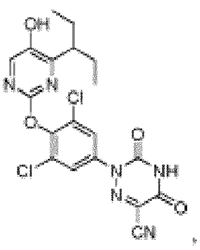 , 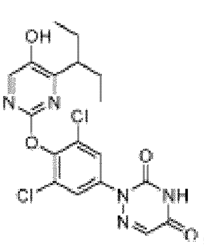 , 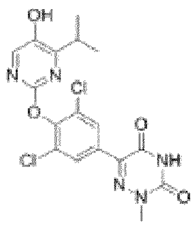 , 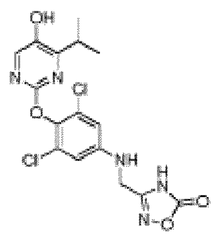 , 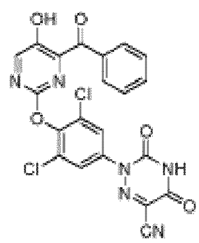 ,

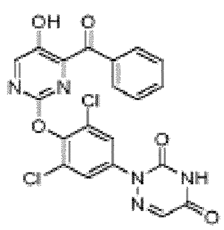 , 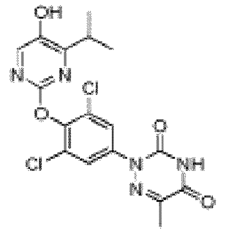 , 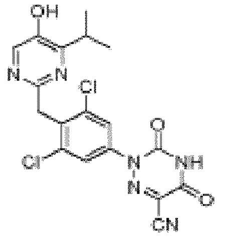 , 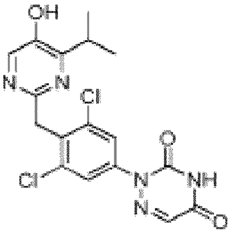 ,

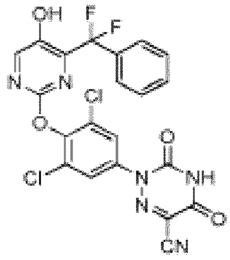 , 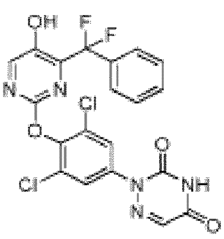 , 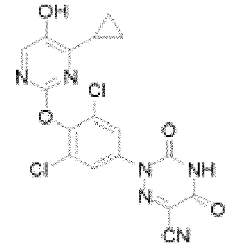 , 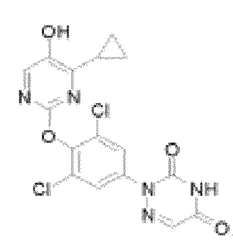 , 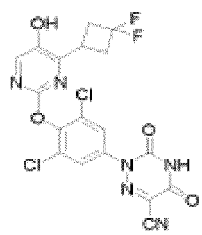 ,

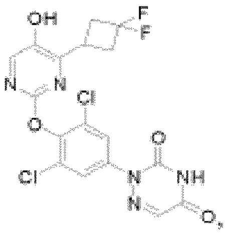 , 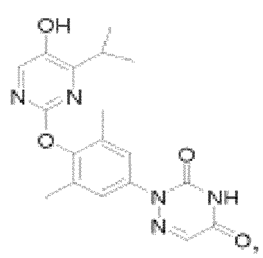 , ou 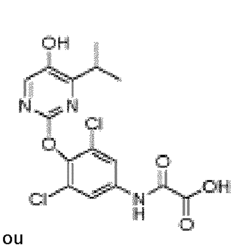 .

**13.** Composition pharmaceutique comprenant le composé ou le sel pharmaceutiquement acceptable de celui-ci selon l'une quelconque des revendications 1 à 12, et un ou plusieurs supports pharmaceutiquement acceptables.

**14.** Composé ou sel pharmaceutiquement acceptable de celui-ci selon l'une quelconque des revendications 1 à 12, ou composition pharmaceutique selon la revendication 13 pour une utilisation comme médicament.

**15.** Composé ou sel pharmaceutiquement acceptable de celui-ci selon l'une quelconque des revendications 1 à 12, ou composition pharmaceutique selon la revendication 13 pour une utilisation dans la prévention ou le traitement d'une maladie associée à une action agoniste sur les récepteurs β, dans lequel, de préférence, la maladie associée à une action agoniste sur les récepteurs β est l'obésité, l'hyperlipidémie, l'hypercholestérolémie, l'hypertriglycéridémie, la dyslipidémie, le cancer de la thyroïde, le syndrome métabolique, les maladies cardiovasculaires, les maladies coronariennes, l'infarctus du myocarde, l'insuffisance ventriculaire, l'insuffisance cardiaque, la stéatose hépatique, la cirrhose, le diabète, la stéatohépatite, la stéatohépatite non alcoolique, la maladie du foie gras non alcoolique, l'athérosclérose, ou l'hypothyroïdie, ou un trouble.

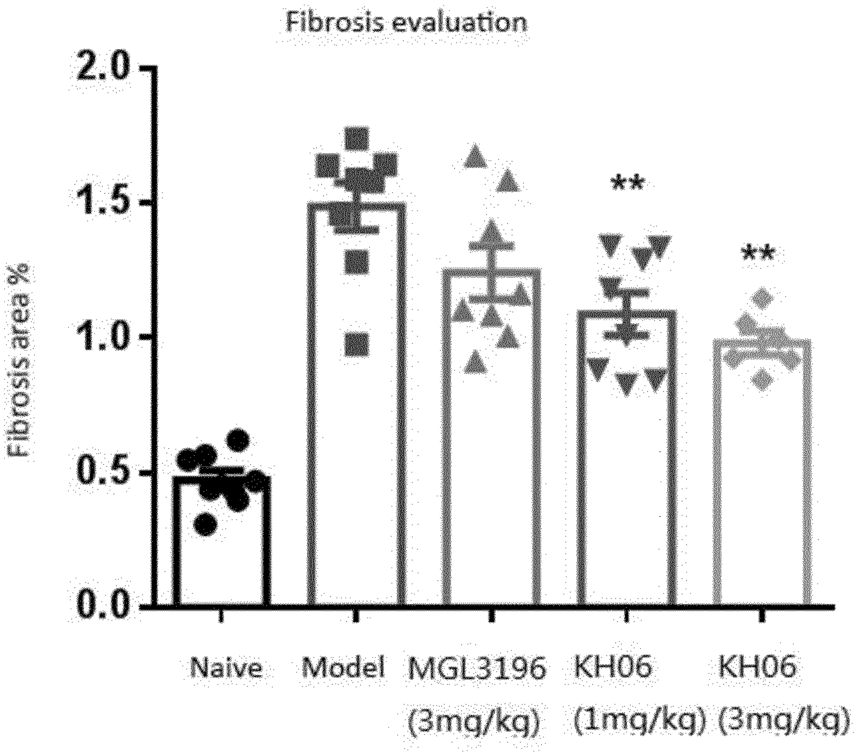
Fibrosis evaluation
Fibrosis area %
2.0
1.5
1.0
0.5
0.0
**
**
Naive
Model
MGL3196 (3mg/kg)
KH06 (1mg/kg)
KH06 (3mg/kg)

Figure 1

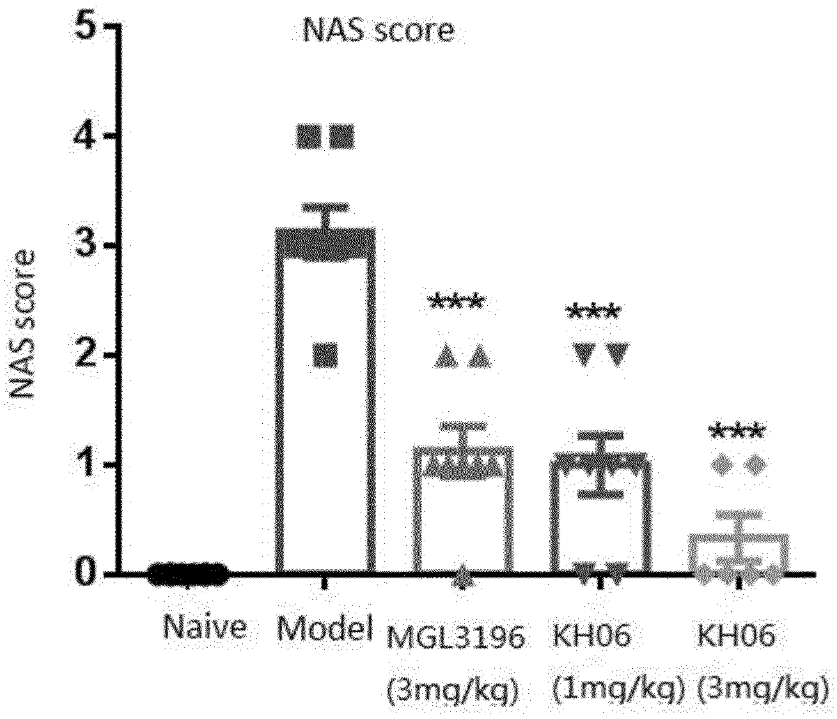
NAS score
NAS score
5
4
3
2
1
0
***
***
***
Naive
Model
MGL3196
(3mg/kg)
KH06
(1mg/kg)
KH06
(3mg/kg)

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 101228135 B
- CN 1882327 C
- CN 1216857 C
- CN 102459185
- WO 2002051805 A
- WO 2013097773 A1

### Non-patent literature cited in the description

- *Journal of Molecular and Celluar Cardiology*, 2004, vol. 37, 1137-1146
- *J. Med. Chem.*, 2014, vol. 57, 3912-3923